(19) Europäisches Patentamt
European Patent Office
Office européen des brevets

(11) EP 4 782 448 A1

(12) EUROPEAN PATENT APPLICATION
published in accordance with Art. 153(4) EPC

(43) Date of publication:
**29.07.2026 Bulletin 2026/31**

(21) Application number: **24867323.8**

(22) Date of filing: **09.09.2024**

(51) International Patent Classification (IPC):
*C07D 471/04* (2006.01) *C07D 519/00* (2006.01)
*A61K 31/437* (2006.01) *A61K 31/5377* (2006.01)
*A61K 31/4545* (2006.01) *A61K 31/438* (2006.01)
*A61P 35/00* (2006.01) *A61P 37/06* (2006.01)
*A61P 25/28* (2006.01) *A61P 29/00* (2006.01)

(86) International application number:
**PCT/CN2024/117673**

(87) International publication number:
**WO 2025/060911 (27.03.2025 Gazette 2025/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.09.2023 CN 202311218595**
**12.07.2024 CN 202410939370**

(71) Applicant: **Origiant Pharmaceutical Co., Ltd.**
**Chengdu, Sichuan 610000 (CN)**

(72) Inventors:
- **YANG, Shengyong**
  **Chengdu, Sichuan 610000 (CN)**
- **LI, Linli**
  **Chengdu, Sichuan 610000 (CN)**
- **JIANG, Zhicheng**
  **Chengdu, Sichuan 610000 (CN)**
- **WU, Jichao**
  **Chengdu, Sichuan 610000 (CN)**
- **ZHONG, Yuanhai**
  **Chengdu, Sichuan 610000 (CN)**
- **GUO, Baosong**
  **Chengdu, Sichuan 610000 (CN)**
- **DU, Junhui**
  **Chengdu, Sichuan 610000 (CN)**
- **XIE, Jiayu**
  **Chengdu, Sichuan 610000 (CN)**
- **YAN, Xiaocai**
  **Chengdu, Sichuan 610000 (CN)**
- **MA, Shuang**
  **Chengdu, Sichuan 610000 (CN)**
- **WU, Xiaoquan**
  **Chengdu, Sichuan 610000 (CN)**
- **YU, Yan**
  **Chengdu, Sichuan 610000 (CN)**
- **HUANG, Qi**
  **Chengdu, Sichuan 610000 (CN)**

(74) Representative: **Straus, Alexander**
**2K Patentanwälte - München**
**Bajuwarenring 14**
**82041 Oberhaching (DE)**

# (54) SUBSTITUTED PYRIDOTRIAZOLE COMPOUND, AND PREPARATION METHOD THEREFOR AND USE THEREOF

(57) The present invention relates to the field of pharmaceutical technology, and specifically to substituted pyridotriazole compounds and the preparation method and use thereof. The present description discloses substituted pyridotriazole compounds having an inhibitory effect on RIPK1 and/or MLK, or a pharmaceutically acceptable salt, stereoisomer, solvate or prodrug thereof. The compound is represented by formula (I), wherein the definitions of respective groups in the formula are detailed in the description. In addition, the present invention further discloses a pharmaceutical composition comprising the compound, and use thereof in the preparation of a medicine for treating RIPK1-related diseases or disorders.

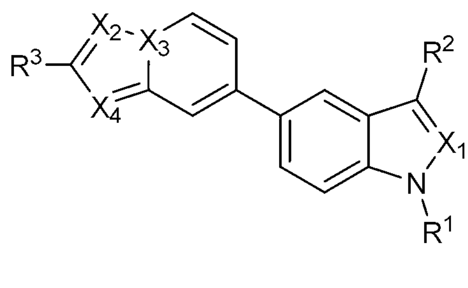

(I)

EP 4 782 448 A1

## Description

The present application claims priority to Chinese Patent Application No. 2023112185953 filed on September 19, 2023, and Chinese Patent Application No. 2024109393705 filed on July 12, 2024, which are incorporated herein by reference in their entireties.

## TECHNICAL FIELD

The present invention relates to the field of pharmaceutical technology, and specifically to substituted pyridotriazole derivatives and use thereof as RIPK1 and/or MLK inhibitors.

## BACKGROUND ART

Necroptosis (programmed necrosis) is a novel caspase-independent form of programmed cell death distinct from apoptosis, which has been discovered in recent years. It is regulated by death signals and exhibits necrosis-like structural characteristics. Compared with apoptosis, necroptosis does not form apoptotic bodies or exhibit chromatin condensation; compared with necrosis, necroptosis is a controlled mode of cell death regulated by multiple genes. In an *in vitro* culture system supplemented with the caspase inhibitor Z-VAD-FMK, TNF can induce necroptosis. The cells exhibit morphological characteristics of necrosis, including swelling, rupture, and the release of cellular contents, which subsequently trigger inflammatory and immune responses. In addition to TNF, ligands of TLR3 and TLR4, as well as certain bacterial and viral infections, can induce necroptosis.

Receptor-interacting protein kinase 1 (RIPK1) is a protein exhibiting specific serine/threonine kinase activity; it possesses an N-terminal kinase domain similar to that of other protein kinases, but features a distinct binding domain. Studies indicate that RIPK1 is a key mediator of necroptosis, modulating this process via the RIPK1/RIPK3/MLKL signaling axis. Various death receptors, such as TNFR, FAS, TRAILR, and Toll-like receptors, can trigger upstream signaling of necroptosis upon stimulation by inflammatory factors or exogenous infections. Under conditions where cIAPs and caspase activity are restricted, RIPK1 interacts with RIPK3 to form a necrosome. RIPK3 further recruits MLKL. Phosphorylated MLKL self-oligomerizes and migrates to the cell membrane to "punch holes," causing the leakage of cellular contents and the disruption of ionization balance, ultimately leading to cell necrosis.

Necroptosis is closely associated with the pathogenesis and progression of inflammation, autoimmune diseases, neurodegenerative diseases, and tumors. For instance, studies indicate that necroptosis is a significant cause of Systemic Inflammatory Response Syndrome (SIRS). SIRS is a systemic inflammatory response syndrome triggered by infectious or non-infectious factors, resulting in a dysregulated and self-destructive reaction in the body. Critically ill patients are prone to SIRS due to diminished compensatory anti-inflammatory capabilities and metabolic dysfunction. Longterm studies have demonstrated that the TNF-$\alpha$-induced SIRS model is highly correlated with RIPK1-dependent necroptosis. Inflammatory bowel diseases (IBD) are abnormal immune-mediated intestinal inflammation caused by environmental, genetic, infectious, and immunological factors; it is a chronic, non-specific inflammatory disease of the intestine. The pathogenesis remains unclear, but excessive apoptosis of intestinal epithelial cells, damage to the intestinal mucosal barrier, and increased permeability of intestinal epithelial cells are considered to be among the causes of IBD. Studies have shown that necroptosis plays an important role in the pathogenesis of IBD. Furthermore, necroptosis plays a significant role in the pathogenesis of various autoimmune diseases, such as rheumatoid arthritis (RA), psoriasis, and multiple sclerosis (MS). Activated microglia play a key role in the development of Alzheimer's disease (AD); RIPK1 is highly expressed in microglia, and RIPK1 inhibitors can effectively protect against A$\beta$-induced neuronal necroptosis *in vitro*. In addition to AD, necroptosis is also involved in the onset and progression of numerous neurodegenerative diseases, such as amyotrophic lateral sclerosis (ALS), frontotemporal dementia (FTD), and Parkinson's disease (PD). In 2016, Strilic *et al.* first revealed that tumor cells can induce necroptosis in vascular endothelial cells. Subsequently, tumor cells penetrate the vascular walls and achieve distant metastasis via blood circulation, which is a major cause of tumor metastasis. Experiments have shown that RIPK1 inhibitors can effectively inhibit tumor metastasis. In addition, studies indicate that RIPK1 kinase can promote the differentiation of tolerogenic macrophages within the pancreatic tumor microenvironment, whereas RIPK1 inhibition facilitates the differentiation of immunogenic macrophages in the pancreatic tumor microenvironment, thereby leading to adaptive immune activation and tumor protection.

Mixed lineage kinase (MLK) is a MAPK kinase that targets JNK and p38 MAPK, activating them in response to various stimuli that induce cellular stress. Thus, MLK regulates a wide range of cellular processes. The MLK family consists of six members: MLK1, MLK2, MLK3, MLK6, dual leucine zipper kinase (DLK), and leucine zipper-bearing kinase (LZK). MLK2 is also known as MST. MLK3 is the most widely expressed member of the MLK family and is present in neurons and resident mononuclear phagocytes in the brain.

MLK3 can regulate neuroinflammation by influencing cytokines produced by microglia and brain macrophages. In particular, CEP-1347-mediated MLK3 inhibition results in a significant reduction in tumor necrosis factor alpha (TNF-$\alpha$)

and interleukin (IL)-6 released by Tat-induced macrophages and microglia. The potent brain-penetrant MLK3 inhibitor URMC-099 has been shown to be effective in vivo in protecting normal synaptic structure and reversing neuroinflammation following CNS exposure to HIV-1 Tat (Journal of Neuroscience the Official Journal of the Society for Neuroscience 33.24(2013):9998-10010.).

Blocking the MLK3 pathway may interfere with the neurotoxic effects of β-amyloid oligomers, and inhibiting the C-jun/JNK pathway blocks tau hyperphosphorylation in cultured hippocampal neurons (Journal of Neuroscience, 2009, 29(28):9078-9089). Therefore, MLK3 inhibitors may be valuable for the treatment of AD and other neurodegenerative diseases involving the C-jun/JNK pathway.

MLK3 inhibitors may also be used for the treatment of various cancers, such as pancreatic and breast cancers. MLK3 regulates the proliferation of certain tumor cell types that overexpress MLK3, including human schwannoma and meningioma cells, and breast cancer cells (Oncogene 29.31(2010):4399-4411).

In summary, RIPK1 and MLK are important therapeutic targets for necroptosis-related diseases such as inflammation, autoimmune diseases, neurodegenerative diseases and tumors, and RIPK1 inhibitors are expected to serve as potential therapeutic drugs for these diseases.

## SUMMARY OF INVENTION

An object of the present invention is to provide a class of structurally novel RIPK1 kinase inhibitors. A first aspect of the present invention provides a compound of formula (I), or a pharmaceutically acceptable salt, solvate or stereoisomer thereof:

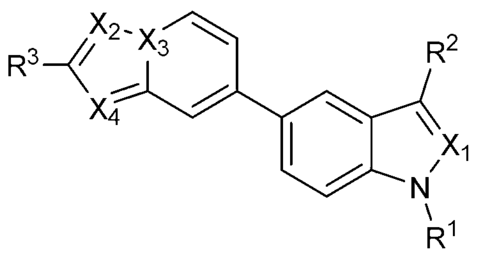

(I)

wherein,

X1 is selected from N or CH;
X2. X3 and X4 are each independently selected from N or CH, provided that at least one of X1, X2 and X3 is N;
$R^1$ is selected from $C_{1-6}$ alkyl, $C_{1-6}$ alkoxy, -$C_{1-6}$ alkyl-$C_{1-6}$ alkoxy or $C_{3-6}$ cycloalkyl, wherein the said $C_{1-6}$ alkyl is optionally substituted with halogen;
$R^2$ is selected from

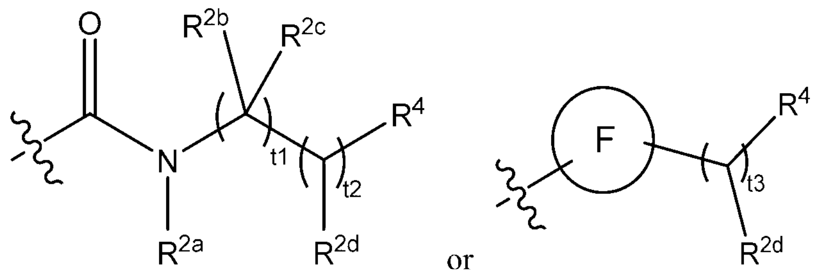

or ;

wherein, $R^4$ is selected from $C_{1-6}$ alkyl, $C_{5-8}$ aryl, 8- to 12-membered partially unsaturated bicyclic group and 5- or 6-membered monocyclic heteroaryl, wherein the said $C_{5-8}$ aryl, 8- to 12-membered partially unsaturated bicyclic group and 5- or 6-membered monocyclic heteroaryl are optionally substituted with one or more substituents selected from $R^{41}$;
$R^{41}$ is selected from halogen, cyano, -$NR^{4a}R^{4b}$, $C_{1-6}$ alkyl and $C_{3-6}$ cycloalkyl;
Ring F is selected from 5- or 6-membered monocyclic heteroaryl and 4- to 8-membered nitrogen-containing partially unsaturated heterocyclyl;
$R^{2a}$. $R^{2b}$, $R^{2c}$ and $R^{2d}$ are each independently selected from hydrogen, deuterium, halogen, $C_{1-6}$ alkyl and $C_{3-6}$ cycloalkyl, wherein the said $C_{1-6}$ alkyl and $C_{3-6}$ cycloalkyl are optionally substituted with one or more halogen or hydroxyl;

Alternatively, $R^{2b}$ and $R^{2c}$, together with the carbon atom to which they are attached, form $C_{3-6}$ cycloalkyl or 4- to 12-membered heterocycloalkyl;
Alternatively, $R^{2a}$ and $R^{2b}$, together with the carbon atom and nitrogen atom to which they are attached, form 4- to 12-membered nitrogen-containing heterocycloalkyl or 4- to 8-membered nitrogen-containing partially unsaturated heterocyclyl;
Alternatively, $R^{2b}$ and $R^{2d}$, together with the carbon atom to which they are attached, form $C_{3-6}$ cycloalkyl or 4- to 12-membered heterocycloalkyl;
The said $C_{3-6}$ cycloalkyl, 4- to 12-membered heterocycloalkyl, 4- to 12-membered nitrogen-containing heterocycloalkyl or 4- to 8-membered nitrogen-containing partially unsaturated heterocyclyl is optionally substituted with one or more substituents selected from halogen;
t1. t2 and t3 are each independently selected from integers of 0 to 6;
$R^3$ is selected from 4- to 12-membered heterocycloalkyl, -NH-$C_{1-6}$ alkyl, -NH-4- to 12-membered heterocycloalkyl or -NH-C(O)$R^5$, wherein the said $C_{1-6}$ alkyl or 4- to 12-membered heterocycloalkyl is optionally substituted with one or more $C_{3-6}$ cycloalkyl;
$R^5$ is selected from hydrogen, deuterium, $C_{1-6}$ alkyl, $C_{1-6}$ alkoxy, $C_{3-6}$ cycloalkyl, 4- to 12-membered heterocycloalkyl, 5- or 6-membered heteroaryl, -$NR^{5a}R^{5b}$, -C(O)O-$C_{1-6}$ alkyl, -C(O)$NR^{5a}R^{5b}$, - $NR^{5a}R^{5b}$-C(O)O-$C_{1-6}$ alkyl, -C(O)-4- to 12-membered heterocycloalkyl, -$C_{1-6}$ alkyl-$(OCH_2CH_2)_mOCH_3$, -$C_{1-6}$ alkyl-$NR^{5a}R^{5b}$, -$C_{1-6}$ alkyl-NH-$C_{1-6}$ alkyl-O-$C_{1-6}$ alkyl, -$C_{1-6}$ alkyl-4- to 12-membered heterocycloalkyl, -$C_{1-6}$ alkyl-NH-4- to 12-membered heterocycloalkyl, -$C_{1-6}$ alkyl-NH-C1-6 alkyl-4- to 12-membered heterocycloalkyl, or -C1-6 alkyl-5- or 6-membered heteroaryl, wherein the said $C_{1-6}$ alkyl, $C_{1-6}$ alkoxy, $C_{3-6}$ cycloalkyl, 4- to 12-membered heterocycloalkyl, and 5-or 6-membered heteroaryl are optionally substituted with one or more substituents selected from deuterium, halogen, cyano, oxo, hydroxyl, carboxyl, $C_{1-6}$ alkyl, -$C_{1-6}$ alkyl-OH, $C_{1-6}$ alkoxy, $C_{3-6}$ cycloalkyl, -C(O)-$C_{1-6}$ alkyl, -C(O)O-$C_{1-6}$ alkyl, -C(O)-$C_{3-6}$ cycloalkyl, or 4- to 12-membered heterocycloalkyl, wherein two hydrogen atoms on an optional carbon atom of the said $C_{1-6}$ alkyl are substituted to form a $C_{3-6}$ cycloalkyl;
m is an integer selected from 1 to 4;
$R^{4a}$. $R^{4b}$, $R^{5a}$ and $R^{5b}$ are each independently selected from hydrogen, halogen, $C_{1-6}$ alkyl or $C_{1-6}$ alkoxy, wherein the said $C_{1-6}$ alkyl is optionally substituted with one or more substituents selected from halogen, hydroxyl or C3-6 cycloalkyl.

In one embodiment, $R^1$ is selected from $C_{1-6}$ alkyl, $C_{1-6}$ alkoxy, -$C_{1-6}$ alkyl-$C_{1-6}$ alkoxy or $C_{3-6}$ cycloalkyl;

preferably, $R^1$ is selected from $C_{1-6}$ alkyl or -$C_{1-3}$ alkyl-$C_{1-3}$ alkoxy;
preferably, $R^1$ is selected from methyl, ethyl or -$CH_2CH_2OCH_3$.

In one embodiment, t1, t2 and t3 are each independently selected from 0, 1, 2 or 3;
In one embodiment, $R^2$ is selected from

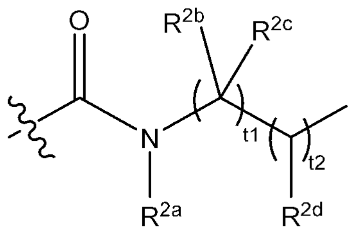
or
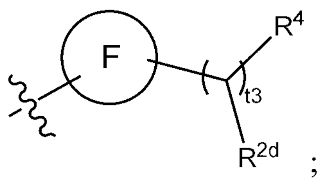
;

$R^4$ is selected from $C_{1-6}$ alkyl,

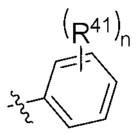
,
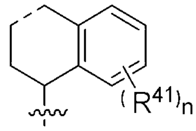
or
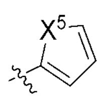
,

wherein the said $R^{41}$ is as defined for the compound of formula (I); $X^5$ is selected from N, O or S;

preferably, $R^{41}$ is selected from halogen, cyano or amino;
preferably, $R^{41}$ is selected from fluoro, chloro or cyano;
preferably, ring F is selected from a 5- or 6-membered monocyclic heteroaryl or a 4- to 8-membered nitrogen-containing partially unsaturated heterocyclyl, wherein the said 5- or 6-membered monocyclic heteroaryl or 4- to 8-membered nitrogen-containing partially unsaturated heterocyclyl optionally contains 1, 2 or 3 heteroatoms selected from N and O as ring atoms;
preferably, ring F is selected from a 5- or 6-membered monocyclic heteroaryl or a 4- to 6-membered nitrogen-containing partially unsaturated heterocyclyl, wherein the said 5- or 6-membered monocyclic heteroaryl or 4- to 8-membered nitrogen-containing partially unsaturated heterocyclyl optionally contains 1, 2 or 3 heteroatoms selected from N and O as ring atoms;
preferably, ring F is selected from triazolyl or dihydrooxazolyl.

In one embodiment, $R^{2a}$, $R^{2b}$, $R^{2c}$ and $R^{2d}$ are each independently selected from hydrogen, deuterium, halogen, $C_{1-6}$ alkyl, halogen-substituted $C_{1-6}$ alkyl or hydroxy-substituted $C_{1-6}$ alkyl; more preferably selected from hydrogen, deuterium, halogen, methyl, ethyl, fluoroethyl, difluoroethyl or hydroxymethyl;

preferably, $R^{2a}$ is selected from hydrogen, deuterium or methyl;
preferably, $R^{2b}$, $R^{2c}$ and $R^{2d}$ are each independently selected from methyl, ethyl, fluoroethyl, difluoroethyl or hydroxymethyl;
preferably, $R^{2b}$ and $R^{2c}$, together with the carbon atom to which they are attached, form $C_{3-6}$ cycloalkyl or 4- to 12-membered heterocycloalkyl;
preferably, $R^{2b}$ and $R^{2c}$, together with the carbon atom to which they are attached, form $C_{3-6}$ cycloalkyl or 4- to 12-membered heterocycloalkyl;
preferably, $R^{2b}$ and $R^{2c}$, together with the carbon atom to which they are attached, form a 4- to 8-membered heterocycloalkyl, wherein the said 4- to 8-membered heterocycloalkyl optionally contains 1 or 2 heteroatoms selected from N, O and S as ring atoms;
preferably, the said 4- to 12-membered heterocycloalkyl is a 4- to 6-membered oxygen-containing heterocycloalkyl, more preferably oxetanyl;

Alternatively, $R^{2a}$ and $R^{2b}$, together with the carbon atom and nitrogen atom to which they are attached, form a 4- to 12-membered nitrogen-containing heterocycloalkyl or a 4- to 8-membered nitrogen-containing partially unsaturated heterocyclyl, wherein the said 4- to 12-membered nitrogen-containing heterocycloalkyl is preferably azetidinyl or morpholinyl; the said 4- to 8-membered nitrogen-containing partially unsaturated heterocyclyl is preferably dihydropyrazolyl, and the said azetidinyl is optionally substituted with one or more halogens;
Alternatively, $R^{2b}$ and $R^{2d}$, together with the carbon atom to which they are attached, form $C_{3-6}$ cycloalkyl or preferably cyclopropyl;
preferably, $R^2$ is selected from

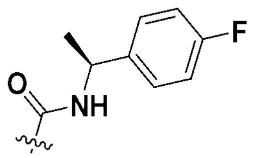 , 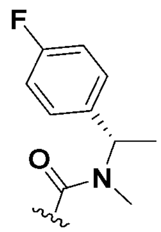 , 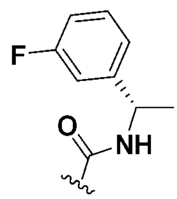 ,

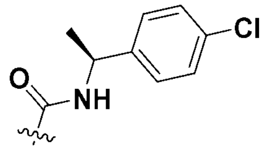 , 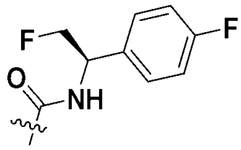 , 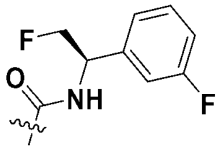 , 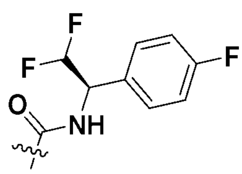 , 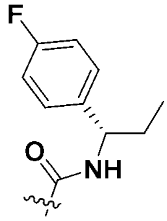 ,

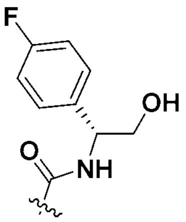, 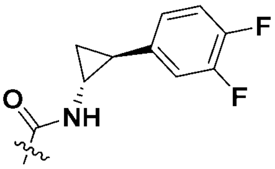, 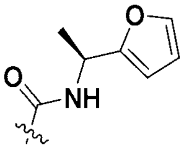, 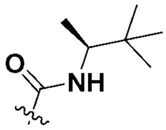, 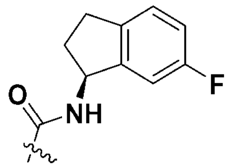,

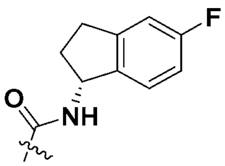, 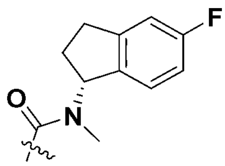, 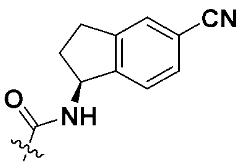, 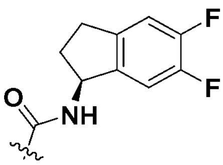,

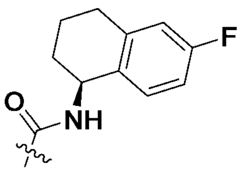, 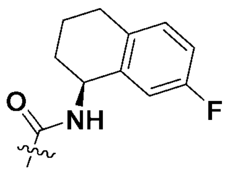, 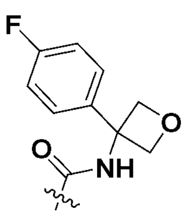, 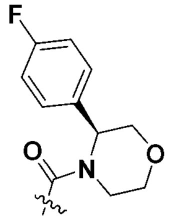, 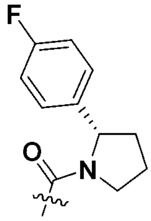,

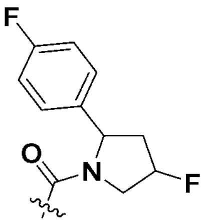, 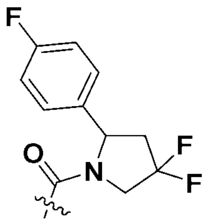, 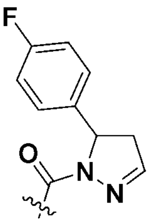, 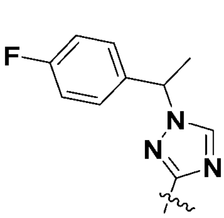, 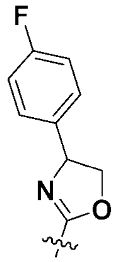, 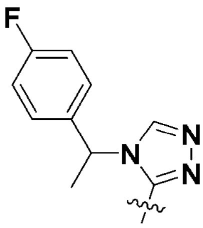.

In one embodiment, $R^3$ is selected from 4- to 6-membered heterocycloalkyl, -NH-$C_{1-6}$ alkyl, -NH-4-to 12-membered heterocycloalkyl or -NH-C(O)$R^5$, wherein the said $C_{1-6}$ alkyl is optionally substituted with one or more $C_{3-6}$ cycloalkyl, and the said 4- to 6-membered heterocycloalkyl is optionally substituted with one or more oxo;

preferably, $R^3$ is selected from morpholinyl or oxomorpholinyl;
preferably, $R^3$ is selected from -NH-isopropyl, -NH-$CH_2$-cyclopropyl or -NH-tetrahydrofuran-3-yl; preferably, $R^5$ is selected from methyl, ethoxy, $-CH_2CH_2COOH$, $-CH_2Cl$, $-CH_2CN$, $-CH_2OCH_3$, - $CH(CH_3)OCH_3$,

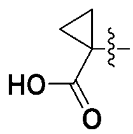,

$-C(O)OCH_2CH_3$, $-C(O)NH_2$, $-CH_2(OCH_2CH_2)_2OCH_3$, - $CH_2(OCH_2CH_2)_3OCH_3$,

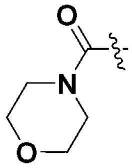, 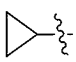, 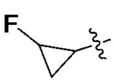, 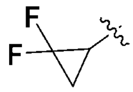, 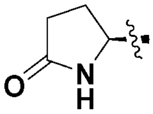, 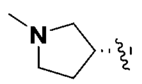,

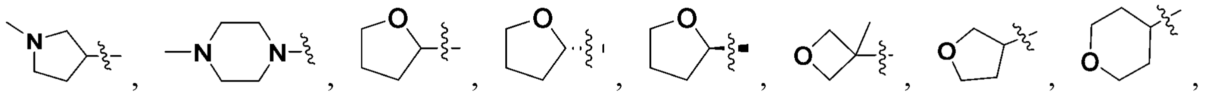
, , , , , , , ,

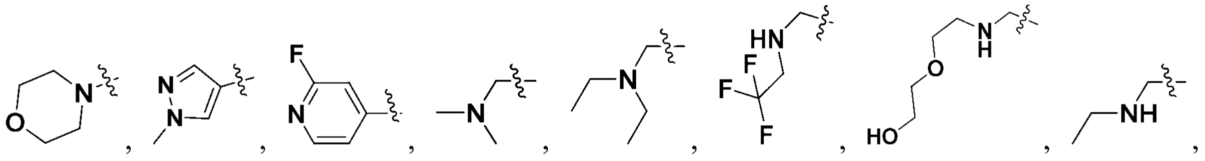
, , , , , , , ,

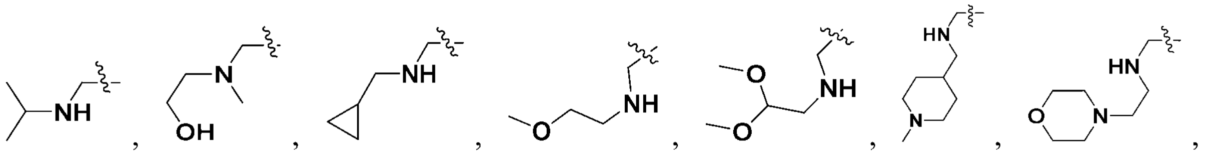
, , , , , , ,

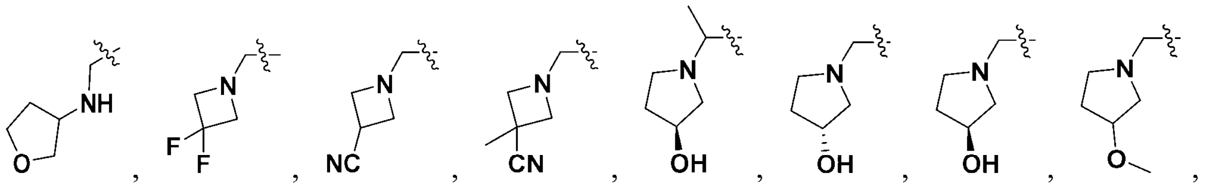
, , , , , , , ,

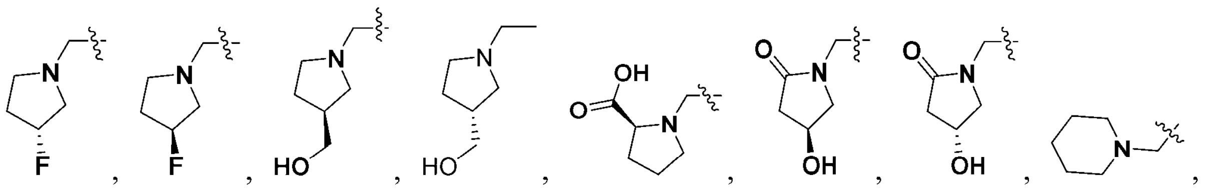
, , , , , , , ,

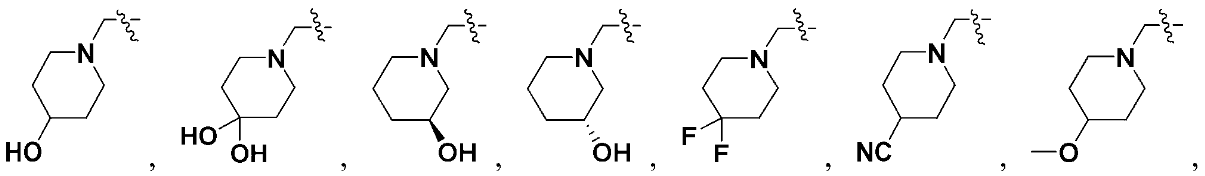
, , , , , , ,

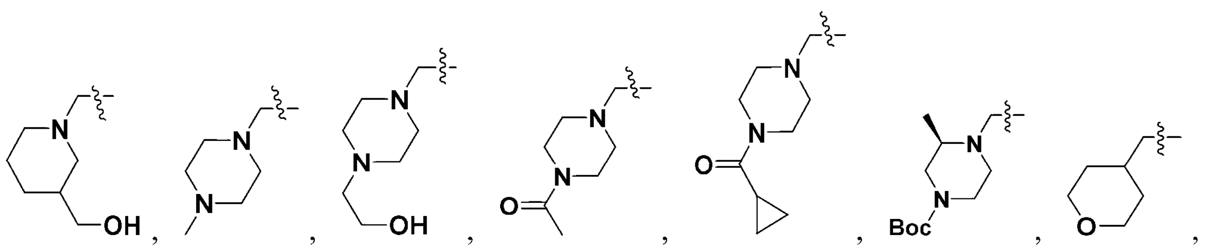
, , , , , , ,

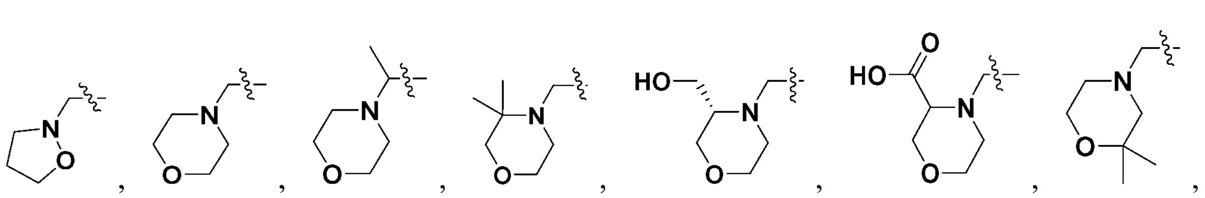
, , , , , , ,

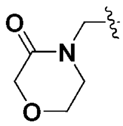, 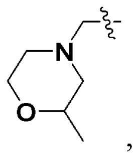, 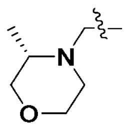, 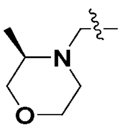, 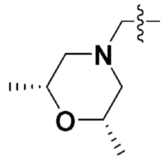, 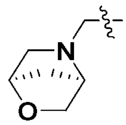, 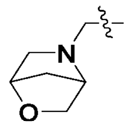, 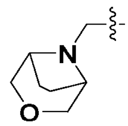,

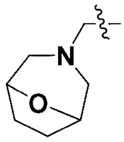, 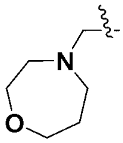, 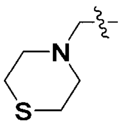, 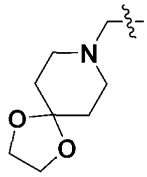, 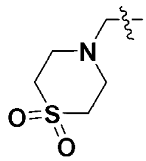, 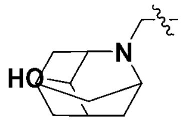, 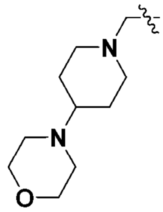,

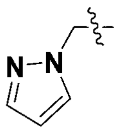, 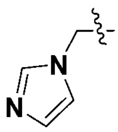, 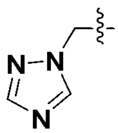 or 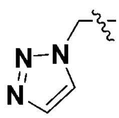.

In one embodiment, $R^3$ is selected from:

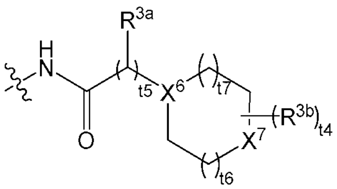, 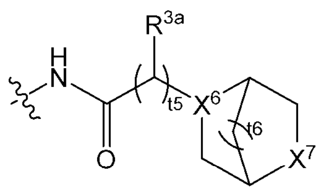, 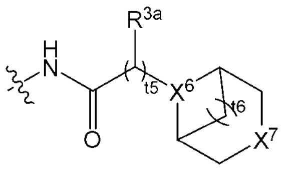,

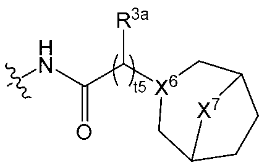, 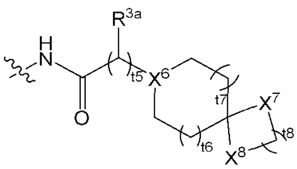, 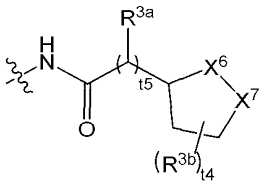, 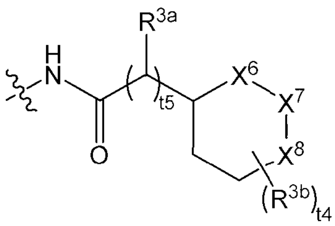,

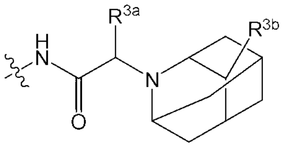, 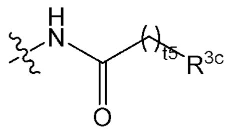, 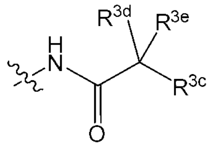, 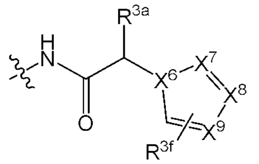,

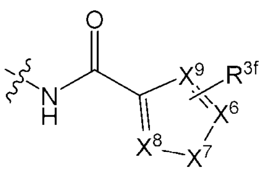, 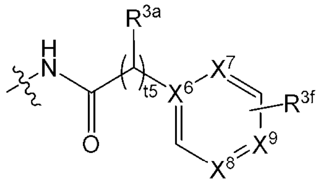, 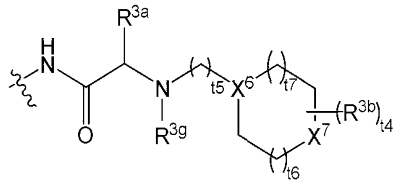,

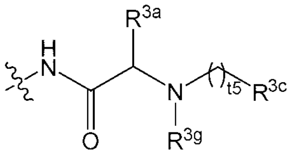;

preferably, $R^3$ is selected from:

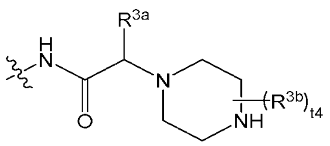, 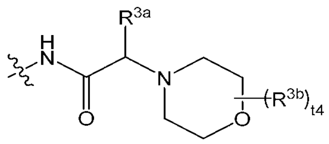, 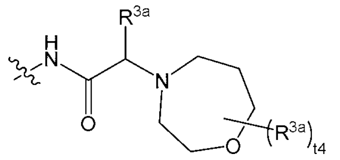,

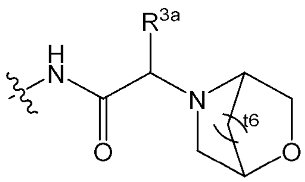, 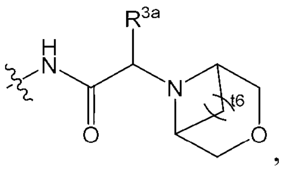, 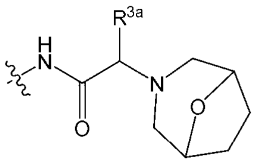, 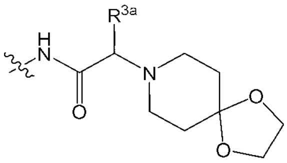,

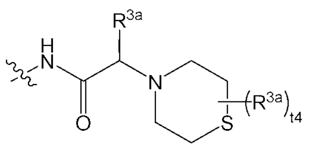, 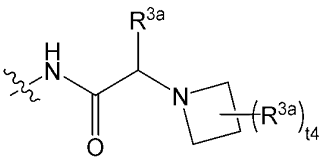, 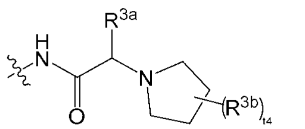,

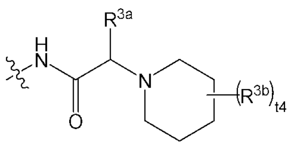, 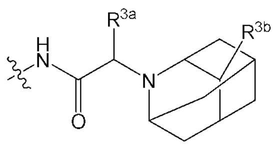, 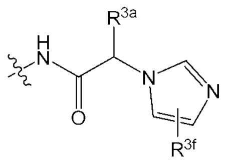, 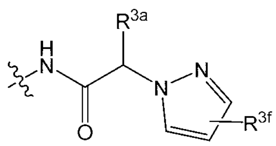,

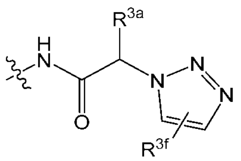, 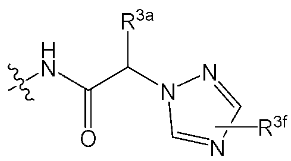, 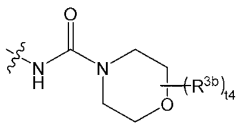, 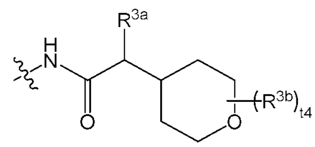,

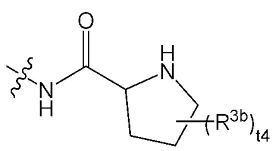, 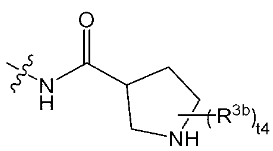, 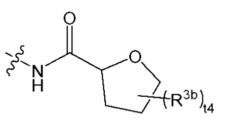, 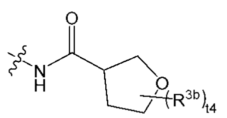,

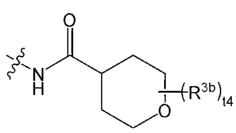, 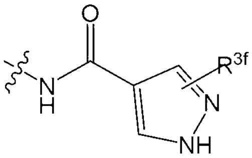, 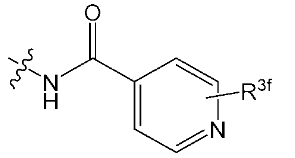,

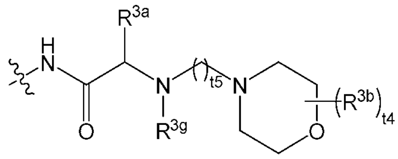, 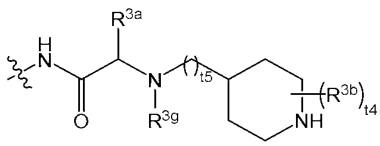, 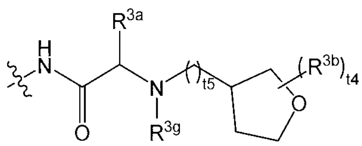,

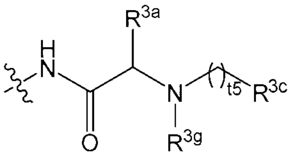, 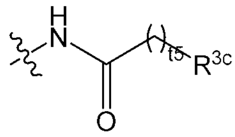, 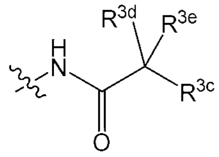;

wherein, $X^6$, $X^7$, $X^8$, and $X^9$ are each independently selected from N, NH, O, S, CH or $CH_2$;
t4 and t5 are each independently selected from integers of 0 to 6;
t6 and t7 are each independently selected from 0, 1 or 2;
$R^{3a}$. $R^{3b}$, $R^{3c}$, $R^{3d}$, $R^{3e}$, $R^{3f}$, and $R^{39}$ are each independently selected from hydrogen, deuterium, halogen, cyano, oxo, hydroxyl, carboxyl, $C_{1-6}$ alkyl, $C_{1-6}$ alkoxy, $C_{3-6}$ cycloalkyl, 4- to 12-membered heterocycloalkyl, $-(OCH_2CH_2)_mOCH_3$, $-NR^{X3a}R^{X3b}$, $-C(O)R^{X3c}$, wherein the said $C_{1-6}$ alkyl, $C_{1-6}$ alkoxy, $C_{3-6}$ cycloalkyl are optionally substituted with one or more substituents selected from halogen, hydroxyl, $C_{1-6}$ alkoxy;
or. $R^{3d}$, $R^{3e}$ and the carbon atom to which they are attached together form $C_{3-6}$ cycloalkyl;
$R^{X3a}$, $R^{X3b}$, and $R^{X3c}$ are each independently selected from hydrogen, $C_{1-6}$ alkyl, $C_{1-6}$ alkoxy or $C_{3-6}$ cycloalkyl;
m is an integer selected from 1 to 4; preferably 2 or 3.
In one embodiment, $R^{3a}$ is selected from hydrogen, deuterium, oxo, or $C_{1-6}$ alkyl; preferably from hydrogen, oxo, or methyl;
preferably, $R^{3b}$ is selected from hydrogen, deuterium, halogen, cyano, oxo, hydroxyl, carboxyl, $C_{1-6}$ alkyl, $C_{1-6}$ alkoxy, $-C_{1-6}$ alkyl-OH, $-C(O)C_{1-6}$ alkyl, $-C(O)C_{3-6}$ cycloalkyl, $C_{3-6}$ cycloalkyl, or 4- to 12-membered heterocycloalkyl; wherein the said 4- to 12-membered heterocycloalkyl optionally contains 1, 2, or 3 heteroatoms selected from N and O as ring atoms;
preferably, $R^{3b}$ is selected from hydrogen, deuterium, halogen, cyano, oxo, hydroxyl, carboxyl, methyl, methoxyl, $-CH_2OH$, $-C(O)CH_3$, -C(O)cyclopropyl, cyclopropyl, or morpholinyl;
preferably, $R^{3c}$ is selected from hydrogen, deuterium, halogen, cyano, oxo, hydroxyl, carboxyl, amino, $-(OCH_2CH_2)_mOCH_3$, $-NR^{X3a}R^{X3b}$, $C_{1-6}$ alkyl, $C_{1-6}$ alkoxy, halogen-substituted $C_{1-6}$ alkyl, $C_{3-6}$ cycloalkyl and halogen-substituted $C_{3-6}$ cycloalkyl, wherein the said $C_{1-6}$ alkyl or $C_{1-6}$ alkoxy is optionally substituted with one or more substituents selected from hydroxyl and $C_{1-6}$ alkoxy;
preferably, $R^{3c}$ is selected from hydrogen, deuterium, halogen, cyano, oxo, hydroxyl, carboxyl, amino, dimethylamino, diethylamino, trifluoromethyl, methoxy, ethoxy, isopropyl, cyclopropyl, halogen-substituted cyclopropyl, $-CH(OCH_3)_2$, $-OCH_2CH_2OH$, $-(OCH_2CH_2)_2OCH_3$ or $-(OCH_2CH_2)_3OCH_3$;
Alternatively, $R^{3d}$ and $R^{3e}$ together with the carbon atom to which they are attached form $C_{3-6}$ cycloalkyl; preferably, $R^{3d}$ and $R^{3e}$ together with the carbon atom to which they are attached form cyclopropyl.

In one embodiment, $R^{3f}$ is hydrogen.

In one embodiment, the compound of formula (I) is a compound represented by formula (II):

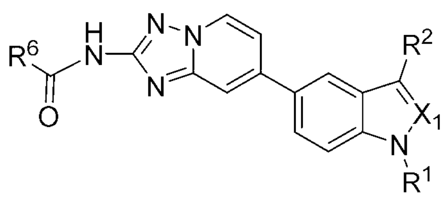 (II)

wherein $X^1$, $R^1$ and $R^2$ are each as defined for the compound of formula (I);
$R^6$ is selected from hydrogen, deuterium, $C_{1-6}$ alkyl, $C_{1-6}$ alkoxy, $C_{3-6}$ cycloalkyl, 4- to 12-membered heterocycloalkyl, 5- or 6-membered heteroaryl, $-C(O)NR^{5a}R^{5b}$, $-C(O)O-C_{1-6}$ alkyl or -C(O)-(4- to 12-membered heterocycloalkyl), wherein the said $C_{1-6}$ alkyl, $C_{3-6}$ cycloalkyl, 4- to 12-membered heterocycloalkyl and 5- or 6-membered heteroaryl are optionally substituted with one or more substituents selected from halogen, oxo, hydroxyl, carboxyl, cyano, $C_{1-6}$ alkyl, $-C_{1-6}$ alkyl-OH, $C_{1-6}$ alkoxy, $C_{3-6}$ cycloalkyl, $-C(O)-C_{1-6}$ alkyl, $-C(O)O-C_{1-6}$ alkyl, $-C(O)-C_{3-6}$ cycloalkyl or 4- to 12-membered heterocycloalkyl, wherein two hydrogen atoms on any one carbon atom of the said $C_{1-6}$ alkyl are substituted to form $C_{3-6}$ cycloalkyl;

$R^{5a}$ and $R^{5b}$ are each independently selected from hydrogen, $C_{1-6}$ alkyl or $C_{1-6}$ alkoxy, wherein the said $C_{1-6}$ alkyl is optionally substituted with one or more halogen or $C_{3-6}$ cycloalkyl.

In one embodiment, $R^6$ is selected from methyl, ethoxy, $-CH_2CH_2COOH$, $-CH_2Cl$, $-CH_2CN$, $-CH_2OCH_3$, $-CH(CH_3)OCH_3$,

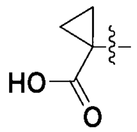,

$-C(O)OCH_2CH_3$, $-C(O)NH_2$,

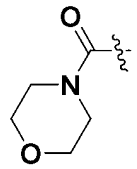,

$-CH_2-(OCH_2CH_2)_2OCH_3$, $-CH_2(OCH_2CH_2)_3OCH_3$,

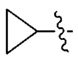, 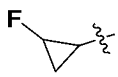, 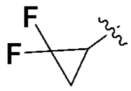, 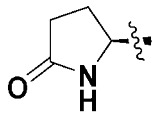,

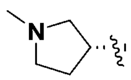, 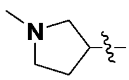, 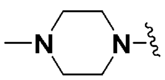, 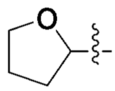, 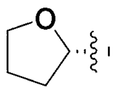, 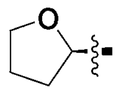, 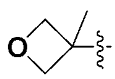, 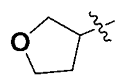,

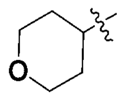, 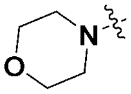, 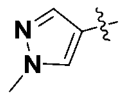 or 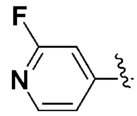.

In one embodiment, the compound of formula (I) is a compound represented by formula (III):

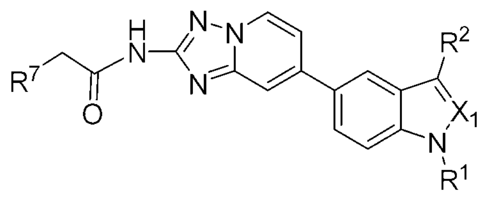

(III)

wherein $X^1$, $R^1$ and $R^2$ are each as defined for the compound of formula (I);

$R^7$ is selected from $-NR^{5a}R^{5b}$, $-(OCH_2CH_2)_mOCH_3$, $-NH-C_{1-6}$ alkyl-O-$C_{1-6}$ alkyl, -4- to 12-membered heterocycloalkyl, -NH-4- to 12-membered heterocycloalkyl, $-NH-C_{1-6}$ alkyl-4- to 12-membered heterocycloalkyl or 5- or 6-membered heteroaryl, wherein the said $C_{1-6}$ alkyl, $C_{3-6}$ cycloalkyl, 4- to 12-membered heterocycloalkyl and 5- or 6-membered heteroaryl are optionally substituted with one or more substituents selected from halogen, oxo, hydroxyl, carboxyl, cyano, $C_{1-6}$ alkyl, $-C_{1-6}$ alkyl-OH, $C_{1-6}$ alkoxy, $C_{3-6}$ cycloalkyl, $-C(O)-C_{1-6}$ alkyl, $-C(O)O-C_{1-6}$ alkyl, $-C(O)-C_{3-6}$ cycloalkyl or 4- to 12-membered heterocycloalkyl;

$R^{5a}$ and $R^{5b}$ are each independently selected from hydrogen, $C_{1-6}$ alkyl or $C_{1-6}$ alkoxy, wherein the said $C_{1-6}$ alkyl is optionally substituted with one or more halogen or $C_{3-6}$ cycloalkyl.

In one embodiment, $R^7$ is selected from

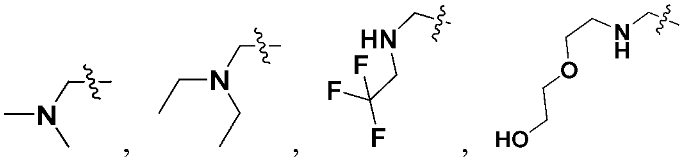 , , , ,

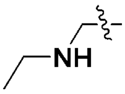 , 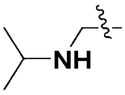 , 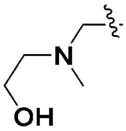 , 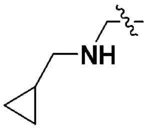 , 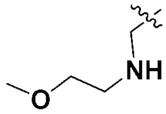 , 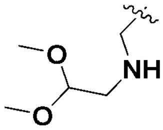 , 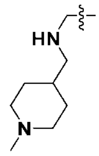 ,

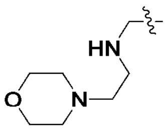 , 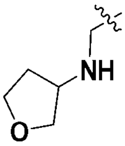 , 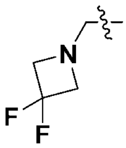 , 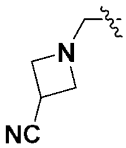 , 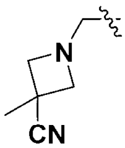 , 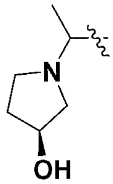 , 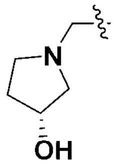 , 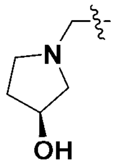 ,

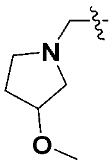 , 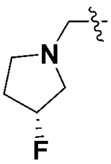 , 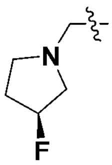 , 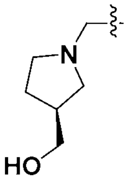 , 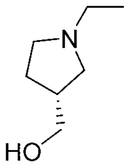 , 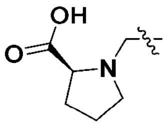 , 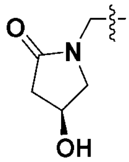 , 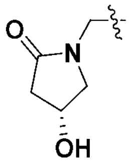 ,

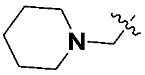 , 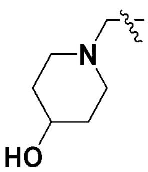 , 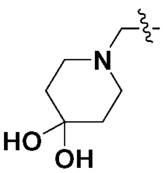 , 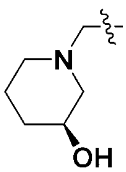 , 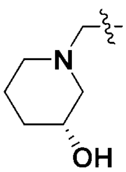 , 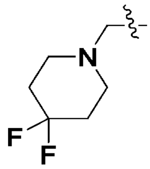 , 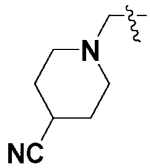 ,

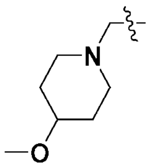 , 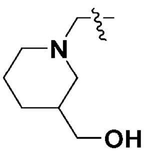 , 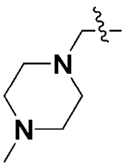 , 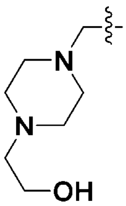 , 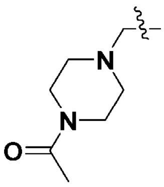 , 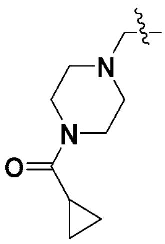 , 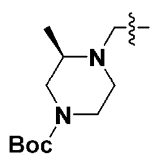 ,

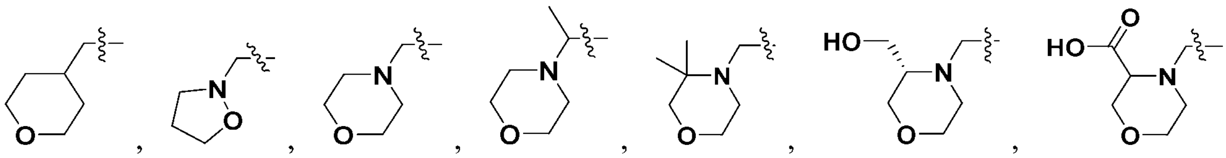 , , , , , , ,

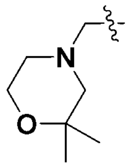, 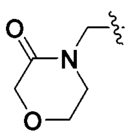, 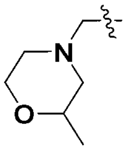, 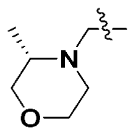, 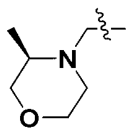, 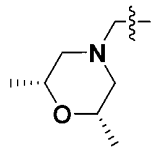, 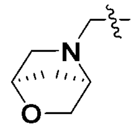, 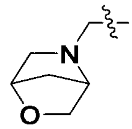,

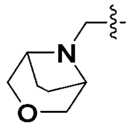, 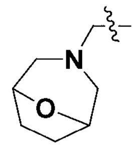, 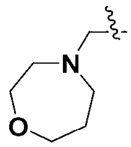, 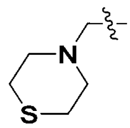, 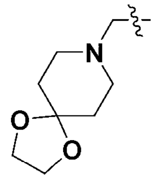, 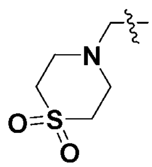, 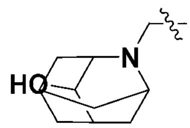,

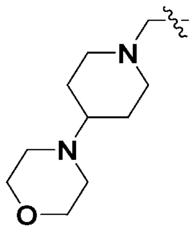, 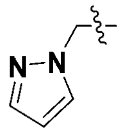, 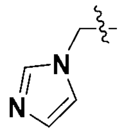 or 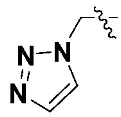.

In one embodiment, the compound of formula (III) is a compound represented by formula (IIIa):

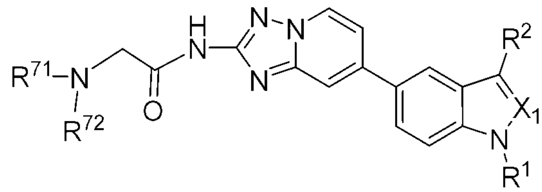

(IIIa)

wherein $X^1$, $R^1$ and $R^2$ are each as defined for the compound of formula (I);
$R^{71}$ and $R^{72}$ are each independently selected from hydrogen, $C_{1-6}$ alkyl, $C_{1-6}$ alkoxy, 4- to 12-membered heterocycloalkyl and 5- or 6-membered heteroaryl, wherein the said $C_{1-6}$ alkyl is optionally substituted with one or more substituents selected from halogen, $C_{1-6}$ alkoxy, $C_{3-6}$ cycloalkyl, 4- to 12-membered heterocycloalkyl or $-CH(OCH_3)_2$.

In one embodiment, the compound of formula (III) is a compound represented by formula (IIIb):

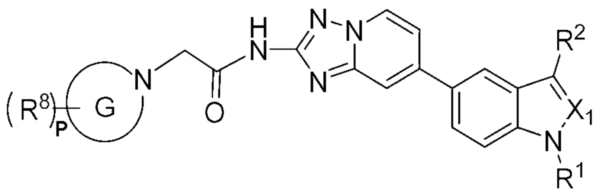

(IIIb)

wherein $X^1$, $R^1$ and $R^2$ are each as defined for the compound of formula (I);
Ring G is selected from 5- or 6-membered nitrogen-containing heteroaryl or 5- to 12-membered nitrogen-containing heterocycloalkyl;
$R^8$ is selected from hydrogen, halogen, cyano, hydroxyl, carboxyl, oxo, $C_{1-6}$ alkyl, $C_{1-6}$ alkoxy, $-C_{1-6}$ alkyl-OH, $-C(O)-C_{1-6}$ alkyl, $-C(O)O-C_{1-6}$ alkyl or $-C(O)-C_{3-6}$ cycloalkyl;
p is selected from 0, 1, 2 or 3, preferably 1 or 2;
preferably, $R^8$ is selected from hydrogen, fluoro, cyano, hydroxyl, carboxyl, oxo, methyl, methoxy, hydroxymethyl, $-C(O)CH_3$, -C(O)-cyclopropyl or $-C(O)OCH(CH_3)_3$;
In one embodiment, ring G is selected from

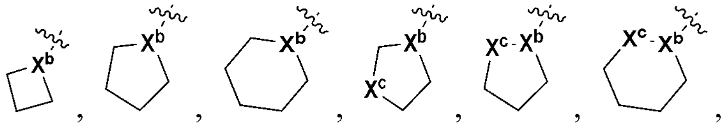

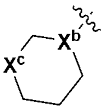, 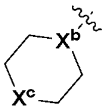, 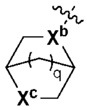, 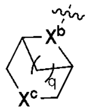, 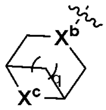, 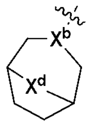, 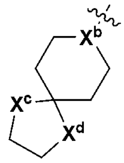, 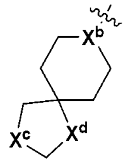, 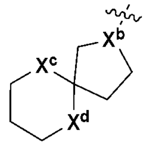,

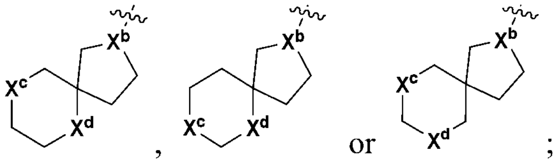

wherein $X^b$ is N, and $X^c$ and $X^d$ are each independently selected from $CH_2$, NH, N, O or S; q is selected from 1, 2 or 3, preferably 1 or 2;
preferably, ring G is selected from

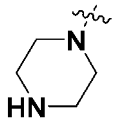, 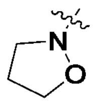, 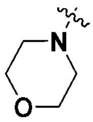, 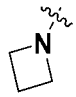, 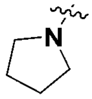, 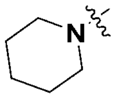,

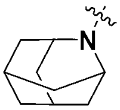, 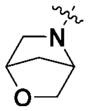, 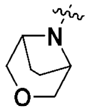, 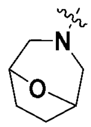, 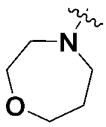, 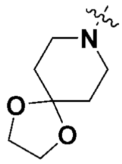, 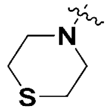, 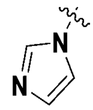, 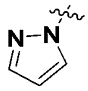,

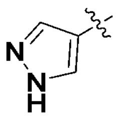, 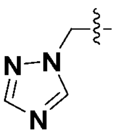, 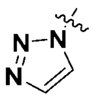 or 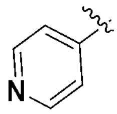.

In one embodiment, the said 4- to 12-membered heterocycloalkyl is selected from 4- to 7-membered monocyclic heterocycloalkyl, 6- to 8-membered bicyclic heterocycloalkyl, 6- to 10-membered bridged heterocycloalkyl, or 6- to 10-membered spiroheterocycloalkyl; the said 4- to 7-membered monocyclic heterocycloalkyl, 6- to 8-membered bicyclic heterocycloalkyl, 6- to 10-membered bridged heterocycloalkyl, or 6- to 10-membered spiroheterocycloalkyl preferably contains 1, 2, or 3 heteroatoms selected from N, S, or O as ring atoms.

In one embodiment, the said 4- to 12-membered heterocycloalkyl is selected from

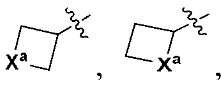

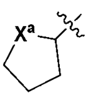, 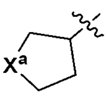, 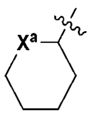, 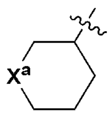, 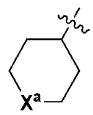, 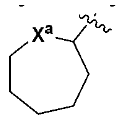, 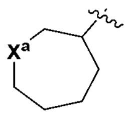, 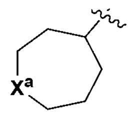, 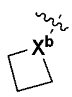,

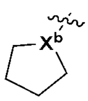, 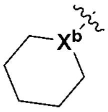, 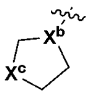, 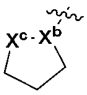, 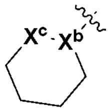, 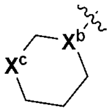, 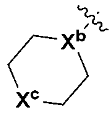, 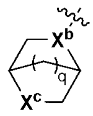, 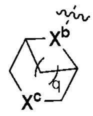, 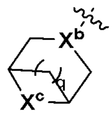,

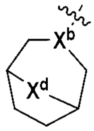, 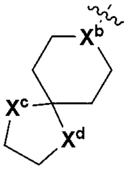, 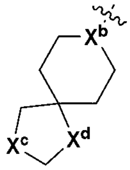, 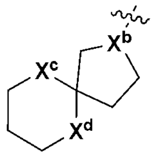, 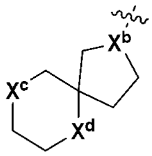, 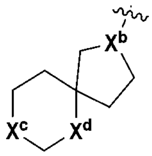 or 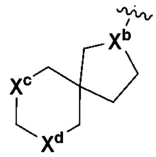;

wherein $X^a$, $X^b$, $X^c$, and $X^d$ are each independently selected from $CH_2$, NH, N, O, or S; q is selected from 1, 2, or 3, preferably 1 or 2;

preferably, $X^b$ is N;

preferably, the said 4- to 12-membered heterocycloalkyl is selected from pyrrolidinyl, piperidinyl, piperazinyl, oxetanyl, tetrahydorfuranyl, oxetanyl, isoxazolidinyl, oxazepanyl, azetidinyl, pyrrolidinyl, piperidinyl, azaadamantyl, oxa-azabicycloheptyl, oxa-azabicyclooctyl, oxaazaspirodecyl or thiomorpholinyl;

preferably, the said 4- to 12-membered heterocycloalkyl is selected from

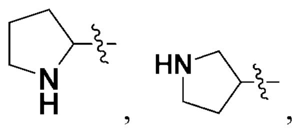

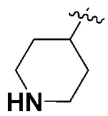, 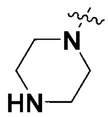, 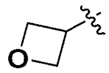, 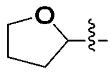, 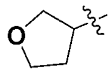, 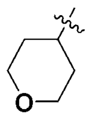 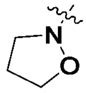, 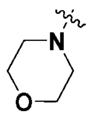, 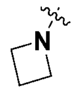, 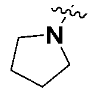,

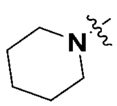, 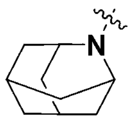, 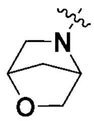, 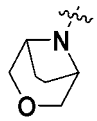, 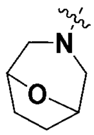, 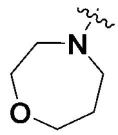, 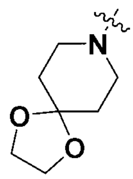 or 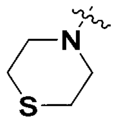.

In one embodiment, the said 5- or 6-membered heteroaryl is selected from 5- or 6-membered nitrogen-containing heteroaryl, wherein the said 5- or 6-membered nitrogen-containing heteroaryl optionally contains 1, 2 or 3 nitrogen atoms as ring atoms;

preferably, the said 5- or 6-membered heteroaryl is selected from imidazolyl, pyrazolyl, triazolyl or pyridyl;

preferably, the said 5- or 6-membered heteroaryl is selected from

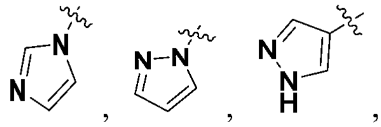

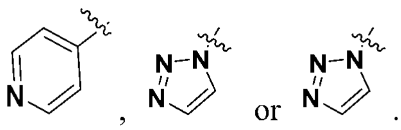

The present invention provides the following compounds, or pharmaceutically acceptable salts, solvates or stereoisomers thereof:

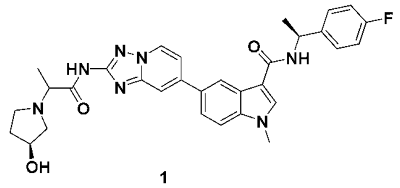
1 ,

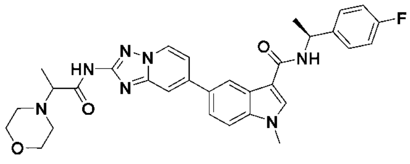
2 ,

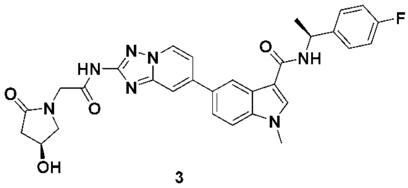
3 ,

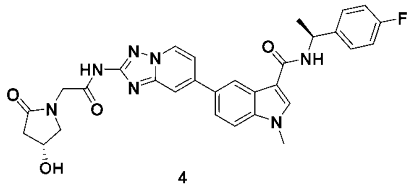
4 ,

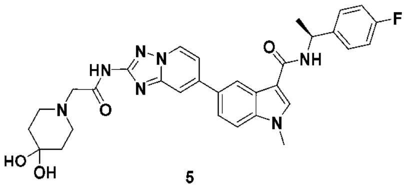
5 ,

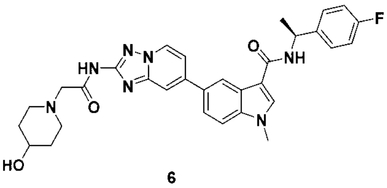
6 ,

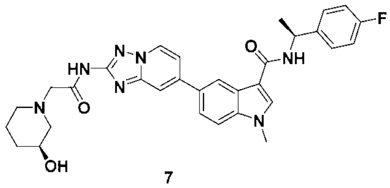
7 ,

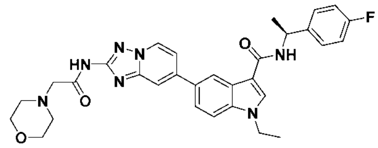
8 ,

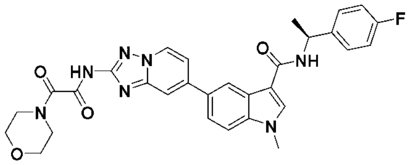
9 ,

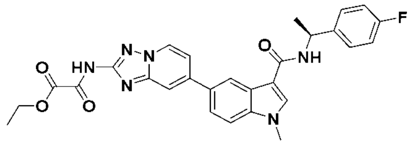
10 ,

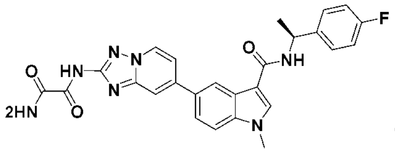
11

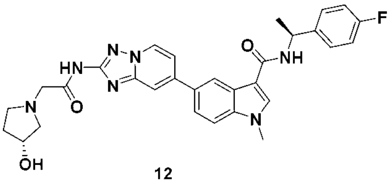
12 ,

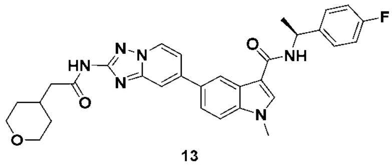
13 ,

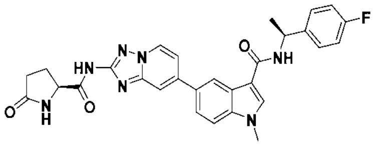
14 ,

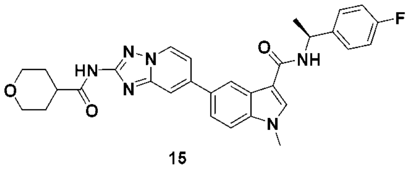
15 ,

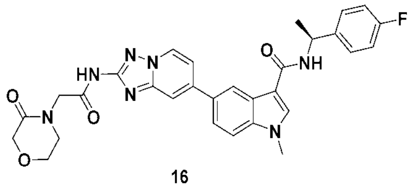
16 ,

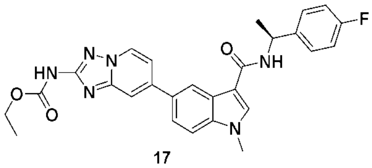
17 ,

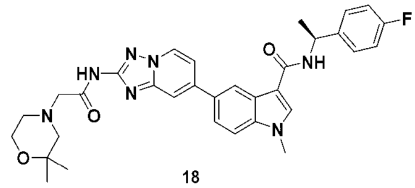
18 ,

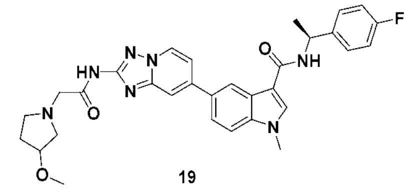
19 ,

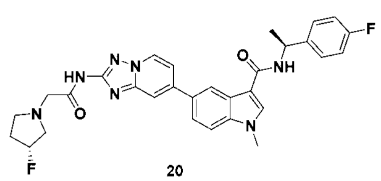
20 ,

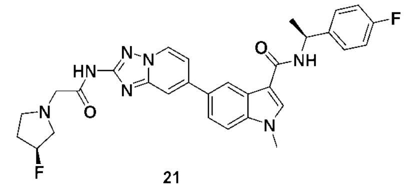
21 ,

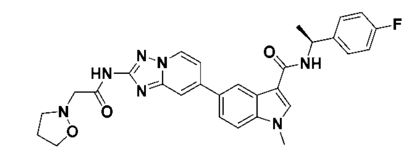
22 ,

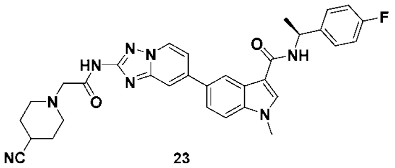
23 ,

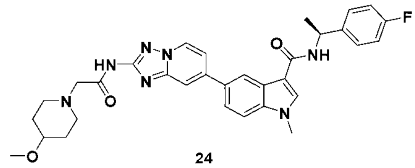
24 ,

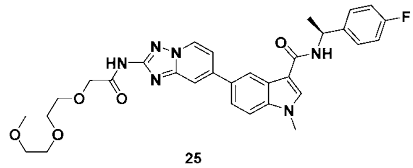
25 ,

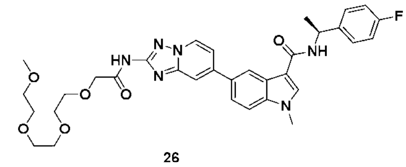
26 ,

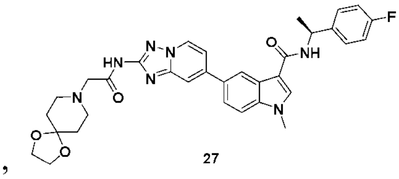
27 ,

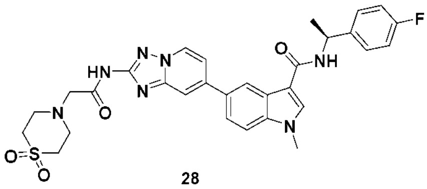
28 ,

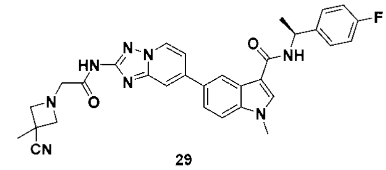
29 ,

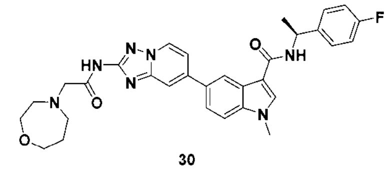
30 ,

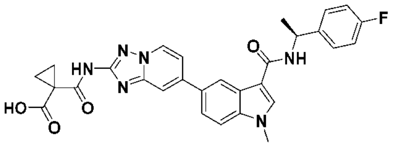
31 ,

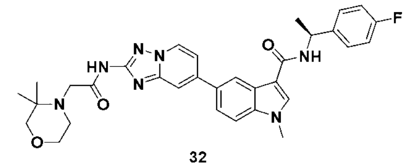
32 ,

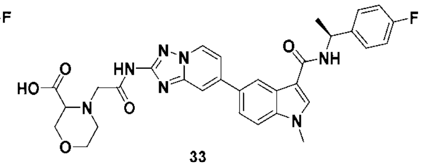
33 ,

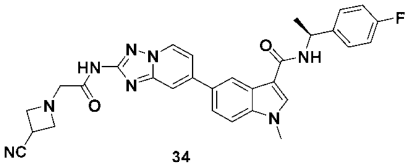
34 ,

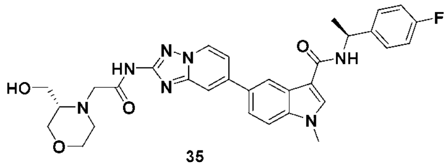
35 ,

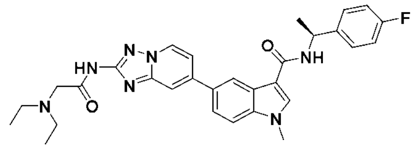
36 ,

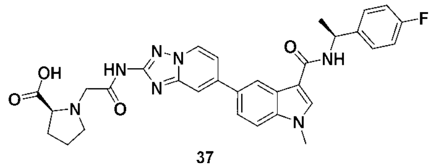
37 ,

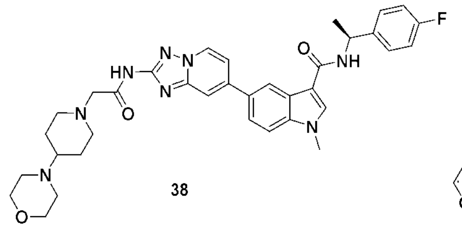

38

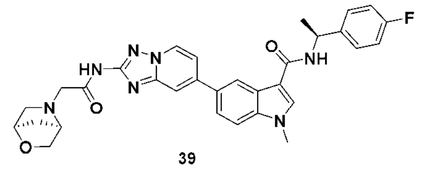

39

,

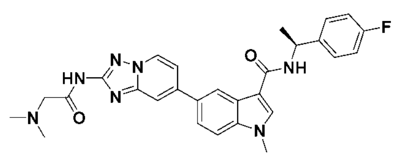

40

,

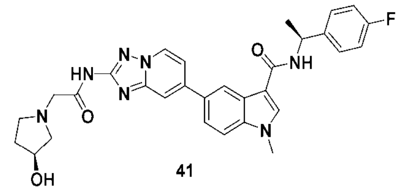

41

,

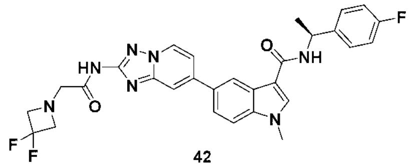

42

,

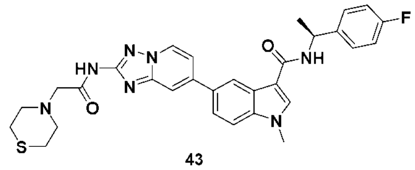

43

,

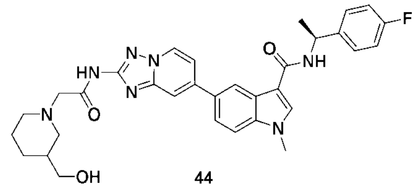

44

,

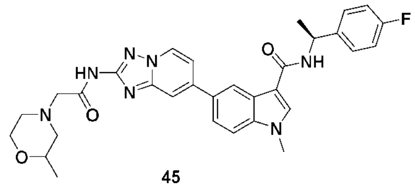

45

,

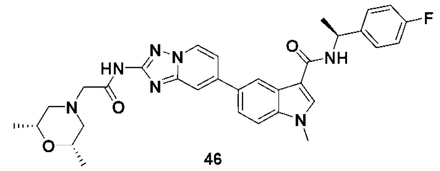

46

,

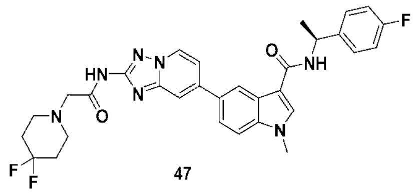

47

,

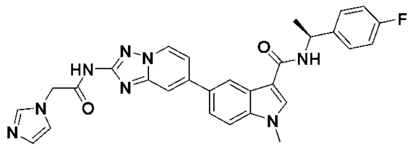

48

,

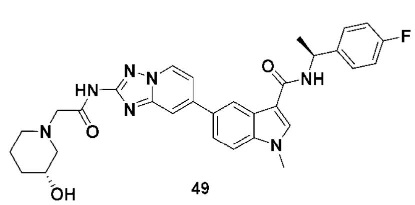

49

,

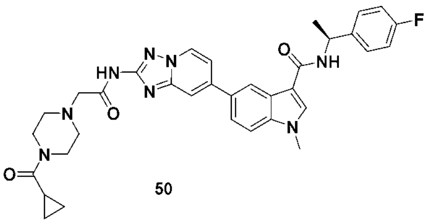

50

,

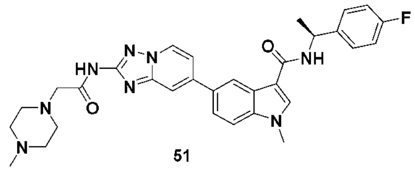

51

,

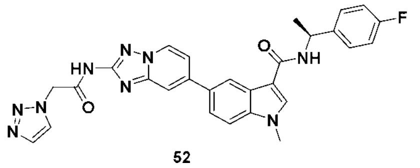

52

,

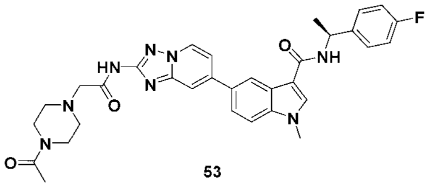
53
,

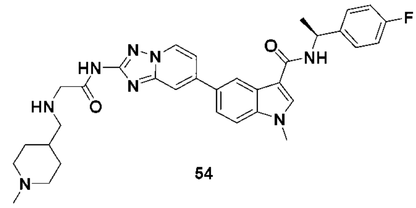
54
,

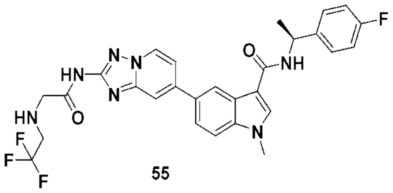
55
,

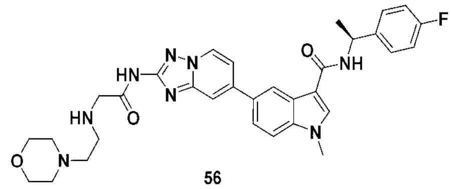
56
,

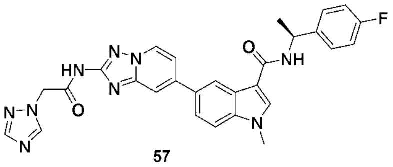
57
,

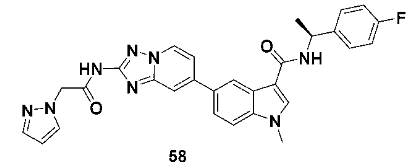
58
,

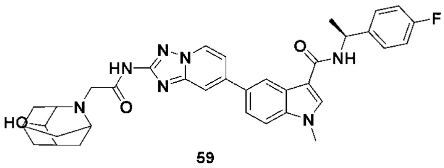
59
,

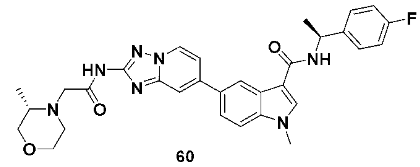
60
,

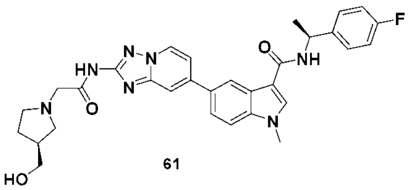
61
,

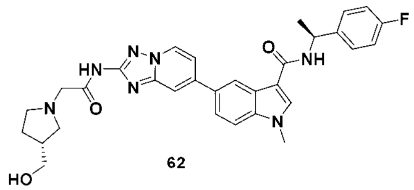
62
,

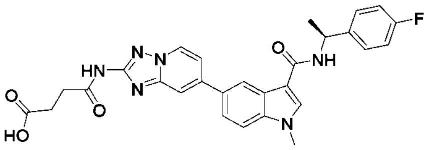
63
,

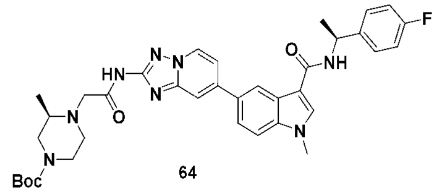
64
,

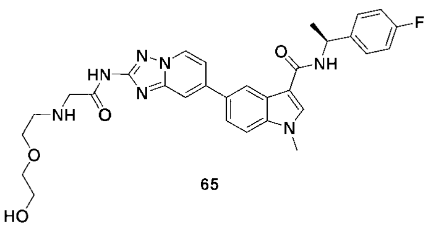
65

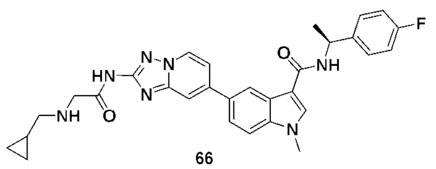
66
,

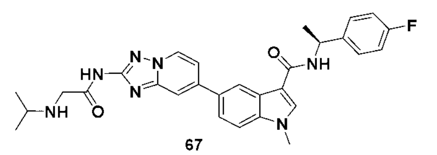
67
,

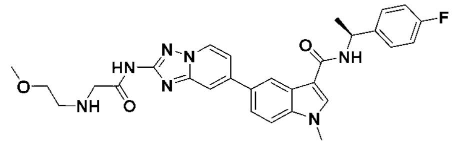
68

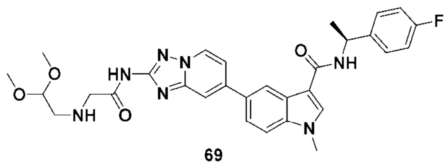
69
,

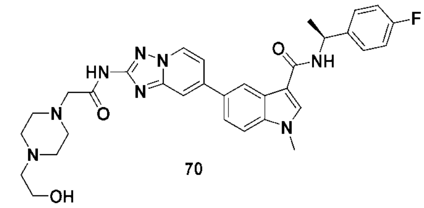
70
,

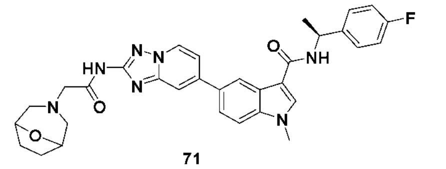
71
,

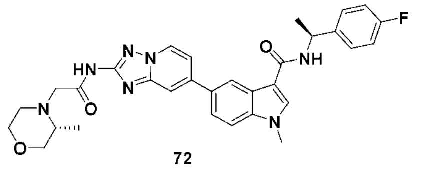
72
,

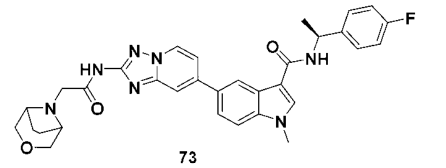
73
,

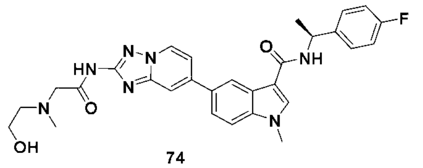
74
,

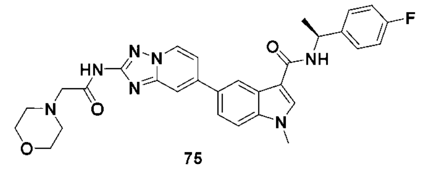
75
,

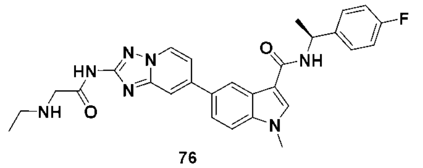
76
,

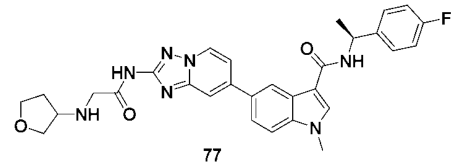
77
,

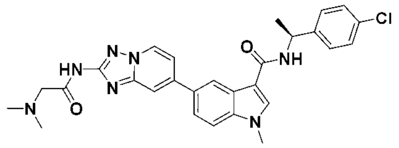
78
,

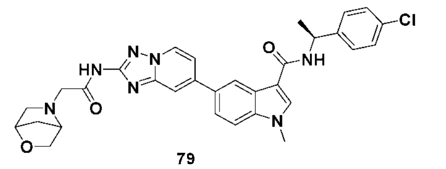
79
,

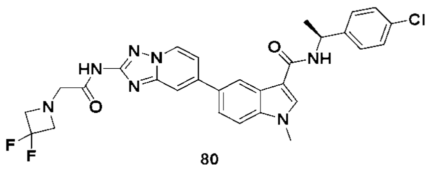
80
,

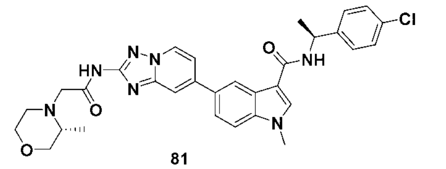
81
,

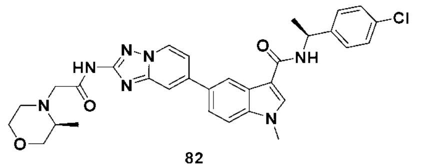
82
,

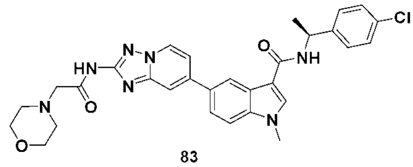

83 ,

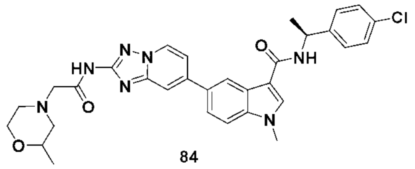

84 ,

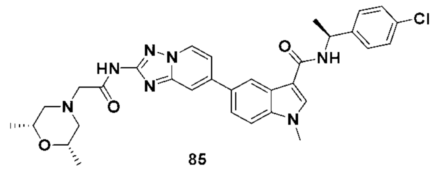

85 ,

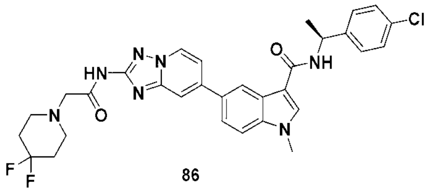

86 ,

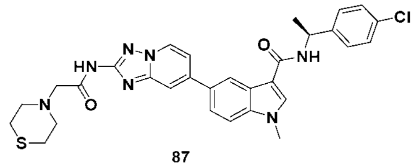

87 ,

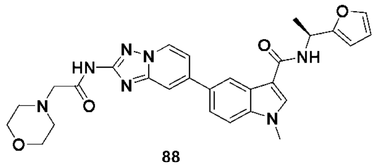

88 ,

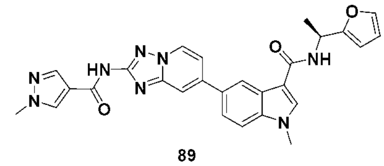

89 ,

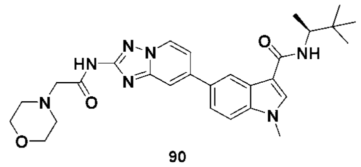

90 ,

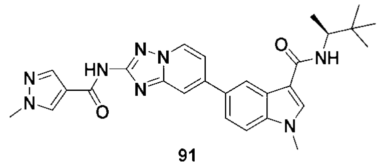

91 ,

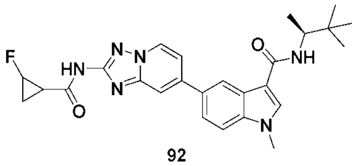

92 ,

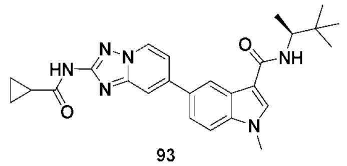

93 ,

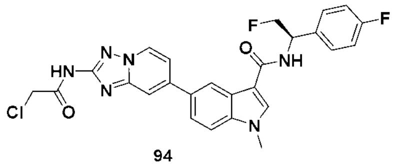

94 ,

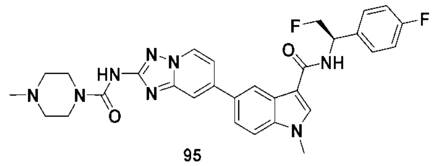

95 ,

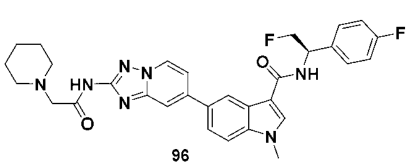

96 ,

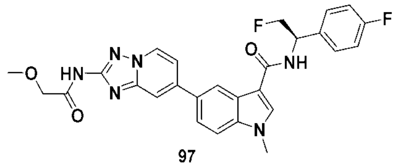

97 ,

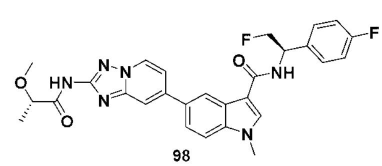

98 ,

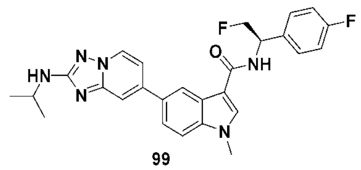

99 ,

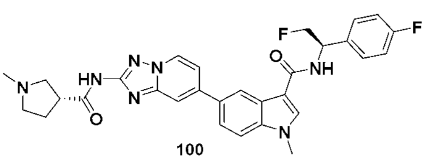

100 ,

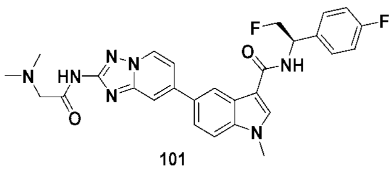

101 ,

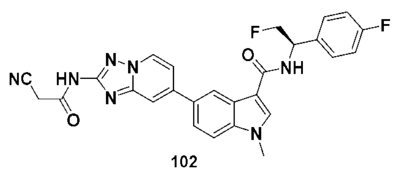

102 ,

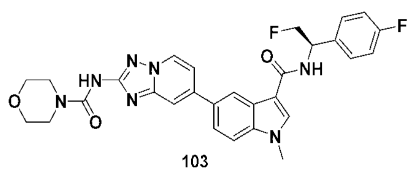

103 ,

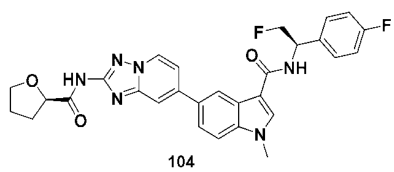

104 ,

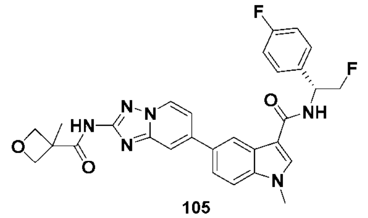
105
,
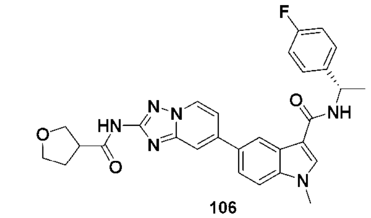
106
,
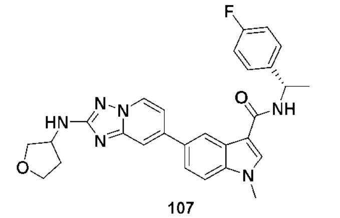
107
,

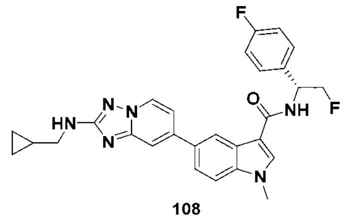
108
,
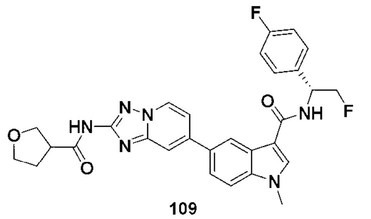
109
,
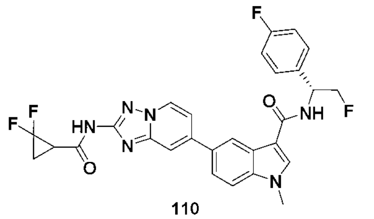
110
,

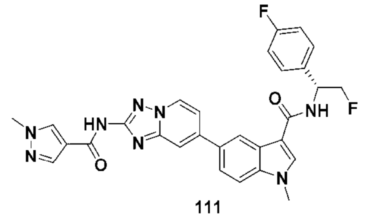
111
,
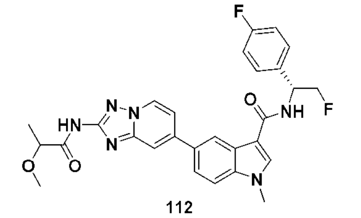
112
,
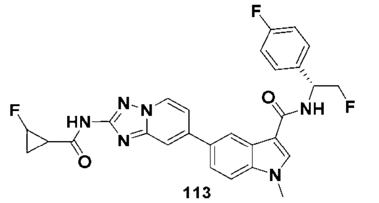
113
,

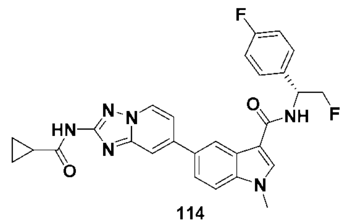
114
,
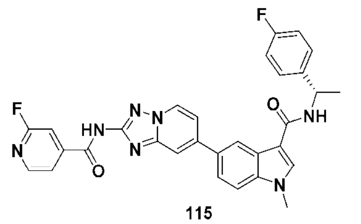
115
,
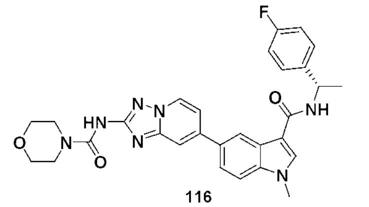
116
,

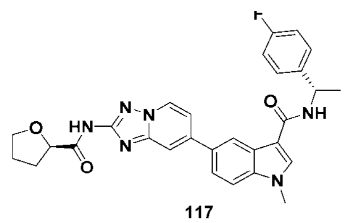
117
,
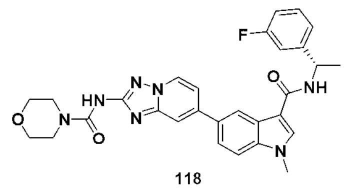
118
,
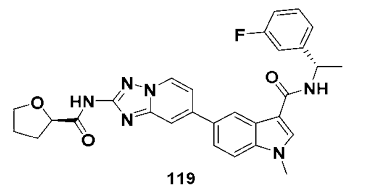
119
,

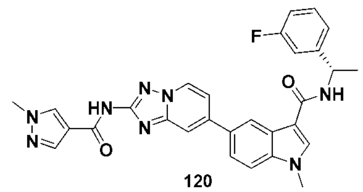
120
,
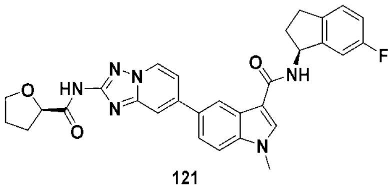
121
,
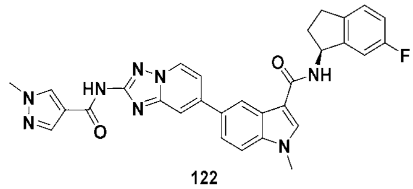
122
,

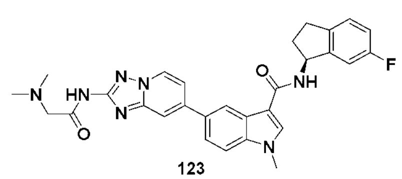
123
,
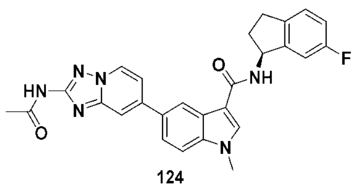
124
,
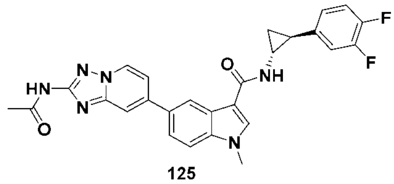
125
,

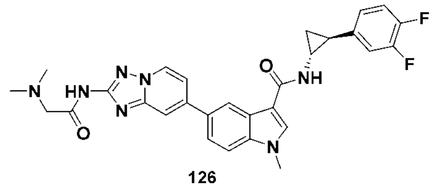
126
,
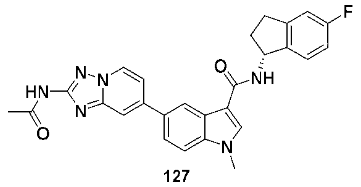
127
,
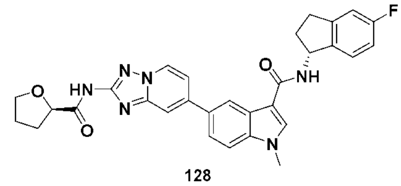
128
,

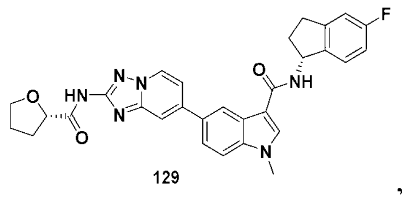
129
,
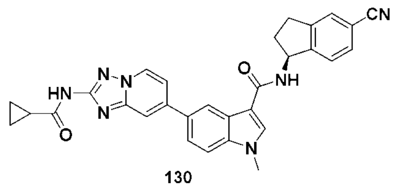
130
,
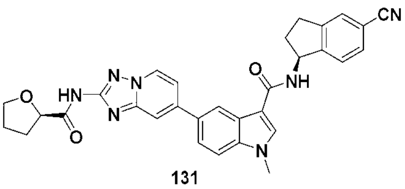
131
,

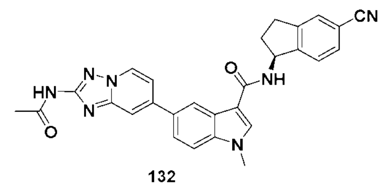
132
,
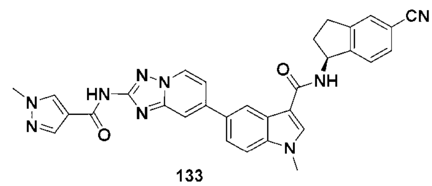
133
,

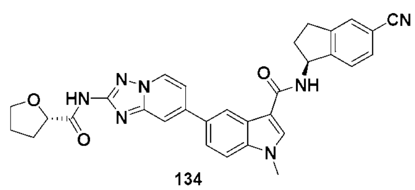
134
,
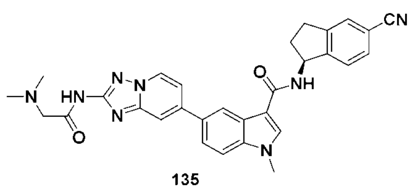
135
,

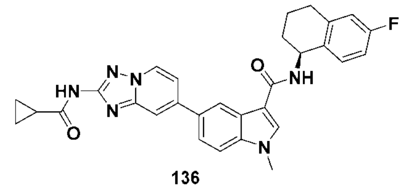
136
,
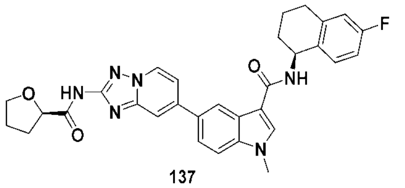
137
,
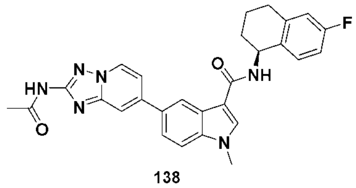
138
,

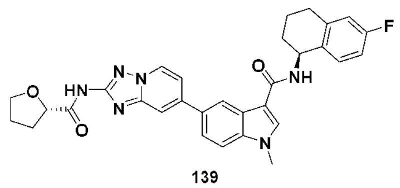
139
,
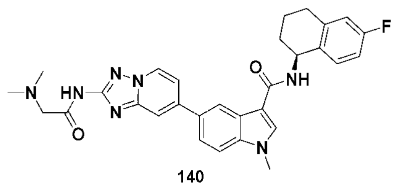
140
,

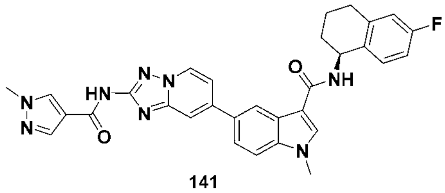
141

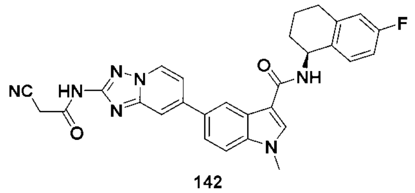
142

, ,

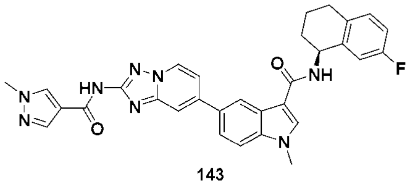

143 ,

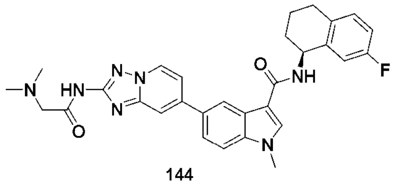

144 ,

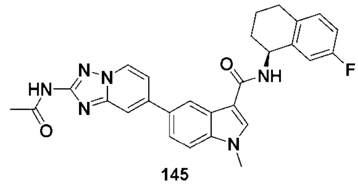

145 ,

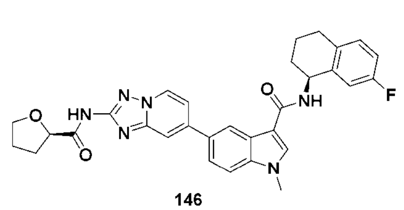

146 ,

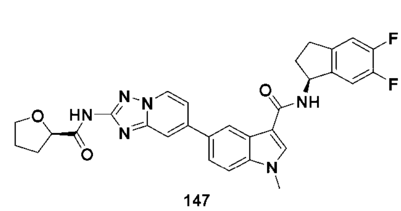

147 ,

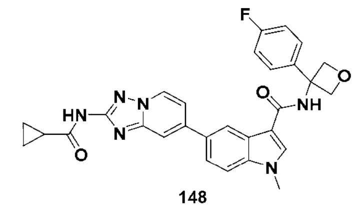

148 ,

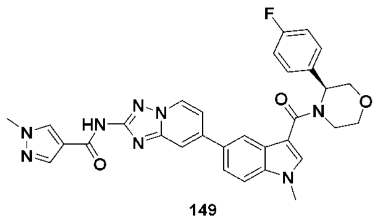

149 ,

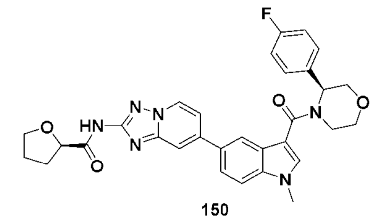

150 ,

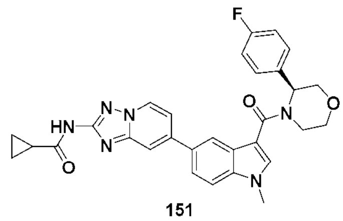

151 ,

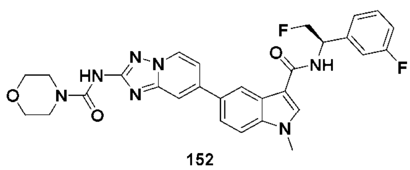

152 ,

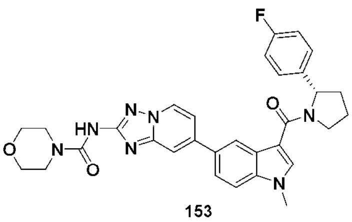

153 ,

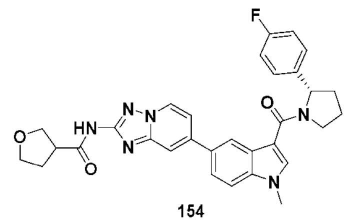

154 ,

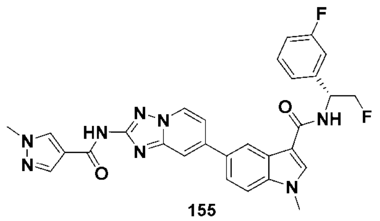

155 ,

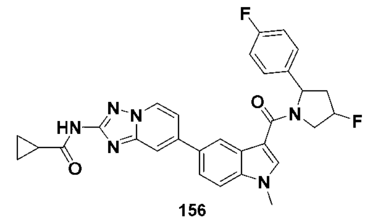

156 ,

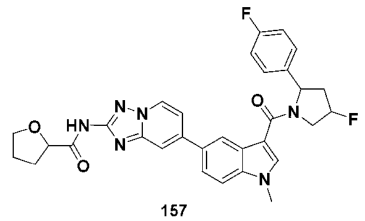

157 ,

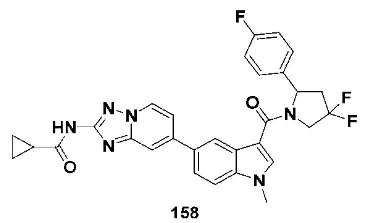

158 ,

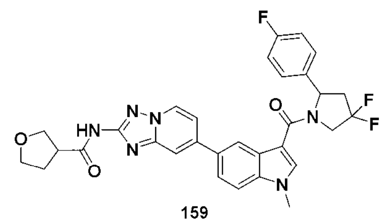

159 ,

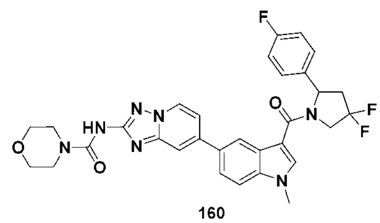

160 ,

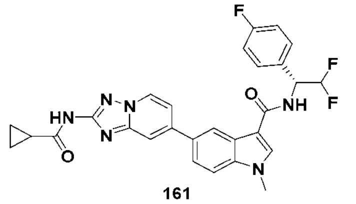
161 ,

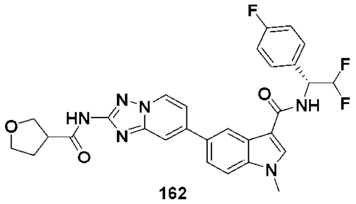
162 ,

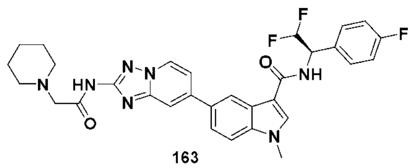
163 ,

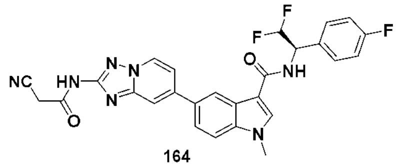
164 ,

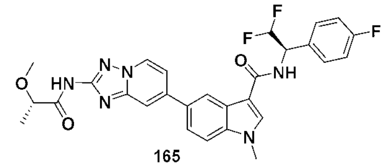
165 ,

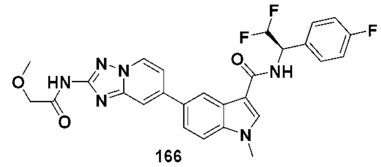
166 ,

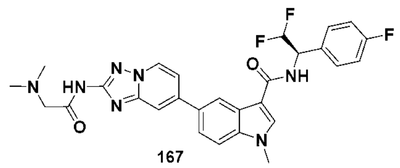
167 ,

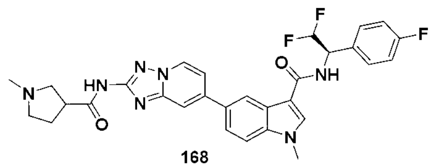
168 ,

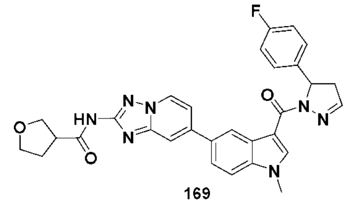
169 ,

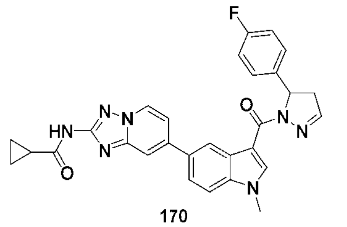
170 ,

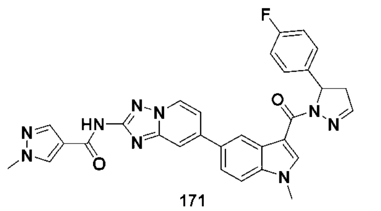
171 ,

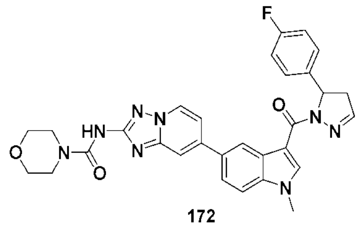
172 ,

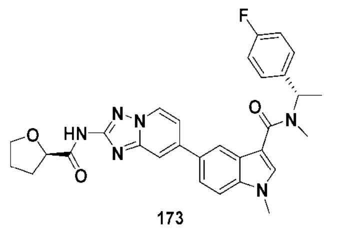
173 ,

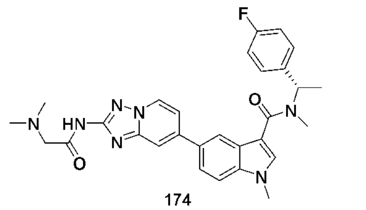
174 ,

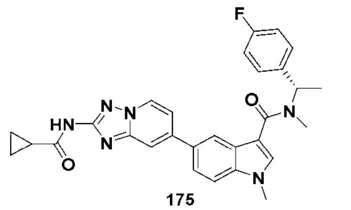
175 ,

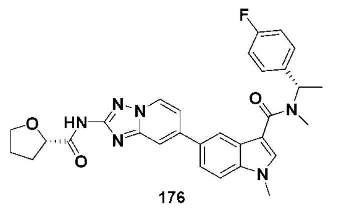
176 ,

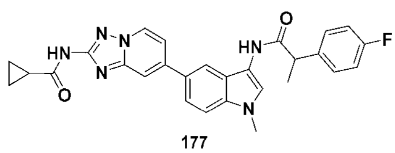
177 ,

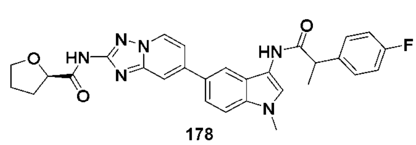
178 ,

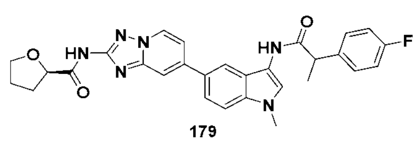
179 ,

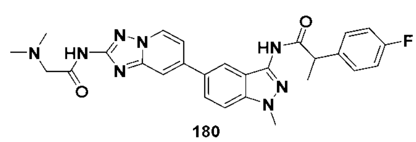
180 ,

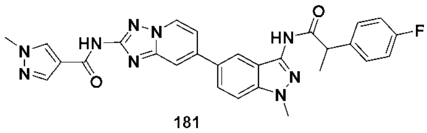
181 ,

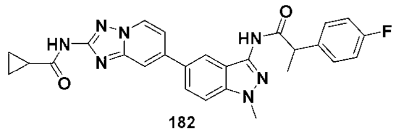
182 ,

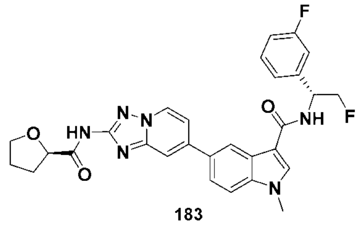
183 ,

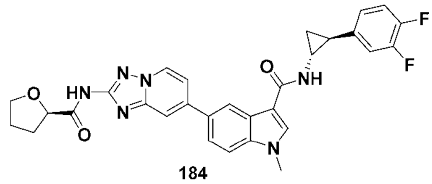
184 ,

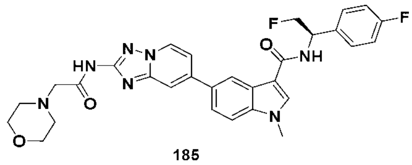
185 ,

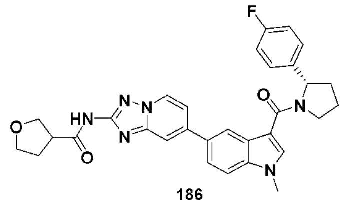
186 ,

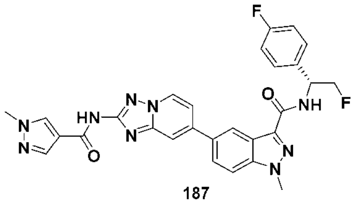
187 ,

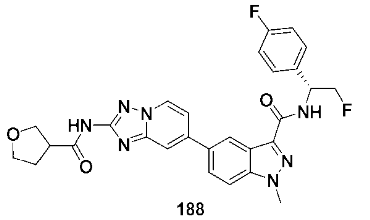
188 ,

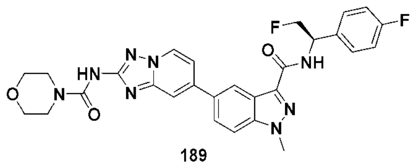
189 ,

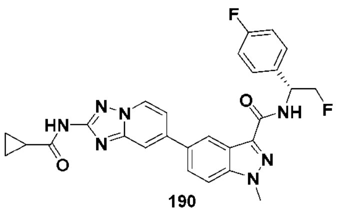
190 ,

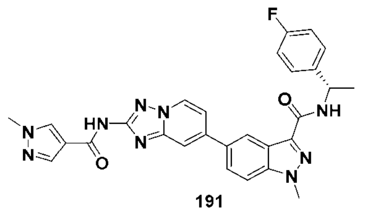
191 ,

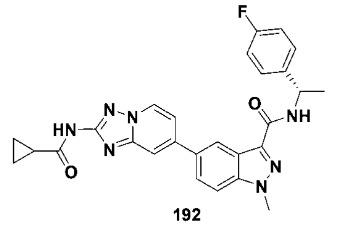
192 ,

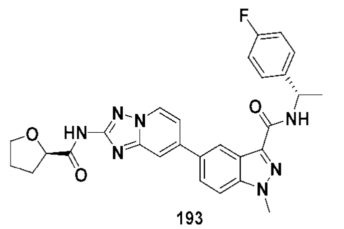
193 ,

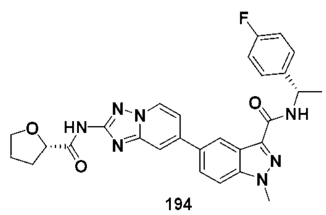
194 ,

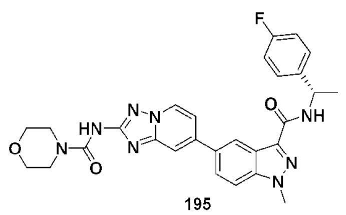
195 ,

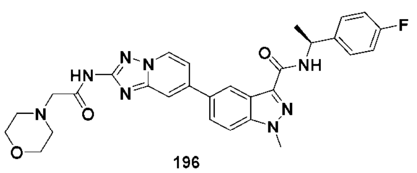
196 ,

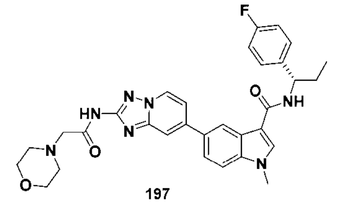
197 ,

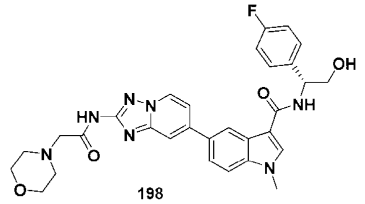
198

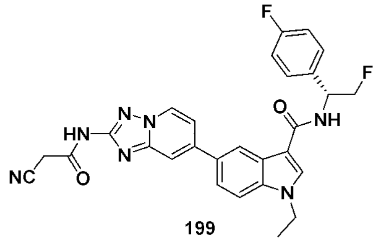
199 ,

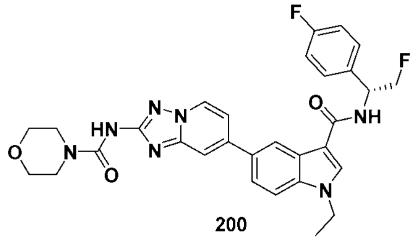
200 ,

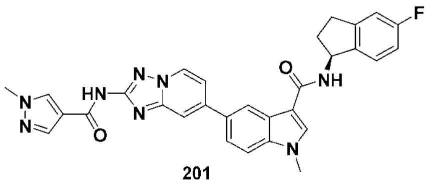
201

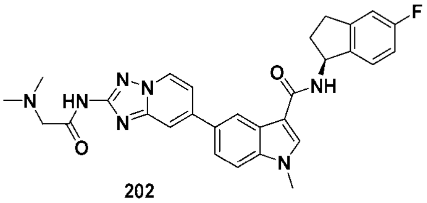
202 ,

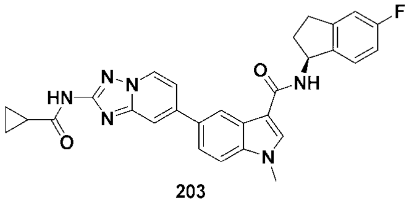
203 ,

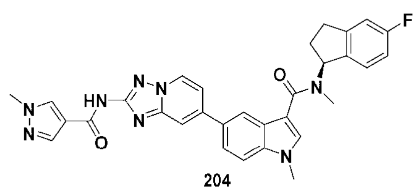
204 ,

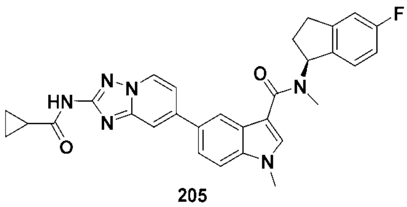
205 ,

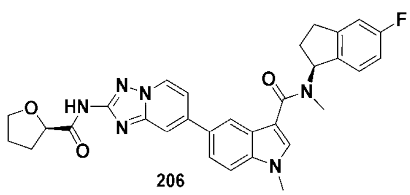
206 ,

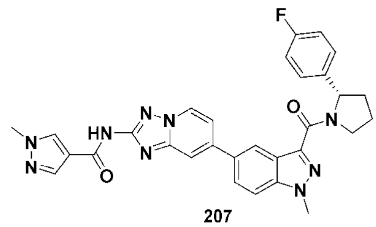
207 ,

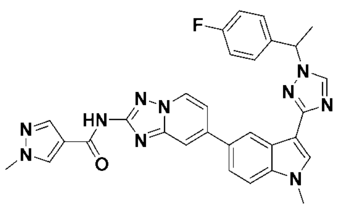
208 ,

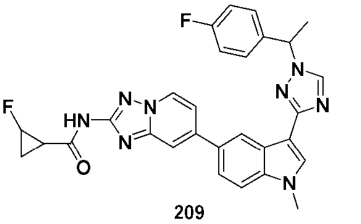
209 ,

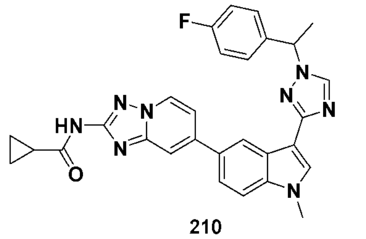
210 ,

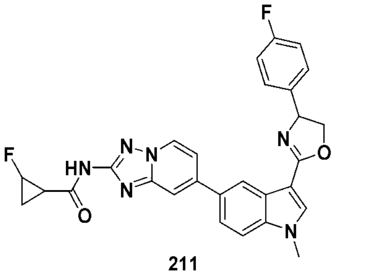
211 ,

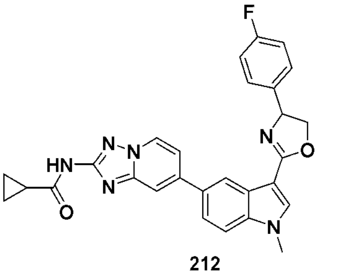
212 ,

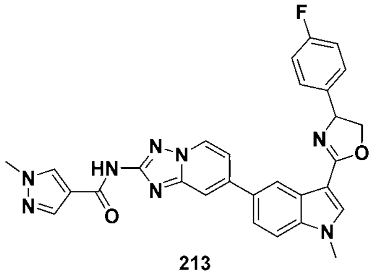
213 ,

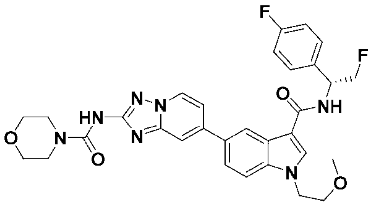
214
,
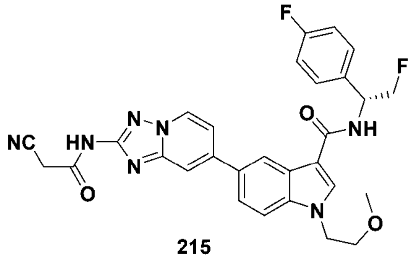
215
,
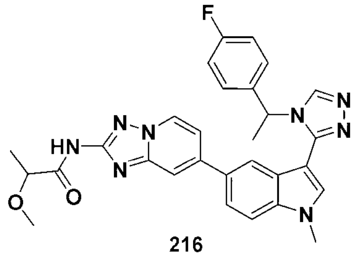
216
,
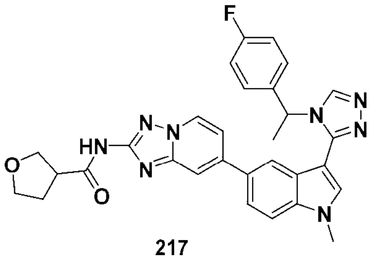
217
,
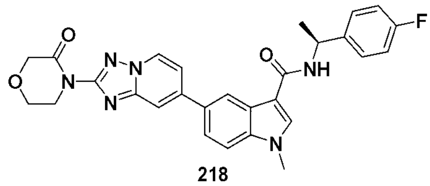
218
,
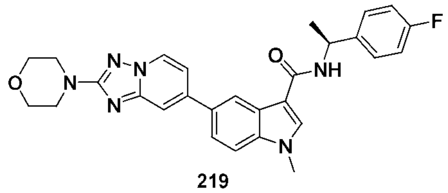
219
.

Another aspect of the present invention provides a pharmaceutical composition comprising the aforementioned compound, or a pharmaceutically acceptable salt, solvate or stereoisomer thereof. Another aspect of the present invention provides the use of the aforementioned compound, or a pharmaceutically acceptable salt, solvate or stereoisomer thereof or a pharmaceutical composition thereof, in the manufacture of a medicament for preventing and/or treating inflammation, autoimmune diseases, neurodegenerative diseases and tumor-related diseases.

Preferably, it provides the use in the manufacture of a drug for treating tumor-related diseases.

Another aspect of the present invention provides the use of the aforementioned compound, or a pharmaceutically acceptable salt, solvate or stereoisomer thereof or a pharmaceutical composition thereof, in the manufacture of a RIPK1 and/or MLK inhibitor.

Preferably, the said MLK inhibitor is an MLK2/3 inhibitor.

A method for preventing and/or treating inflammation, autoimmune diseases, neurodegenerative diseases and tumor-related diseases, comprising the step of administering to a patient in need thereof the aforementioned compound, or a pharmaceutically acceptable salt, solvate or stereoisomer thereof or a pharmaceutical composition thereof.

Preferably, it comprises the step of administering a therapeutically effective dose of the aforementioned compound, or a pharmaceutically acceptable salt, solvate, stereoisomer or pharmaceutical composition thereof, to a patient in need thereof.

## DESCRIPTION OF EMBODIMENTS

### I. Definitions

In the present invention, scientific and technical terms used herein have the meanings commonly understood by those skilled in the art, unless otherwise specified. Furthermore, the relevant terms and laboratory operations used herein are widely used terms and routine steps in the corresponding field. Meanwhile, for a better understanding of the present invention, definitions and explanations of relevant terms are provided below.

As used herein and unless otherwise specified, the term "about" or "approximately" means within ± 10% of a given value or range. Where an integer is required, the term refers to rounding to the nearest integer within ± 10% of the given value or range.

In the description herein, references to "some embodiments", "some implementations" or "some schemes"

describe a subset of all possible embodiments; however, it is understood that "some embodiments" may be the same subset or different subsets of all possible embodiments and may be combined with each other without conflict.

As used herein and unless otherwise specified, the terms "comprising", "including", "having", "containing", and grammatical equivalents thereof, should generally be understood as open-ended and non-restrictive, for example, not excluding other unrecited elements or steps.

As used herein, the term "pharmaceutically acceptable salt" refers to a salt of the compound in the present invention that is pharmaceutically acceptable and possesses the pharmacological activity of the parent compound. Such salt includes acid addition salt formed with inorganic acids or organic acids, such as nitric acid, phosphoric acid, carbonic acid, etc. as the said inorganic acids, or propionic acid, hexanoic acid, cyclopentylpropionic acid, glycolic acid, pyruvic acid, gluconic acid, stearic acid, muconic acid, etc. as the said organic acids; or salt formed when an acidic proton present in the parent compound is substituted with a metal ion, such as an alkali metal ion or an alkaline earth metal ion; or coordination compounds formed with an organic base, such as ethanolamine. The pharmaceutically acceptable salt in the present invention can be synthesized from a parent compound containing a basic or acidic moiety by conventional chemical methods. Generally, such salt is prepared by reacting the free acid or base forms of these compounds with a stoichiometric amount of the appropriate base or acid in water or in an organic solvent, or in a mixture thereof. Generally, non-aqueous media such as ether, ethyl acetate, ethanol, isopropanol, or acetonitrile are preferred. In addition to salt forms, the compound in the present invention also exists in the form of prodrug. The prodrug of the compound described herein readily undergo chemical changes under physiological conditions to convert into the compound in the present invention. In addition, the prodrug can be converted into the compound in the present invention using chemical or biochemical methods in an *in vivo* environment.

As used herein, the term "solvate" refers to a substance formed by the combination of a compound in the present invention with a pharmaceutically acceptable solvent. Pharmaceutically acceptable solvents include acetic acid, and the like. Solvates include stoichiometric solvates and nonstoichiometric solvates. Some compounds provided in the present invention may exist in non-solvate forms or solvate forms. In general, the solvate forms are equivalent to the non-solvate forms and are encompassed within the scope of the present invention.

As used herein, the term "stereoisomer" includes conformational isomers and configurational isomers, wherein the configurational isomers mainly include cis-trans isomers and optical isomers. The compound in the present invention may exist in the form of stereoisomer, and thus encompasses all possible stereoisomer forms, including but not limited to cis-trans isomers, tautomers, enantiomers, diastereoisomers, atropisomers. The compound in the present invention may also exist in the form of any combination or mixture of the aforementioned stereoisomers, such as meso compounds, racemates, equimolar mixture of atropisomers. For example, a single enantiomer, a single diastereomer or a mixture thereof, or a single atropisomer or a mixture thereof. When the compound in the present invention contains an olefinic double bond, unless otherwise specified, it includes cisisomers and trans-isomers, as well as any combination thereof. The atropisomer in the present invention is a stereoisomer based on axial or planar chirality resulting from restricted intramolecular rotation. Furthermore, as a drug, the stereoisomer with excellent activity is preferred. The compound in the present invention possesses optical isomers derived from asymmetric carbons, etc. If necessary, a single isomer can be obtained by resolution using methods known in the art, such as crystallization or chiral chromatography.

As used herein, the term "heteroatom" is selected from nitrogen, oxygen, or sulfur. Wherein, the nitrogen may be optionally substituted; and the sulfur may also be optionally substituted, for example with oxo, i.e., forming S(O)m3 (wherein m3 is an integer from 0 to 2).

As used herein, the term "alkyl" refers to a straight or branched saturated aliphatic hydrocarbon group. The term "$C_{1-20}$ alkyl" refers to a straight or branched alkyl group having 1 to 20 carbon atoms. $C_{1-10}$ alkyl is preferred. $C_{1-6}$ alkyl (i.e., a straight or branched alkyl group having 1, 2, 3, 4, 5, or 6 carbon atoms) is more preferred. $C_{1-4}$ alkyl is more preferred. $C_{1-3}$ alkyl is more preferred. Specific examples include, but are not limited to, methyl, ethyl, n-propyl, isopropyl, n-butyl, isobutyl, tert-butyl, secbutyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and various branched isomers thereof.

As used herein, the term "alkoxy" refers to a group having an -O-alkyl structure, wherein alkyl is as defined above. The term "$C_{1-6}$ alkoxy" refers to alkoxy having 1 to 6 carbon atoms. Preferably, it is $C_{1-4}$ alkoxy, and more preferably $C_{1-3}$ alkoxy. Specific examples include, but are not limited to, methoxy, ethoxy, n-propoxy, isopropoxy, n-butoxy, tert-butoxy, isobutoxy, n-pentyloxy, and the like. As used herein, the term "cycloalkyl" refers to a saturated monocyclic or polycyclic hydrocarbon group, including, for example, monocyclic cycloalkyl, spirocycloalkyl, fused cycloalkyl, and bridged cycloalkyl. The ring carbon atoms of the cycloalkyl in the present invention may optionally be substituted with 1, 2 or 3 oxo groups to form a cyclic ketone structure. The term "3- to 20-membered cycloalkyl" or "$C_{3-20}$ cycloalkyl" refers to cycloalkyl having 3 to 20 ring carbon atoms, including monocyclic cycloalkyl, spirocycloalkyl, fused cycloalkyl and bridged cycloalkyl. $C_{3-12}$ cycloalkyl, $C_{5-20}$ spirocycloalkyl, $C_{5-20}$ fused cycloalkyl or $C_{5-20}$ bridged cycloalkyl is preferred. $C_{3-8}$ monocyclic cycloalkyl is more preferred.

As used herein, the term "spirocycloalkyl" refers to a saturated or partially unsaturated polycyclic hydrocarbon group in which one carbon atom (called a spiro atom) is shared between the rings. The term "spiroheterocycloalkyl" refers to spirocycloalkyl containing a C atom and at least one heteroatom selected from N, O and S as ring members. Non-limiting examples of spiroheterocycloalkyl include:

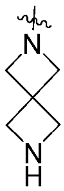, 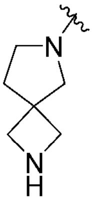, 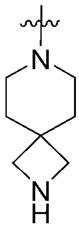, 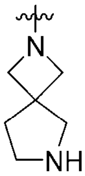, 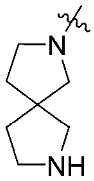, 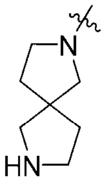, 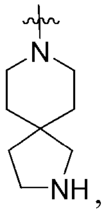, 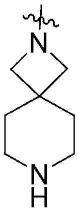, 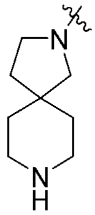, 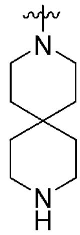, 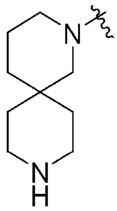,

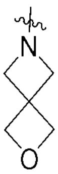, 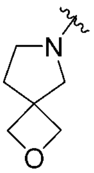, 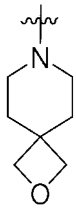, 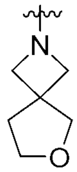, 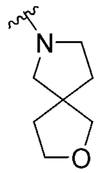, 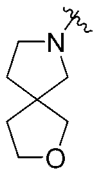, 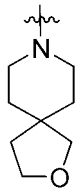, 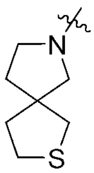 and 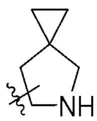.

As used herein, the term "bridged cycloalkyl" refers to a saturated or partially unsaturated polycyclic hydrocarbon group in which any two rings share two carbon atoms that are not directly connected. The term "bridged heterocycloalkyl" refers to a bridged cycloalkyl containing C atoms and at least one heteroatom selected from N, O and S as ring members.

As used herein, the term "heterocycloalkyl" refers to a saturated ring substituent containing heteroatoms in the ring skeleton, wherein one or more (preferably 1 to 4, 1 to 3, or 1 to 2) ring atoms are heteroatoms selected from N, O, and S. The term "4- to 12-membered heterocycloalkyl" refers to a saturated ring substituent optionally containing 1, 2, 3, or 4 heteroatoms selected from N, O, and S as ring atoms. The term "4- to 12-membered nitrogen-containing heterocycloalkyl" refers to a 4- to 12-membered heterocycloalkyl in which one ring atom must be a nitrogen atom, and the remaining ring atoms are all carbon atoms or 0, 1, 2, or 3 of the remaining ring atoms are independently heteroatoms selected from N, O, and S. The said "4- to 12-membered nitrogen-containing heterocycloalkyl" is preferably attached to the remainder of the molecule via the mandatory nitrogen atom.

As used herein, the term "partially unsaturated heterocycle" refers to a cyclic group containing a C atom and at least one heteroatom selected from N, O, or S as ring members; wherein the bicyclic group is partially unsaturated and contains at least one C-C double bond, preferably one C-C double bond. As used herein, the term "partially unsaturated bicyclic ring" includes "partially unsaturated bicyclic carbocycle" and "partially unsaturated bicyclic heterocycle", wherein the term "partially unsaturated bicyclic carbocycle" refers to a bicyclic group containing only C atoms as ring members, which is partially unsaturated and contains at least one C-C double bond; a maximally unsaturated carbocycle contains the maximum number of conjugated C-C double bonds allowed by the ring size, while a partially unsaturated bicyclic carbocycle contains fewer than the maximum number of C-C double bonds allowed by the ring size. The said "partially unsaturated bicyclic carbocycle" is preferably formed by the fusion of $C_{5-8}$ aryl with 5- or 6-membered monocyclic ring, wherein the said 5- or 6-membered monocyclic ring is selected from the following group:

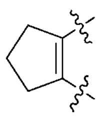, 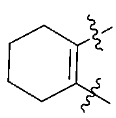.

As used herein, the term "partially unsaturated bicyclic heterocycle" refers to a bicyclic group comprising a C atom and at least one heteroatom selected from N, O, and S as ring members, wherein the bicyclic group is partially unsaturated and contains at least one C-C double bond. A maximally unsaturated heterocycle comprises the maximum number of C-C double bonds and double bonds between C atoms and heteroatoms allowed by the ring size, whereas a partially unsaturated bicyclic heterocycle comprises fewer double bonds than the number allowed by the ring size.

As used herein, the term "aryl" refers to an all-carbon monocyclic, all-carbon non-fused polycyclic (rings connected via covalent bonds and not fused) or all-carbon fused polycyclic (i.e., rings sharing adjacent pairs of carbon atoms) group, wherein at least one ring in the group is aromatic, i.e., possessing a conjugated π-electron system. The term

"$C_{6-14}$ aryl" refers to aryl having 6 to 14 ring atoms. $C_{6-10}$ aryl is preferred. In the present invention, $C_{6-14}$ aryl includes monocyclic aryl, non-fused polycyclic aryl and aromatic fused polycyclic aryl, wherein examples of monocyclic aryl include phenyl, and examples of non-fused polycyclic aryl include biphenyl, etc.

As used herein, the term "5- or 6-membered monocyclic heteroaryl" refers to monocyclic heteroaryl having 5 or 6 ring atoms, wherein 1, 2 or 3 ring atoms are heteroatoms selected from N, O and S(=O)m' (where m' is an integer from 0 to 2). Specific examples of monocyclic heteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine.

"Optionally substituted with one or more ..." means that it may be substituted with one or more specified substituents (preferably 1, 2 or 3), or may be unsubstituted; regarding "more" in the phrase "one or more", unless otherwise specified, the minimum value is 2, and the maximum value is the number of substitutable sites of the group to be substituted.

If substituents are described as being "each selected from" or "independently selected from" a group, each substituent is selected independently of the other. Therefore, each substituent may be the same as or different from the other substituent(s).

"Substitution" refers to the replacement of a hydrogen atom in a molecule with a different group. "Member" refers to the number of skeletal atoms constituting a ring.

The term "bond" in the present invention refers to a direct bond, which can also be understood as "absent".

"Azacycloalkyl" refers to cycloalkyl containing a nitrogen atom in the ring skeleton.

"Bridged cycloalkyl" refers to polycyclic cycloalkyl in which two rings share two adjacent carbon atoms.

"Spirocycloalkyl" refers to cycloalkyl in which two carbon rings in the molecule share one carbon atom, and the shared carbon atom is referred to as a "spiro carbon atom".

Unless otherwise specified, a ring formed by "connecting to form a ring" in the present invention includes unsubstituted rings and rings substituted with substituents.

"Alkoxy" refers to -O-alkyl.

"Halogen" refers to fluorine, chlorine, bromine, or iodine.

"(O)" or "oxo" refers to =O.

"〰" represents the connecting position of a chemical bond.

Although the term "optionally substituted" may not appear, if a substitution occurs at any position on a ring (such as an aliphatic ring or an aromatic ring), it should be understood that the position and number of the substituents are arbitrary, provided that they are chemically feasible.

## II. Examples

In order to make the purpose, technical solutions, and advantages of the present invention clearer, the present invention is described in further detail below. The described embodiments should not be construed as limiting the present invention. All other embodiments obtained by those ordinarily skilled persons in the art without creative efforts fall within the protection scope of the present invention.

Before describing the embodiments of the present invention in further detail, terms and terminology involved in the embodiments of the present invention are explained. The terms and terminology involved in the embodiments of the present invention are applicable to the following explanations. The raw materials and equipment used in the specific embodiments of the present disclosure are all known products and are obtained by purchasing commercially available products.

### Preparation Examples:

Preparation of Intermediate **INT-1**:

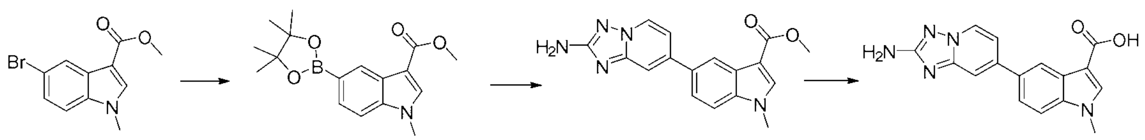

INT-1-1 INT-1-2 INT-1-3 INT-1

Intermediate **INT-1-2**: Preparation of methyl 1-methyl-5-(4,4,5,5-tetramethyl-1,3,2-dioxaborolan-2-yl)-1H-indol-3-carboxylate:

Under an argon atmosphere, Intermediate **INT-1-1** (1335 mg, 5.0 mmol) and bis(pinacolato) diboron (1651 mg, 6.5 mmol) were dissolved in 1,4-dioxane (20 mL), and potassium acetate (4.9 g, 10.0 mmol) and $PdCl_2(dppf)_2$ (160 mg) were added; three cycles of argon purging was performed, and the mixture was heated to 100°C and stirred for 10 h. The reaction mixture was concentrated under reduced pressure and separated and purified by silica gel column (petroleum ether/ethyl acetate = 5/1) to obtain Intermediate **INT-1-2** with a yield of 80%. MS (ESI, positive ion) m/z: 316.3 $[M + H]^+$.

Intermediate **INT-1-3**: Preparation of methyl 5-(2-amino-[1,2,4]triazolo[1,5-a]pyridin-7-yl)-1-methyl-1H-indol-3-carboxylate:

Under an argon atmosphere, Intermediate **INT-1-2** (1260 mg, 4.0 mmol) and 7-bromo-[1,2,4]triazolo[1,5-a]pyridin-2-amine (848 mg, 4.0 mmol) were dissolved in 1,4-dioxane (20 mL), potassium phosphate (1.7 g, 8.0 mmol), $PdCl_2(dppf)_2$ (120 mg), and water (2.0 mL) were added; three cycles of argon purging was performed, the mixture was heated to 95°C and stirred for 8 h. The reaction mixture was concentrated under reduced pressure and separated and purified by silica gel column (dichloromethane/methanol = 20/1) to obtain Intermediate **INT-1-3** with a yield of 70%. MS (ESI, positive ion) m/z: 322.2 $[M + H]^+$.

Intermediate **INT-1**: Preparation of 5-(2-amino-[1,2,4]triazolo[1,5-a]pyridin-7-yl)-1-methyl-1H-indol-3-carboxylic acid:

Intermediate **INT-1-3** (963 mg, 3.0 mmol) was dissolved in ethanol (3 mL), tetrahydrofuran (3 mL), and water (15 mL); sodium hydroxide (0.96 g, 24 mmol) was added; the mixture was heated to 75°C and stirred for 5 h. The pH was adjusted to slightly acidic with dilute hydrochloric acid (1 M). The mixture was filtered and dried to obtain Intermediate **INT-1** with a yield of 92%. MS (ESI, positive ion) m/z: 308.3 $[M + H]^+$.

Preparation of Intermediate **INT-2**:

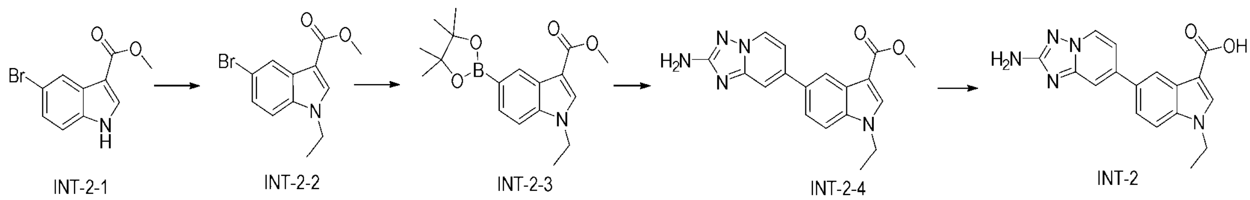

INT-2-1 INT-2-2 INT-2-3 INT-2-4 INT-2

Intermediate **INT-2-2**: Preparation of methyl 5-bromo-1-ethyl-1H-indol-3-carboxylate:

Intermediate **INT-2-1** (2.0 g, 8.0 mmol) was dissolved in N,N-dimethylformamide (20 mL), ethyl iodide (2.5 g, 16.0 mmol) and sodium hydride (640 mg, 16 mmol, 60%) were added in an ice bath; the mixture was stirred at room temperature for 2 h. Water (20 mL) was added in an ice bath, and the mixture was extracted with ethyl acetate twice. The pooled organic phases were dried over anhydrous sodium sulfate, filtered, and concentrated under reduced pressure. The residue was separated by silica gel column chromatography (petroleum ether/ethyl acetate = 10/1) to obtain Intermediate **INT-2-2** with a yield of 83%. MS (ESI, positive ion) m/z: 282.2 $[M + H]^+$.

Intermediate **INT-2-3**: Preparation of 1-ethyl-5-(4,4,5,5-tetramethyl-1,3,2-dioxaborolan-2-yl)-1H-indol-3-carboxylic acid methyl ester:

Under an argon atmosphere, Intermediate **INT-2-2** (1.4 g, 5.0 mmol) and bis(pinacolato) diboron (1651 mg, 6.5 mmol) were dissolved in 1,4-dioxane (20 mL), and potassium acetate (4.9 g, 10.0 mmol) and $PdCl_2(dppf)_2$ (160 mg) were added; three cycles of argon purging was performed, and the mixture was heated to 100°C and stirred for 10 h. The reaction mixture was concentrated under reduced pressure and separated and purified by silica gel column (petroleum ether/ethyl acetate = 5/1) to obtain Intermediate **INT-2-3** with a yield of 80%. MS (ESI, positive ion) m/z: 330.4 $[M + H]^+$.

Intermediate **INT-2-4**: Preparation of 5-(2-amino-[1,2,4]triazolo[1,5-a]pyridin-7-yl)-1-ethyl-1H-indol-3-carboxylic acid methyl ester:

Under an argon atmosphere, Intermediate **INT-2-3** (1.3 g, 4.0 mmol) and 7-bromo-[1,2,4]triazolo[1,5-a]pyridin-2-amine (848 mg, 4.0 mmol) were dissolved in 1,4-dioxane (20 mL), potassium phosphate (1.7 g, 8.0 mmol), $PdCl_2(dppf)_2$ (120 mg), and water (2.0 mL) were added; three cycles of argon purging was performed, the mixture was heated to 95°C and stirred for 8 h. The reaction mixture was concentrated under reduced pressure and separated and purified by silica gel column (dichloromethane/methanol = 20/1) to obtain Intermediate **INT-2-4** with a yield of 70%. MS (ESI, positive ion) m/z: 322.2 $[M + H]^+$.

Intermediate **INT-2**: Preparation of 5-(2-amino-[1,2,4]triazolo[1,5-a]pyridin-7-yl)-1-ethyl-1H-indol-3-carboxylic acid:

Intermediate **INT-2-4** (1.0 g, 3.0 mmol) was dissolved in ethanol (3 mL), tetrahydrofuran (3 mL), and water (15 mL); sodium hydroxide (0.96 g, 24 mmol) was added; the mixture was heated to 75°C and stirred for 5 h. The pH was adjusted to slightly acidic with dilute hydrochloric acid (1 M). The mixture was filtered and dried to obtain Intermediate **INT-2** with a yield of 92%. MS (ESI, positive ion) m/z: 322.2 $[M + H]^+$.

Preparation of Intermediate **INT-3**:

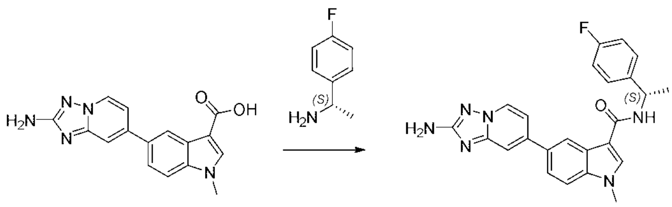

INT-1-4 INT-3

Intermediate **INT-3**: Preparation of (S)-5-(2-amino-[1,2,4]triazolo[1,5-a]pyridin-7-yl)-N-[1-(4-fluorophenyl)ethyl]-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-1-(4-fluorophenyl)ethan-1-amine (167 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-3** with a yield of 70%. MS (ESI, positive ion) m/z: 429.3 $[M + H]^+$.

Preparation of Intermediate **INT-4**:

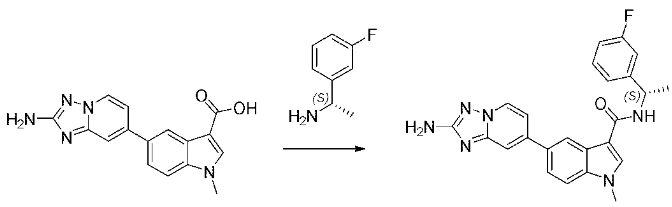

INT-1-4 INT-4

Intermediate **INT-4**: Preparation of (S)-5-(2-amino-[1,2,4]triazolo[1,5-a]pyridin-7-yl)-N-[1-(3-fluorophenyl)ethyl]-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-1-(3-fluorophenyl)ethan-1-amine (167 mg, 1.2 mmol) was added, and the mixture was stirred for reaction at room

temperature for 2 h. The reaction mixture was added dropwise to water and stirred for 0.5 h. The mixture was filtered and dried to obtain Intermediate **INT-4** with a yield of 71%. MS (ESI, positive ion) m/z: 429.3 [M + H]$^+$.

Preparation of Intermediate **INT-5**:

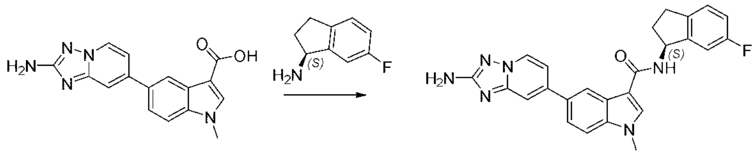

INT-1-4 INT-5

Intermediate **INT-5**: Preparation of (S)-5-(2-amino-[1,2,4]triazolo[1,5-a]pyridin-7-yl)-N-(6-fluoro-2,3-dihydro-1H-inden-1-yl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-6-fluoro-2,3-dihydro-1H-inden-1-amine (181 mg, 1.2 mmol) was added, and the mixture was stirred for reaction at room temperature for 2 h; the reaction mixture was added dropwise into water and stirred for 0.5 h; the mixture was filtered and dried to obtain Intermediate **INT-5** with a yield of 73%. MS (ESI, positive ion) m/z: 441.2 [M + H]$^+$.

Preparation of Intermediate **INT-6**:

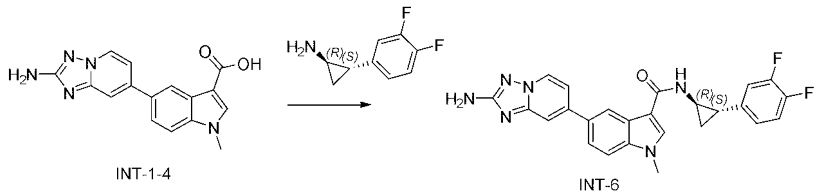

INT-1-4 INT-6

Intermediate **INT-6**: Preparation of 5-(2-amino-[1,2,4]triazolo[1,5-a]pyridin-7-yl)-N-[(1R,2S)-2-(3,4-difluorophenyl)cyclopropyl]-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (1R,2S)-2-(3,4-difluorophenyl)cyclopropan-1-amine (202 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-6** with a yield of 68%. MS (ESI, positive ion) m/z: 459.3 [M + H]$^+$.

Preparation of Intermediate **INT-7**:

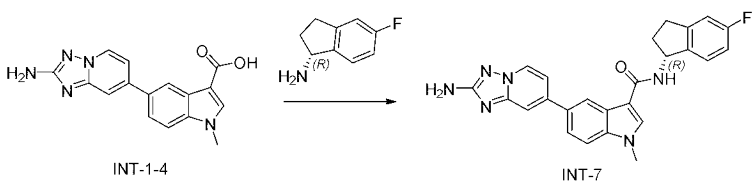

INT-1-4 INT-7

Intermediate **INT-7**: Preparation of (R)-5-(2-amino-[1,2,4]triazolo[1,5-a]pyridin-7-yl)-N-(5-fluoro-2,3-dihydro-1H-inden-1-yl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (R)-5-fluoro-2,3-dihydro-1H-inden-1-amine (181 mg, 1.2 mmol) was added, and the mixture was stirred for reaction at room temperature for 2 h; the reaction mixture was added dropwise into water and stirred for 0.5 h; the mixture was filtered and dried to obtain Intermediate **INT-7** with a yield of 68%. MS (ESI, positive ion) m/z: 443.3 $[M + H]^+$.

Preparation of Intermediate **INT-8**:

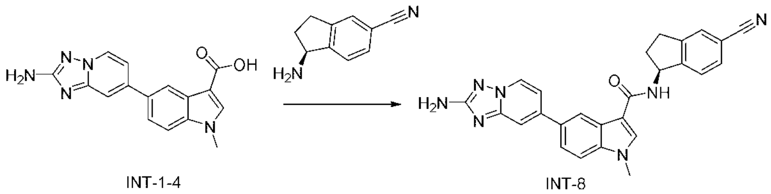

INT-1-4 INT-8

Intermediate **INT-8**: Preparation of (S)-5-(2-amino-[1,2,4]triazolo[1,5-a]pyridin-7-yl)-N-(5-cyano-2,3-dihydro-1H-inden-1-yl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-1-amino-2,3-dihydro-1H-inden-5-carbonitrile (181 mg, 1.2 mmol) was added, and the mixture was stirred for reaction at room temperature for 2 h; the reaction mixture was added dropwise into water and stirred for 0.5 h; the mixture was filtered and dried to obtain Intermediate **INT-8** with a yield of 66%. MS (ESI, positive ion) m/z: 448.3 $[M + H]^+$.

Preparation of Intermediate **INT-9**:

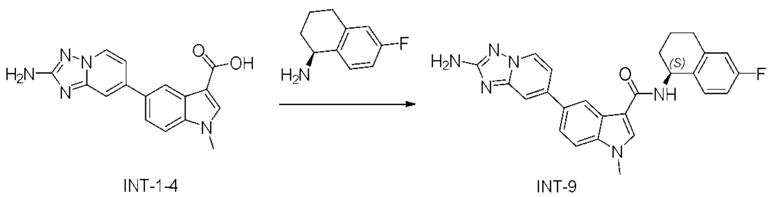

INT-1-4 INT-9

Intermediate **INT-9**: Preparation of (S)-5-(2-amino-[1,2,4]triazolo[1,5-a]pyridin-7-yl)-N-(6-fluoro-1,2,3,4-tetrahydronaphthalen-1-yl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-6-fluoro-1,2,3,4-tetrahydronaphthalen-1-amine (198 mg, 1.2 mmol) was added, and the mixture was stirred for reaction at room temperature for 2 h; the reaction mixture was added dropwise into water and stirred for 0.5 h; the mixture was filtered and dried to obtain Intermediate **INT-9** with a yield of 69%. MS (ESI, positive ion) m/z: 455.3 $[M + H]^+$.

Preparation of Intermediate **INT-10**:

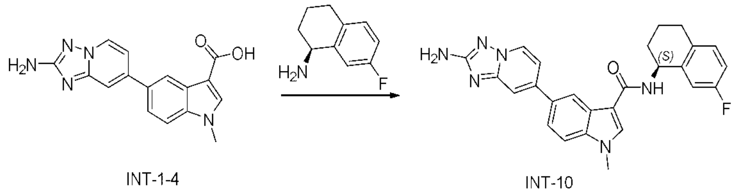

INT-1-4 INT-10

Intermediate **INT-10**: Preparation of (S)-5-(2-amino-[1,2,4]triazolo[1,5-a]pyridin-7-yl)-N-(7-fluoro-1,2,3,4-tetrahydronaphthalen-1-yl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-7-fluoro-1,2,3,4-tetrahydronaphthalen-1-amine (198 mg, 1.2 mmol) was added, and the mixture was stirred for reaction at room temperature for 2 h; the reaction mixture was added dropwise into water and stirred for 0.5 h; the mixture was filtered and dried to obtain Intermediate **INT-10** with a yield of 68%. MS (ESI, positive ion) m/z: 455.3 [M + H]$^+$.

Preparation of Intermediate **INT-11**:

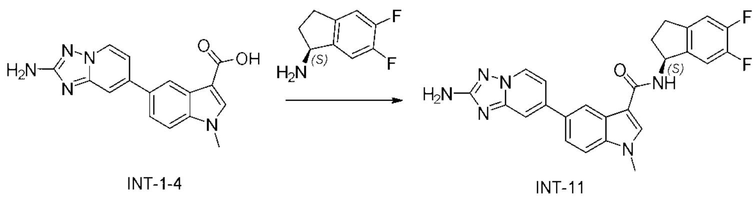

INT-1-4 INT-11

Intermediate **INT-11**: Preparation of (S)-5-(2-amino-[1,2,4]triazolo[1,5-a]pyridin-7-yl)-N-(5,6-difluoro-2,3-dihydro-1H-inden-1-yl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-5,6-difluoro-2,3-dihydro-1H-inden-1-amine (203 mg, 1.2 mmol) was added, and the mixture was stirred for reaction at room temperature for 2 h; the reaction mixture was added dropwise into water and stirred for 0.5 h; the mixture was filtered and dried to obtain Intermediate **INT-11** with a yield of 65%. MS (ESI, positive ion) m/z: 459.3 [M + H]$^+$.

Preparation of Intermediate **INT-12**:

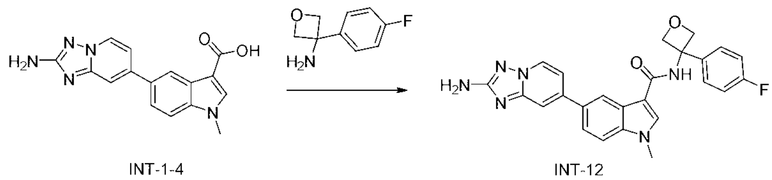

INT-1-4 INT-12

Intermediate **INT-12**: Preparation of 5-(2-amino-[1,2,4]triazolo[1,5-a]pyridin-7-yl)-N-(3-(4-fluorophenyl)oxetan-3-yl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. 3-(4-fluorophenyl)oxetan-3-amine (200 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-12** with a yield of 65%. MS (ESI, positive ion) m/z: 457.3 [M + H]$^+$.

Preparation of Intermediate **INT-13**:

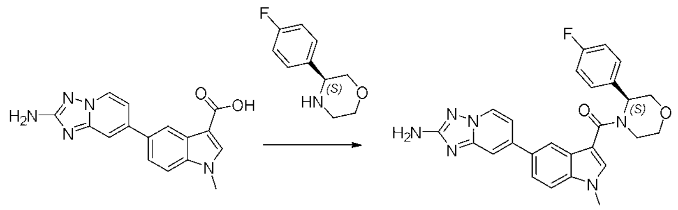

INT-1-4 INT-13

Intermediate **INT-13**: Preparation of (S)-(5-(2-amino-[1,2,4]triazolo[1,5-a]pyridin-7-yl)-1-methyl-1H-indol-3-yl)(3-(4-fluorophenyl)morpholino)methanone:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-3-(4-fluorophenyl)morpholine (217 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-13** with a yield of 71%. MS (ESI, positive ion) m/z: 471.3 $[M + H]^+$.

Preparation of Intermediate **INT-14**:

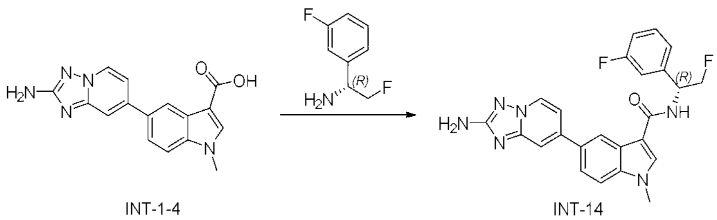

INT-1-4 INT-14

Intermediate **INT-14**: Preparation of (R)-5-(2-amino-[1,2,4]triazolo[1,5-a]pyridin-7-yl)-N-(2-fluoro-1-(3-fluorophenyl)ethyl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (R)-2-fluoro-1-(3-fluorophenyl)ethan-1-amine (188 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-14** with a yield of 72%. MS (ESI, positive ion) m/z: 447.3 $[M + H]^+$.

Preparation of Intermediate **INT-15**:

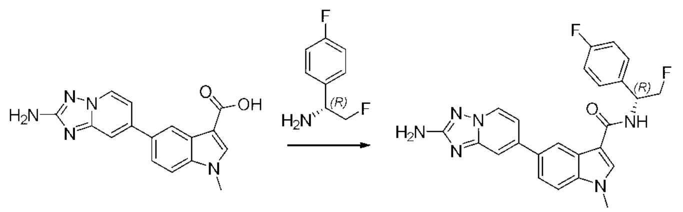

INT-1-4 INT-15

Intermediate **INT-15**: Preparation of (R)-5-(2-amino-[1,2,4]triazolo[1,5-a]pyridin-7-yl)-N-(2-fluoro-1-(4-fluorophenyl)ethyl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (R)-2-fluoro-1-(4-fluorophenyl)ethan-1-amine (188 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-15** with a yield of 72%. MS (ESI, positive ion) m/z: 447.3 [M + H]$^+$.

Preparation of Intermediate **INT-16**:

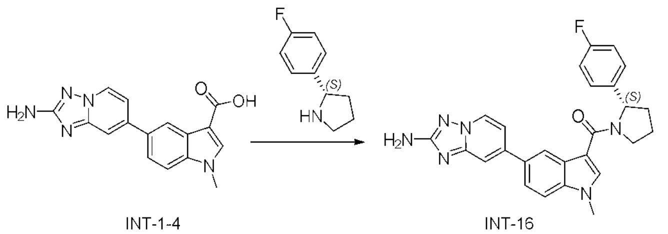

INT-1-4 INT-16

Intermediate **INT-16**: Preparation of (S)-(5-(2-amino-[1,2,4]triazolo[1,5-a]pyridin-7-yl)-1-methyl-1H-indol-3-yl)(2-(4-fluorophenyl)pyrrolidin-1-yl)methanone:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-2-(4-fluorophenyl)pyrrolidine (198 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-16** with a yield of 73%. MS (ESI, positive ion) m/z: 455.3 [M + H]$^+$.

Preparation of Intermediate **INT-17**:

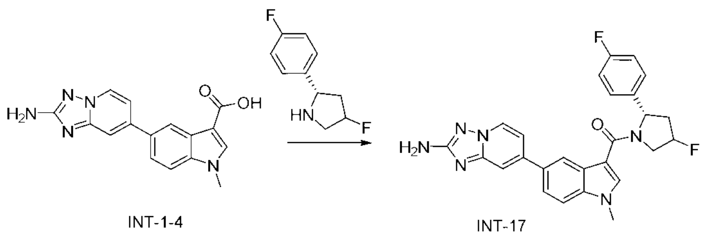

INT-1-4 INT-17

Intermediate **INT-17**: Preparation of (5-(2-amino-[1,2,4]triazolo[1,5-a]pyridin-7-yl)-1-methyl-1H-indol-3-yl)((2S)-4-fluoro-2-(4-fluorophenyl)pyrrolidin-1-yl)methanone:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (2S)-4-fluoro-2-(4-fluorophenyl)pyrrolidine (220 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-17** with a yield of 70%. MS (ESI, positive ion) m/z: 473.3 [M + H]$^+$.

Preparation of Intermediate **INT-18**:

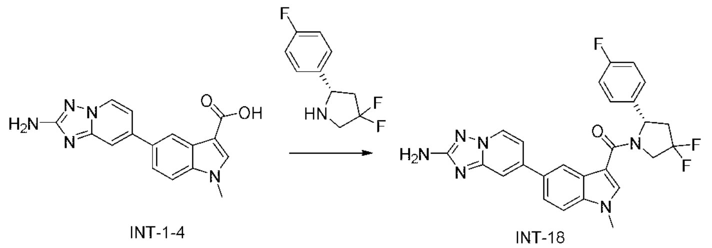

INT-1-4 INT-18

Intermediate **INT-18**: Preparation of (S)-(5-(2-amino-[1,2,4]triazolo[1,5-a]pyridin-7-yl)-1-methyl-1H-indol-3-yl)(4,4-difluoro-2-(4-fluorophenyl)pyrrolidin-1-yl)methanone:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-4,4-difluoro-2-(4-fluorophenyl)pyrrolidine (241 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-18** with a yield of 70%. MS (ESI, positive ion) m/z: 491.3 $[M + H]^+$.

Preparation of Intermediate **INT-19**:

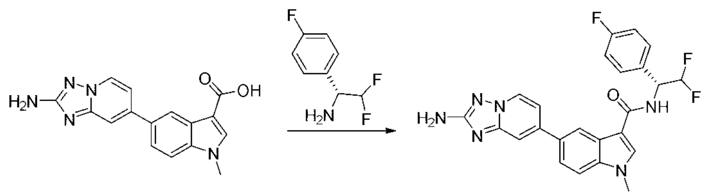

INT-1-4 INT-19

Intermediate **INT-19**: Preparation of (R)-5-(2-amino-[1,2,4]triazolo[1,5-a]pyridin-7-yl)-N-(2,2-difluoro-1-(4-fluorophenyl)ethyl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (R)-2,2-difluoro-1-(4-fluorophenyl)ethan-1-amine (210 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-19** with a yield of 70%. MS (ESI, positive ion) m/z: 464.3 $[M + H]^+$.

Preparation of Intermediate **INT-20**:

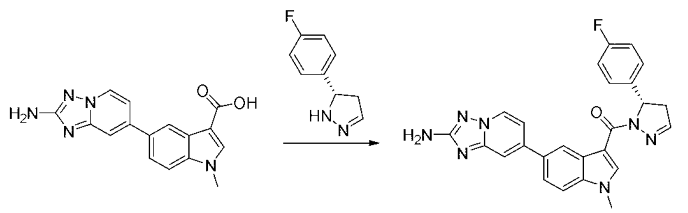

INT-1-4 INT-20

Intermediate **INT-20**: Preparation of (S)-(5-(2-amino-[1,2,4]triazolo[1,5-a]pyridin-7-yl)-1-methyl-1H-indol-3-yl)(5-(4-fluorophenyl)-4,5-dihydro-1H-pyrazol-1-yl)methanone:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-5-(4-fluorophenyl)-4,5-dihydro-1H-pyrazole (197 mg, 1.2 mmol) was added and stirred for reaction at room temperature for 2 h. The reaction mixture was added dropwise to water and stirred for 0.5 h. The mixture was filtered and dried to obtain Intermediate **INT-20** with a yield of 70%. MS (ESI, positive ion) m/z: 464.3 [M + H]$^+$.

Preparation of Intermediate **INT-21**:

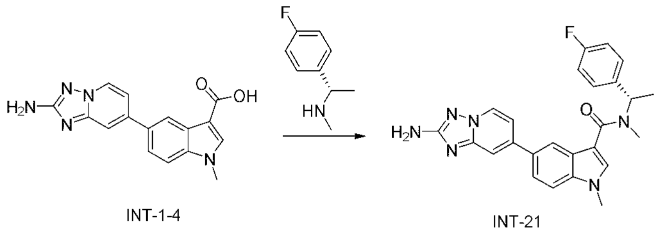

INT-1-4 INT-21

Intermediate **INT-21**: Preparation of (S)-5-(2-amino-[1,2,4]triazolo[1,5-a]pyridin-7-yl)-N-(1-(4-fluorophenyl)ethyl)-N,1-dimethyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-1-(4-fluorophenyl)-N-methylethan-1-amine (184 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-21** with a yield of 70%. MS (ESI, positive ion) m/z: 443.3 [M + H]$^+$.

Preparation of Intermediate **INT-22**:

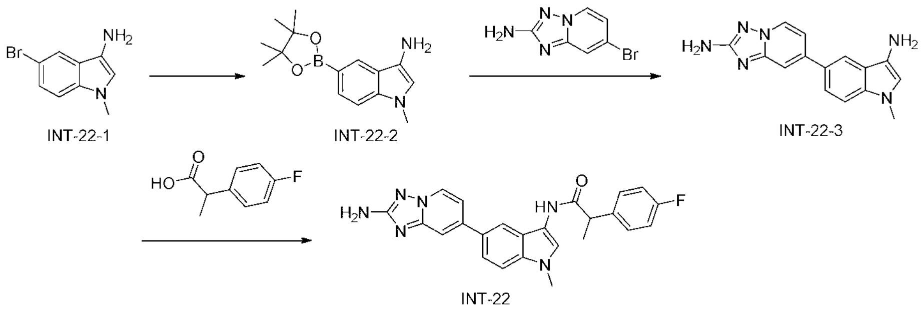

INT-22-1 INT-22-2 INT-22-3

INT-22

Intermediate **INT-22-2**: Preparation of 1-methyl-5-(4,4,5,5-tetramethyl-1,3,2-dioxaborolan-2-yl)-1H-indol-3-amine:

Under an argon atmosphere, Intermediate **INT-22-1** (1.12 g, 5.0 mmol) and bis(pinacolato) diboron (1651 mg, 6.5 mmol) were dissolved in 1,4-dioxane (20 mL), and potassium acetate (4.9 g, 10.0 mmol) and $PdCl_2(dppf)_2$ (160 mg) were added; three cycles of argon purging was performed, and the mixture was heated to 100°C and stirred for 10 h. The reaction mixture was concentrated under reduced pressure and separated and purified by silica gel column (petroleum ether/ethyl acetate = 1/1) to obtain Intermediate **INT-22-2** with a yield of 70%. MS (ESI, positive ion) m/z: 273.3 [M + H]$^+$.

Intermediate **INT-22-3**: Preparation of 7-(3-amino-1-methyl-1H-indol-5-yl)-[1,2,4]triazolo[1,5-a]pyridin-2-amine:

Under an argon atmosphere, Intermediate **INT-22-2** (1.09 g, 4.0 mmol) and 7-bromo-[1,2,4]triazolo[1,5-a]pyridin-2-amine (848 mg, 4.0 mmol) were dissolved in 1,4-dioxane (20 mL), potassium phosphate (1.7 g, 8.0 mmol), $PdCl_2(dppf)_2$ (120 mg), and water (2.0 mL) were added; three cycles of argon purging was performed, the mixture was heated to 95°C and stirred for 8 h. The reaction mixture was concentrated under reduced pressure and separated and purified by silica gel column (dichloromethane/methanol = 20/1) to obtain Intermediate **INT-22-3** with a yield of 61%. MS (ESI, positive ion) m/z: 279.2 [M + H]$^+$.

Intermediate **INT-22**: Preparation of N-(5-(2-amino-[1,2,4]triazolo[1,5-a]pyridin-7-yl)-1-methyl-1H-indol-3-yl)-2-(4-fluorophenyl)propanamide:

Intermediate **INT-22-3** (278 mg, 1.0 mmol) and 2-(4-fluorophenyl)propionic acid (168 mg, 1.0 mmol) were dissolved in dimethyl sulfoxide (10 mL); N,N-diisopropylethylamine (258 mg, 2 mmol) and HATU (570 mg, 1.5 mmol) were added, and the mixture was stirred at room temperature for 2 h. The reaction mixture was added dropwise into water and extracted with ethyl acetate. The organic phase was dried over anhydrous sodium sulfate, filtered, and concentrated under reduced pressure. The residue was separated and purified by silica gel column (dichloromethane/methanol = 20/1) to obtain Intermediate **INT-22** with a yield of 73%. MS (ESI, positive ion) m/z: 429.3. $[M + H]^+$.

Preparation of Intermediate **INT-23**:

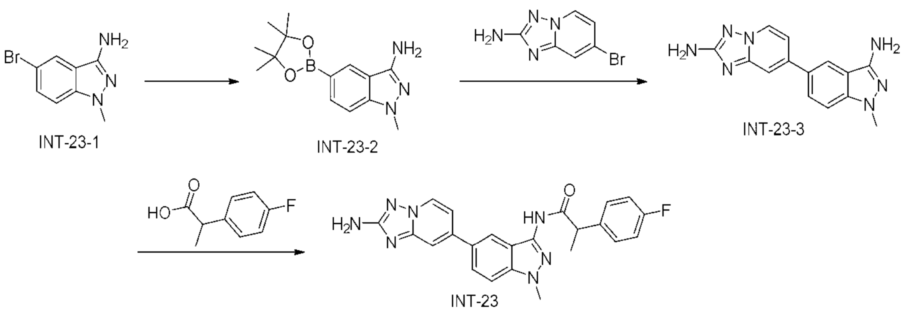


Intermediate **INT-23-2**: Preparation of 1-methyl-5-(4,4,5,5-tetramethyl-1,3,2-dioxaborolan-2-yl)-1H-indazol-3-amine:

Under an argon atmosphere, Intermediate **INT-23-1** (1.12 g, 5.0 mmol) and bis(pinacolato) diboron (1651 mg, 6.5 mmol) were dissolved in 1,4-dioxane (20 mL), and potassium acetate (4.9 g, 10.0 mmol) and PdCl2(dppf)$_2$ (160 mg) were added; three cycles of argon purging was performed, and the mixture was heated to 100°C and stirred for 10 h. The reaction mixture was concentrated under reduced pressure and separated and purified by silica gel column (petroleum ether/ethyl acetate = 1/1) to obtain Intermediate **INT-23-2** with a yield of 67%. MS (ESI, positive ion) m/z: 274.3 $[M + H]^+$.

Intermediate **INT-23-3**: Preparation of 7-(3-amino-1-methyl-1H-indazol-5-yl)-[1,2,4]triazolo[1,5-a]pyridin-2-amine:

Under an argon atmosphere, Intermediate **INT-23-2** (1.09 g, 4.0 mmol) and 7-bromo-[1,2,4]triazolo[1,5-a]pyridin-2-amine (848 mg, 4.0 mmol) were dissolved in 1,4-dioxane (20 mL), potassium phosphate (1.7 g, 8.0 mmol), $PdCl_2(dppf)_2$ (120 mg), and water (2.0 mL) were added; three cycles of argon purging was performed, the mixture was heated to 95°C and stirred for 8 h. The reaction mixture was concentrated under reduced pressure and separated and purified by silica gel column (dichloromethane/methanol = 20/1) to obtain Intermediate **INT-23-3** with a yield of 61%. MS (ESI, positive ion) m/z: 280.2 $[M + H]^+$.

Intermediate **INT-23**: Preparation of N-(5-(2-amino-[1,2,4]triazolo[1,5-a]pyridin-7-yl)-1-methyl-1H-indazol-3-yl)-2-(4-fluorophenyl)propanamide:

Intermediate **INT-23-3** (278 mg, 1.0 mmol) and 2-(4-fluorophenyl)propionic acid (168 mg, 1.0 mmol) were dissolved in dimethyl sulfoxide (10 mL); N,N-diisopropylethylamine (258 mg, 2 mmol) and HATU (570 mg, 1.5 mmol) were added, and the mixture was stirred at room temperature for 2 h. The reaction mixture was added dropwise into water and extracted with ethyl acetate. The organic phase was dried over anhydrous sodium sulfate, filtered, and concentrated under reduced pressure. The residue was separated and purified by silica gel column (dichloromethane/methanol = 20/1) to obtain Intermediate **INT-23** with a yield of 70%. MS (ESI, positive ion) m/z: 430.3. $[M + H]^+$.

Preparation of Intermediate **INT-24**:

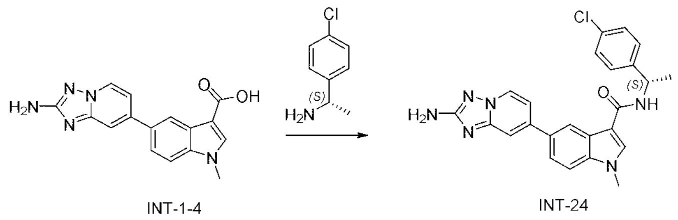

Intermediate **INT-24**: Preparation of (S)-5-(2-amino-[1,2,4]triazolo[1,5-a]pyridin-7-yl)-N-(1-(4-chlorophenyl)ethyl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-1-(4-chlorophenyl)ethan-1-amine (186 mg, 1.2 mmol) was added, and the mixture was stirred for reaction at room temperature for 2 h. The reaction mixture was added dropwise to water and stirred for 0.5 h. The mixture was filtered and dried to obtain Intermediate **INT-24** with a yield of 70%. MS (ESI, positive ion) m/z: 445.1 $[M + H]^+$.

Preparation of Intermediate **INT-25**:

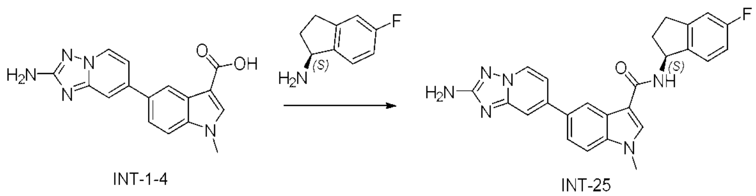

Intermediate **INT-25**: Preparation of (S)-5-(2-amino-[1,2,4]triazolo[1,5-a]pyridin-7-yl)-N-(5-fluoro-2,3-dihydro-1H-inden-1-yl)-1-methyl-1H-indol-3-carboxamide:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-5-fluoro-2,3-dihydro-1H-inden-1-amine (181 mg, 1.2 mmol) was added, and the mixture was stirred for reaction at room temperature for 2 h; the reaction mixture was added dropwise into water and stirred for 0.5 h; the mixture was filtered and dried to obtain Intermediate **INT-25** with a yield of 68%. MS (ESI, positive ion) m/z: 443.3 $[M + H]^+$.

Preparation of Intermediate **INT-26**:

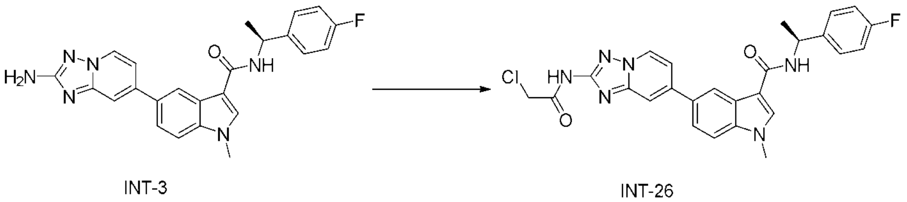

Preparation of Intermediate **INT-26**:

Intermediate **INT-3** (42.8 g, 100 mmol) was dissolved in dichloromethane (1 L), chloroacetyl chloride (13.5 g, 120 mmol) and N,N-diisopropylethylamine (25.8 g, 200 mmol) were added, and the mixture was stirred at room temperature for 2 h. Petroleum ether (3 L) was added to the reaction mixture for crystallization; the solid was filtered with suction, washed with water, and dried to obtain Intermediate **INT-26** with a yield of 82%. MS (ESI, positive ion) m/z: 505.2 $[M + H]^+$.

Preparation of Intermediate **INT-27**:

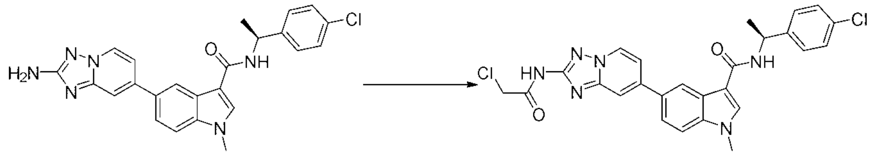

INT-24 INT-27

Preparation of Intermediate **INT-27**:

Intermediate **INT-24** (44.4 g, 100 mmol) was dissolved in dichloromethane (1 L), chloroacetyl chloride (13.5 g, 120 mmol) and N,N-diisopropylethylamine (25.8 g, 200 mmol) were added, and the mixture was stirred at room temperature for 2 h. Petroleum ether (3 L) was added to the reaction mixture for crystallization; the solid was filtered with suction, washed with water, and dried to obtain Intermediate **INT-27** with a yield of 78%. MS (ESI, positive ion) m/z: 521.2 $[M + H]^+$.

Preparation of Intermediate **INT-28**:

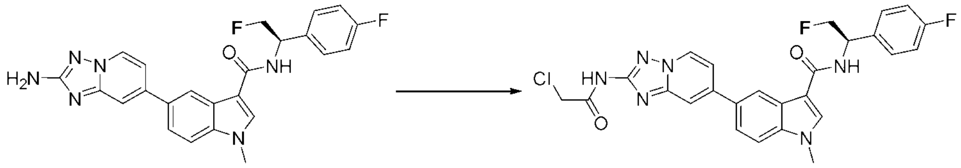

INT-15 INT-28

Preparation of Intermediate **INT-28**:

Intermediate **INT-15** (44.6 g, 100 mmol) was dissolved in dichloromethane (1 L), chloroacetyl chloride (13.5 g, 120 mmol) and N,N-diisopropylethylamine (25.8 g, 200 mmol) were added, and the mixture was stirred at room temperature for 2 h. Petroleum ether (3 L) was added to the reaction mixture for crystallization; the solid was filtered with suction, washed with water, and dried to obtain Intermediate **INT-28** with a yield of 75%. MS (ESI, positive ion) m/z: 523.2 $[M + H]^+$.

Preparation of Intermediate **INT-29**:

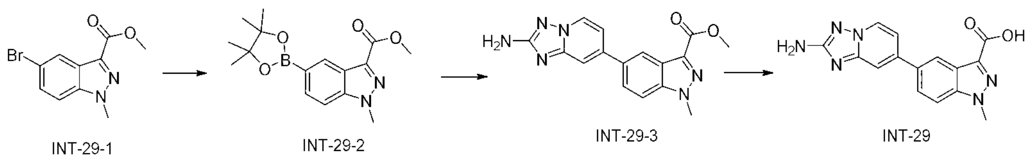

INT-29-1 INT-29-2 INT-29-3 INT-29

Intermediate **INT-29-2**: Preparation of methyl 1-methyl-5-(4,4,5,5-tetramethyl-1,3,2-dioxaborolan-2-yl)-1H-indazole-3-carboxylate:

Under an argon atmosphere, Intermediate **INT-29-1** (1335 mg, 5.0 mmol) and bis(pinacolato) diboron (1651 mg, 6.5 mmol) were dissolved in 1,4-dioxane (20 mL), and potassium acetate (4.9 g, 10.0 mmol) and $PdCl_2(dppf)_2$ (160 mg) were added; three cycles of argon purging was performed, and the mixture was heated to 100°C and stirred for 10 h. The reaction mixture was concentrated under reduced pressure and separated and purified by silica gel column (petroleum ether/ethyl acetate = 5/1) to obtain Intermediate **INT-29-2** with a yield of 68%. MS (ESI, positive ion) m/z: 317.3 $[M + H]^+$.

Intermediate **INT-29-3**: Preparation of 5-(2-amino-[1,2,4]triazolo[1,5-a]pyridin-7-yl)-1-methyl-1H-indazole-3-carboxylic acid methyl ester:

Under an argon atmosphere, Intermediate **INT-29-2** (1260 mg, 4.0 mmol) and 7-bromo-[1,2,4]triazolo[1,5-a]pyridin-2-amine (848 mg, 4.0 mmol) were dissolved in 1,4-dioxane (20 mL), potassium phosphate (1.7 g, 8.0 mmol), $PdCl_2(dppf)_2$ (120 mg), and water (2.0 mL) were added; three cycles of argon purging was performed, the mixture was heated to 95°C and stirred for 8 h. The reaction mixture was concentrated under reduced pressure and separated and purified by silica gel column (dichloromethane/methanol = 20/1) to obtain Intermediate **INT-29-3** with a yield of 70%. MS (ESI, positive ion) m/z: 323.2 $[M + H]^+$.

Intermediate **INT-29:** Preparation of 5-(2-amino-[1,2,4]triazolo[1,5-a]pyridin-7-yl)-1-methyl-1H-indazole-3-carboxylic acid:

Intermediate **INT-29-3** (963 mg, 3.0 mmol) was dissolved in ethanol (3 mL), tetrahydrofuran (3 mL), and water (15 mL); sodium hydroxide (0.96 g, 24 mmol) was added; the mixture was heated to 75°C and stirred for 5 h. The pH was adjusted to slightly acidic with dilute hydrochloric acid (1 M). The mixture was filtered and dried to obtain Intermediate **INT-29** with a yield of 62%. MS (ESI, positive ion) m/z: 309.3 $[M + H]^+$.

**Example 1: Preparation of Compound 1**

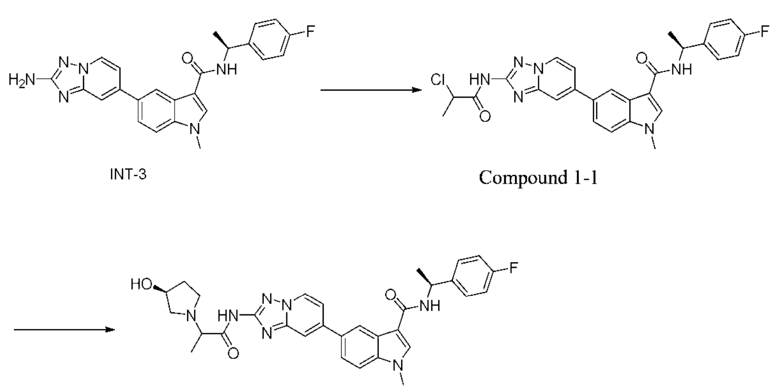

INT-3

Compound 1-1

Compound 1

Preparation of Compound 1-1:

Intermediate **INT-3** (214 mg, 0.5 mmol) was dissolved in pyridine (2 mL); 2-chloropropionic acid (65 mg, 0.6 mmol) and 1-propylphosphonic anhydride (316 mg, 1.0 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 1-1 with a yield of 61%. MS (ESI, positive ion) m/z: 519.2 $[M + H]^+$.

Preparation of Compound 1

Compound 1-1 (100 mg, 0.2 mmol) was dissolved in acetonitrile (1 mL); (S)-3-pyrrolidinol (43 mg, 0.5 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 1 with a yield of 45%. MS (ESI, positive ion) m/z: 570.2 $[M + H]^+$.

$^1H$ NMR (400 MHz, DMSO-$d_6$) δ 10.62 (s, 1H), 8.89 (dd, *J* = 7.1, 0.8 Hz, 1H), 8.56 (d, *J* = 1.9 Hz, 1H), 8.36 (d, *J* = 8.0 Hz, 1H), 8.23 (s, 1H), 7.91 (dd, *J* = 1.9, 0.9 Hz, 1H), 7.74 (dd, *J* = 8.7, 1.9 Hz, 1H), 7.68 (d, *J* = 8.6 Hz, 1H), 7.48 (dd, *J* = 7.4, 2.0 Hz, 3H), 7.21 - 7.14 (m, 2H), 5.25 - 5.20 (m, 1H), 3.92 (s, 3H), 3.67 (m, 1H), 2.48 - 2.34 (m, 4H), 1.82-1.63 (m, 4H), 1.51 (d, *J*= 7.1 Hz, 3H), 1.29 (d, *J* = 7.4 Hz, 3H).

**Example 2: Preparation of Compound 2**

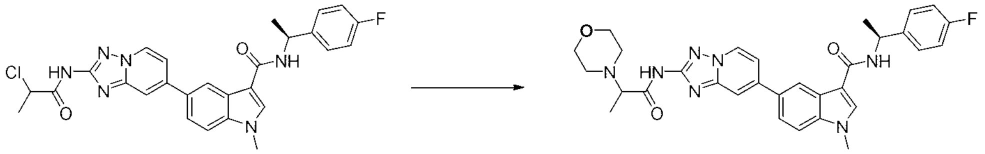

Compound 1-1 Compound 2

Preparation of Compound 2:

Compound 1-1 (100 mg, 0.2 mmol) was dissolved in acetonitrile (1 mL); morpholine (43 mg, 0.5 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 1 with a yield of 62%. MS (ESI, positive ion) m/z: 570.2 $[M + H]^+$.

$^1H$ NMR (400 MHz, DMSO-$d_6$) δ 10.62 (s, 1H), 8.89 (dd, *J*= 7.1, 0.8 Hz, 1H), 8.56 (d, *J*= 1.9 Hz, 1H), 8.36 (d, *J* = 8.0 Hz, 1H), 8.23 (s, 1H), 7.91 (dd, *J* = 1.9, 0.9 Hz, 1H), 7.74 (dd, *J* = 8.7, 1.9 Hz, 1H), 7.68 (d, *J* = 8.6 Hz, 1H), 7.48 (dd, *J* = 7.4, 2.0 Hz, 3H), 7.21 - 7.14 (m, 2H), 5.25 - 5.20 (m, 1H), 3.92 (s, 3H), 3.67 (m, 1H), 3.48 -3.34 (m, 4H), 2.82-2.63 (m, 4H), 1.51 (d, *J*= 7.1 Hz, 3H), 1.29 (d, *J* =7.4 Hz, 3H).

**Example 3: Preparation of Compound 3**

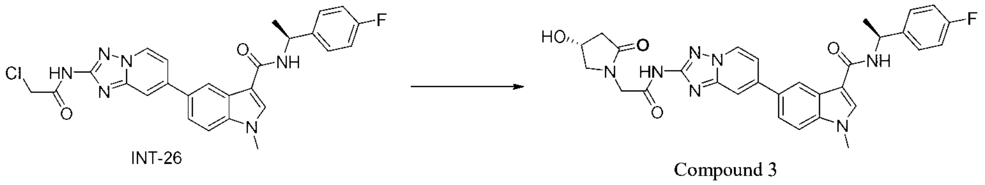

INT-26 Compound 3

Preparation of Compound 3:

(R)-4-hydroxy-2-pyrrolidone (101 mg, 1 mmol) was dissolved in dry tetrahydrofuran (2 mL), and sodium hydride (80 mg, 2 mmol) was added. After 5 min, Intermediate **INT-26** (252 mg, 0.5 mmol) was added, and the mixture was stirred at room temperature for 3 h. The reaction mixture was quenched with water and extracted with ethyl acetate; the organic phase was concentrated under reduced pressure and purified by C-18 reverse phase silica gel column (acetonitrile/0.1% aqueous ammonium bicarbonate solution = 60%) to obtain Compound 3 with a yield of 41%. MS (ESI, positive ion) m/z: 570.2 $[M + H]^+$.

$^1H$ NMR (400 MHz, DMSO-$d_6$) δ 10.62 (s, 1H), 8.89 (dd, *J*= 7.1, 0.8 Hz, 1H), 8.56 (d, *J*= 1.9 Hz, 1H), 8.36 (d, *J*= 8.0 Hz, 1H), 8.23 (s, 1H), 7.91 (dd, *J*= 1.9, 0.9 Hz, 1H), 7.74 (dd, *J*= 8.7, 1.9 Hz, 1H), 7.68 (d, *J*= 8.6 Hz, 1H), 7.48 (dd, *J*= 7.4, 2.0 Hz, 3H), 7.21 - 7.14 (m, 2H), 5.40 (s, 1H), 5.25 - 5.20 (m, 1H), 3.92 (s, 3H), 3.56-3.43 (m, 3H), 3.67-3.42 (m, 2H), 2.50 - 2.25 (m, 2H), 1.51 (d, *J* = 7.1 Hz, 3H).

**Example 4: Preparation of Compound 4**

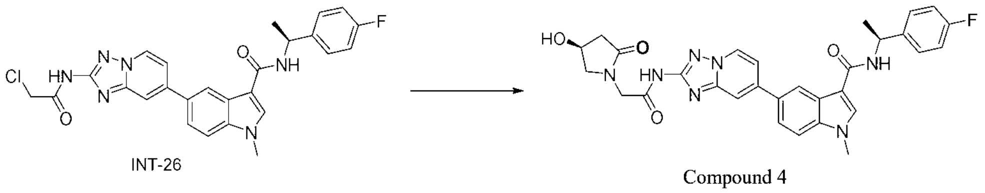

INT-26 Compound 4

Preparation of Compound 4:

(S)-4-hydroxy-2-pyrrolidone (101 mg, 1 mmol) was dissolved in dry tetrahydrofuran (2 mL), and sodium hydride (80 mg, 2 mmol) was added. After 5 min, Intermediate **INT-26** (252 mg, 0.5 mmol) was added, and the mixture was stirred at

room temperature for 3 h. The reaction mixture was quenched with water and extracted with ethyl acetate; the organic phase was concentrated under reduced pressure and purified by C-18 reverse phase silica gel column (acetonitrile/0.1% aqueous ammonium bicarbonate solution = 60%) to obtain Compound 4 with a yield of 43%. MS (ESI, positive ion) m/z: 570.2 [M + H]+.

$^1$H NMR (400 MHz, DMSO-$d_6$) δ 10.62 (s, 1H), 8.89 (dd, *J*= 7.1, 0.8 Hz, 1H), 8.56 (d, *J*= 1.9 Hz, 1H), 8.36 (d, *J* = 8.0 Hz, 1H), 8.23 (s, 1H), 7.91 (dd, *J* = 1.9, 0.9 Hz, 1H), 7.74 (dd, *J* = 8.7, 1.9 Hz, 1H), 7.68 (d, *J*= 8.6 Hz, 1H), 7.48 (dd, *J*= 7.4, 2.0 Hz, 3H), 7.21 - 7.14 (m, 2H), 5.40 (s, 1H), 5.25 - 5.20 (m, 1H), 3.92 (s, 3H), 3.56-3.43 (m, 3H), 3.67-3.42 (m, 2H), 2.50 - 2.25 (m, 2H), 1.51 (d, *J* = 7.1 Hz, 3H).

**Example 5: Preparation of Compound 5**

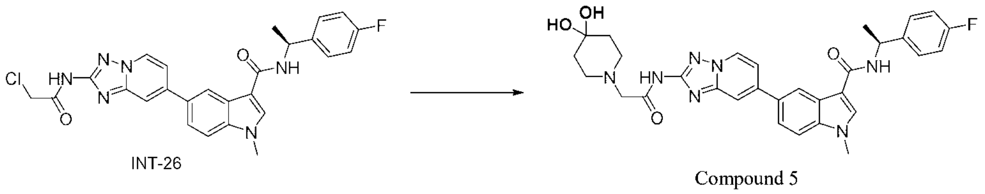

INT-26

Compound 5

Preparation of Compound 5:

Intermediate **INT-26** (101 mg, 0.2 mmol) was dissolved in acetonitrile (1 mL); 4,4-piperidinediol hydrochloride (60 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 5 with a yield of 32%. MS (ESI, positive ion) m/z: 586.2 [M + H]+.

$^1$H NMR (400 MHz, DMSO-$d_6$) δ 10.62 (s, 1H), 8.89 (dd, *J* = 7.1, 0.8 Hz, 1H), 8.56 (d, *J* = 1.9 Hz, 1H), 8.36 (d, *J* = 8.0 Hz, 1H), 8.23 (s, 1H), 7.91 (dd, *J* = 1.9, 0.9 Hz, 1H), 7.74 (dd, *J* = 8.7, 1.9 Hz, 1H), 7.68 (d, *J* = 8.6 Hz, 1H), 7.48 (dd, *J* = 7.4, 2.0 Hz, 3H), 7.21 - 7.14 (m, 2H), 5.25 - 5.20 (m, 1H), 3.92 (s, 3H), 3.46 (s, 2H), 2.92 (t, *J* = 6.0 Hz, 4H), 2.55 (d, *J* = 6.8 Hz, 2H), 2.48 - 2.34 (m, 4H), 1.51 (d, *J* = 7.1 Hz, 3H).

**Example 6: Preparation of Compound 6**

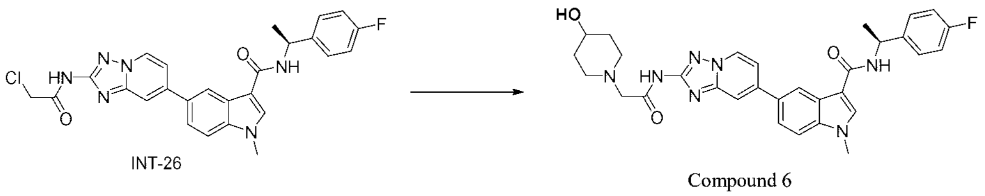

INT-26

Compound 6

Preparation of Compound 6:

Intermediate **INT-26** (101 mg, 0.2 mmol) was dissolved in acetonitrile (1 mL); 4-piperidinol (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 6 with a yield of 42%. MS (ESI, positive ion) m/z: 570.2 [M + H]+.

$^1$H NMR (400 MHz, DMSO-d6) δ 10.38 (s, 1H), 8.89 (d, J = 7.1 Hz, 1H), 8.55 (d, J = 1.8 Hz, 1H), 8.36 (d, J = 8.0 Hz, 1H), 8.22 (s, 1H), 7.90 (dd, J = 1.9, 0.9 Hz, 1H), 7.73 (d, J = 1.9 Hz, 1H), 7.68 (d, J = 8.7 Hz, 1H), 7.50 - 7.46 (m, 3H), 7.17 (t, J = 8.9 Hz, 2H), 5.28 - 5.18 (m, 1H), 4.58 (d, J = 4.2 Hz, 1H), 3.92 (s, 3H), 3.55 - 3.45 (m, 1H), 3.21 (s, 2H), 2.85 - 2.76 (m, 2H), 2.30 (t, J = 10.2 Hz, 2H), 1.76 (d, J = 10.9 Hz, 2H), 1.51 (d, J = 7.0 Hz, 5H).

**Example 7: Preparation of Compound 7**

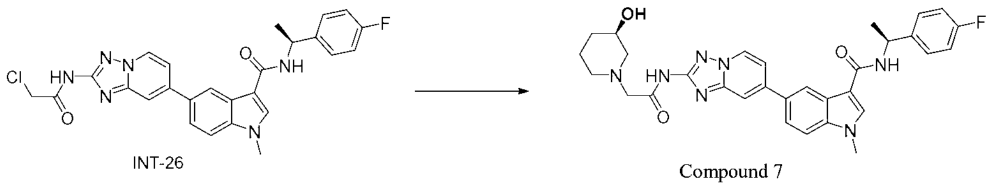

INT-26 Compound 7

Preparation of Compound 7:

Intermediate **INT-26** (101 mg, 0.2 mmol) was dissolved in acetonitrile (1 mL); S-3-hydroxypiperidine (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 7 with a yield of 46%. MS (ESI, positive ion) m/z: 570.2 $[M + H]^+$.

$^1$H NMR (400 MHz, DMSO-$d_6$) δ 10.42 (s, 1H), 8.89 (dd, *J* = 7.1, 0.8 Hz, 1H), 8.55 (d, *J* = 1.8 Hz, 1H), 8.36 (d, *J* = 8.0 Hz, 1H), 8.22 (s, 1H), 7.90 (dd, *J* = 1.9, 0.9 Hz, 1H), 7.74 (dd, *J* = 8.7, 1.8 Hz, 1H), 7.68 (d, *J* = 8.6 Hz, 1H), 7.50 - 7.46 (m, 3H), 7.17 (t, *J* = 8.9 Hz, 2H), 5.28 - 5.18 (m, 1H), 4.74 (d, *J* = 5.5 Hz, 1H), 3.92 (s, 3H), 3.58 (dd, *J* = 9.0, 4.6 Hz, 1H), 3.22 (d, *J* = 8.7 Hz, 2H), 2.83 (d, *J* = 9.6 Hz, 1H), 2.66 (dd, *J* = 15.1, 9.2 Hz, 1H), 2.23 (t, *J* = 10.2 Hz, 1H), 2.12 (t, *J* = 9.4 Hz, 1H), 1.80 - 1.66 (m, 2H), 1.50 (d, *J* = 7.0 Hz, 4H), 1.18 (q, *J* = 9.0, 8.3 Hz, 1H).

**Example 8: Preparation of Compound 8**

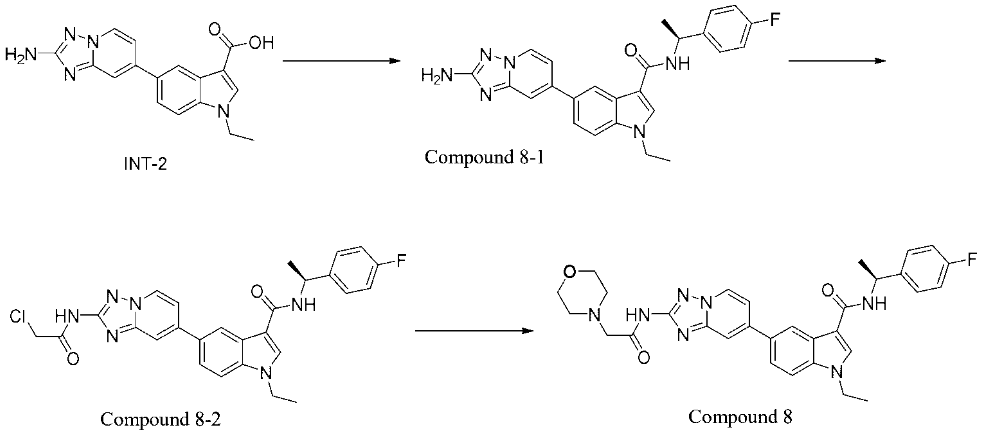

INT-2 Compound 8-1

Compound 8-2 Compound 8

Preparation of Compound 8-1:

Intermediate **INT-2** (321 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-1-(4-fluorophenyl)ethan-1-amine (167 mg, 1.2 mmol) was added, and the mixture was stirred for reaction at room temperature for 2 h. The reaction mixture was added dropwise to water and stirred for 0.5 h. The mixture was filtered and dried to obtain Compound 8-1 with a yield of 73%. MS (ESI, positive ion) m/z: 443.3 $[M + H]^+$.

Preparation of Compound 8-2:

Compound 8-1 (442 mg, 1.0 mmol) was dissolved in dichloromethane (10 mL); chloroacetyl chloride (135 mg, 1.2 mmol) and N,N-diisopropylethylamine (258 mg, 2.0 mmol) were added, and the mixture was stirred at room temperature for 2 h. Petroleum ether (30 mL) was added to the reaction mixture for crystallization; the solid was filtered with suction, washed with water, and dried to obtain Compound 8-2 with a yield of 79%. MS (ESI, positive ion) m/z: 519.2 $[M + H]^+$.

Preparation of Compound 8:

Compound 8-2 (104 mg, 0.2 mmol) was dissolved in acetonitrile (1 mL); morpholine (43 mg, 0.5 mmol) was added, and the mixture was heated to 80°C and stirred for 6 h. The reaction mixture was concentrated under reduced

pressure and purified by C-18 reverse phase silica gel column (acetonitrile/0.1% aqueous ammonium bicarbonate solution = 60%) to obtain Compound 8 with a yield of 52%. MS (ESI, positive ion) m/z: 570.2 $[M + H]^+$.

$^1$H NMR (400 MHz, DMSO-$d_6$) δ 10.51 (s, 1H), 8.89 (d, *J* = 7.1 Hz, 1H), 8.56 (s, 1H), 8.35 (d, *J* = 8.0 Hz, 1H), 8.30 (s, 1H), 7.89 (d, *J* = 1.9 Hz, 1H), 7.72 (d, *J* = 1.3 Hz, 2H), 7.48 (dd, *J* = 8.4, 5.4 Hz, 3H), 7.20 - 7.15 (m, 2H), 5.23 (t, *J* = 7.3 Hz, 1H), 4.32 (q, *J* = 7.3 Hz, 2H), 3.65 (t, *J* = 4.6 Hz, 4H), 2.57 (t, *J* = 4.4 Hz, 4H), 1.52 - 1.46 (m, 6H).

**Example 9: Preparation of Compound 9**

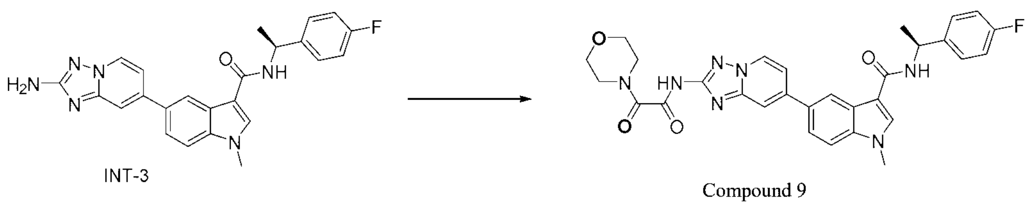

INT-3

Compound 9

Preparation of Compound 9:

Intermediate **INT-3** (85 mg, 0.2 mmol) was dissolved in pyridine (1 mL), 2-morpholin-4-yl-2-oxo-acetic acid (48 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 9 with a yield of 56%. MS (ESI, positive ion) m/z: 570.2 $[M + H]^+$.

$^1$H NMR (400 MHz, DMSO-$d_6$) δ 11.60 (s, 1H), 8.89 (d, *J* = 7.3 Hz, 1H), 8.55 (d, *J* = 1.8 Hz, 1H), 8.36 (d, *J* = 8.0 Hz, 1H), 8.22 (s, 1H), 7.93 (s, 1H), 7.74 (dd, *J* = 8.5, 1.9 Hz, 1H), 7.66 (d, *J* = 8.6 Hz, 1H), 7.48 (dtd, *J* = 12.1, 6.4, 5.7, 2.0 Hz, 3H), 7.19 - 7.13 (m, 2H), 5.22 (t, *J* = 7.3 Hz, 1H), 3.91 (s, 3H), 3.70 (d, *J* = 22.8 Hz, 4H), 3.56 (t, *J* = 4.9 Hz, 2H), 3.46 (s, 2H), 1.50 (d, *J* = 7.0 Hz, 3H).

**Example 10: Preparation of Compound 10**

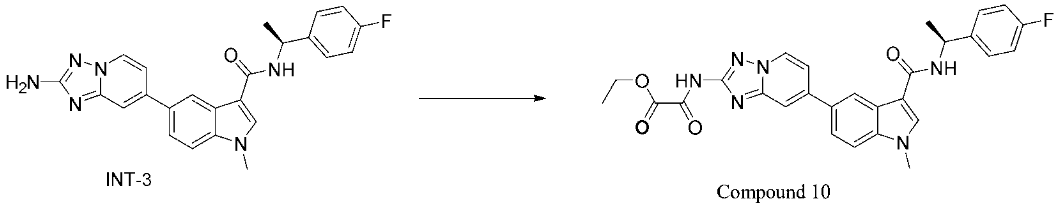

INT-3

Compound 10

Preparation of Compound 10:

Intermediate **INT-3** (85 mg, 0.2 mmol) was dissolved in pyridine (1 mL), monoethyl oxalate (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 10 with a yield of 46%. MS (ESI, positive ion) m/z: 529.2 $[M + H]^+$.

$^1$H NMR (400 MHz, DMSO-$d_6$) δ 11.76 - 11.52 (m, 1H), 8.87 (d, *J*= 6.9 Hz, 1H), 8.53 (d, *J*= 1.9 Hz, 1H), 8.34 (d, *J*= 8.0 Hz, 1H), 8.19 (s, 1H), 7.91 (s, 1H), 7.69 (dd, *J*= 8.7, 1.8 Hz, 1H), 7.61 (d, *J* = 8.6 Hz, 1H), 7.49 - 7.41 (m, 3H), 7.15 - 7.09 (m, 2H), 5.19 (t, *J*= 7.3 Hz, 1H), 4.30 (q, *J* = 7.1 Hz, 2H), 3.86 (s, 3H), 1.46 (d, *J*= 7.0 Hz, 3H), 1.28 (t, *J*= 7.0 Hz, 3H).

**Example 11: Preparation of Compound 11**

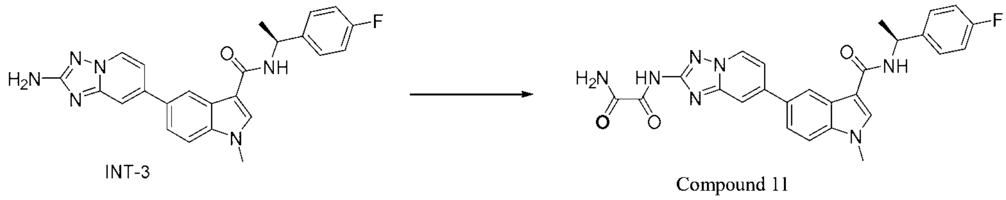

INT-3

Compound 11

Preparation of Compound 11:

Intermediate **INT-3** (85 mg, 0.2 mmol) was dissolved in pyridine (1 mL); oxamic acid (27 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 11 with a yield of 51%. MS (ESI, positive ion) m/z: 500.2 $[M + H]^+$.

$^1H$ NMR (400 MHz, DMSO-$d_6$) δ 10.87 (s, 1H), 8.93 (d, *J* = 7.2 Hz, 1H), 8.56 (d, *J* = 1.8 Hz, 1H), 8.36 (d, *J* = 7.8 Hz, 2H), 8.22 (s, 1H), 8.08 - 7.95 (m, 2H), 7.76 (dd, *J* = 8.7, 1.9 Hz, 1H), 7.68 (d, *J* = 8.6 Hz, 1H), 7.54 - 7.45 (m, 3H), 7.19 - 7.14 (m, 2H), 5.27 - 5.18 (m, 1H), 3.91 (s, 3H), 1.50 (d, *J* = 7.0 Hz, 3H).

**Example 12: Preparation of Compound 12**

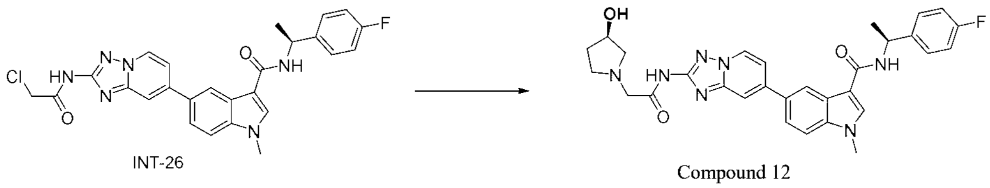

INT-26

Compound 12

Preparation of Compound 12:

Intermediate **INT-26** (101 mg, 0.2 mmol) was dissolved in acetonitrile (1 mL); (R)-3-pyrrolidine (35 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 12 with a yield of 46%. MS (ESI, positive ion) m/z: 556.2 $[M + H]^+$.

$^1H$ NMR (400 MHz, DMSO-$d_6$) δ 10.44 (s, 1H), 8.89 (d, *J* = 7.1 Hz, 1H), 8.55 (d, *J* = 1.8 Hz, 1H), 8.36 (d, *J* = 8.0 Hz, 1H), 8.23 (s, 1H), 7.90 (d, *J* = 1.9 Hz, 1H), 7.74 (dd, *J* = 8.7, 1.9 Hz, 1H), 7.67 (d, *J* = 8.6 Hz, 1H), 7.50 - 7.45 (m, 3H), 7.21 - 7.14 (m, 2H), 5.22 (p, *J* = 7.1 Hz, 1H), 4.83 (d, *J* = 5.5 Hz, 1H), 4.23 (s, 1H), 3.91 (s, 3H), 3.36 (s, 2H), 2.87 - 2.79 (m, 2H), 2.60 - 2.55 (m, 2H), 2.06 (dq, *J* = 14.0, 7.2 Hz, 1H), 1.69 - 1.59 (m, 1H), 1.50 (d, *J* = 7.0 Hz, 3H).

**Example 13: Preparation of Compound 13**

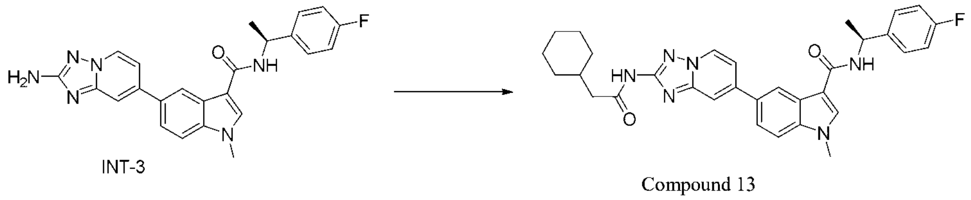

INT-3

Compound 13

Preparation of Compound 13:

Intermediate **INT-3** (85 mg, 0.2 mmol) was dissolved in pyridine (1 mL); cyclohexylacetic acid (42 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column

(acetonitrile/0.1% aqueous ammonium bicarbonate solution = 60%) to obtain Compound 13 with a yield of 61%. MS (ESI, positive ion) m/z: 553.2 [M + H]+.

$^1$H NMR (400 MHz, DMSO-$d_6$) δ 10.78 (s, 1H), 8.86 (d, *J* = 7.1 Hz, 1H), 8.55 (d, *J* = 1.8 Hz, 1H), 8.37 (d, *J* = 8.0 Hz, 1H), 8.23 (s, 1H), 7.88 (d, *J* = 1.9 Hz, 1H), 7.73 (dd, *J* = 8.6, 1.9 Hz, 1H), 7.66 (d, *J* = 8.6 Hz, 1H), 7.47 (ddd, *J* = 8.6, 5.5, 2.4 Hz, 3H), 7.17 (t, *J* = 8.9 Hz, 2H), 5.27 - 5.17 (m, 1H), 3.91 (s, 3H), 3.85 (ddd, *J* = 11.3, 4.4, 1.9 Hz, 2H), 3.31 (dd, *J* = 11.6, 2.1 Hz, 2H), 2.40 (s, 1H), 2.04 (dt, *J* = 7.4, 3.5 Hz, 1H), 1.67 - 1.59 (m, 2H), 1.50 (d, *J* = 7.1 Hz, 3H), 1.28 (tt, *J* = 12.1, 6.0 Hz, 2H).

**Example 14: Preparation of Compound 14**

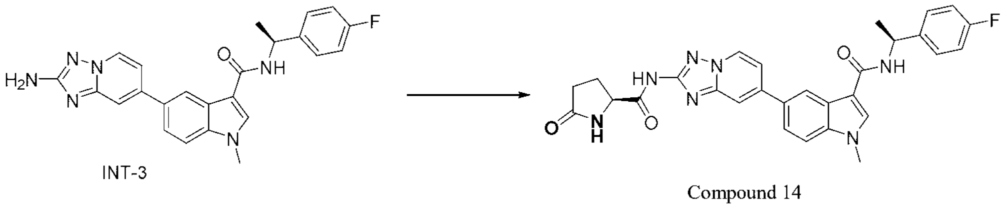
INT-3
Compound 14

Preparation of Compound 14:

Intermediate **INT-3** (85 mg, 0.2 mmol) was dissolved in pyridine (1 mL); L-pyroglutamic acid (39 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 14 with a yield of 53%. MS (ESI, positive ion) m/z: 540.2 [M + H]+.

$^1$H NMR (400 MHz, DMSO-$d_6$) δ 8.42 (t, *J* = 1.2 Hz, 1H), 8.31 (d, *J* = 8.0 Hz, 1H), 8.19 (s, 1H), 7.88 (d, *J* = 1.6 Hz, 1H), 7.66 - 7.59 (m, 4H), 7.51 - 7.42 (m, 2H), 7.22 - 7.11 (m, 2H), 5.22 (p, *J* = 7.1 Hz, 1H), 4.11 (m, 1H), 3.90 (s, 3H), 2.46 - 2.08(m, 4H), 1.50 (d, *J* = 7.0 Hz, 3H).

**Example 15: Preparation of Compound 15**

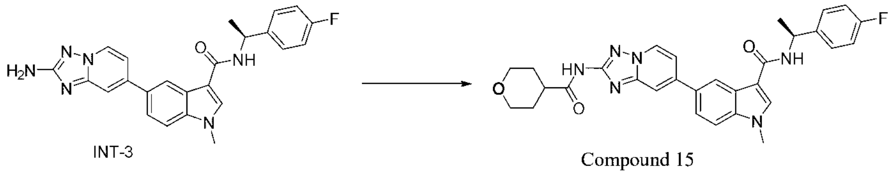
INT-3
Compound 15

Preparation of Compound 15:

Intermediate **INT-3** (85 mg, 0.2 mmol) was dissolved in pyridine (1 mL); cyclohexanecarboxylic acid (38 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 15 with a yield of 51%. MS (ESI, positive ion) m/z: 540.2 [M + H]+.

$^1$H NMR (400 MHz, DMSO-$d_6$) δ 8.42 (t, *J* = 1.2 Hz, 1H), 8.31 (d, *J* = 8.0 Hz, 1H), 8.19 (s, 1H), 7.88 (d, *J*= 1.6 Hz, 1H), 7.66 - 7.59 (m, 3H), 7.51 - 7.42 (m, 2H), 7.22 - 7.11 (m, 2H), 5.22 (p, *J* = 7.1 Hz, 1H), 3.90 (s, 3H), 3.63 (t, *J* = 4.6 Hz, 4H), 2.60 - 2.55 (m, 4H), 1.50 (d, *J* = 7.0 Hz, 3H).

**Example 16: Preparation of Compound 16**

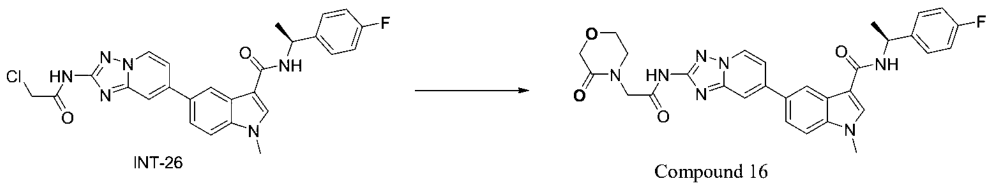

INT-26

Compound 16

Preparation of Compound 16:

3-morpholinone (101 mg, 1 mmol) was dissolved in dry tetrahydrofuran (2 mL), and sodium hydride (80 mg, 2 mmol) was added. After 5 min, Intermediate **INT-26** (252 mg, 0.5 mmol) was added, and the mixture was stirred at room temperature for 3 h. The reaction mixture was quenched with water and extracted with ethyl acetate; the organic phase was concentrated under reduced pressure and purified by C-18 reverse phase silica gel column (acetonitrile/0.1% aqueous ammonium bicarbonate solution = 60%) to obtain Compound 16 with a yield of 33%. MS (ESI, positive ion) m/z: 570.2 [M + H]$^+$.

$^1$H NMR (400 MHz, DMSO-$d_6$) δ 8.56 (d, *J*= 1.9 Hz, 1H), 8.37 (d, *J*= 8.0 Hz, 1H), 8.23 (s, 1H), 7.98 (s, 1H), 7.78 - 7.66 (m, 3H), 7.53 (dd, *J* = 7.2, 2.0 Hz, 1H), 7.47 (dd, *J*= 8.5, 5.6 Hz, 3H), 7.18 (d, *J*= 8.9 Hz, 2H), 4.97 - 4.82 (m, 1H), 4.30 (s, 2H), 3.91 (s, 3H), 3.65 - 3.52 (m, 2H), 2.57 (m, 2H), 2.35 (s, 2H), 1.50 (d, *J* = 7.1 Hz, 3H), 1.25 (s, 6H),.

**Example 17: Preparation of Compound 17**

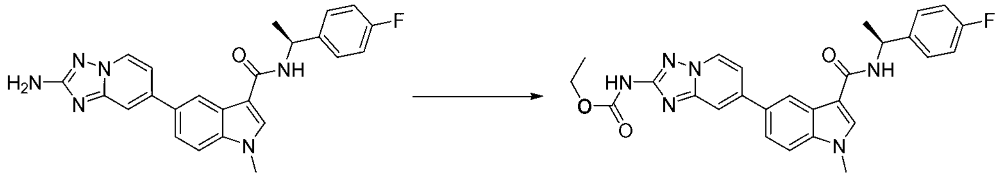

INT-3

Compound 17

Preparation of Compound 17:

Intermediate **INT-3** (43 mg, 0.10 mmol) was dissolved in dichloromethane (1 mL); ethyl chloroformate (13 mg, 0.12 mmol) and N,N-diisopropylethylamine (26 mg, 0.20 mmol) were added, and the mixture was stirred at room temperature for 2 h. Petroleum ether (3 mL) was added to the reaction mixture for crystallization; the solid was filtered with suction, washed with water, and dried to obtain Compound 17 with a yield of 82%. MS (ESI, positive ion) m/z: 501.2 [M + H]$^+$.

$^1$H NMR (400 MHz, DMSO-d6) δ 10.46 (s, 1H), 8.83 (d, J = 7.1 Hz, 1H), 8.52 (d, J = 1.8 Hz, 1H), 8.34 (d, J = 8.0 Hz, 1H), 8.20 (s, 1H), 7.85 (d, J = 1.9 Hz, 1H), 7.71 (dd, J = 8.6, 1.9 Hz, 1H), 7.65 (d, J = 8.7 Hz, 1H), 7.49 - 7.37 (m, 3H), 7.19 - 7.10 (m, 2H), 5.25 - 5.15 (m, 1H), 4.16 (q, J = 7.1 Hz, 2H), 1.48 (d, J = 7.1 Hz, 3H), 1.25 (t, J = 7.1 Hz, 3H).

**Example 18: Preparation of Compound 18**

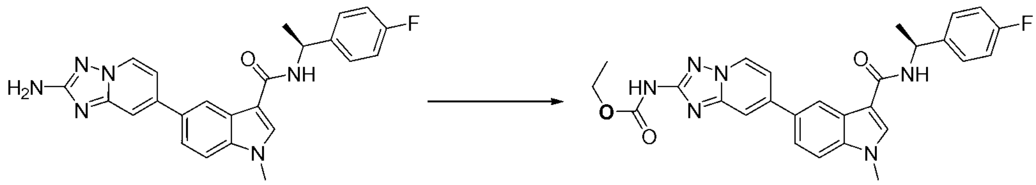

INT-3

Compound 17

Preparation of Compound 18:

Intermediate **INT-26** (101 mg, 0.2 mmol) was dissolved in acetonitrile (1 mL); 2,2-dimethylmorpholine (45 mg, 0.4

mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 18 with a yield of 66%. MS (ESI, positive ion) m/z: 584.2 [M + H]$^+$.

$^1$H NMR (400 MHz, DMSO-*d*$_6$) δ 8.56 (d, *J* = 1.9 Hz, 1H), 8.37 (d, *J* = 8.0 Hz, 1H), 8.23 (s, 1H), 7.98 (s, 1H), 7.78 - 7.66 (m, 3H), 7.53 (dd, *J* = 7.2, 2.0 Hz, 1H), 7.47 (dd, *J* = 8.5, 5.6 Hz, 3H), 7.18 (d, *J* = 8.9 Hz, 2H), 4.97 - 4.82 (m, 1H), 3.91 (s, 3H), 3.65 - 3.52 (m, 2H), 3.25 (s, 2H), 2.57 (m, 2H), 2.35 (s, 2H), 1.50 (d, *J*= 7.1 Hz, 3H), 1.25 (s, 6H),.

**Example 19: Preparation of Compound 19**

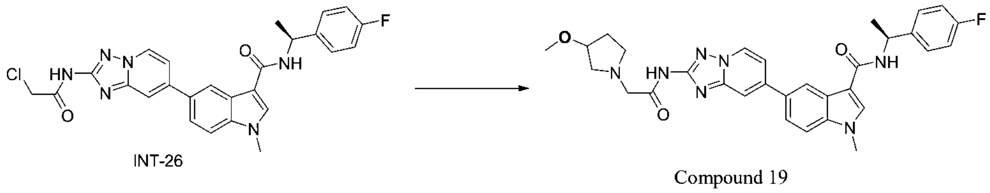

INT-26

Compound 19

Preparation of Compound 19:

Intermediate **INT-26** (101 mg, 0.2 mmol) was dissolved in acetonitrile (1 mL); 2-methoxypyrrolidine (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 19 with a yield of 76%. MS (ESI, positive ion) m/z: 570.2 [M + H]$^+$.

$^1$H NMR (400 MHz, DMSO-*d*$_6$) δ 8.56 (d, *J* = 1.9 Hz, 1H), 8.37 (d, *J* = 8.0 Hz, 1H), 8.23 (s, 1H), 7.98 (s, 1H), 7.78 - 7.66 (m, 3H), 7.53 (dd, *J* = 7.2, 2.0 Hz, 1H), 7.47 (dd, *J* = 8.5, 5.6 Hz, 3H), 7.18 (d, *J* = 8.9 Hz, 2H), 4.97 - 4.82 (m, 1H), 3.91 (s, 3H), 3.42 - 3.38 (m, 4H), 3.25 (s, 2H), 2.62 - 2.32 (m, 4H), 1.82 - 1.64 (m, 2H), 1.50 (d, *J* = 7.1 Hz, 3H).

**Example 20: Preparation of Compound 20**

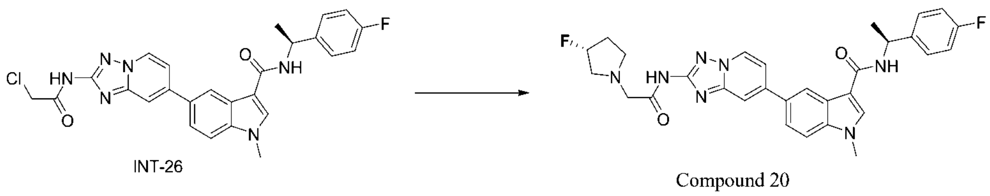

INT-26

Compound 20

Preparation of Compound 20:

Intermediate **INT-26** (101 mg, 0.2 mmol) was dissolved in acetonitrile (1 mL); 3-(S)-pyrrolidinone (35 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 20 with a yield of 76%. MS (ESI, positive ion) m/z: 558.2 [M + H]$^+$.

$^1$H NMR (400 MHz, DMSO-*d*$_6$) δ 8.56 (d, *J*= 1.9 Hz, 1H), 8.37 (d, *J*= 8.0 Hz, 1H), 8.23 (s, 1H), 7.98 (s, 1H), 7.78 - 7.66 (m, 3H), 7.53 (dd, *J*= 7.2, 2.0 Hz, 1H), 7.47 (dd, *J*= 8.5, 5.6 Hz, 3H), 7.18 (d, *J* = 8.9 Hz, 2H), 4.97 - 4.82 (m, 1H), 3.91 (s, 3H), 3.42 - 3.38 (m, 1H), 3.25 (s, 2H), 2.62 - 2.32 (m, 4H), 1.82 - 1.64 (m, 2H), 1.50 (d, *J* = 7.1 Hz, 3H).

**Example 21: Preparation of Compound 21**

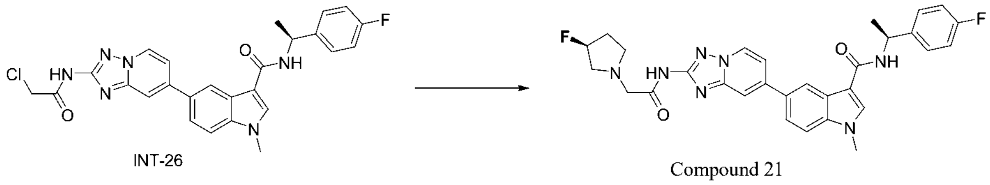

INT-26

Compound 21

Preparation of Compound 21:

Intermediate **INT-26** (101 mg, 0.2 mmol) was dissolved in acetonitrile (1 mL); 3-(R)-pyrrolidinone (35 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 21 with a yield of 66%. MS (ESI, positive ion) m/z: 558.2 $[M + H]^+$.

$^1H$ NMR (400 MHz, DMSO-$d_6$) δ 8.56 (d, *J* = 1.9 Hz, 1H), 8.37 (d, *J* = 8.0 Hz, 1H), 8.23 (s, 1H), 7.98 (s, 1H), 7.78 - 7.66 (m, 3H), 7.53 (dd, *J* = 7.2, 2.0 Hz, 1H), 7.47 (dd, *J* = 8.5, 5.6 Hz, 3H), 7.18 (d, *J*= 8.9 Hz, 2H),4.97 - 4.82 (m, 1H), 3.91 (s, 3H), 3.42 - 3.38 (m, 1H), 3.25 (s, 2H), 2.62 - 2.32 (m, 4H), 1.82 - 1.64 (m, 2H), 1.50 (d, *J* = 7.1 Hz, 3H).

**Example 22: Preparation of Compound 22**

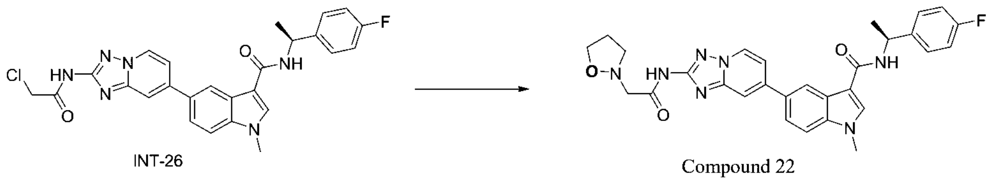

INT-26

Compound 22

Preparation of Compound 22:

Intermediate **INT-26** (101 mg, 0.2 mmol) was dissolved in acetonitrile (1 mL); isoxazolidine (29 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 22 with a yield of 56%. MS (ESI, positive ion) m/z: 541.2 $[M + H]^+$.

$^1H$ NMR (400 MHz, DMSO-$d_6$) δ 8.56 (d, *J*= 1.9 Hz, 1H), 8.37 (d, *J*= 8.0 Hz, 1H), 8.23 (s, 1H), 7.98 (s, 1H), 7.78 - 7.66 (m, 3H), 7.53 (dd, *J* = 7.2, 2.0 Hz, 1H), 7.47 (dd, *J*= 8.5, 5.6 Hz, 3H), 7.18 (d, *J* = 8.9 Hz, 2H), 5.27 - 5.17 (m, 1H), 4.07 - 3.98 (m, 1H), 3.91 (s, 3H), 3.70 (s, 2H), 2.01 (s, 2H), 1.57 - 1.54 (m, 1H), 1.50 (d, *J*= 7.1 Hz, 3H), 0.88 (d, *J*= 2.1 Hz, 2H).

**Example 23: Preparation of Compound 23**

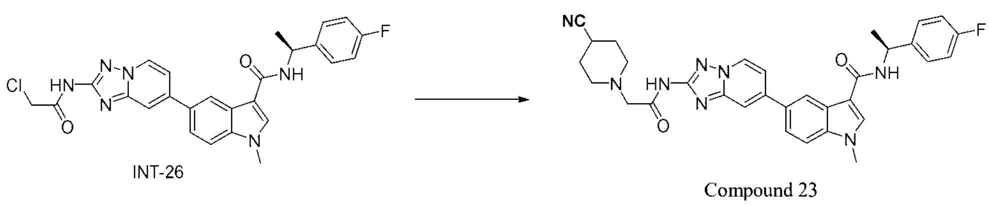

INT-26

Compound 23

Preparation of Compound 23:

Intermediate INT-26 (101 mg, 0.2 mmol) was dissolved in acetonitrile (1 mL); 4-cyanopiperidine (44 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 23 with a yield of 66%. MS (ESI, positive ion) m/z: 579.2 $[M + H]^+$.

$^1H$ NMR (400 MHz, DMSO) δ 10.52 (s, 1H), 8.89 (d, *J* = 7.1 Hz, 1H), 8.55 (d, *J* = 1.4 Hz, 1H), 8.39 (d, *J* = 8.0 Hz, 1H), 8.23 (s, 1H), 7.90 (d, *J* = 1.1 Hz, 1H), 7.78 - 7.64 (m, 2H), 7.51 - 7.43 (m, 3H), 7.17 (t, *J* = 8.9 Hz, 2H), 5.22 (p, *J* = 6.9 Hz,

1H), 3.91 (s, 3H), 3.26 (d, *J* = 11.5 Hz, 2H), 2.97 - 2.86 (m, 1H), 2.77 - 2.62 (m, 2H), 2.48 (s, 2H), 2.01 - 1.84 (m, 2H), 1.79 (ddd, *J* = 12.9, 8.4, 4.0 Hz, 2H), 1.50 (d, *J* = 7.0 Hz, 3H).

**Example 24: Preparation of Compound 24**

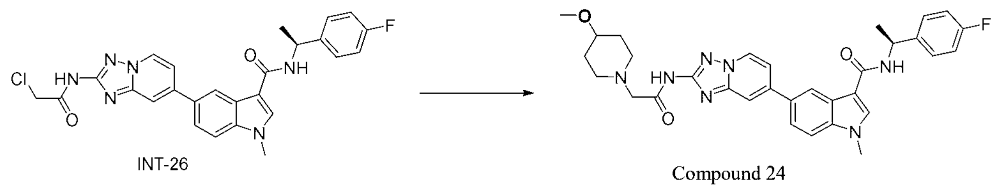

INT-26

Compound 24

Preparation of Compound 24:

Intermediate **INT-26** (101 mg, 0.2 mmol) was dissolved in acetonitrile (1 mL); 4-methoxypiperidine (45 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 24 with a yield of 76%. MS (ESI, positive ion) m/z: 584.2 [M + H]$^+$.

$^1$H NMR (400 MHz, DMSO) δ 10.44 (s, 1H), 8.89 (d, *J* = 7.1 Hz, 1H), 8.55 (d, *J* = 1.5 Hz, 1H), 8.39 (d, *J* = 8.0 Hz, 1H), 8.23 (s, 1H), 7.90 (d, *J* = 1.2 Hz, 1H), 7.78 - 7.63 (m, 2H), 7.52 - 7.43 (m, 3H), 7.26 - 7.11 (m, 2H), 5.22 (p, *J* = 7.1 Hz, 1H), 3.91 (s, 3H), 3.38 (s, 3H), 3.23 (s, 3H), 2.77 (dd, *J* = 13.7, 8.1 Hz, 2H), 2.38 - 2.29 (m, 2H), 1.87 (d, *J* = 9.7 Hz, 2H), 1.50 (d, *J* = 7.0 Hz, 5H).

**Example 25: Preparation of Compound 25**

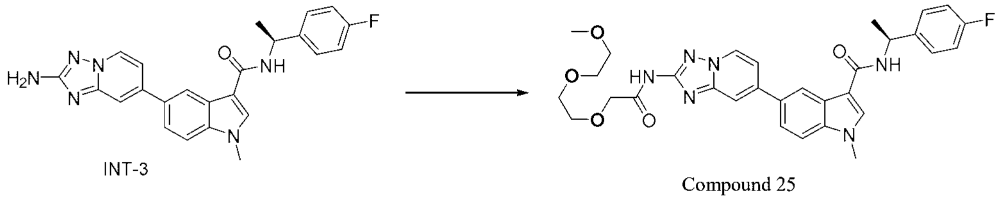

INT-3

Compound 25

Preparation of Compound 25:

Intermediate **INT-3** (85 mg, 0.2 mmol) was dissolved in pyridine (1 mL); 2-(2-(2-methoxyethoxy)ethoxy)acetic acid (53 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 25 with a yield of 51%. MS (ESI, positive ion) m/z: 589.2 [M + H]$^+$.

$^1$H NMR (400 MHz, DMSO) δ 10.56 (s, 1H), 8.90 (d, *J* = 7.1 Hz, 1H), 8.56 (d, *J* = 1.2 Hz, 1H), 8.39 (d, *J* = 8.0 Hz, 1H), 8.23 (s, 1H), 7.91 (s, 1H), 7.79 - 7.64 (m, 2H), 7.47 (dd, *J* = 8.2, 5.9 Hz, 3H), 7.17 (t, *J* = 8.9 Hz, 2H), 5.22 (p, *J* = 7.1 Hz, 1H), 4.37 - 4.19 (m, 2H), 3.91 (s, 3H), 3.72 - 3.66 (m, 2H), 3.64 - 3.57 (m, 4H), 3.49 (dd, *J* = 5.7, 3.7 Hz, 2H), 3.26 (s, 3H), 1.50 (d, *J* = 7.0 Hz, 3H).

**Example 26: Preparation of Compound 26**

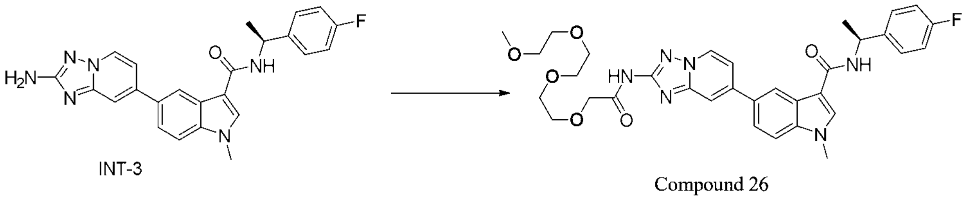

INT-3

Compound 26

Preparation of Compound 26:

Intermediate **INT-3** (85 mg, 0.2 mmol) was dissolved in pyridine (1 mL); triethylene glycol monomethyl ether acetate (66 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 26 with a yield of 48%. MS (ESI, positive ion) m/z: 633.2 [M + H]$^+$.

$^1$H NMR (400 MHz, DMSO) δ 10.54 (s, 1H), 8.90 (d, *J* = 7.1 Hz, 1H), 8.55 (d, *J* = 1.2 Hz, 1H), 8.38 (d, *J* = 8.0 Hz, 1H), 8.23 (s, 1H), 7.93 (d, *J* = 15.9 Hz, 1H), 7.78 - 7.64 (m, 2H), 7.47 (dd, *J* = 8.1, 6.0 Hz, 3H), 7.17 (t, *J* = 8.9 Hz, 2H), 5.22 (p, *J* = 7.0 Hz, 1H), 4.25 (s, 2H), 3.91 (s, 3H), 3.70 (dd, *J* = 5.8, 3.3 Hz, 2H), 3.62 (dd, *J* = 5.6, 3.4 Hz, 2H), 3.58 (s, 4H), 3.53 (dd, *J* = 5.8, 3.6 Hz, 2H), 3.43 (dd, *J* = 5.7, 3.8 Hz, 2H), 3.23 (s, 3H), 1.50 (d, *J* = 7.0 Hz, 3H).

**Example 27: Preparation of Compound 27**

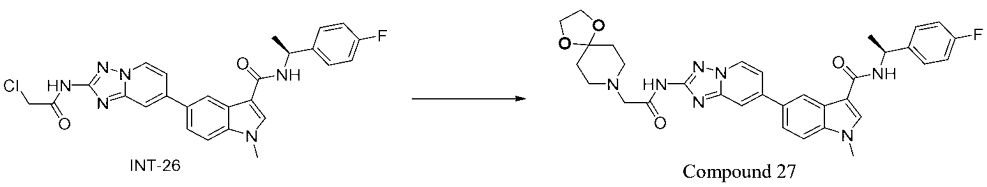

INT-26

Compound 27

Preparation of Compound 27:

Intermediate **INT-26** (101 mg, 0.2 mmol) was dissolved in acetonitrile (1 mL); 4-piperidone ethylene ketal (57 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 27 with a yield of 86%. MS (ESI, positive ion) m/z: 612.2 [M + H]$^+$.

$^1$H NMR (400 MHz, DMSO) δ 10.48 (s, 1H), 8.89 (d, *J* = 7.1 Hz, 1H), 8.55 (d, *J* = 1.4 Hz, 1H), 8.39 (d, *J* = 8.0 Hz, 1H), 8.23 (s, 1H), 7.90 (d, *J* = 1.2 Hz, 1H), 7.78 - 7.64 (m, 2H), 7.52 - 7.43 (m, 3H), 7.17 (t, *J* = 8.9 Hz, 2H), 5.22 (p, *J* = 7.1 Hz, 1H), 3.90 (d, *J* = 10.6 Hz, 7H), 3.27 (s, 2H), 2.68 - 2.58 (m, 4H), 1.69 (t, *J* = 5.4 Hz, 4H), 1.50 (d, *J* = 7.0 Hz, 3H).

**Example 28: Preparation of Compound 28**

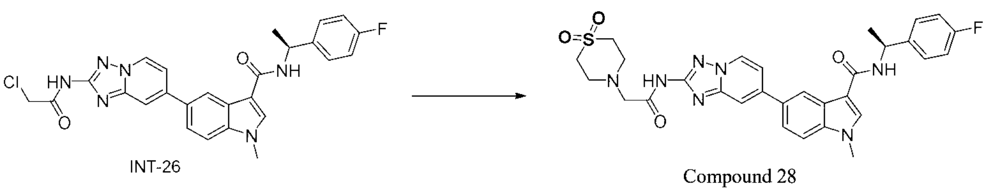

INT-26

Compound 28

Preparation of Compound 28:

Intermediate **INT-26** (101 mg, 0.2 mmol) was dissolved in acetonitrile (1 mL); thiomorpholine 1,1-dioxide (53 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 28 with a yield of 72%. MS (ESI, positive ion) m/z: 604.2 [M + H]+.

$^1$H NMR (400 MHz, DMSO) δ 10.70 (s, 1H), 8.89 (d, *J* = 7.1 Hz, 1H), 8.55 (d, *J* = 1.4 Hz, 1H), 8.39 (d, *J* = 8.0 Hz, 1H), 8.23 (s, 1H), 7.90 (d, *J* = 1.2 Hz, 1H), 7.78 - 7.59 (m, 2H), 7.52 - 7.43 (m, 3H), 7.22 - 7.12 (m, 2H), 5.22 (p, *J* = 7.0 Hz, 1H), 3.91 (s, 3H), 3.52 (s, 2H), 3.19 (d, *J* = 4.8 Hz, 4H), 3.13 (d, *J* = 5.3 Hz, 4H), 1.50 (d, *J* = 7.0 Hz, 3H).

**Example 29: Preparation of Compound 29**

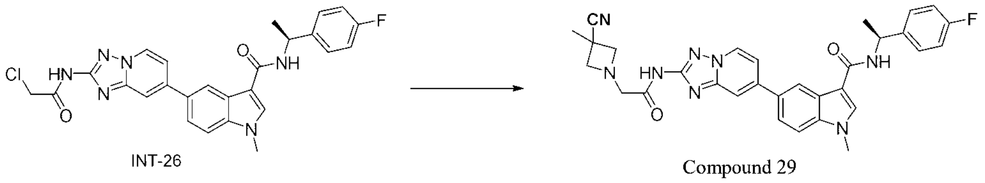

INT-26

Compound 29

Preparation of Compound 29:

Intermediate **INT-26** (101 mg, 0.2 mmol) was dissolved in acetonitrile (1 mL); 3-methylazetidine-3-carbonitrile (38 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 29 with a yield of 68%. MS (ESI, positive ion) m/z: 565.2 [M + H]+.

$^1$H NMR (400 MHz, DMSO) δ 10.68 (s, 1H), 8.87 (d, *J*= 7.1 Hz, 1H), 8.54 (s, 1H), 8.38 (d, *J*= 8.0 Hz, 1H), 8.22 (s, 1H), 7.89 (s, 1H), 7.77 - 7.63 (m, 2H), 7.52 - 7.42 (m, 3H), 7.16 (t, *J* = 8.9 Hz, 2H), 5.21 (p, *J* = 7.2 Hz, 1H), 3.90 (s, 3H), 3.67 (d, *J* = 7.2 Hz, 2H), 3.45 (s, 2H), 3.33 (s, 2H), 1.58 (s, 3H), 1.49 (d, *J* = 7.0 Hz, 3H).

**Example 30: Preparation of Compound 30**

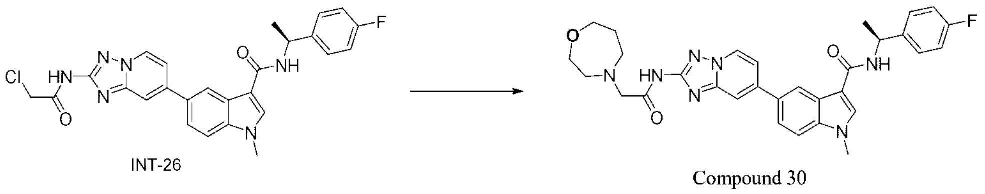

INT-26

Compound 30

Preparation of Compound 30:

Intermediate **INT-26** (101 mg, 0.2 mmol) was dissolved in acetonitrile (1 mL); 1,4-oxazepane (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 30 with a yield of 78%. MS (ESI, positive ion) m/z: 570.2 [M + H]+.

$^1$H NMR (400 MHz, DMSO) δ 10.47 (s, 1H), 8.89 (d, *J*= 7.1 Hz, 1H), 8.55 (d, *J*= 1.5 Hz, 1H), 8.39 (d, *J* = 8.0 Hz, 1H), 8.23 (s, 1H), 7.90 (d, *J* = 1.2 Hz, 1H), 7.78 - 7.64 (m, 2H), 7.52 - 7.43 (m, 3H), 7.17 (dd, *J* = 12.4, 5.4 Hz, 2H), 5.22 (p, *J* = 7.1 Hz, 1H), 3.91 (s, 3H), 3.73 (t, *J* = 6.0 Hz, 2H), 3.70 - 3.64 (m, 2H), 3.44 (s, 2H), 2.87 - 2.80 (m, 4H), 1.92 - 1.81 (m, 2H), 1.50 (d, *J*= 7.0 Hz, 3H).

**Example 31: Preparation of Compound 31**

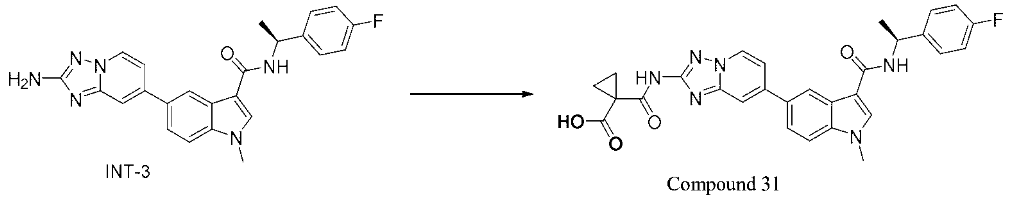

Preparation of Compound 31:

Intermediate **INT-3** (85 mg, 0.2 mmol) was dissolved in pyridine (1 mL); 1,1-cyclopropanedicarboxylic acid (39 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 31 with a yield of 39%. MS (ESI, positive ion) m/z: 541.2 $[M + H]^+$.

$^1H$ NMR (400 MHz, DMSO-$d_6$) δ 8.81 (d, *J* = 7.2 Hz, 1H), 8.52 (s, 1H), 8.44 (d, *J* = 7.9 Hz, 1H), 8.28 (s, 1H), 7.86 (s, 1H), 7.64 (q, *J* = 8.8 Hz, 2H), 7.43 (t, *J* = 7.4 Hz, 3H), 7.11 (t, *J* = 8.7 Hz, 2H), 5.24 - 5.11 (m, 1H), 3.85 (s, 3H), 1.45 (d, *J* = 7.0 Hz, 3H), 1.23 (d, *J* = 41.5 Hz, 4H).

**Example 32: Preparation of Compound 32**

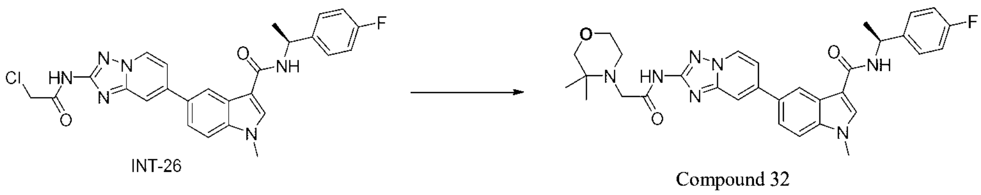

Preparation of Compound 32:

Intermediate **INT-26** (101 mg, 0.2 mmol) was dissolved in acetonitrile (1 mL); 3,3-dimethylmorpholine (46 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 32 with a yield of 88%. MS (ESI, positive ion) m/z: 583.2 $[M + H]^+$.

$^1H$ NMR (400 MHz, DMSO) δ 10.32 - 10.25 (m, 1H), 8.94 - 8.87 (m, 1H), 8.58 - 8.52 (m, 1H), 8.43 - 8.34 (m, 1H), 8.26 - 8.21 (m, 1H), 7.94 - 7.89 (m, 1H), 7.78 - 7.64 (m, 2H), 7.53 - 7.43 (m, 3H), 7.22 - 7.13 (m, 2H), 5.23 (p, *J* = 7.0 Hz, 1H), 3.92 (s, 3H), 3.76 - 3.65 (m, 2H), 3.37 (s, 2H), 3.22 (s, 2H), 2.66 - 2.57 (m, 2H), 1.50 (d, *J* = 7.0 Hz, 3H), 1.04 (s, 6H).

**Example 33: Preparation of Compound 33**

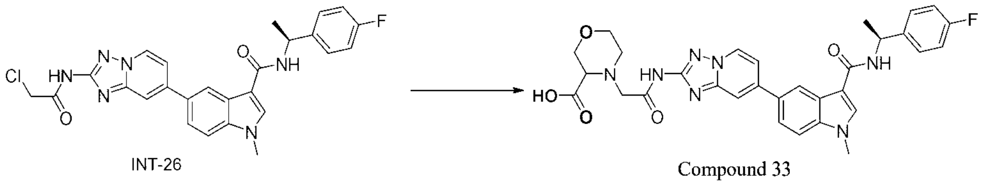

Preparation of Compound 33:

Intermediate **INT-26** (108 mg, 0.2 mmol) was dissolved in acetonitrile (1 mL); 3-morpholinecarboxylic acid (52 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 33 with a yield of 48%. MS (ESI, positive ion) m/z: 600.2 $[M + H]^+$.

$^1H$ NMR (400 MHz, DMSO-$d_6$) $\delta$11.3 (s, 1H), 10.25 (s, 1H), 8.87 (d, $J$ = 7.1 Hz, 1H), 8.54 (d, $J$ = 1.9 Hz, 1H), 8.39 (d, $J$ = 8.0 Hz, 1H), 8.23 (s, 1H), 7.88 (s, 1H), 7.76 - 7.64 (m, 2H), 7.47 (ddd, $J$ = 8.9, 5.7, 2.8 Hz, 3H), 7.20 - 7.14 (m, 2H), 5.22 (p, $J$ = 7.2 Hz, 1H), 3.91 (s, 3H), 3.85 (d, $J$= 9.8 Hz, 1H), 3.72 - 3.50 (m, 5H), 3.18 (s, 2H), 2.90 (d, $J$= 11.4 Hz, 1H), 1.50 (d, $J$= 7.0 Hz, 3H).

**Example 34: Preparation of Compound 34**

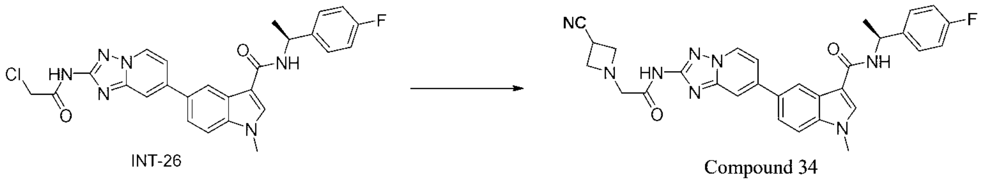

INT-26

Compound 34

Preparation of Compound 34:

Intermediate **INT-26** (101 mg, 0.2 mmol) was dissolved in acetonitrile (1 mL); 3-azetidinecarbonitrile (32 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 34 with a yield of 38%. MS (ESI, positive ion) m/z:551.2 [M + H]$^+$.

$^1H$ NMR (400 MHz, DMSO-$d_6$) $\delta$ 10.65 (s, 1H), 8.86 (d, $J$= 7.1 Hz, 1H), 8.54 (d, $J$= 1.8 Hz, 1H), 8.36 (d, $J$= 8.0 Hz, 1H), 8.22 (s, 1H), 7.89 (d, $J$ = 1.9 Hz, 1H), 7.72 (d, $J$ = 1.9 Hz, 1H), 7.66 (d, $J$ = 8.6 Hz, 1H), 7.47 (td, $J$= 5.5, 2.5 Hz, 3H), 7.16 (t, $J$ = 8.9 Hz, 2H), 5.22 (t, $J$= 7.3 Hz, 1H), 3.91 (s, 3H), 3.64 (dd, $J$= 7.8, 5.9 Hz, 2H), 3.59 - 3.53 (m, 1H), 3.48 (d, $J$ = 6.3 Hz, 2H), 1.50 (d, $J$ = 7.1 Hz, 3H), 0.95 (d, $J$= 6.5 Hz, 2H).

**Example 35: Preparation of Compound 35**

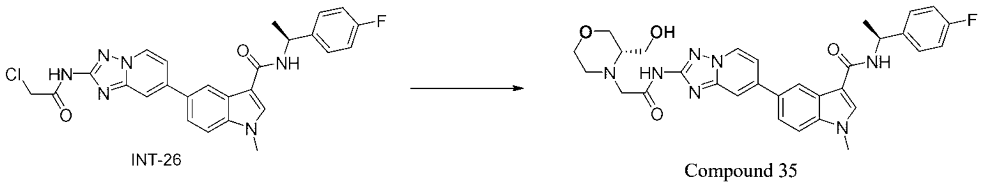

INT-26

Compound 35

Preparation of Compound 35:

Intermediate **INT-26** (101 mg, 0.2 mmol) was dissolved in acetonitrile (1 mL), and (S)-3-(hydroxymethyl) morpholine (46 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 35 with a yield of 58%. MS (ESI, positive ion) m/z:586.2 [M + H]$^+$.

$^1H$ NMR (400 MHz, DMSO-$d_6$) $\delta$ 10.47 (s, 1H), 8.89 (d, $J$= 7.1 Hz, 1H), 8.59 - 8.53 (m, 1H), 8.36 (t, $J$= 7.6 Hz, 1H), 8.22 (d, $J$= 7.6 Hz, 1H), 7.90 (d, $J$= 2.0 Hz, 1H), 7.76 - 7.61 (m, 2H), 7.51 - 7.43 (m, 3H), 7.17 (t, $J$ = 8.8 Hz, 2H), 5.22 (p, $J$ = 7.1 Hz, 1H), 4.71 (t, $J$ = 5.2 Hz, 1H), 3.91 (d, $J$ = 4.8 Hz, 3H), 3.80 - 3.38 (m, 6H), 3.27 (d, $J$ = 16.3 Hz, 2H), 3.06 - 2.77 (m, 1H), 2.60 (d, $J$ = 8.7 Hz, 1H), 2.46 (q, $J$= 7.2 Hz, 1H), 1.50 (dd, $J$= 7.1, 1.9 Hz, 3H).

**Example 36: Preparation of Compound 36**

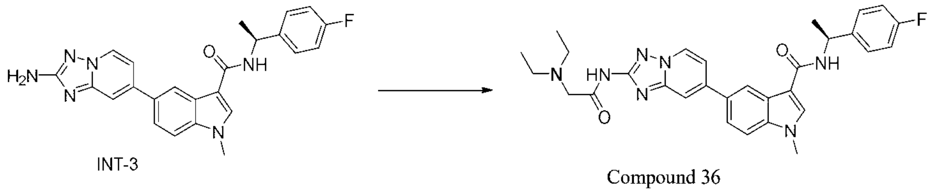

INT-3

Compound 36

Preparation of Compound 36:

Intermediate **INT-3** (85 mg, 0.2 mmol) was dissolved in pyridine (1 mL); 2-diethylaminoacetic acid (39 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 36 with a yield of 39%. MS (ESI, positive ion) m/z: 542.2 $[M + H]^+$.

$^1H$ NMR (400 MHz, DMSO-$d_6$) δ 10.28 (s, 1H), 8.89 (d, *J*= 7.1 Hz, 1H), 8.55 (d, *J*= 1.8 Hz, 1H), 8.37 (d, *J* = 8.0 Hz, 1H), 8.23 (s, 1H), 7.90 (d, *J* = 1.9 Hz, 1H), 7.73 (d, *J*= 1.9 Hz, 1H), 7.67 (d, *J* = 8.6 Hz, 1H), 7.50 - 7.46 (m, 3H), 7.17 (t, *J*= 8.9 Hz, 2H), 5.23 (t, *J*= 7.4 Hz, 1H), 3.91 (s, 3H), 3.28 (s, 2H), 2.65 (q, *J* = 7.2 Hz, 4H), 1.51 (d, *Jc*= 7.1 Hz, 3H), 1.05 (t, *J* = 7.1 Hz, 6H).

**Example 37: Preparation of Compound 37**

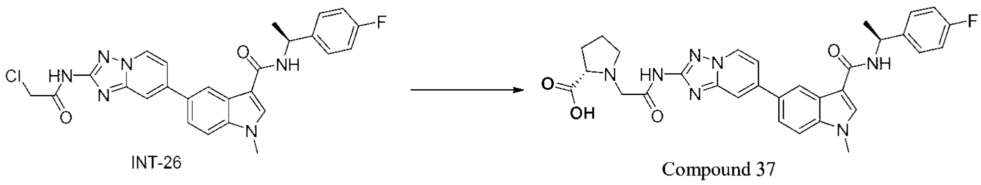

INT-26

Compound 37

Preparation of Compound 37:

Intermediate **INT-26** (101 mg, 0.2 mmol) was dissolved in acetonitrile (1 mL); L-proline (34 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 37 with a yield of 48%. MS (ESI, positive ion) m/z:584.2 $[M + H]^+$.

$^1H$ NMR (400 MHz, DMSO-$d_6$) δ 8.87 (d, *J*= 7.2 Hz, 1H), 8.55 (s, 1H), 8.37 (d, *J*= 7.9 Hz, 1H), 8.22 (s, 1H), 7.89 (s, 1H), 7.78 - 7.64 (m, 2H), 7.47 (dd, *J*= 8.6, 5.6 Hz, 3H), 7.17 (t, *J*= 8.8 Hz, 2H), 5.22 (t, *J*= 7.3 Hz, 1H), 3.91 (s, 3H), 3.50 - 3.42 (m, 2H), 3.28 - 3.09 (m, 4H), 2.67 (d, *J*= 15.5 Hz, 1H), 2.19 - 2.06 (m, 1H), 1.50 (d, *J*= 7.1 Hz, 3H).

**Example 38: Preparation of Compound 38**

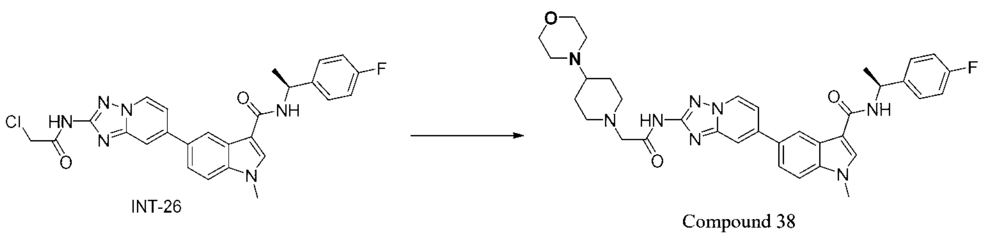

INT-26

Compound 38

Preparation of Compound 38:

Intermediate **INT-26** (101 mg, 0.2 mmol) was dissolved in acetonitrile (1 mL); 4-(4-piperidinyl)morpholine (68 mg, 0.4 mmol) was added, and the mixture was heated to 80°C and stirred for 6 h. The reaction mixture was concentrated under

reduced pressure and purified by C-18 reverse phase silica gel column (acetonitrile/0.1% aqueous ammonium bicarbonate solution = 60%) to obtain Compound 38 with a yield of 78%. MS (ESI, positive ion) m/z:639.2 [M + H]+.

$^1$H NMR (400 MHz, DMSO-$d_6$) δ 10.40 (s, 1H), 8.88 (dd, *J* = 7.1, 0.8 Hz, 1H), 8.55 (d, *J*= 1.8 Hz, 1H), 8.36 (d, *J* = 8.0 Hz, 1H), 8.22 (s, 1H), 7.90 (dd, *J* = 2.0, 0.9 Hz, 1H), 7.74 (dd, *J* = 8.7, 1.9 Hz, 1H), 7.67 (d, *J* = 8.6 Hz, 1H), 7.50 - 7.45 (m, 3H), 7.21 - 7.13 (m, 2H), 5.23 (t, *J*=7.4 Hz, 1H), 3.91 (s, 3H), 3.58 (t, *J* = 4.6 Hz, 4H), 3.22 (s, 2H), 2.95 (d, *J* = 11.0 Hz, 2H), 2.47 (t, *J* = 4.7 Hz, 4H), 2.24 - 2.16 (m, 2H), 1.77 (d, *J*= 12.2 Hz, 2H), 1.49 (t, *J*= 8.3 Hz, 5H).

**Example 39: Preparation of Compound 39**

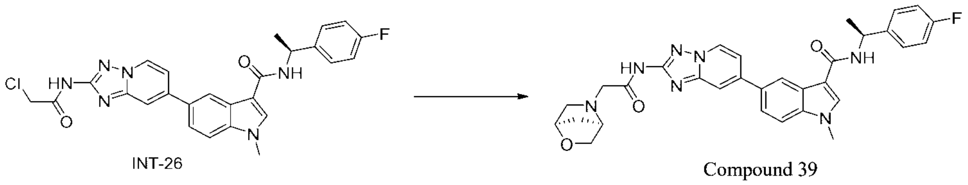

INT-26

Compound 39

Preparation of Compound 39:

Intermediate **INT-26** (101 mg, 0.2 mmol) was dissolved in acetonitrile (1 mL), and (1R,4R)-2-oxa-5-azabicyclo [2.2.1]heptane (39 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 39 with a yield of 76%. MS (ESI, positive ion) m/z:568.2 [M + H]+.

$^1$H NMR (400 MHz, DMSO-$d_6$) δ 10.39 (s, 1H), 8.89 (d, *J* = 7.1 Hz, 1H), 8.55 (d, *J* = 1.8 Hz, 1H), 8.37 (d, *J* = 8.0 Hz, 1H), 8.23 (s, 1H), 7.90 (d, *J* = 1.9 Hz, 1H), 7.77 - 7.71 (m, 1H), 7.67 (d, *J* = 8.6 Hz, 1H), 7.50 - 7.45 (m, 3H), 7.17 (t, *J* = 8.7 Hz, 2H), 5.23 (t, *J* = 7.3 Hz, 1H), 4.40 (s, 1H), 3.90 (d, *J* = 10.6 Hz, 4H), 3.62 (s, 1H), 3.58 (dd, *J* = 7.6, 1.7 Hz, 1H), 3.48 (d, *J* = 5.3 Hz, 2H), 2.97 (dd, *J* = 10.0, 1.7 Hz, 1H), 2.57 (d, *J* = 9.9 Hz, 1H), 1.87 (dd, *J* = 9.6, 2.1 Hz, 1H), 1.65 (d, *J* = 9.5 Hz, 1H), 1.51 (d, *J* = 7.0 Hz, 3H).

**Example 40: Preparation of Compound 40**

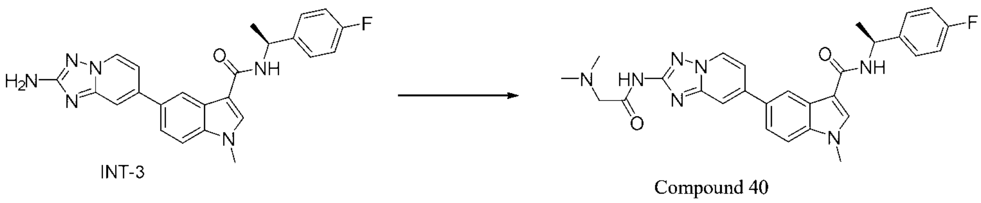

INT-3

Compound 40

Preparation of Compound 40:

Intermediate **INT-3** (85 mg, 0.2 mmol) was dissolved in pyridine (1 mL); 2-dimethylaminoacetic acid (31 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 40 with a yield of 39%. MS (ESI, positive ion) m/z: 514.2 [M + H]+.

$^1$H NMR (400 MHz, DMSO-$d_6$) δ 10.76 (s, 1H), 8.87 (d, *J* = 7.1 Hz, 1H), 8.52 (d, *J* = 1.8 Hz, 1H), 8.37 (d, *J* = 8.0 Hz, 1H), 8.23 (s, 1H), 7.89 (d, *J* = 1.9 Hz, 1H), 7.76 (dd, *J* = 8.6, 1.9 Hz, 1H), 7.65 (d, *J* = 8.6 Hz, 1H), 7.57 (td, *J* = 5.3, 2.4 Hz, 3H), 7.19 - 7.14 (m, 2H), 5.22 (p, *J* = 7.2 Hz, 1H), 3.91 (s, 3H), 3.80 (t, *J* = 12.5 Hz, 4H), 3.61 (s, 6H), 1.50 (d, *J* = 7.0 Hz, 3H), 0.97 (d, *J* = 6.5 Hz, 2H).

**Example 41: Preparation of Compound 41**

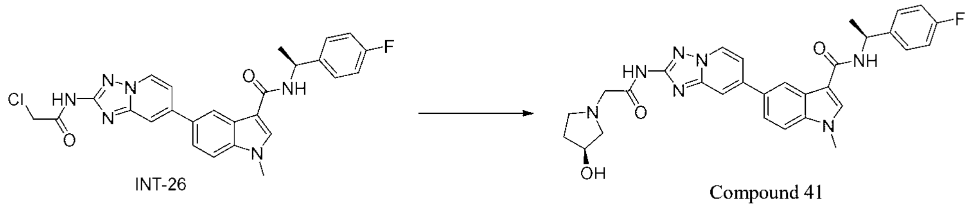

Compound 41

Preparation of Compound 41:

Intermediate **INT-26** (101 mg, 0.2 mmol) was dissolved in acetonitrile (1 mL); (S)-3-pyrrolidinol (35 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 41 with a yield of 66%. MS (ESI, positive ion) m/z:556.2 $[M + H]^+$.

$^1H$ NMR (400 MHz, DMSO-$d_6$) δ 10.74 (s, 1H), 8.89 (d, *J* = 7.1 Hz, 1H), 8.54 (d, *J* = 1.8 Hz, 1H), 8.36 (d, *J* = 8.0 Hz, 1H), 8.22 (s, 1H), 7.89 (d, *J* = 1.9 Hz, 1H), 7.74 (dd, *J* = 8.6, 1.9 Hz, 1H), 7.67 (d, *J* = 8.6 Hz, 1H), 7.47 (td, *J* = 5.3, 2.4 Hz, 3H), 7.19 - 7.14 (m, 2H), 5.22 (p, *J* = 7.2 Hz, 1H), 3.91 (s, 3H), 3.80 (t, *J* = 12.5 Hz, 4H), 2.06 (m,1H), 1.50 (d, *J* = 7.0 Hz, 3H), 1.22 (m, 2H), 0.97 (d, *J* = 6.5 Hz, 2H).

**Example 42: Preparation of Compound 42**

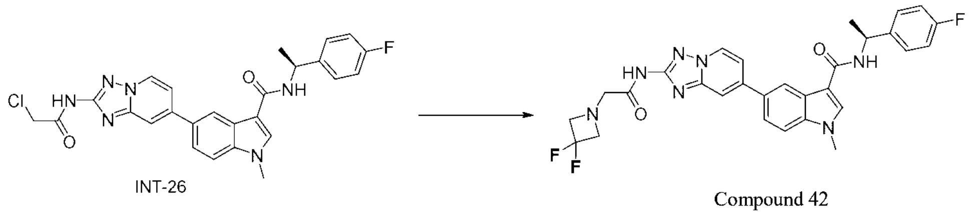

Compound 42

Preparation of Compound 42:

Intermediate **INT-26** (101 mg, 0.2 mmol) was dissolved in acetonitrile (1 mL); 3,3-difluoroazetidine (37 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 42 with a yield of 71%. MS (ESI, positive ion) m/z:562.2 $[M + H]^+$.

$^1H$ NMR (400 MHz, DMSO-$d_6$) δ 10.74 (s, 1H), 8.87 (d, *J* = 7.1 Hz, 1H), 8.55 (d, *J* = 1.8 Hz, 1H), 8.37 (d, *J* = 8.0 Hz, 1H), 8.23 (s, 1H), 7.89 (d, *J* = 1.9 Hz, 1H), 7.74 (dd, *J* = 8.6, 1.9 Hz, 1H), 7.67 (d, *J* = 8.6 Hz, 1H), 7.47 (td, *J* = 5.3, 2.4 Hz, 3H), 7.19 - 7.14 (m, 2H), 5.22 (p, *J* = 7.2 Hz, 1H), 3.91 (s, 3H), 3.80 (t, *J* = 12.5 Hz, 4H), 1.50 (d, *J* = 7.0 Hz, 3H), 0.97 (d, *J* = 6.5 Hz, 2H).

**Example 43: Preparation of Compound 43**

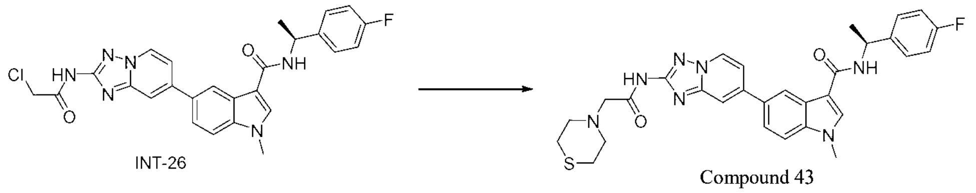

Compound 43

Preparation of Compound 43:

Intermediate **INT-26** (101 mg, 0.2 mmol) was dissolved in acetonitrile (1 mL); thiomorpholine (41 mg, 0.4 mmol) was added, and the mixture was heated to 80°C and stirred for 6 h. The reaction mixture was concentrated under reduced

pressure and purified by C-18 reverse phase silica gel column (acetonitrile/0.1% aqueous ammonium bicarbonate solution = 60%) to obtain Compound 43 with a yield of 71%. MS (ESI, positive ion) m/z:562.2 $[M + H]^+$.

$^1H$ NMR (400 MHz, DMSO-$d_6$) δ 10.47 (s, 1H), 8.89 (d, *J* = 7.1 Hz, 1H), 8.56 (d, *J* = 1.9 Hz, 1H), 8.37 (d, *J* = 8.0 Hz, 1H), 8.23 (s, 1H), 7.90 (d, *J* = 1.9 Hz, 1H), 7.74 (dd, *J* = 8.6, 1.9 Hz, 1H), 7.67 (d, *J* = 8.6 Hz, 1H), 7.48 (td, *J* = 5.5, 2.5 Hz, 3H), 7.22 - 7.12 (m, 2H), 5.23 (p, *J* = 7.1 Hz, 1H), 3.91 (s, 3H), 2.84 (dd, *J* = 6.5, 3.4 Hz, 4H), 2.71 - 2.65 (m, 4H), 1.51 (d, *J* = 7.0 Hz, 3H).

**Example 44: Preparation of Compound 44**

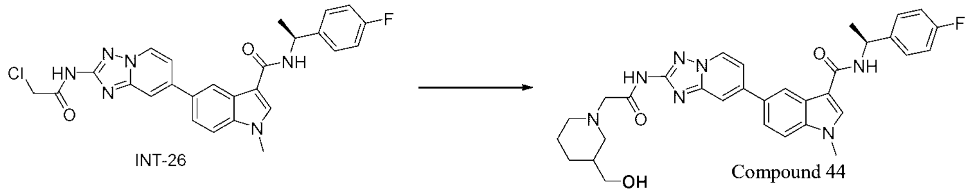
INT-26
Compound 44

Preparation of Compound 44:

Intermediate **INT-26** (101 mg, 0.2 mmol) was dissolved in acetonitrile (1 mL); 3-(hydroxymethyl)piperidine (46 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 44 with a yield of 61%. MS (ESI, positive ion) m/z:584.2 $[M + H]^+$.

$^1H$ NMR (400 MHz, DMSO-$d_6$) δ 10.37 (s, 1H), 8.89 (d, *J* = 7.3 Hz, 1H), 8.55 (d, *J* = 2.0 Hz, 1H), 8.38 (d, *J* = 8.0 Hz, 1H), 8.23 (s, 1H), 7.90 (d, *J* = 1.9 Hz, 1H), 7.74 (dd, *J* = 8.7, 1.9 Hz, 1H), 7.68 (d, *J* = 8.6 Hz, 1H), 7.52 - 7.43 (m, 3H), 7.22 - 7.12 (m, 2H), 5.22 (p, *J* = 7.2 Hz, 1H), 4.48 (t, *J* = 5.3 Hz, 1H), 3.92 (s, 3H), 3.34 - 3.29 (m, 1H), 3.27 - 3.16 (m, 3H), 2.98 - 2.91 (m, 1H), 2.83 (d, *J* = 11.0 Hz, 1H), 2.21 - 2.11 (m, 1H), 1.91 (t, *J* = 10.4 Hz, 1H), 1.75 - 1.62 (m, 3H), 1.50 (d, *J* = 7.1 Hz, 4H), 0.95 (dd, *J* = 19.6, 9.2 Hz, 1H).

**Example 45: Preparation of Compound 45**

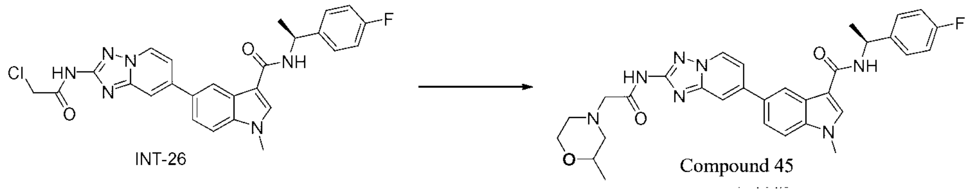
INT-26
Compound 45

Preparation of Compound 45:

Intermediate **INT-26** (101 mg, 0.2 mmol) was dissolved in acetonitrile (1 mL); 3-(hydroxymethyl)piperidine (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 45 with a yield of 61%. MS (ESI, positive ion) m/z:570.2 $[M + H]^+$.

$^1H$ NMR (400 MHz, DMSO-$d_6$) δ 10.53 (s, 1H), 8.89 (d, *J*= 7.1 Hz, 1H), 8.55 (d, *J*= 1.8 Hz, 1H), 8.39 (d, *J* = 8.0 Hz, 1H), 8.23 (s, 1H), 7.90 (d, *J* = 1.9 Hz, 1H), 7.74 (dd, *J* = 8.7, 1.9 Hz, 1H), 7.67 (d, *J* = 8.6 Hz, 1H), 7.49 - 7.45 (m, 3H), 7.17 (t, *J* = 8.9 Hz, 2H), 5.22 (p, *J* = 7.2 Hz, 1H), 3.91 (s, 3H), 3.77 (dt, *J* = 10.4, 2.5 Hz, 1H), 3.64 - 3.54 (m, 2H), 3.26 (s, 2H), 2.82 (dd, *J* = 11.1, 2.4 Hz, 1H), 2.75 (d, *J* = 11.2 Hz, 1H), 2.28 (td, *J* = 11.3, 3.3 Hz, 1H), 1.98 (t, *J* = 10.5 Hz, 1H), 1.50 (d, *J* = 7.0 Hz, 3H), 1.07 (d, *J* = 6.3 Hz, 3H).

**Example 46: Preparation of Compound 46**

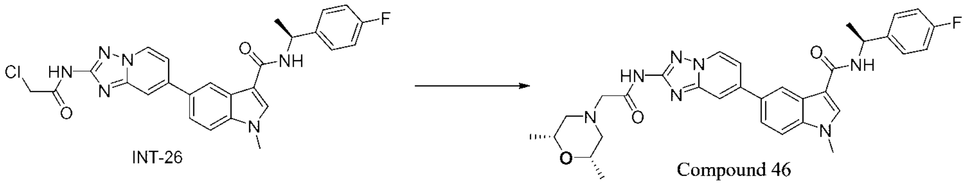
INT-26
Compound 46

Preparation of Compound 46:

Intermediate **INT-26** (101 mg, 0.2 mmol) was dissolved in acetonitrile (1 mL), and cis-2,6-dimethylmorpholine (46 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 46 with a yield of 74%. MS (ESI, positive ion) m/z:584.2 $[M + H]^+$.

$^1$H NMR (400 MHz, DMSO-$d_6$) δ 10.49 (s, 1H), 8.88 (dd, *J* = 7.1, 0.8 Hz, 1H), 8.55 (d, *J*= 1.8 Hz, 1H), 8.36 (d, *J* = 8.0 Hz, 1H), 8.22 (s, 1H), 7.89 (dd, *J* = 1.9, 0.9 Hz, 1H), 7.73 (dd, *J*= 8.7, 1.9 Hz, 1H), 7.67 (d, *J* = 8.6 Hz, 1H), 7.49 - 7.44 (m, 3H), 7.21 - 7.12 (m, 2H), 5.22 (t, *J* = 7.3 Hz, 1H), 3.91 (s, 3H), 3.64 (ddd, *J* = 10.0, 6.2, 2.0 Hz, 2H), 3.25 (s, 2H), 2.82 - 2.77 (m, 2H), 1.91 (t, *J* = 10.6 Hz, 2H), 1.50 (d, *J* = 7.0 Hz, 3H), 1.07 (d, *J* = 6.2 Hz, 6H).

**Example 47: Preparation of Compound 47**

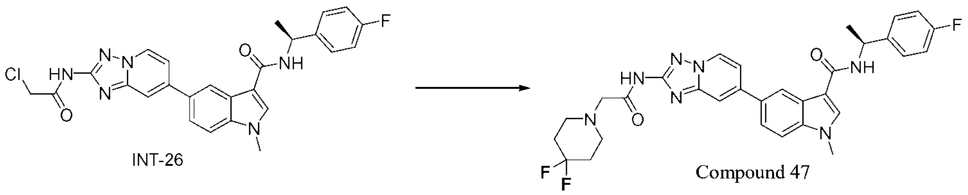
INT-26
Compound 47

Preparation of Compound 47:

Intermediate **INT-26** (101 mg, 0.2 mmol) was dissolved in acetonitrile (1 mL); 4,4-difluoropiperidine (48 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 47 with a yield of 74%. MS (ESI, positive ion) m/z:590.2 $[M + H]^+$.

$^1$H NMR (400 MHz, DMSO-$d_6$) δ 10.60 (s, 1H), 8.89 (d, *J* = 7.1 Hz, 1H), 8.55 (d, *J* = 1.9 Hz, 1H), 8.39 (d, *J* = 8.0 Hz, 1H), 8.24 (s, 1H), 7.90 (d, *J* = 2.0 Hz, 1H), 7.74 (dd, *J* = 8.7, 1.9 Hz, 1H), 7.67 (d, *J* = 8.6 Hz, 1H), 7.50 - 7.45 (m, 3H), 7.22 - 7.12 (m, 2H), 5.22 (p, *J* = 7.2 Hz, 1H), 3.91 (s, 3H), 2.72 (t, *J* = 5.7 Hz, 4H), 2.53 (d, *J* = 1.9 Hz, 2H), 2.04 (dq, *J* = 14.3, 8.0, 6.8 Hz, 4H), 1.50 (d, *J* = 7.0 Hz, 3H).

**Example 48: Preparation of Compound 48**

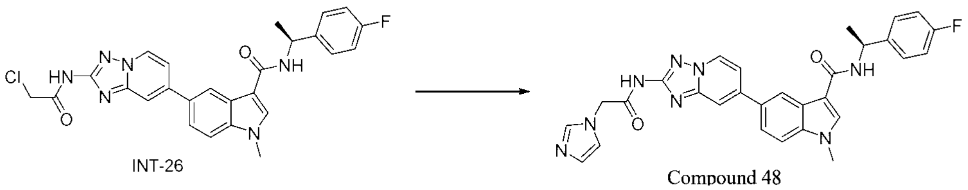
INT-26
Compound 48

Preparation of Compound 48:

Intermediate **INT-26** (101 mg, 0.2 mmol) was dissolved in acetonitrile (1 mL); 4,4-difluoropiperidine (27 mg, 0.4 mmol) and potassium carbonate (55 mg, 0.4 mmol) were added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 48 with a yield of 69%. MS (ESI,

positive ion) m/z:537.2 [M + H]+.

$^1$H NMR (400 MHz, DMSO-$d_6$) δ 11.25 (s, 1H), 8.90 (d, *J* = 7.1 Hz, 1H), 8.56 (d, *J* = 1.9 Hz, 1H), 8.38 (d, *J* = 8.0 Hz, 1H), 8.23 (s, 1H), 7.93 (t, *J* = 1.4 Hz, 1H), 7.76 (dd, *J* = 8.7, 1.9 Hz, 1H), 7.71 - 7.64 (m, 2H), 7.47 (tt, *J* = 9.0, 2.6 Hz, 3H), 7.22 - 7.12 (m, 3H), 6.92 (t, *J* = 1.1 Hz, 1H), 5.23 (q, *J* = 7.3 Hz, 1H), 3.92 (s, 3H), 2.56 (s, 2H), 1.50 (d, *J* = 7.0 Hz, 3H).

**Example 49: Preparation of Compound 49**

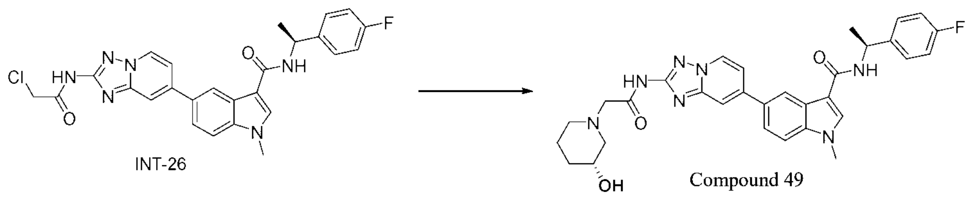

INT-26 Compound 49

Preparation of Compound 49:

Intermediate **INT-26** (101 mg, 0.2 mmol) was dissolved in acetonitrile (1 mL); (R)-3-piperidinol (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 49 with a yield of 62%. MS (ESI, positive ion) m/z:570.2 [M + H]+.

$^1$H NMR (400 MHz, DMSO-$d_6$) δ 10.45 (s, 1H), 8.89 (d, *J* = 7.1 Hz, 1H), 8.55 (d, *J* = 1.8 Hz, 1H), 8.38 (d, *J* = 8.0 Hz, 1H), 8.23 (s, 1H), 7.90 (d, *J* = 1.9 Hz, 1H), 7.74 (dd, *J* = 8.7, 1.9 Hz, 1H), 7.68 (d, *J* = 8.6 Hz, 1H), 7.48 (dt, *J* = 6.6, 2.7 Hz, 3H), 7.17 (dd, *J* = 10.0, 7.8 Hz, 2H), 5.23 (q, *J* = 7.3 Hz, 1H), 4.77 (d, *J* = 5.6 Hz, 1H), 3.91 (s, 3H), 3.58 (s, 1H), 3.30 - 3.15 (m, 2H), 2.83 (d, *J* = 10.0 Hz, 1H), 2.64 (d, *J* = 10.9 Hz, 1H), 2.17 (dt, *J* = 42.9, 9.8 Hz, 2H), 1.74 (q, *J* = 12.9, 9.1 Hz, 2H), 1.50 (d, *J* = 7.0 Hz, 4H), 1.24 - 1.12 (m, 1H).

**Example 50: Preparation of Compound 50**

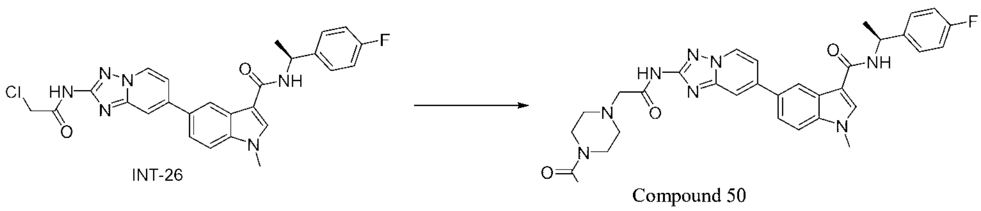

INT-26 Compound 50

Preparation of Compound 50:

Intermediate **INT-26** (101 mg, 0.2 mmol) was dissolved in acetonitrile (1 mL); 1-cyclopropanecarbonylpiperazine (61 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 50 with a yield of 58%. MS (ESI, positive ion) m/z:623.2 [M + H]+.

$^1$H NMR (400 MHz, DMSO-$d_6$) δ 10.58 (s, 1H), 8.89 (d, *J* = 7.1 Hz, 1H), 8.55 (d, *J* = 1.9 Hz, 1H), 8.38 (d, *J* = 8.0 Hz, 1H), 8.23 (s, 1H), 7.91 (d, *J* = 2.0 Hz, 1H), 7.74 (dd, *J* = 8.7, 1.9 Hz, 1H), 7.67 (d, *J* = 8.7 Hz, 1H), 7.50 - 7.45 (m, 3H), 7.20 - 7.14 (m, 2H), 5.22 (p, *J* = 7.2 Hz, 1H), 3.91 (s, 3H), 3.74 (s, 2H), 3.53 (s, 2H), 3.34 (s, 4H), 2.62 (s, 2H), 1.99 (tt, *J* = 7.8, 4.9 Hz, 1H), 1.50 (d, *J* = 7.0 Hz, 3H), 0.73 (ddt, *J* = 10.7, 4.8, 2.9 Hz, 4H).

**Example 51: Preparation of Compound 51**

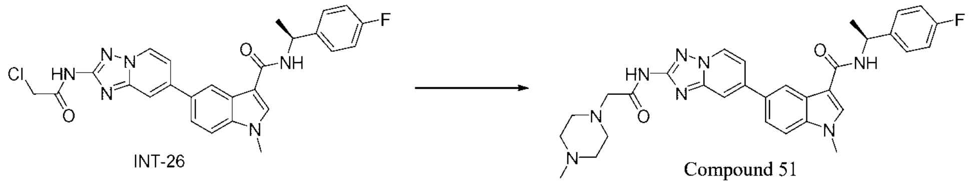

INT-26

Compound 51

Preparation of Compound 51:

Intermediate **INT-26** (101 mg, 0.2 mmol) was dissolved in acetonitrile (1 mL); 1-methylpiperazine (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 51 with a yield of 61%. MS (ESI, positive ion) m/z:569.2 $[M + H]^+$.

$^1H$ NMR (400 MHz, DMSO-$d_6$) δ 10.44 (s, 1H), 8.89 (d, *J* = 7.1 Hz, 1H), 8.55 (d, *J* = 1.8 Hz, 1H), 8.39 (d, *J* = 8.0 Hz, 1H), 8.23 (s, 1H), 7.90 (d, *J* = 2.0 Hz, 1H), 7.74 (dd, *J* = 8.6, 1.9 Hz, 1H), 7.67 (d, *J* = 8.6 Hz, 1H), 7.48 (td, *J* = 5.6, 2.6 Hz, 3H), 7.17 (t, *J* = 8.9 Hz, 2H), 5.23 (p, *J* = 7.1 Hz, 1H), 3.92 (s, 3H), 3.36 (s, 6H), 2.79 - 2.54 (m, 4H), 2.38 (s, 3H), 1.50 (d, *J* = 7.1 Hz, 3H).

**Example 52: Preparation of Compound 52**

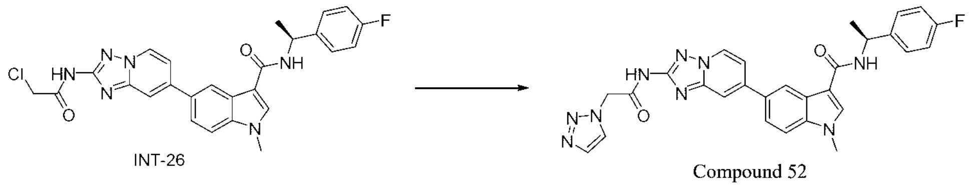

INT-26

Compound 52

Preparation of Compound 52:

Intermediate **INT-26** (101 mg, 0.2 mmol) was dissolved in acetonitrile (1 mL); 1,2,3-triazole (27 mg, 0.4 mmol) and potassium carbonate (55 mg, 0.4 mmol) were added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 52 with a yield of 41%. MS (ESI, positive ion) m/z:538.2 $[M + H]^+$.

$^1H$ NMR (400 MHz, DMSO-$d_6$) δ10.54 (s, 1H), 8.91 (d, *J* = 7.2 Hz, 1H), 8.56 (d, *J* = 1.9 Hz, 1H), 8.38 (d, *J* = 8.0 Hz, 1H), 8.23 (s, 1H), 8.19 (s, 1H), 7.97 - 7.93 (m, 1H), 7.79 (d, *J* = 9.1 Hz, 1H), 7.68 (d, *J* = 8.6 Hz, 1H), 7.47 (dd, *J* = 8.5, 5.7 Hz, 3H), 7.17 (dd, *J* = 10.1, 7.7 Hz, 2H), 5.22 (t, *J* = 7.3 Hz, 1H), 3.92 (s, 3H), 2.56 (s, 2H), 1.50 (d, *J* = 7.0 Hz, 3H), 0.97 (d, *J* = 6.5 Hz, 1H).

**Example 53: Preparation of Compound 53**

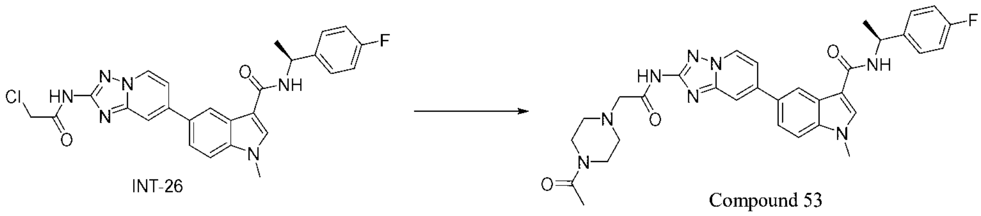

INT-26

Compound 53

Preparation of Compound 53:

Intermediate **INT-26** (101 mg, 0.2 mmol) was dissolved in acetonitrile (1 mL); 1-acetylpiperazine (51 mg, 0.4 mmol) was added, and the mixture was heated to 80°C and stirred for 6 h. The reaction mixture was concentrated under

reduced pressure and purified by C-18 reverse phase silica gel column (acetonitrile/0.1% aqueous ammonium bicarbonate solution = 60%) to obtain Compound 53 with a yield of 58%. MS (ESI, positive ion) m/z:597.2 $[M + H]^+$.

$^1$H NMR (400 MHz, DMSO-$d_6$) δ 10.54 (s, 1H),8.89 (d, *J* = 7.1 Hz, 1H), 8.55 (d, *J* = 1.8 Hz, 1H), 8.39 (d, *J* = 8.0 Hz, 1H), 8.23 (s, 1H), 7.91 (d, *J* = 2.0 Hz, 1H), 7.74 (dd, *J* = 8.7, 1.9 Hz, 1H), 7.68 (d, *J* = 8.6 Hz, 1H), 7.50 - 7.45 (m, 3H), 7.20 - 7.15 (m, 2H), 5.22 (p, *J* = 7.2 Hz, 1H), 3.92 (s, 3H), 3.50 (q, *J* = 5.1 Hz, 4H), 3.32 (s, 4H), 2.58 (t, *J* = 5.0 Hz, 2H), 2.02 (s, 3H), 1.50 (d, *J* = 7.0 Hz, 3H).

**Example 54: Preparation of Compound 54**

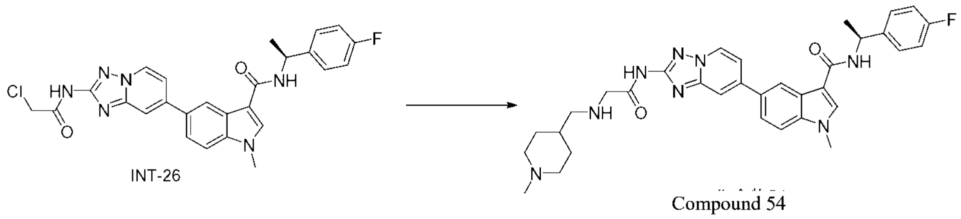

INT-26

Compound 54

Preparation of Compound 54:

Intermediate **INT-26** (101 mg, 0.2 mmol) was dissolved in acetonitrile (1 mL); (1-methylpiperidin-4-yl)methanamine (51 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 54 with a yield of 54%. MS (ESI, positive ion) m/z:597.2 $[M + H]^+$.

$^1$H NMR (400 MHz, DMSO-$d_6$) δ 10.53 (s, 1H), 8.88 (d, *J* = 7.1 Hz, 1H), 8.55 (d, *J* = 1.9 Hz, 1H), 8.38 (d, *J* = 8.1 Hz, 1H), 8.22 (s, 1H), 7.90 (d, *J* = 1.9 Hz, 1H), 7.76 (dd, *J* = 8.6, 1.8 Hz, 1H), 7.67 (d, *J* = 8.7 Hz, 1H), 7.47 (ddt, *J* = 5.8, 4.1, 1.7 Hz, 3H), 7.22 - 7.12 (m, 2H), 5.22 (p, *J* = 7.2 Hz, 1H),4.32 (m, 1H), 3.91 (s, 3H), 3.64 (t, *J* = 4.6 Hz, 4H), 3.5 (s, 3H), 3.27 (s, 2H), 3.10(m, 2H) 1.50 (d, *J* = 7.0 Hz, 3H).1.23 (m, 4H), 1.15 (m, 1H).

**Example 55: Preparation of Compound 55**

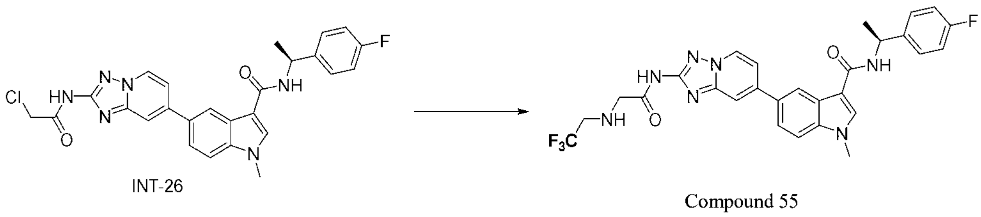

INT-26

Compound 55

Preparation of Compound 55:

Intermediate **INT-26** (101 mg, 0.2 mmol) was dissolved in acetonitrile (1 mL); trifluoroethylamine (39 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 55 with a yield of 54%. MS (ESI, positive ion) m/z:568.2 $[M + H]^+$.

$^1$H NMR (400 MHz, DMSO-$d_6$) δ 10.74 (s, 1H), 8.91 - 8.87 (m, 1H), 8.55 (d, *J* = 1.9 Hz, 1H), 8.38 (d, *J* = 8.0 Hz, 1H), 8.23 (s, 1H), 7.92 - 7.89 (m, 1H), 7.75 (dd, *J* = 8.7, 1.9 Hz, 1H), 7.67 (d, *J* = 8.7 Hz, 1H), 7.47 (td, *J* = 5.5, 2.5 Hz, 3H), 7.19 - 7.15 (m, 2H), 5.22 (t, *J* = 7.3 Hz, 1H), 3.91 (s, 3H), 3.61 (s, 1H), 3.40 (td, *J* = 10.1, 6.5 Hz, 2H), 1.50 (d, *J* = 7.1 Hz, 3H), 0.97 (d, *J* = 6.5 Hz, 2H).

**Example 56: Preparation of Compound 56**

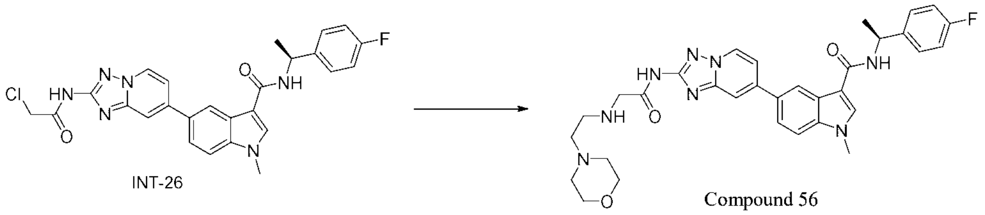

INT-26 Compound 56

Preparation of Compound 56:

Intermediate **INT-26** (101 mg, 0.2 mmol) was dissolved in acetonitrile (1 mL); N-(2-aminoethyl)morpholine (52 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 56 with a yield of 64%. MS (ESI, positive ion) m/z:599.2 $[M + H]^+$.

$^1$H NMR (400 MHz, DMSO-$d_6$) δ 8.89 (d, *J* = 7.1 Hz, 1H), 8.56 (d, *J* = 1.8 Hz, 1H), 8.37 (d, *J* = 8.0 Hz, 1H), 8.23 (s, 1H), 7.90 (d, *J* = 2.0 Hz, 1H), 7.75 (dd, *J* = 8.6, 1.9 Hz, 1H), 7.67 (d, *J* = 8.6 Hz, 1H), 7.48 (td, *J* = 5.4, 2.4 Hz, 3H), 7.17 (t, *J* = 8.8 Hz, 2H), 5.23 (p, *J* = 7.2 Hz, 1H), 3.92 (s, 3H), 3.60 (t, *J* = 4.7 Hz, 4H), 3.47 (s, 2H), 2.70 (t, *J* = 6.1 Hz, 2H), 2.41 (dt, *J* = 10.4, 5.4 Hz, 6H), 1.51 (d, *J* = 7.0 Hz, 3H).

**Example 57: Preparation of Compound 57**

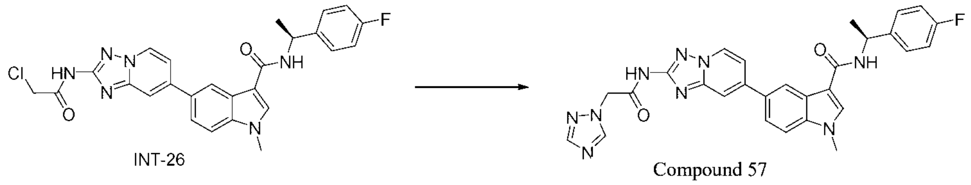

INT-26 Compound 57

Preparation of Compound 57:

Intermediate **INT-26** (101 mg, 0.2 mmol) was dissolved in acetonitrile (1 mL); 1,2,4-triazole (27 mg, 0.4 mmol) and potassium carbonate (55 mg, 0.4 mmol) were added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 57 with a yield of 41%. MS (ESI, positive ion) m/z:538.2 $[M + H]^+$.

$^1$H NMR (400 MHz, DMSO-$d_6$) δ 8.89 (d, *J* = 7.2 Hz, 1H), 8.55 (d, *J* = 1.9 Hz, 1H), 8.52 (s, 1H), 8.40 (d, *J* = 8.0 Hz, 1H), 8.22 (s, 1H), 7.93 (d, *J* = 1.9 Hz, 1H), 7.75 (dd, *J* = 8.7, 1.9 Hz, 1H), 7.67 (d, *J* = 8.6 Hz, 1H), 7.47 (dt, *J* = 8.5, 4.0 Hz, 3H), 7.19 - 7.14 (m, 2H), 5.78 (p, *J* = 7.1 Hz, 2H), 5.21 (t, *J* = 7.3 Hz, 1H), 3.91 (s, 3H), 2.56 (s, 2H), 1.50 (d, *J* = 7.0 Hz, 3H).

**Example 58: Preparation of Compound 58**

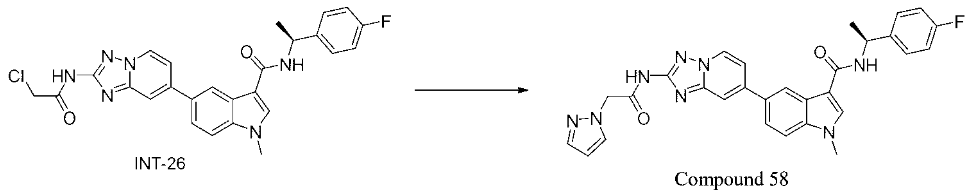

INT-26 Compound 58

Preparation of Compound 58:

Intermediate **INT-26** (101 mg, 0.2 mmol) was dissolved in acetonitrile (1 mL); pyrazole (27 mg, 0.4 mmol) and potassium carbonate (55 mg, 0.4 mmol) were added, and the mixture was heated to 80°C and stirred for 6 h. The reaction mixture was concentrated under reduced pressure and purified by C-18 reverse phase silica gel column (acetonitrile/0.1%

aqueous ammonium bicarbonate solution = 60%) to obtain Compound 58 with a yield of 57%. MS (ESI, positive ion) m/z:537.2 [M + H]$^+$.

$^1$H NMR (400 MHz, DMSO-$d_6$) δ 11.24 (d, *J* = 13.2 Hz, 1H), 8.91 (dd, *J* = 7.0, 5.4 Hz, 1H), 8.56 (d, *J* = 1.9 Hz, 1H), 8.38 (d, *J* = 8.0 Hz, 1H), 8.23 (s, 1H), 7.96 - 7.91 (m, 1H), 7.83 - 7.72 (m, 2H), 7.68 (d, *J* = 8.6 Hz, 1H), 7.53 - 7.43 (m, 3H), 7.23 - 7.12 (m, 2H),5.79 (p, *J* = 7.1 Hz, 2H), 5.22 (p, *J* = 7.1 Hz, 1H), 3.92 (s, 3H), 2.55 (s, 2H), 1.50 (d, *J* = 7.0 Hz, 3H).

**Example 59: Preparation of Compound 59**

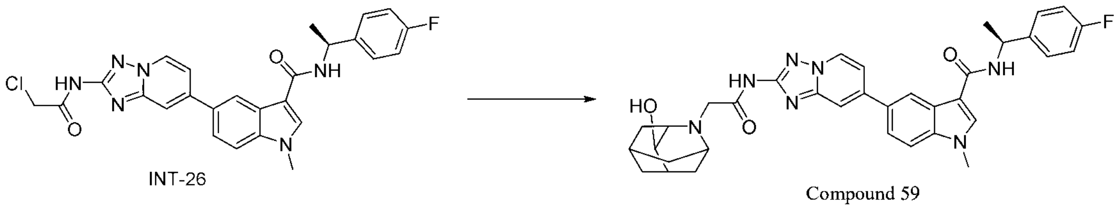

INT-26

Compound 59

Preparation of Compound 59:

Intermediate **INT-26** (101 mg, 0.2 mmol) was dissolved in acetonitrile (1 mL); 3-amino-1-adamantanol (61 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 59 with a yield of 34%. MS (ESI, positive ion) m/z:622.2 [M + H]$^+$.

$^1$H NMR (400 MHz, DMSO-$d_6$) δ10.44 (s, 1H), 8.89 (d, *J* = 7.1 Hz, 1H), 8.55 (d, *J* = 1.8 Hz, 1H), 8.38 (d, *J* = 8.0 Hz, 1H), 8.23 (s, 1H), 7.90 (d, *J* = 2.0 Hz, 1H), 7.74 (dd, *J* = 8.7, 1.9 Hz, 1H), 7.67 (d, *J* = 8.7 Hz, 1H), 7.47 (td, *J* = 5.3, 2.4 Hz, 3H), 7.22 - 7.12 (m, 2H), 5.22 (p, *J* = 7.2 Hz, 1H), 4.47 (s, 1H), 3.91 (s, 3H), 3.35 (dd, *J* = 7.1, 3.7 Hz, 3H),2.16 (d, *J* = 4.4 Hz, 2H), 1.55 - 1.47 (m, 13H), 1.44 (d, *J* = 3.4 Hz, 2H).

**Example 60: Preparation of Compound 60**

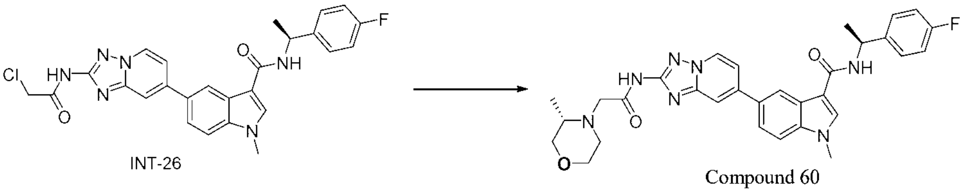

INT-26

Compound 60

Preparation of Compound 60:

Intermediate **INT-26** (101 mg, 0.2 mmol) was dissolved in acetonitrile (1 mL), and (S)-3-methylmorpholine and cis-2,6-dimethylmorpholine (40 mg, 0.4 mmol) were added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 60 with a yield of 71%. MS (ESI, positive ion) m/z:570.2 [M + H]$^+$.

$^1$H NMR (400 MHz, DMSO-$d_6$) δ 10.43 (s, 1H), 8.89 (d, *J* = 7.1 Hz, 1H), 8.55 (d, *J* = 1.8 Hz, 1H), 8.37 (d, *J* = 8.0 Hz, 1H), 8.23 (s, 1H), 7.90 (d, *J* = 1.9 Hz, 1H), 7.74 (dd, *J* = 8.7, 1.9 Hz, 1H), 7.67 (d, *J* = 8.6 Hz, 1H), 7.50 - 7.45 (m, 3H), 7.22 - 7.12 (m, 2H), 5.23 (p, *J* = 7.2 Hz, 1H), 3.92 (s, 3H), 3.73 (dt, *J* = 11.3, 3.0 Hz, 1H), 3.66 (dd, *J* = 11.1, 3.1 Hz, 1H), 3.57 (td, *J* = 10.7, 2.5 Hz, 1H), 3.50 (d, *J* = 16.3 Hz, 1H), 3.28 - 3.12 (m, 2H), 2.80 (dt, *J* = 11.6, 2.7 Hz, 1H), 2.68 - 2.54 (m, 2H), 1.51 (d, *J* = 7.0 Hz, 3H), 0.97 (d, *J* = 6.3 Hz, 3H).

**Example 61: Preparation of Compound 61**

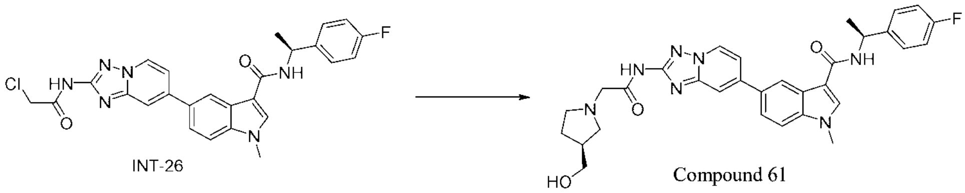

INT-26

Compound 61

Preparation of Compound 61:

Intermediate **INT-26** (101 mg, 0.2 mmol) was dissolved in acetonitrile (1 mL); (S)-pyrrolidin-3-ylmethanol (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 61 with a yield of 58%. MS (ESI, positive ion) m/z:570.2 $[M + H]^+$.

$^1H$ NMR (400 MHz, DMSO-$d_6$) δ 10.44 (s, 1H), 8.89 (d, $J$ = 7.1 Hz, 1H), 8.55 (d, $J$ = 1.8 Hz, 1H), 8.39 (d, $J$ = 8.0 Hz, 1H), 8.23 (s, 1H), 7.90 (d, $J$ = 1.9 Hz, 1H), 7.74 (dd, $J$ = 8.7, 1.9 Hz, 1H), 7.67 (d, $J$ = 8.6 Hz, 1H), 7.47 (td, $J$ = 5.5, 2.4 Hz, 3H), 7.22 - 7.12 (m, 2H), 5.22 (p, $J$ = 7.1 Hz, 1H), 4.60 (s, 1H), 3.91 (s, 3H), 3.35 (dd, $J$ = 7.1, 3.7 Hz, 4H),2.74 (dd, $J$ = 9.1, 7.7 Hz, 1H), 2.64 (t, $J$ = 6.9 Hz, 2H), 2.45 (dd, $J$ = 9.1, 5.6 Hz, 1H), 2.30 - 2.25 (m, 1H), 1.93 - 1.75 (m, 1H), 1.50 (d, $J$ = 7.1 Hz, 3H), 1.44 (dt, $J$ = 12.9, 6.5 Hz, 1H).

**Example 62: Preparation of Compound 62**

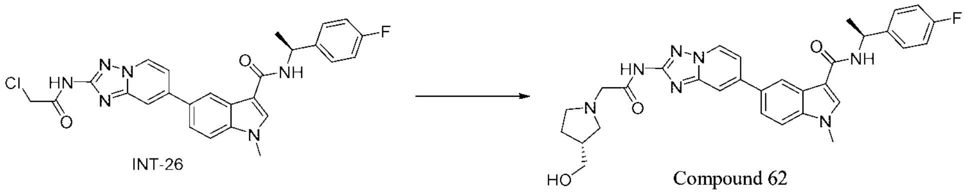

INT-26

Compound 62

Preparation of Compound 62:

Intermediate **INT-26** (101 mg, 0.2 mmol) was dissolved in acetonitrile (1 mL); (R)-pyrrolidin-3-ylmethanol (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 62 with a yield of 54%. MS (ESI, positive ion) m/z:570.2 $[M + H]^+$.

$^1H$ NMR (400 MHz, DMSO-$d_6$) δ 10.44 (s, 1H), 8.86 (d, $J$ = 7.1 Hz, 1H), 8.54 (d, $J$ = 1.8 Hz, 1H), 8.41 (d, $J$ = 8.0 Hz, 1H), 8.21 (s, 1H), 7.89 (d, $J$ = 1.9 Hz, 1H), 7.72 (dd, $J$ = 8.7, 1.8 Hz, 1H), 7.66 (d, $J$ = 8.6 Hz, 1H), 7.47 (td, $J$ = 6.9, 5.7, 2.2 Hz, 3H), 7.20 - 7.11 (m, 2H), 5.21 (p, $J$ = 7.2 Hz, 1H), 4.68 (s, 1H), 3.90 (s, 3H), 3.35 (dd, $J$ = 7.1, 3.7 Hz, 4H), 2.73 (dd, $J$ = 9.1, 7.8 Hz, 1H), 2.64 (t, $J$ = 6.9 Hz, 2H), 2.44 (dd, $J$ = 9.1, 5.7 Hz, 1H), 2.27 (t, $J$ = 7.8 Hz, 1H), 1.93 - 1.76 (m, 1H), 1.49 (d, $J$ = 7.0 Hz, 3H), 1.44 (dt, $J$ = 12.9, 6.4 Hz, 1H).

**Example 63: Preparation of Compound 63**

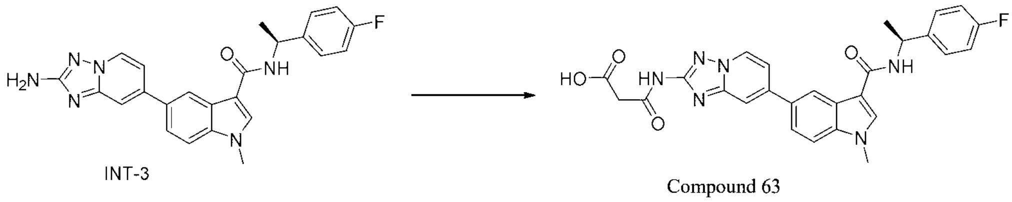

INT-3

Compound 63

Preparation of Compound 63:

Intermediate **INT-3** (85 mg, 0.2 mmol) was dissolved in pyridine (1 mL); 2-dimethylaminoacetic acid (31 mg, 0.3

mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 63 with a yield of 35%. MS (ESI, positive ion) m/z: 515.2 [M + H]$^+$.

$^1$H NMR (400 MHz, DMSO-$d_6$) δ 11.03 (s, 1H), 8.86 (d, *J* = 7.1 Hz, 1H), 8.55 (d, *J* = 1.9 Hz, 1H), 8.39 (d, *J* = 8.0 Hz, 1H), 8.23 (s, 1H), 7.89 (d, *J* = 2.0 Hz, 1H), 7.74 (dd, *J* = 8.6, 1.9 Hz, 1H), 7.67 (d, *J* = 8.6 Hz, 1H), 7.47 (qd, *J* = 6.9, 6.3, 2.1 Hz, 3H), 7.17 (t, *J* = 8.9 Hz, 2H), 5.22 (p, *J* = 7.2 Hz, 1H), 3.91 (s, 3H), 2.70 (s, 2H), 2.50 (s, 2H), 1.50 (d, *J* = 7.0 Hz, 3H).

**Example 64: Preparation of Compound 64**

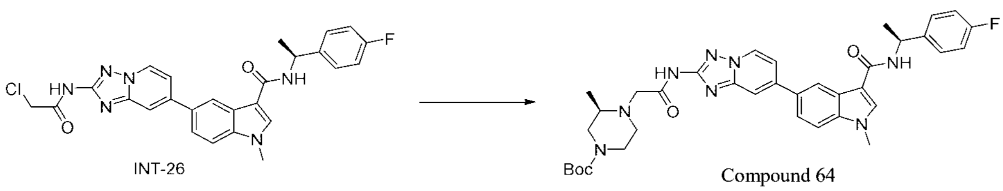

INT-26

Compound 64

Preparation of Compound 64:

Intermediate **INT-26** (101 mg, 0.2 mmol) was dissolved in acetonitrile (1 mL), and (R)-4-Boc-2-methylpiperazine (80 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 64 with a yield of 61%. MS (ESI, positive ion) m/z:669.2 [M + H]$^+$.

$^1$H NMR (400 MHz, DMSO-$d_6$) δ 10.45 (s, 1H), 8.88 (d, *J* = 7.1 Hz, 1H), 8.55 (d, *J* = 1.9 Hz, 1H), 8.36 (d, *J* = 8.1 Hz, 1H), 8.22 (s, 1H), 7.90 (d, *J* = 1.9 Hz, 1H), 7.74 (dd, *J* = 8.7, 1.9 Hz, 1H), 7.67 (d, *J* = 8.7 Hz, 1H), 7.49 - 7.45 (m, 3H), 7.17 (t, *J* = 8.9 Hz, 2H), 5.22 (p, *J* = 7.1 Hz, 1H), 3.91 (s, 3H), 3.67 (d, *J* = 13.0 Hz, 2H), 3.46 (d, *J* = 15.7 Hz, 1H), 3.09 (d, *J* = 11.9 Hz, 1H), 2.83 (d, *J* = 11.5 Hz, 1H), 2.62 (s, 1H), 1.50 (d, *J* = 7.1 Hz, 3H), 1.41 (d, *J* = 7.3 Hz, 12H), 1.03 (d, *J* = 6.2 Hz, 3H).

**Example 65: Preparation of Compound 65**

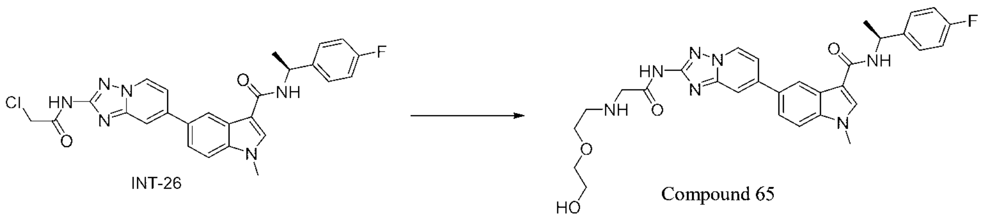

INT-26

Compound 65

Preparation of Compound 65:

Intermediate **INT-26** (101 mg, 0.2 mmol) was dissolved in acetonitrile (1 mL); diglycolamine (42 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 65 with a yield of 71%. MS (ESI, positive ion) m/z:574.2 [M + H]$^+$.

$^1$H NMR (400 MHz, DMSO-$d_6$) δ 8.92 (dd, *J* = 23.7, 7.1 Hz, 1H), 8.56 (d, *J* = 1.9 Hz, 1H), 8.37 (d, *J* = 8.0 Hz, 1H), 8.23 (s, 1H), 7.89 (t, *J* = 1.3 Hz, 1H), 7.75 (dd, *J* = 8.7, 2.0 Hz, 1H), 7.71 - 7.64 (m, 1H), 7.56 - 7.44 (m, 3H), 7.22 - 7.13 (m, 2H), 5.23 (p, *J* = 7.2 Hz, 1H), 3.92 (s, 3H), 3.58 - 3.43 (m, 8H), 2.75 (t, *J* = 5.5 Hz, 2H), 1.51 (d, *J* = 7.0 Hz, 3H).

**Example 66: Preparation of Compound 66**

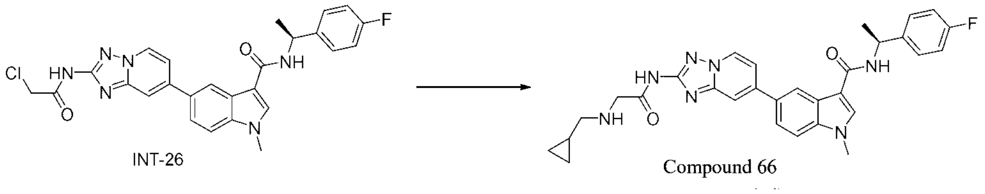


Preparation of Compound 66:

Intermediate **INT-26** (101 mg, 0.2 mmol) was dissolved in acetonitrile (1 mL); cyclopropylmethylamine (28 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 66 with a yield of 75%. MS (ESI, positive ion) m/z:540.2 $[M + H]^+$.

$^1H$ NMR (400 MHz, DMSO-$d_6$) δ10.43 (s, 1H), 8.93 (dd, *J* = 13.2, 7.1 Hz, 1H), 8.56 (dd, *J* = 4.8, 1.8 Hz, 1H), 8.48 (t, *J* = 8.1 Hz, 1H), 8.31 (d, *J* = 6.8 Hz, 1H), 7.97 (dd, *J* = 24.3, 1.9 Hz, 1H), 7.75 (dd, *J* = 8.7, 1.9 Hz, 1H), 7.68 (d, *J* = 8.7 Hz, 2H), 7.48 (d, *J* = 2.9 Hz, 2H), 7.21 - 7.12 (m, 2H), 5.20 (q, *J* = 7.3 Hz, 1H),4.44 (t, *J* = 5.4 Hz, 1H), 3.91 (s, 3H), 3.46 (s, 1H),2.88 (d, *J* = 7.3 Hz, 1H), 2.74 (t, *J* = 5.5 Hz, 2H),1.50 (d, *J* = 7.1 Hz, 3H), 1.17 - 0.85 (m, 1H), 0.63 - 0.16 (m, 4H).

**Example 67: Preparation of Compound 67**

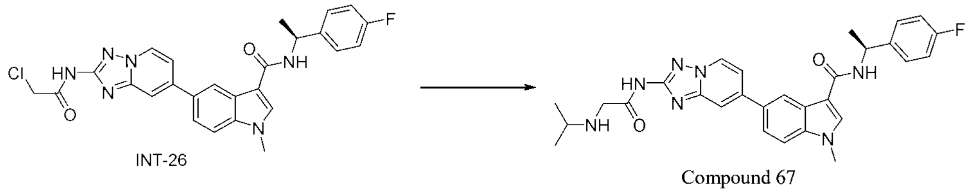


Preparation of Compound 67:

Intermediate **INT-26** (101 mg, 0.2 mmol) was dissolved in acetonitrile (1 mL); isopropylamine (23 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 67 with a yield of 65%. MS (ESI, positive ion) m/z:528.2 $[M + H]^+$.

$^1H$ NMR (400 MHz, DMSO-$d_6$) δ 8.91 (dd, *J* = 22.1, 7.1 Hz, 1H), 8.55 (d, *J* = 1.9 Hz, 1H), 8.36 (d, *J* = 8.0 Hz, 1H), 8.22 (s, 1H), 7.90 (d, *J* = 1.9 Hz, 1H), 7.74 (dd, *J* = 8.7, 1.9 Hz, 1H), 7.68 (d, *J* = 8.7 Hz, 1H), 7.47 (td, *J* = 5.5, 2.5 Hz, 3H), 7.22 - 7.13 (m, 2H), 5.24 (h, *J* = 7.3, 6.7 Hz, 1H), 3.92 (s, 3H), 3.51 - 3.39 (m, 2H), 2.78 (p, *J* = 6.2 Hz, 1H), 1.51 (d, *J* = 7.0 Hz, 3H), 1.04 (d, *J* = 6.2 Hz, 6H).

**Example 68: Preparation of Compound 68**

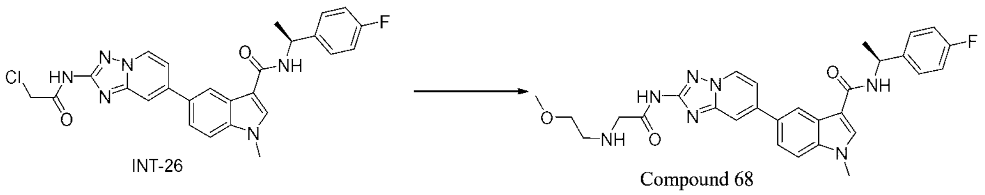


Preparation of Compound 68:

Intermediate **INT-26** (101 mg, 0.2 mmol) was dissolved in acetonitrile (1 mL); 2-methoxyethylamine (30 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 68 with a yield of 75%. MS (ESI, positive ion) m/z:544.2 [M + H]$^+$.

$^1$H NMR (400 MHz, DMSO-$d_6$) δ10.43 (s, 1H),8.89 (d, *J* = 7.2 Hz, 1H), 8.55 (d, *J* = 1.8 Hz, 1H), 8.38 (d, *J* = 7.9 Hz, 1H), 8.23 (s, 1H), 7.90 (d, *J* = 2.1 Hz, 1H), 7.73 (d, *J* = 1.9 Hz, 1H), 7.67 (d, *J* = 8.7 Hz, 1H), 7.48 (dd, *J* = 5.6, 3.2 Hz, 3H), 7.19 - 7.16 (m, 2H), 5.21 (d, *J* = 7.4 Hz, 1H), 4.44 (t, *J* = 5.4 Hz, 1H),3.91 (s, 3H), 3.46 (s, 1H),3.43 (t, *J* = 5.5 Hz, 3H), 3.27 - 3.24 (m, 3H), 2.74 (t, *J* = 5.5 Hz, 2H), 1.50 (d, *J* = 7.1 Hz, 3H).

**Example 69: Preparation of Compound 69**

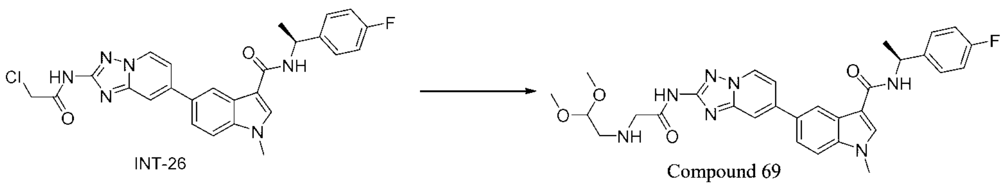

INT-26

Compound 69

Preparation of Compound 69:

Intermediate **INT-26** (101 mg, 0.2 mmol) was dissolved in acetonitrile (1 mL); aminoacetaldehyde dimethyl acetal (42 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 69 with a yield of 66%. MS (ESI, positive ion) m/z:574.2 [M + H]$^+$.

$^1$H NMR (400 MHz, DMSO-$d_6$) δ10.43 (s, 1H), 8.89 (d, *J* = 7.1 Hz, 1H), 8.55 (d, *J* = 1.9 Hz, 1H), 8.38 (d, *J* = 8.0 Hz, 1H), 8.23 (s, 1H), 7.90 (d, *J* = 2.0 Hz, 1H), 7.75 (dd, *J* = 8.7, 1.9 Hz, 1H), 7.68 (d, *J* = 8.6 Hz, 1H), 7.47 (td, *J* = 5.5, 2.5 Hz, 3H), 7.23 - 7.13 (m, 2H), 5.22 (p, *J* = 7.1 Hz, 1H), 4.44 (t, *J* = 5.4 Hz, 1H), 3.92 (s, 3H), 3.46 (s, 1H), 3.31 (s, 6H), 3.29 - 3.26 (m, 2H), 2.69 (d, *J* = 5.4 Hz, 2H), 1.50 (d, *J* = 7.1 Hz, 3H).

**Example 70: Preparation of Compound 70**

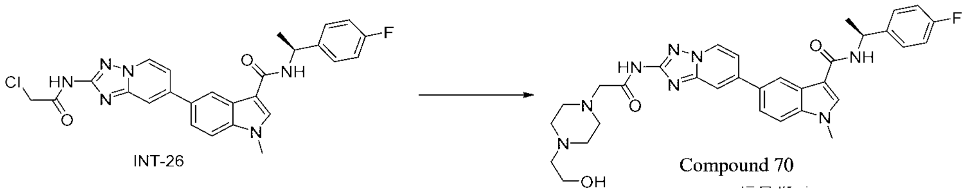

INT-26

Compound 70

Preparation of Compound 70:

Intermediate **INT-26** (101 mg, 0.2 mmol) was dissolved in acetonitrile (1 mL); N-hydroxyethylpiperazine (52 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 70 with a yield of 56%. MS (ESI, positive ion) m/z:599.2 [M + H]$^+$.

$^1$H NMR (400 MHz, DMSO-$d_6$) δ10.43 (s, 1H), 8.89 (d, *J* = 7.1 Hz, 1H), 8.55 (d, *J* = 1.8 Hz, 1H), 8.38 (d, *J* = 8.0 Hz, 1H), 8.23 (s, 1H), 7.90 (d, *J* = 2.0 Hz, 1H), 7.74 (dd, *J* = 8.6, 1.9 Hz, 1H), 7.67 (d, *J* = 8.6 Hz, 1H), 7.51 - 7.42 (m, 3H), 7.22 - 7.12 (m, 2H), 5.22 (p, *J* = 7.1 Hz, 1H), 4.39 (t, *J* = 5.4 Hz, 1H), 3.91 (s, 3H), 3.51 (q, *J* = 6.1 Hz, 2H), 3.23 (s, 2H), 2.56 (s, 3H), 2.47 (d, *J* = 7.7 Hz, 3H), 2.40 (t, *J* = 6.3 Hz, 2H), 1.50 (d, *J* = 7.0 Hz, 3H).

**Example 71: Preparation of Compound 71**

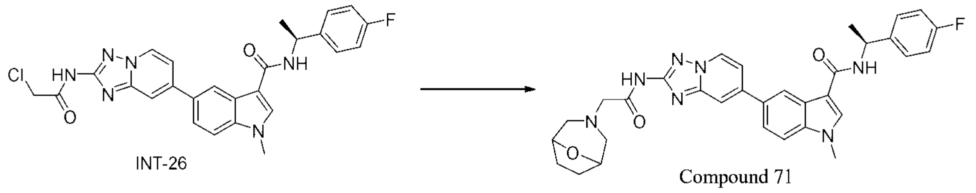

Preparation of Compound 71:

Intermediate **INT-26** (101 mg, 0.2 mmol) was dissolved in acetonitrile (1 mL); 8-oxa-3-azabicyclo[3.2.1]octane hydrochloride (45 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 71 with a yield of 56%. MS (ESI, positive ion) m/z:582.2 $[M + H]^+$.

$^1H$ NMR (400 MHz, DMSO-$d_6$) δ 10.41 (s, 1H), 8.93 - 8.86 (m, 1H), 8.55 (d, *J* = 1.9 Hz, 1H), 8.38 (d, *J* = 8.0 Hz, 1H), 8.23 (s, 1H), 7.92 (dd, *J* = 2.0, 0.9 Hz, 1H), 7.75 (dd, *J* = 8.7, 1.9 Hz, 1H), 7.68 (d, *J* = 8.6 Hz, 1H), 7.49 - 7.45 (m, 3H), 7.20 - 7.15 (m, 2H), 5.22 (p, *J* = 7.1 Hz, 1H), 4.25 (s, 2H), 3.92 (s, 4H), 3.22 (s, 2H), 2.64 (d, *J* = 10.7 Hz, 2H), 2.47 (dd, *J* = 11.1, 2.1 Hz, 3H), 1.77 (s, 2H), 1.50 (d, *J* = 7.1 Hz, 3H).

**Example 72: Preparation of Compound 72**

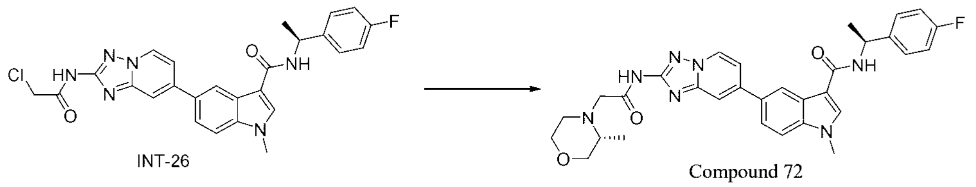

Preparation of Compound 72:

Intermediate **INT-26** (101 mg, 0.2 mmol) was dissolved in acetonitrile (1 mL); (R)-3-methylmorpholine (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 72 with a yield of 76%. MS (ESI, positive ion) m/z:570.2 $[M + H]^+$.

$^1H$ NMR (400 MHz, DMSO-$d_6$) δ 10.43 (s, 1H), 8.89 (dd, *J*=7.1, 0.8 Hz, 1H), 8.55 (d, *J* = 1.8 Hz, 1H), 8.37 (d, *J*= 8.0 Hz, 1H), 8.23 (s, 1H), 7.90 (dd, *J*= 1.9, 0.9 Hz, 1H), 7.74 (dd, *J*= 8.7, 1.9 Hz, 1H), 7.67 (d, *J* = 8.7 Hz, 1H), 7.52 - 7.43 (m, 3H), 7.22 - 7.12 (m, 2H), 5.23 (p, *J* = 7.2 Hz, 1H), 3.91 (s, 3H), 3.73 (dt, *J* = 11.2, 3.1 Hz, 1H), 3.66 (dd, *J* = 11.0, 3.1 Hz, 1H), 3.62 - 3.53 (m, 1H), 3.53 - 3.42 (m, 1H), 3.28 - 3.12 (m, 2H), 2.80 (dt, *J* = 11.7, 2.7 Hz, 1H), 2.70 - 2.54 (m, 2H), 1.50 (d, *J* = 7.1 Hz, 3H), 0.97 (d, *J*= 6.3 Hz, 3H).

**Example 73: Preparation of Compound 73**

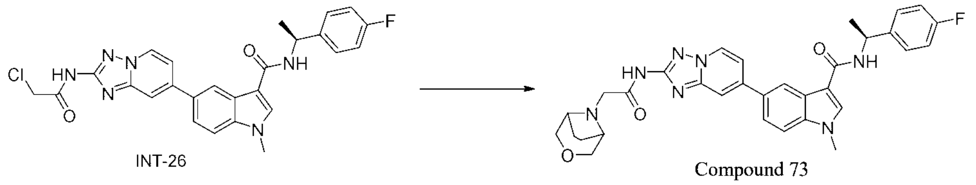

Preparation of Compound 73:

Intermediate **INT-26** (101 mg, 0.2 mmol) was dissolved in acetonitrile (1 mL); nortropane (45 mg, 0.4 mmol) was added, and the mixture was heated to 80°C and stirred for 6 h. The reaction mixture was concentrated under reduced

pressure and purified by C-18 reverse phase silica gel column (acetonitrile/0.1% aqueous ammonium bicarbonate solution = 60%) to obtain Compound 73 with a yield of 65%. MS (ESI, positive ion) m/z:582.2 $[M + H]^+$.

$^1H$ NMR (400 MHz, DMSO-$d_6$) δ 10.45 (s, 1H), 8.90 (d, *J*= 7.2 Hz, 1H), 8.55 (d, *J*= 1.9 Hz, 1H), 8.38 (d, *J* = 8.0 Hz, 1H), 8.22 (s, 1H), 7.91 (d, *J* = 2.0 Hz, 1H), 7.74 (dd, *J* = 8.7, 1.9 Hz, 1H), 7.68 (d, *J*= 8.6 Hz, 1H), 7.48 (dd, *J*= 6.5, 2.7 Hz, 3H), 7.19 - 7.15 (m, 2H), 5.22 (t, *J* = 7.3 Hz, 1H), 3.91 (s, 4H), 3.69 (d, *J* = 10.3 Hz, 2H), 3.49 (dd, *J* = 10.7, 2.1 Hz, 2H), 3.15 (s, 3H), 1.90 (dd, *J* = 9.0, 4.6 Hz, 2H), 1.82 - 1.76 (m, 2H), 1.50 (d, *J* = 7.0 Hz, 3H).

**Example 74: Preparation of Compound 74**

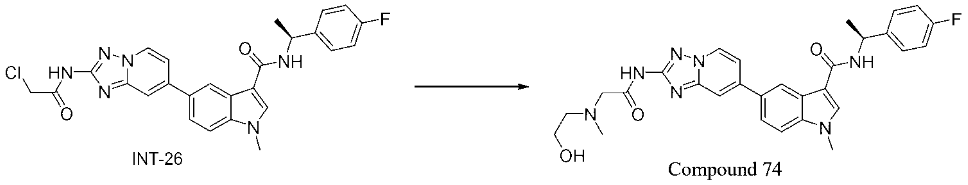

INT-26

Compound 74

Preparation of Compound 74:

Intermediate **INT-26** (101 mg, 0.2 mmol) was dissolved in acetonitrile (1 mL); N-methyl-2-hydroxyethylamine (30 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 74 with a yield of 55%. MS (ESI, positive ion) m/z:544.2 $[M + H]^+$.

$^1H$ NMR (400 MHz, DMSO-$d_6$) δ 10.51 (s, 1H), 8.89 (d, *J* = 7.1 Hz, 1H), 8.60 - 8.52 (m, 1H), 8.37 (t, *J* = 7.5 Hz, 1H), 8.22 (d, *J* = 7.4 Hz, 1H), 7.90 (d, *J* = 1.9 Hz, 1H), 7.74 (dd, *J* = 8.7, 1.9 Hz, 1H), 7.71 - 7.62 (m, 1H), 7.51 - 7.43 (m, 3H), 7.23 - 7.12 (m, 2H), 5.22 (p, *J* = 7.3 Hz, 1H), 4.71 (s, 1H), 3.91 (d, *J* = 4.8 Hz, 3H), 3.56 - 3.42 (m, 3H), 2.63 - 2.53 (m, 2H), 2.35 (s, 3H), 2.30 (s, 1H), 1.50 (d, *J* = 7.1 Hz, 3H).

**Example 75: Preparation of Compound 75**

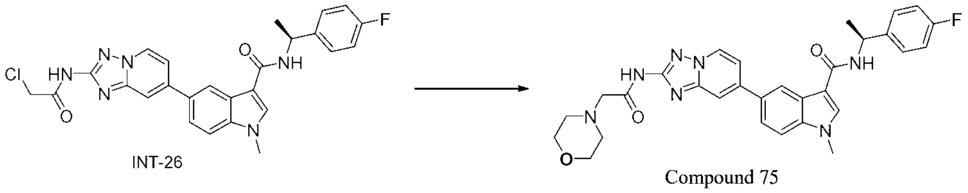

INT-26

Compound 75

Preparation of Compound 75:

Intermediate **INT-26** (101 mg, 0.2 mmol) was dissolved in acetonitrile (1 mL); morpholine (35 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 75 with a yield of 85%. MS (ESI, positive ion) m/z:556.2 $[M + H]^+$.

$^1H$ NMR (400 MHz, DMSO-$d_6$) δ 10.54 (s, 1H), 8.89 (d, *J* = 7.1 Hz, 1H), 8.55 (d, *J* = 1.9 Hz, 1H), 8.38 (d, *J* = 8.1 Hz, 1H), 8.23 (s, 1H), 7.90 (d, *J* = 1.9 Hz, 1H), 7.74 (dd, *J* = 8.6, 1.8 Hz, 1H), 7.67 (d, *J* = 8.7 Hz, 1H), 7.47 (ddt, *J* = 5.8, 4.1, 1.7 Hz, 3H), 7.22 - 7.12 (m, 2H), 5.22 (p, *J* = 7.2 Hz, 1H), 3.91 (s, 3H), 3.64 (t, *J* = 4.6 Hz, 4H), 3.27 (s, 2H), 2.56 (dd, *J* = 5.4, 3.4 Hz, 4H), 1.50 (d, *J* = 7.0 Hz, 3H).

**Example 76: Preparation of Compound 76**

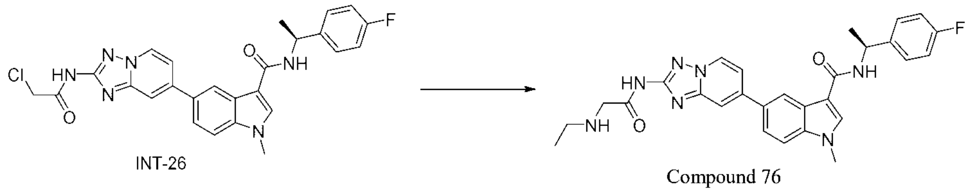

Preparation of Compound 76:

Intermediate **INT-26** (101 mg, 0.2 mmol) was dissolved in acetonitrile (1 mL); ethylamine hydrochloride (32 mg, 0.4 mmol) and DIPEA (103 mg, 0.8 mmol) were added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 76 with a yield of 68%. MS (ESI, positive ion) m/z:514.2 $[M + H]^+$.

$^1H$ NMR (400 MHz, DMSO-$d_6$) δ 10.54 (s, 1H), 8.89 (d, *J* = 7.1 Hz, 1H), 8.55 (d, *J* = 1.9 Hz, 1H), 8.38 (d, *J* = 8.1 Hz, 1H), 8.23 (s, 1H), 7.90 (d, *J* = 1.9 Hz, 1H), 7.74 (dd, *J* = 8.6, 1.8 Hz, 1H), 7.67 (d, *J* = 8.7 Hz, 1H), 7.47 (ddt, *J* = 5.8, 4.1, 1.7 Hz, 3H), 7.22 - 7.12 (m, 2H), 4.97 (m, 1H), 3.91 (s, 3H), 3.44 (s, 1H), 3.22 (s, 2H), 2.63 (dd, *J* = 6.6, 1.8 Hz, 2H), 1.56 (d, *J* = 5.4 Hz, 3H), 1.10 (dd, *J* = 7.6, 2.8 Hz, 3H).

**Example 77: Preparation of Compound 77**

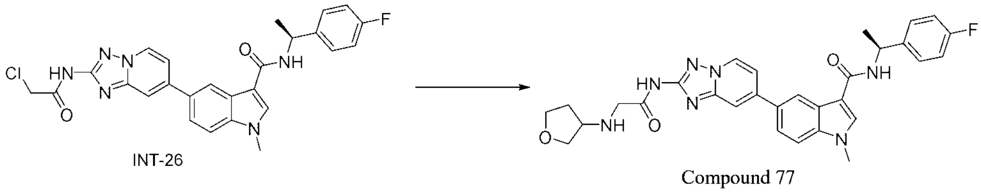

Preparation of Compound 77:

Intermediate **INT-26** (101 mg, 0.2 mmol) was dissolved in acetonitrile (1 mL); 3-aminotetrahydrofuran (35 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 77 with a yield of 61%. MS (ESI, positive ion) m/z:556.2 $[M + H]^+$.

$^1H$ NMR (400 MHz, DMSO-$d_6$) δ 10.54 (s, 1H), 8.89 (d, *J* = 7.1 Hz, 1H), 8.55 (d, *J* = 1.9 Hz, 1H), 8.38 (d, *J* = 8.1 Hz, 1H), 8.23 (s, 1H), 7.90 (d, *J* = 1.9 Hz, 1H), 7.74 (dd, *J* = 8.6, 1.8 Hz, 1H), 7.67 (d, *J* = 8.7 Hz, 1H), 7.47 (ddt, *J* = 5.8, 4.1, 1.7 Hz, 3H), 7.22 - 7.12 (m, 2H), 5.13 (s, 1H), 4.97 (m, 1H), 3.91-3.75 (m, 7H), 3.01-2.95 (m, 1H), 2.63 (dd, *J* = 6.6, 1.8 Hz, 2H), 2.06-1.83 (m, 2H), 1.56 (d, *J* = 5.4 Hz, 3H).

**Example 78: Preparation of Compound 78**

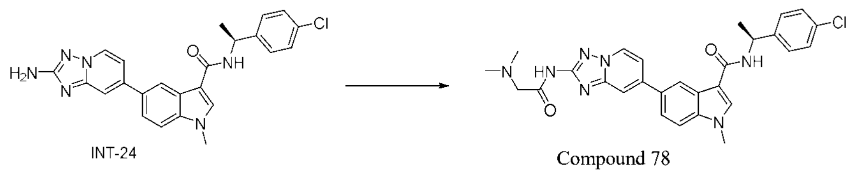

Preparation of Compound 78:

Intermediate **INT-24** (89 mg, 0.2 mmol) was dissolved in pyridine (1 mL); 2-dimethylaminoacetic acid (31 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel

column (acetonitrile/0.1% aqueous ammonium bicarbonate solution = 60%) to obtain Compound 78 with a yield of 49%. MS (ESI, positive ion) m/z: 530.2 [M + H]$^+$.

$^1$H NMR (400 MHz, DMSO-$d_6$) δ 10.44 (s, 1H), 8.88 (dd, *J* = 7.1, 0.8 Hz, 1H), 8.55 (d, *J* = 1.8 Hz, 1H), 8.39 (d, *J* = 7.9 Hz, 1H), 8.23 (s, 1H), 7.90 (dd, *J* = 2.0, 0.9 Hz, 1H), 7.74 (dd, *J* = 8.7, 1.9 Hz, 1H), 7.67 (d, *J*= 8.6 Hz, 1H), 7.50 - 7.45 (m, 3H), 7.43 - 7.39 (m, 2H), 5.21 (p, *J* = 7.1 Hz, 1H), 3.92 (s, 3H), 3.21 (s, 2H), 2.33 (s, 6H), 1.50 (d, *J*= 7.1 Hz, 3H).

**Example 79: Preparation of Compound 79**

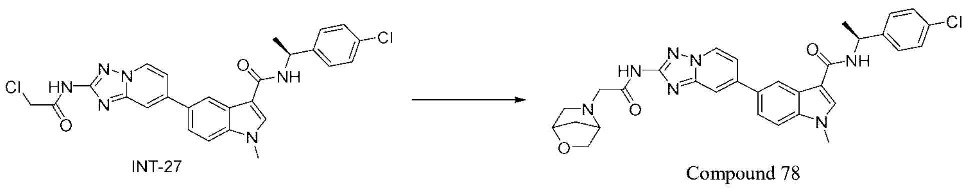

INT-27

Compound 78

Preparation of Compound 79:

Intermediate **INT-27** (104 mg, 0.2 mmol) was dissolved in acetonitrile (1 mL), and 2-oxa-5-azabicyclo[2.2.1] heptane (39 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 79 with a yield of 71%. MS (ESI, positive ion) m/z:584.2 [M + H]$^+$.

$^1$H NMR (400 MHz, DMSO-$d_6$) δ 10.39 (s, 1H), 8.88 (d, *J*=7.1 Hz, 1H), 8.55 (d, *J*= 1.8 Hz, 1H), 8.39 (d, *J* = 7.9 Hz, 1H), 8.23 (s, 1H), 7.90 (d, *J* = 1.9 Hz, 1H), 7.74 (dd, *J* = 8.6, 1.9 Hz, 1H), 7.67 (d, *J*= 8.6 Hz, 1H), 7.49 - 7.45 (m, 3H), 7.42 - 7.39 (m, 2H), 5.26 - 5.15 (m, 1H), 4.39 (t, *J* = 2.1 Hz, 1H), 3.90 (d, *J* = 11.3 Hz, 4H), 3.62 (s, 1H), 3.58 (dd, *J* = 7.7, 1.8 Hz, 1H), 3.48 (d, *J* = 5.2 Hz, 2H), 2.96 (dd, *J* = 9.9, 1.7 Hz, 1H), 2.57 (d, *J* = 10.0 Hz, 1H), 1.87 (dd, *J* = 9.7, 2.2 Hz, 1H), 1.68 - 1.61 (m, 1H), 1.50 (d, *J* = 7.1 Hz, 3H).

**Example 80: Preparation of Compound 80**

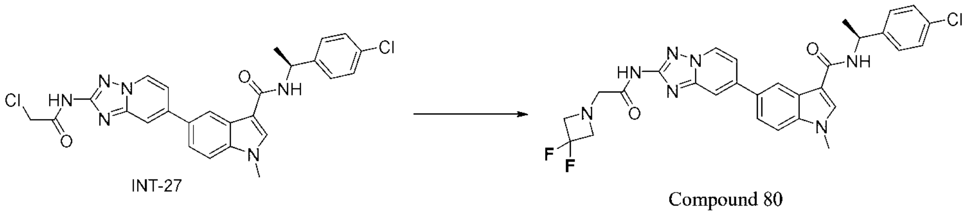

INT-27

Compound 80

Preparation of Compound 80:

Intermediate **INT-27** (104 mg, 0.2 mmol) was dissolved in acetonitrile (1 mL); 3,3-difluoroazetidine (37 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 80 with a yield of 65%. MS (ESI, positive ion) m/z:578.2 [M + H]$^+$.

$^1$H NMR (400 MHz, DMSO) δ 10.77 (s, 1H), 8.89 (t, *J* = 10.7 Hz, 1H), 8.55 (s, 1H), 8.41 (d, *J* = 7.9 Hz, 1H), 8.24 (s, 1H), 7.91 (d, *J* = 11.7 Hz, 1H), 7.79 - 7.64 (m, 2H), 7.47 (dd, *J* = 9.4, 5.0 Hz, 3H), 7.41 (d, *J* = 8.5 Hz, 2H), 5.20 (p, *J* = 7.0 Hz, 1H), 3.92 (s, 3H), 3.80 (t, *J* = 12.5 Hz, 4H), 3.61 (s, 2H), 1.50 (d, *J* = 7.0 Hz, 3H).

**Example 81: Preparation of Compound 81**

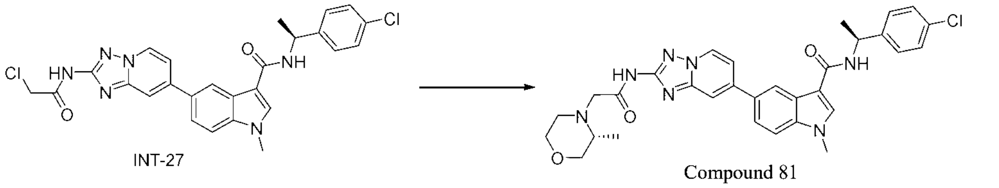

Compound 81

Preparation of Compound 81:

Intermediate **INT-27** (104 mg, 0.2 mmol) was dissolved in acetonitrile (1 mL); (R)-3-methylmorpholine (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 81 with a yield of 73%. MS (ESI, positive ion) m/z:586.2 $[M + H]^+$.

$^1$H NMR (400 MHz, DMSO-$d_6$) δ 10.43 (s, 1H), 8.89 (d, *J* = 7.1 Hz, 1H), 8.55 (d, *J* = 1.8 Hz, 1H), 8.39 (d, *J* = 7.9 Hz, 1H), 8.24 (s, 1H), 7.90 (d, *J* = 2.0 Hz, 1H), 7.74 (dd, *J* = 8.7, 1.9 Hz, 1H), 7.68 (d, *J* = 8.7 Hz, 1H), 7.49 - 7.44 (m, 3H), 7.43 - 7.38 (m, 2H), 5.25 - 5.17 (m, 1H), 3.92 (s, 3H), 3.76 - 3.70 (m, 1H), 3.66 (dd, *J* = 10.9, 3.1 Hz, 1H), 3.61 - 3.46 (m, 2H), 3.22 - 3.15 (m, 1H), 2.82 - 2.78 (m, 1H), 2.66 - 2.54 (m, 2H), 1.50 (d, *J* = 7.1 Hz, 3H), 0.97 (d, *J* = 6.3 Hz, 3H).

**Example 82: Preparation of Compound 82**

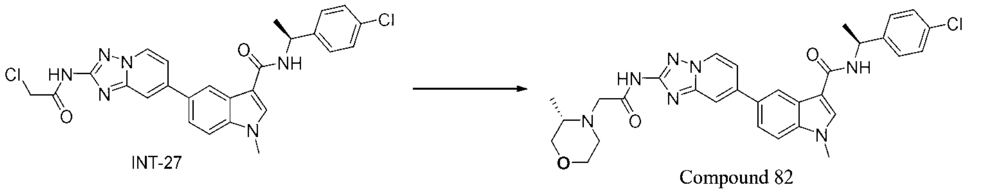

Compound 82

Preparation of Compound 82:

Intermediate **INT-27** (104 mg, 0.2 mmol) was dissolved in acetonitrile (1 mL), and (S)-3-methylmorpholine and cis-2,6-dimethylmorpholine (40 mg, 0.4 mmol) were added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 82 with a yield of 68%. MS (ESI, positive ion) m/z:586.2 $[M + H]^+$.

$^1$H NMR (400 MHz, DMSO) δ 10.45 (s, 1H), 8.89 (d, *J* = 7.1 Hz, 1H), 8.55 (s, 1H), 8.43 (t, *J* = 13.8 Hz, 1H), 8.24 (s, 1H), 7.90 (s, 1H), 7.71 (dd, *J* = 25.0, 8.6 Hz, 2H), 7.50 - 7.44 (m, 3H), 7.41 (d, *J* = 8.4 Hz, 2H), 5.20 (p, *J* = 6.9 Hz, 1H), 3.92 (s, 3H), 3.69 (dd, *J* = 27.9, 9.8 Hz, 2H), 3.61 - 3.46 (m, 2H), 3.34 - 3.10 (m, 2H), 2.79 (d, *J* = 11.7 Hz, 1H), 2.60 (dd, *J* = 18.8, 8.3 Hz, 2H), 1.50 (d, *J* = 7.0 Hz, 3H), 0.96 (d, *J* = 6.3 Hz, 3H).

**Example 83: Preparation of Compound 83**

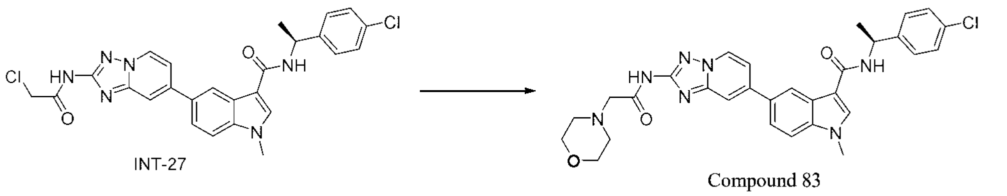

Compound 83

Preparation of Compound 83:

Intermediate **INT-27** (104 mg, 0.2 mmol) was dissolved in acetonitrile (1 mL); morpholine (35 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate

solution = 60%) to obtain Compound 83 with a yield of 82%. MS (ESI, positive ion) m/z:572.2 [M + H]+.

$^1$H NMR (400 MHz, DMSO-$d_6$) δ 10.53 (s, 1H), 8.89 (d, *J*= 7.1 Hz, 1H), 8.54 (d, *J*= 2.0 Hz, 1H), 8.40 (d, *J*= 7.9 Hz, 1H), 8.23 (s, 1H), 7.90 (d, *J*= 1.9 Hz, 1H), 7.74 (dd, *J*= 8.6, 1.9 Hz, 1H), 7.67 (d, *J*= 8.7 Hz, 1H), 7.51 - 7.44 (m, 3H), 7.44 - 7.37 (m, 2H), 5.20 (p, *J* = 7.2 Hz, 1H), 3.91 (s, 3H), 3.64 (t, *J* = 4.6 Hz, 4H), 3.27 (s, 2H), 2.58 - 2.54 (m, 4H), 1.50 (d, *J* = 7.1 Hz, 3H).

**Example 84: Preparation of Compound 84**

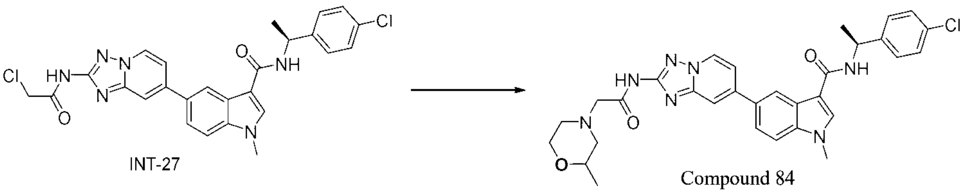

INT-27

Compound 84

Preparation of Compound 84:

Intermediate **INT-27** (104 mg, 0.2 mmol) was dissolved in acetonitrile (1 mL); 3-(hydroxymethyl)piperidine (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 84 with a yield of 66%. MS (ESI, positive ion) m/z:586.2 [M + H]+.

$^1$H NMR (400 MHz, DMSO-$d_6$) δ 10.52 (s, 1H), 8.88 (d, *J*= 7.1 Hz, 1H), 8.54 (d, *J*= 1.8 Hz, 1H), 8.40 (d, *J* = 7.9 Hz, 1H), 8.23 (s, 1H), 7.90 (d, *J* = 1.9 Hz, 1H), 7.74 (dd, *J* = 8.6, 1.9 Hz, 1H), 7.67 (d, *J* = 8.7 Hz, 1H), 7.49 - 7.44 (m, 3H), 7.40 (d, *J* = 8.6 Hz, 2H), 5.20 (p, *J* = 7.2 Hz, 1H), 3.91 (s, 3H), 3.77 (dt, *J*= 10.0, 2.4 Hz, 1H), 3.58 (ddd, *J* = 13.8, 8.9, 2.8 Hz, 2H), 3.26 (s, 2H), 2.82 (dt, *J* = 11.1, 2.1 Hz, 1H), 2.75 (dd, *J*= 11.1, 2.1 Hz, 1H), 2.28 (td, *J*= 11.3, 3.3 Hz, 1H), 1.98 (dd, *J*= 11.1, 9.9 Hz, 1H), 1.50 (d, *J*= 7.1 Hz, 3H), 1.07 (d, *J*= 6.2 Hz, 3H).

**Example 85: Preparation of Compound 85**

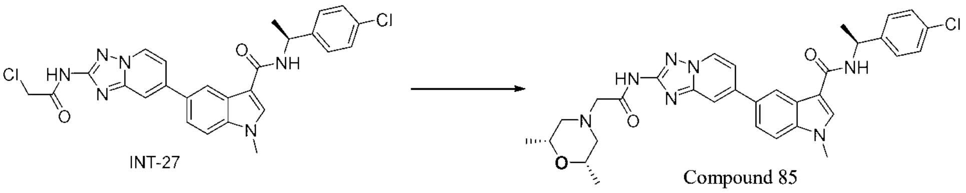

INT-27

Compound 85

Preparation of Compound 85:

Intermediate **INT-27** (104 mg, 0.2 mmol) was dissolved in acetonitrile (1 mL), and cis-2,6-dimethylmorpholine (46 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 85 with a yield of 64%. MS (ESI, positive ion) m/z:600.2 [M + H]+.

$^1$H NMR (400 MHz, DMSO-$d_6$) δ 10.50 (s, 1H), 8.86 (d, *J*= 7.1 Hz, 1H), 8.52 (d, *J*= 1.9 Hz, 1H), 8.39 (d, *J*= 7.9 Hz, 1H), 8.21 (s, 1H), 7.88 (d, *J* = 2.0 Hz, 1H), 7.72 (dd, *J* = 8.6, 1.9 Hz, 1H), 7.66 (d, *J* = 8.7 Hz, 1H), 7.47 - 7.42 (m, 3H), 7.38 (d, *J* = 8.6 Hz, 2H), 5.18 (t, *J*= 7.3 Hz, 1H), 3.90 (s, 3H), 3.67 - 3.59 (m, 2H), 3.23 (s, 2H), 2.81 - 2.77 (m, 2H), 1.89 (t, *J*= 10.6 Hz, 2H), 1.48 (d, *J* = 7.0 Hz, 3H), 1.05 (d, *J* = 6.3 Hz, 6H).

**Example 86: Preparation of Compound 86**

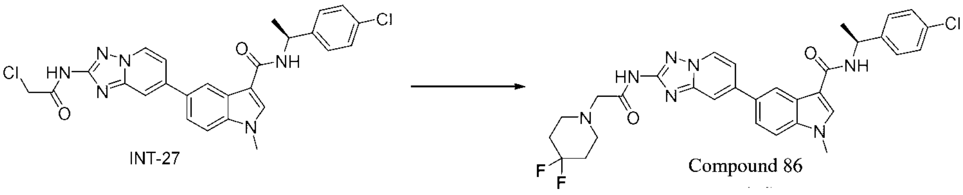

INT-27

Compound 86

Preparation of Compound 86:

Intermediate **INT-27** (104 mg, 0.2 mmol) was dissolved in acetonitrile (1 mL); 4,4-difluoropiperidine (48 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 86 with a yield of 71%. MS (ESI, positive ion) m/z:606.2 [M + H]$^+$.

$^1$H NMR (400 MHz, DMSO-$d_6$) δ 8.89 (d, *J*= 7.1 Hz, 1H), 8.55 (d, *J*= 1.9 Hz, 1H), 8.42 (d, *J* = 7.9 Hz, 1H), 8.24 (s, 1H), 7.90 (d, *J* = 2.0 Hz, 1H), 7.74 (dd, *J* = 8.7, 1.9 Hz, 1H), 7.68 (d, *J* = 8.7 Hz, 1H), 7.49 - 7.44 (m, 3H), 7.41 (d, *J*= 8.5 Hz, 2H), 5.20 (p, *J*= 7.2 Hz, 1H), 3.92 (s, 3H), 3.39 (s, 2H), 2.72 (t, *J*= 5.7 Hz, 4H), 2.03 (dt, *J*= 14.6, 7.8 Hz, 4H), 1.50 (d, *J*= 7.0 Hz, 3H).

**Example 87: Preparation of Compound 87**

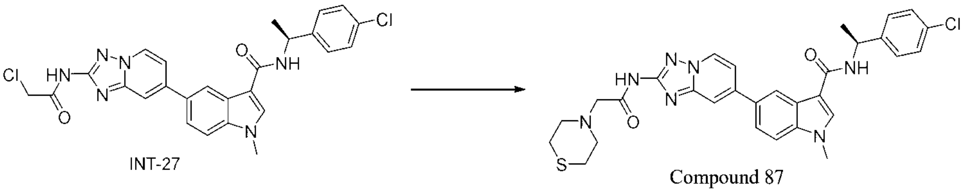

INT-27

Compound 87

Preparation of Compound 87:

Intermediate **INT-27** (104 mg, 0.2 mmol) was dissolved in acetonitrile (1 mL); thiomorpholine (41 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 87 with a yield of 61%. MS (ESI, positive ion) m/z:588.2 [M + H]$^+$.

$^1$H NMR (400 MHz, DMSO-$d_6$) δ 10.47 (s, 1H), 8.88 (d, *J* = 7.1 Hz, 1H), 8.55 (d, *J* = 1.8 Hz, 1H), 8.39 (d, *J* = 7.9 Hz, 1H), 8.24 (s, 1H), 7.90 (d, *J* = 1.9 Hz, 1H), 7.74 (dd, *J* = 8.6, 1.9 Hz, 1H), 7.68 (d, *J* = 8.7 Hz, 1H), 7.49 - 7.45 (m, 3H), 7.42 - 7.39 (m, 2H), 5.21 (p, *J* = 7.2 Hz, 1H), 3.92 (s, 3H), 3.31 (s, 2H), 2.84 (dd, *J* = 6.4, 3.5 Hz, 4H), 2.70 - 2.66 (m, 4H), 1.50 (d, *J* = 7.1 Hz, 3H).

**Example 88: Preparation of Compound 88**

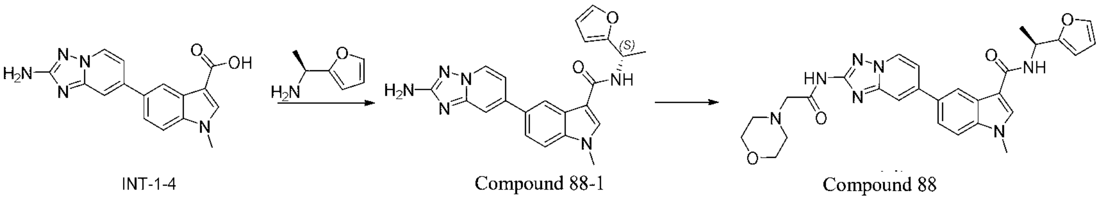

INT-1-4

Compound 88-1

Compound 88

Preparation of Compound 88-1:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-1-(furan-2-yl)ethan-1-amine (133 mg, 1.2 mmol) was added, and the mixture was stirred for reaction at room

temperature for 2 h; the reaction mixture was added dropwise into water and stirred for 0.5 h, filtered, and dried to obtain intermediate compound 88-1 with a yield of 76%. MS (ESI, positive ion) m/z: 401.3 [M + H]$^+$.

Preparation of Compound 88:

Compound 88-1 (80 mg, 0.2 mmol) was dissolved in pyridine (1 mL), 2-(morpholin-4-yl)acetic acid (43 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 88 with a yield of 69%. MS (ESI, positive ion) m/z: 528.2 [M + H]$^+$.

$^1$H NMR (400 MHz, DMSO-$d_6$) δ 10.52 (s, 1H), 8.90 (d, *J* = 7.1 Hz, 1H), 8.61 (d, *J* = 1.8 Hz, 1H), 8.31 (d, *J* = 8.4 Hz, 1H), 8.19 (s, 1H), 7.92 (d, *J* = 1.8 Hz, 1H), 7.75 (dd, *J* = 8.6, 1.9 Hz, 1H), 7.67 (d, *J* = 8.6 Hz, 1H), 7.60 (d, *J* = 1.8 Hz, 1H), 7.50 (dd, *J* = 7.2, 2.0 Hz, 1H), 6.42 (dd, *J* = 3.3, 1.8 Hz, 1H), 6.30 (d, *J* = 3.2 Hz, 1H), 5.34 (p, *J* = 7.2 Hz, 1H), 3.90 (s, 3H), 3.65 (t, *J* = 4.6 Hz, 4H), 3.28 (s, 2H), 2.57 (dd, *J* = 5.6, 3.7 Hz, 4H), 1.52 (d, *J* = 7.0 Hz, 3H).

**Example 89: Preparation of Compound 89**

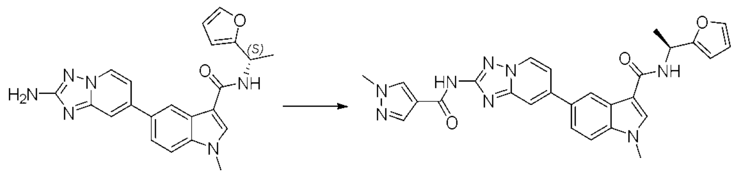

Compound 88-1 Compound 89

Preparation of Compound 89:

Compound 88-1 (80 mg, 0.2 mmol) was dissolved in pyridine (1 mL), 1-methylpyrazole-4-carboxylic acid (38 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 89 with a yield of 62%. MS (ESI, positive ion) m/z: 509.2 [M + H]$^+$.

$^1$H NMR (400 MHz, DMSO-$d_6$) δ 10.93 (s, 1H), 8.96 - 8.90 (m, 1H), 8.62 (d, *J* = 1.9 Hz, 1H), 8.43 (s, 1H), 8.33 (d, *J* = 8.4 Hz, 1H), 8.20 (s, 1H), 8.13 (s, 1H), 7.96 - 7.91 (m, 1H), 7.77 (dd, *J* = 8.6, 1.9 Hz, 1H), 7.69 (d, *J* = 8.7 Hz, 1H), 7.65 - 7.58 (m, 1H), 7.51 (dd, *J* = 7.2, 2.0 Hz, 1H), 6.43 (dd, *J* = 3.2, 1.8 Hz, 1H), 6.31 (dt, *J* = 3.3, 1.0 Hz, 1H), 5.34 (p, *J* = 7.1 Hz, 1H), 3.91 (d, *J* = 5.9 Hz, 6H), 1.52 (d, *J* = 7.0 Hz, 3H).

**Example 90: Preparation of Compound 90**

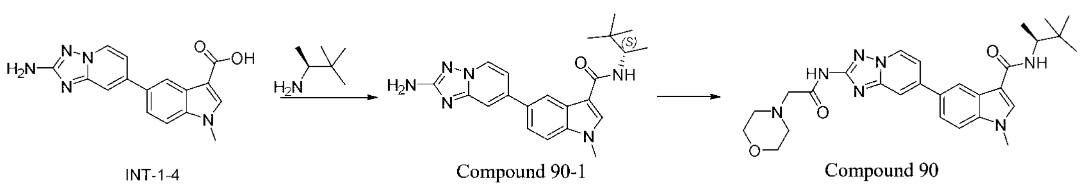

INT-1-4 Compound 90-1 Compound 90

Preparation of Compound 90-1:

Intermediate **INT-1-4** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-1-(tert-butyl)ethan-1-amine (121 mg, 1.2 mmol) was added, and the mixture was stirred for reaction at room temperature for 2 h; the reaction mixture was added dropwise into water and stirred for 0.5 h, filtered, and dried to obtain intermediate compound 90-1 with a yield of 56%. MS (ESI, positive ion) m/z: 391.3 [M + H]$^+$.

Preparation of Compound 90:

Compound 90-1 (78 mg, 0.2 mmol) was dissolved in pyridine (1 mL), 2-(morpholin-4-yl)acetic acid (43 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 90 with a yield of 59%. MS (ESI, positive ion) m/z: 518.2 $[M + H]^+$.

$^1H$ NMR (400 MHz, DMSO-$d_6$) δ 10.54 (s, 1H), 8.92 - 8.89 (m, 1H), 8.57 (d, *J* = 1.8 Hz, 1H), 8.22 (s, 1H), 7.91 (dd, *J* = 2.0, 0.9 Hz, 1H), 7.74 (dd, *J* = 8.6, 1.9 Hz, 1H), 7.67 (d, *J* = 8.6 Hz, 1H), 7.52 - 7.47 (m, 2H), 4.09 - 4.00 (m, 1H), 3.90 (s, 3H), 3.65 (t, *J* = 4.6 Hz, 4H), 3.28 (s, 2H), 2.57 (t, *J* = 4.7 Hz, 4H), 1.12 (d, *J* = 6.9 Hz, 3H), 0.95 (s, 9H).

**Example 91: Preparation of Compound 91**

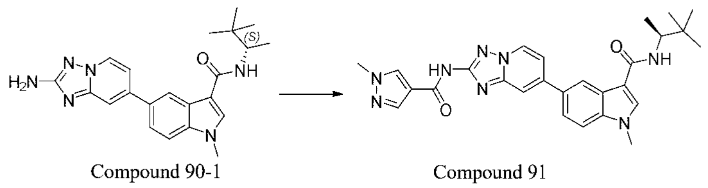

Compound 90-1 Compound 91

Preparation of Compound 91:

Compound 90-1 (78 mg, 0.2 mmol) was dissolved in pyridine (1 mL), 1-methylpyrazole-4-carboxylic acid (38 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 91 with a yield of 58%. MS (ESI, positive ion) m/z: 499.2 $[M + H]^+$.

$^1H$ NMR (400 MHz, DMSO-$d_6$) δ 10.93 (s, 1H), 8.93 (d, *J* = 7.1 Hz, 1H), 8.59 (d, *J* = 1.8 Hz, 1H), 8.43 (s, 1H), 8.22 (s, 1H), 8.13 (s, 1H), 7.93 (t, *J* = 1.3 Hz, 1H), 7.76 (dd, *J* = 8.6, 1.9 Hz, 1H), 7.67 (d, *J* = 8.6 Hz, 1H), 7.54 - 7.47 (m, 2H), 4.11 - 3.99 (m, 1H), 3.91 (d, *J* = 4.1 Hz, 6H), 1.12 (d, *J* = 6.9 Hz, 3H), 0.95 (s, 9H).

**Example 92: Preparation of Compound 92**

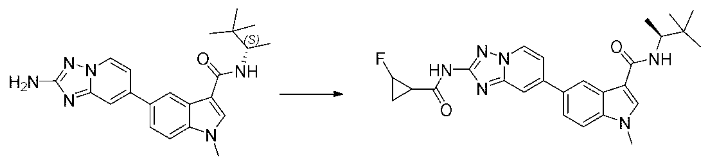

Compound 90-1 Compound 92

Preparation of Compound 92:

Compound 90-1 (78 mg, 0.2 mmol) was dissolved in pyridine (1 mL), 2-fluorocyclopropanecarboxylic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 92 with a yield of 58%. MS (ESI, positive ion) m/z: 477.2 $[M + H]^+$.

$^1H$ NMR (400 MHz, DMSO-$d_6$) δ 8.89 (d, *J* = 7.1 Hz, 1H), 8.58 (d, *J* = 1.8 Hz, 1H), 8.22 (s, 1H), 7.90 (d, *J* = 2.2 Hz, 1H), 7.75 (dd, *J* = 8.7, 1.9 Hz, 1H), 7.67 (d, *J* = 8.7 Hz, 1H), 7.55 - 7.45 (m, 2H), 4.05 (dd, *J* = 9.3, 6.9 Hz, 1H), 3.90 (s, 3H), 1.75 - 1.60 (m, 1H), 1.26 - 1.16 (m, 1H), 1.12 (d, *J* = 6.9 Hz, 3H), 0.95 (s, 11H).

**Example 93: Preparation of Compound 93**

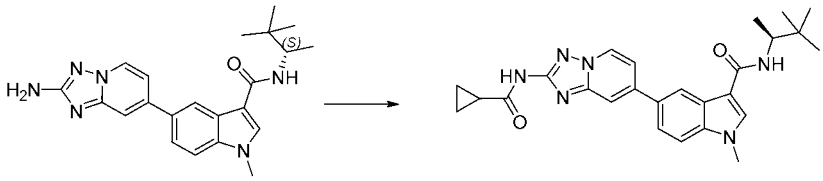

Compound 90-1 Compound 93

Preparation of Compound 93:

Compound 90-1 (78 mg, 0.2 mmol) was dissolved in pyridine (1 mL), cyclopropanecarboxylic acid (28 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 93 with a yield of 58%. MS (ESI, positive ion) m/z: 459.2 $[M + H]^+$.

$^1H$ NMR (400 MHz, DMSO-$d_6$) δ 11.10 (s, 1H), 8.88 (d, *J* = 7.0 Hz, 1H), 8.58 (d, *J* = 1.8 Hz, 1H), 8.22 (s, 1H), 7.92 - 7.87 (m, 1H), 7.74 (dd, *J* = 8.7, 1.9 Hz, 1H), 7.67 (d, *J* = 8.6 Hz, 1H), 7.53 - 7.46 (m, 2H), 4.09 - 4.00 (m, 1H), 3.90 (s, 3H), 1.12 (d, *J* = 6.9 Hz, 3H), 0.95 (s, 10H), 0.86 (d, *J* = 6.1 Hz, 4H).

**Example 94: Preparation of Compound 94**

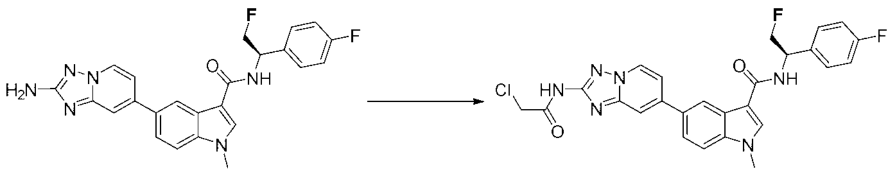

INT-15

化合物94
Compound 94

**Preparation of Compound 94:**

Intermediate **INT-15** (44.6 g, 100 mmol) was dissolved in dichloromethane (1 L), chloroacetyl chloride (13.5 g, 120 mmol) and N,N-diisopropylethylamine (25.8 g, 200 mmol) were added, and the mixture was stirred at room temperature for 2 h. Petroleum ether (3 L) was added to the reaction mixture for crystallization; the solid was filtered with suction, washed with water, and dried to obtain Compound 94 with a yield of 73%. MS (ESI, positive ion) m/z: 523.2 $[M + H]^+$.

$^1H$ NMR (400 MHz, DMSO-$d_6$) δ 11.26 (s, 1H), 8.92 (d, *J* = 7.1 Hz, 1H), 8.62 (d, *J* = 8.3 Hz, 1H), 8.55 (d, *J* = 1.8 Hz, 1H), 8.27 (s, 1H), 7.94 (d, *J* = 1.9 Hz, 1H), 7.77 (dd, *J* = 8.6, 1.9 Hz, 1H), 7.69 (d, *J* = 8.7 Hz, 1H), 7.59 - 7.53 (m, 2H), 7.50 (dd, *J* = 7.2, 2.0 Hz, 1H), 7.23 (t, *J* = 8.9 Hz, 2H), 5.48 (dq, *J* = 14.6, 7.2 Hz, 1H), 4.84 - 4.58 (m, 2H), 4.46 (s, 2H), 3.93 (s, 3H).

**Example 95: Preparation of Compound 95**

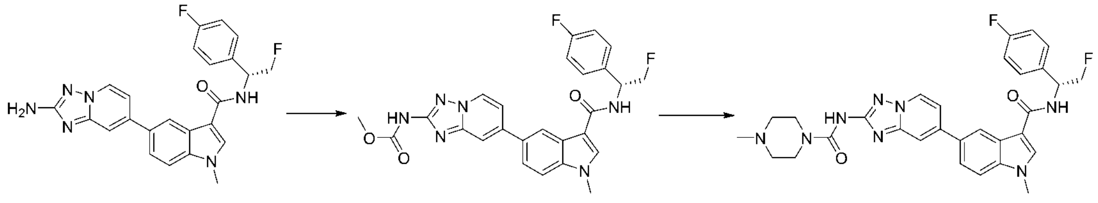

INT-15

Compound 95-1

Compound 95

Preparation of Compound 95-1:

Intermediate **INT-15** (44.6 g, 100 mmol) was dissolved in dichloromethane (1 L), methyl chloroformate (11.2 g, 120 mmol) and N,N-diisopropylethylamine (25.8 g, 200 mmol) were added, and the mixture was stirred at room temperature for 2 h. Petroleum ether (3 L) was added to the reaction mixture for crystallization; the solid was filtered with suction, washed with water, and dried to obtain Compound 95-1 with a yield of 69%. MS (ESI, positive ion) m/z: 505.2 $[M + H]^+$.

Preparation of Compound 95:

Compound 95-1 (100 mg, 0.2 mmol) was dissolved in acetonitrile (1 mL); N-methylpiperazine (40 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 95 with a yield of 61%. MS (ESI, positive ion) m/z:573.2 $[M + H]^+$.

$^1$H NMR (400 MHz, DMSO-$d_6$) δ 9.65 (s, 1H), 8.83 (d, $J$ = 7.1 Hz, 1H), 8.61 (d, $J$ = 8.3 Hz, 1H), 8.52 (d, $J$ = 1.8 Hz, 1H), 8.27 (s, 1H), 7.86 (dd, $J$ = 1.9, 0.9 Hz, 1H), 7.78 (dd, $J$ = 8.7, 1.9 Hz, 1H), 7.69 (d, $J$ = 8.6 Hz, 1H), 7.59 - 7.53 (m, 2H), 7.43 (dd, $J$ = 7.1, 2.0 Hz, 1H), 7.23 (t, $J$ = 8.9 Hz, 2H), 5.48 (dq, $J$ = 14.9, 7.2 Hz, 1H), 4.75 (dd, $J$ = 6.6, 3.3 Hz, 1H), 4.68 - 4.60 (m, 1H),4.23(s, 3H), 3.93 (s, 3H), 3.55 - 3.60 (m, 4H), 3.48 (t, $J$ = 4.8 Hz, 4H).

**Example 96: Preparation of Compound 96**

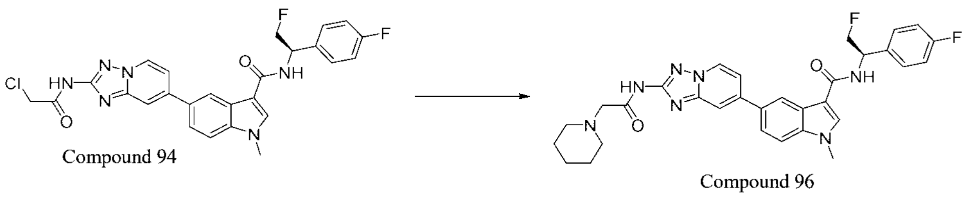

Compound 94

Compound 96

Preparation of Compound 96:

Intermediate Compound 96 (104 mg, 0.2 mmol) was dissolved in acetonitrile (1 mL); piperidine (34 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 96 with a yield of 58%. MS (ESI, positive ion) m/z:572.2 $[M + H]^+$.

$^1$H NMR (400 MHz, DMSO-$d_6$) δ 9.64 (s, 1H), 8.85 (d, $J$ = 7.1 Hz, 1H), 8.61 (d, $J$ = 8.3 Hz, 1H), 8.54 (d, $J$ = 1.8 Hz, 1H), 8.26 (s, 1H), 7.85 (dd, $J$ = 1.9, 0.9 Hz, 1H), 7.75 (dd, $J$ = 8.7, 1.9 Hz, 1H), 7.67 (d, $J$ = 8.6 Hz, 1H), 7.56 - 7.53 (m, 2H), 7.43 (dd, $J$ = 7.1, 2.0 Hz, 1H), 7.23 (t, $J$ = 8.9 Hz, 2H), 5.46 (dq, $J$ = 14.9, 7.2 Hz, 1H), 4.75 (dd, $J$ = 6.6, 3.3 Hz, 1H), 4.65 - 4.60 (m, 1H), 3.93 (s, 3H),3.67 (m, 2H), 3.55 - 3.60 (m, 4H), 1.32 (m, 4H).1.10 (m, 2H).

**Example 97: Preparation of Compound 97**

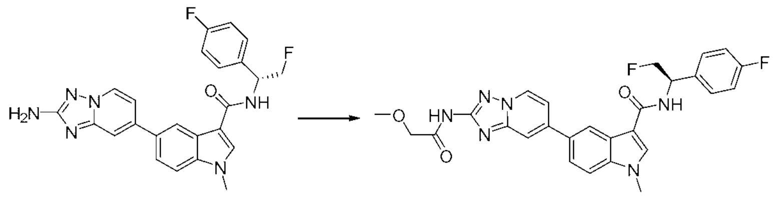

INT-15 Compound 97

Preparation of Compound 97:

**INT-15** (89 mg, 0.2 mmol) was dissolved in pyridine (1 mL), 2-methoxyacetic acid (26 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 97 with a yield of 68%. MS (ESI, positive ion) m/z: 519.2 $[M + H]^+$.

$^1$H NMR (400 MHz, DMSO-$d_6$) δ 9.65 (s, 1H), 8.86 (d, *J* = 7.1 Hz, 1H), 8.65 (d, *J* = 8.3 Hz, 1H), 8.54 (d, *J* = 1.8 Hz, 1H), 8.25 (s, 1H), 7.86 (dd, *J* = 1.9, 0.9 Hz, 1H), 7.75 (dd, *J* = 8.7, 1.9 Hz, 1H), 7.72 (d, *J* = 8.6 Hz, 1H), 7.59 - 7.53 (m, 2H), 7.42 (dd, *J* = 7.1, 2.0 Hz, 1H), 7.21 (t, *J* = 8.9 Hz, 2H), 5.48 (dq, *J* = 14.9, 7.2 Hz, 1H), 4.75 (dd, *J* = 6.6, 3.3 Hz, 1H), 4.68 - 4.60 (m, 1H), 3.93 (s, 3H), 3.12(m, 2H), 2.87 (m, 2H).

**Example 98: Preparation of Compound 98**

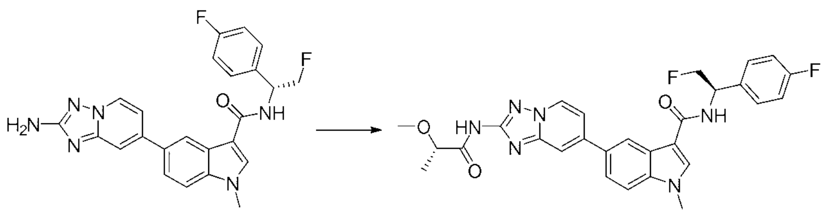

INT-15 Compound 98

Preparation of Compound 98:

**INT-15** (89 mg, 0.2 mmol) was dissolved in pyridine (1 mL), (S)-2-methoxypropanoic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 98 with a yield of 61%. MS (ESI, positive ion) m/z: 533.2 $[M + H]^+$.

$^1$H NMR (400 MHz, DMSO-$d_6$) δ 8.61 (t, *J* = 8.3 Hz, 2H), 8.49 (d, *J* = 1.8 Hz, 1H), 8.25 (s, 1H), 7.73 - 7.63 (m, 2H), 7.61 (d, *J* = 2.0 Hz, 1H), 7.55 (dd, *J* = 8.6, 5.6 Hz, 2H), 7.27 - 7.17 (m, 3H), 6.58 (t, *J* = 6.1 Hz, 1H), 5.49 (dt, *J* = 15.5, 7.0 Hz, 1H), 4.75 (d, *J* = 7.7 Hz, 1H), 4.67 - 4.60 (m, 1H), 3.92 (s, 3H), 3.55 (q, *J* = 6.1 Hz, 1H), 3.22 (dt, *J* = 12.9, 6.1 Hz, 1H), 1.25 (s, 3H), 1.13 (d, *J* = 6.1 Hz, 3H).

**Example 99: Preparation of Compound 99**

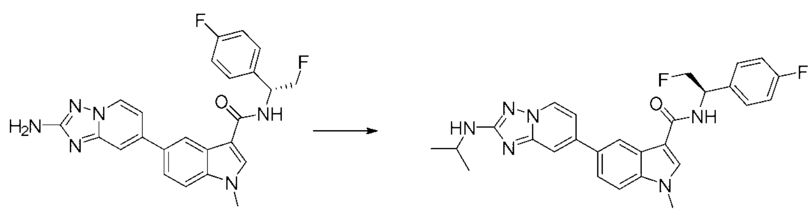

INT-15 Compound 99

Preparation of Compound 99:

**INT-15** (89 mg, 0.2 mmol) was dissolved in THF (1 mL), acetone (0.2 mL) and tetraisopropyl titanate (0.1 mL) were added, and the mixture was heated to 80°C and stirred for 2 h. After cooling to room temperature, sodium borohydride (23 mg, 0.6 mmol) was added. After 1 h, the reaction mixture was concentrated under reduced pressure and purified by C18 reverse phase silica gel column (acetonitrile/0.1% aqueous ammonium bicarbonate solution = 60%) to obtain Compound 99 with a yield of 21%. MS (ESI, positive ion) m/z: 489.2 $[M + H]^+$.

$^1H$ NMR (400 MHz, DMSO-$d_6$) δ 9.64 (s, 1H), 8.85 (d, *J* = 7.1 Hz, 1H), 8.64 (d, *J* = 8.3 Hz, 1H), 8.53 (d, *J* = 1.8 Hz, 1H), 8.27 (s, 1H), 7.86 (dd, *J* = 1.9, 0.9 Hz, 1H), 7.75 (dd, *J* = 8.7, 1.9 Hz, 1H), 7.67 (d, *J* = 8.6 Hz, 1H), 7.61 - 7.53 (m, 2H), 7.45 (dd, *J* = 7.1, 2.0 Hz, 1H), 7.22 (t, *J* = 8.9 Hz, 2H), 4.75 (dd, *J* = 6.6, 3.3 Hz, 1H), 3.96 (m, 1H), 3.76 (m, 2H), 3.50 (s, 3H), 1.21 (d, *J* = 8.6 Hz, 6H).

**Example 100: Preparation of Compound 100**

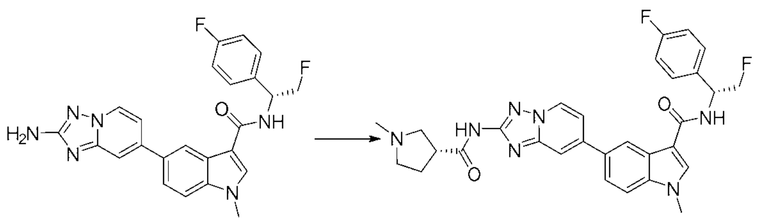

INT-15 Compound 100

Preparation of Compound 100:

**INT-15** (89 mg, 0.2 mmol) was dissolved in pyridine (1 mL), (R)-1-methylpyrrolidine-3-carboxylic acid (39 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 100 with a yield of 78%. MS (ESI, positive ion) m/z: 558.2 $[M + H]^+$.

$^1H$ NMR (400 MHz, DMSO-$d_6$) δ 9.63 (s, 1H), 8.85 (d, *J* = 7.1 Hz, 1H), 8.64 (d, *J* = 8.3 Hz, 1H), 8.55 (d, *J* = 1.8 Hz, 1H), 8.27 (s, 1H), 7.83 (dd, *J* = 1.9, 0.9 Hz, 1H), 7.75 (dd, *J* = 8.7, 1.9 Hz, 1H), 7.69 (d, *J* = 8.6 Hz, 1H), 7.59 - 7.53 (m, 2H), 7.43 (dd, *J* = 7.1, 2.0 Hz, 1H), 7.24 (t, *J* = 8.9 Hz, 2H), 5.48 (dq, *J* = 14.9, 7.2 Hz, 1H), 4.75 (dd, *J* = 6.6, 3.3 Hz, 1H),3.59 (m, 1H), 1.50 (d, *J* = 7.1 Hz, 6H).

**Example 101: Preparation of Compound 101**

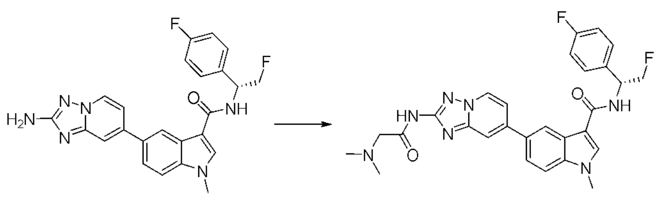

INT-15 Compound 101

Preparation of Compound 101:

INT-15 (89 mg, 0.2 mmol) was dissolved in pyridine (1 mL), 2-(dimethylamino)acetic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 101 with a yield of 78%. MS (ESI, positive ion) m/z: 532.2 $[M + H]^+$.

$^1H$ NMR (400 MHz, DMSO-$d_6$) δ 9.64 (s, 1H), 8.85 (d, *J* = 7.1 Hz, 1H), 8.64 (d, *J* = 8.3 Hz, 1H), 8.53 (d, *J* = 1.8 Hz,

1H), 8.27 (s, 1H), 7.86 (dd, $J$ = 1.9, 0.9 Hz, 1H), 7.75 (dd, $J$ = 8.7, 1.9 Hz, 1H), 7.67 (d, $J$ = 8.6 Hz, 1H), 7.61 - 7.53 (m, 2H), 7.45 (dd, $J$ = 7.1, 2.0 Hz, 1H), 7.22 (t, $J$ = 8.9 Hz, 2H), 5.49 (dq, $J$ = 14.9, 7.2 Hz, 1H), 4.75 (dd, $J$ = 6.6, 3.3 Hz, 1H), 3.76 (m, 2H), 3.66 (m, 2H), 3.50 (s, 3H), 2.87 (m, 2H), 2.32 (m, 1H).

**Example 102: Preparation of Compound 102**

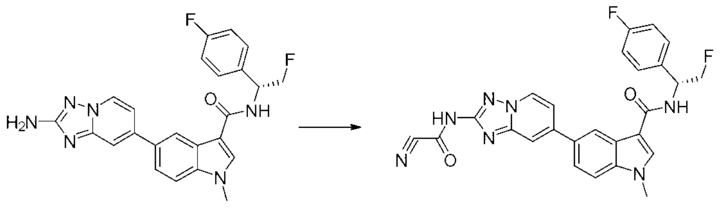

INT-15

Compound 102

Preparation of Compound 102:

**INT-15** (89 mg, 0.2 mmol) was dissolved in pyridine (1 mL), cyanoacetic acid (29 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 102 with a yield of 78%. MS (ESI, positive ion) m/z: 500.2 $[M + H]^+$.

$^1$H NMR (400 MHz, DMSO-$d_6$) δ 11.29 (s, 1H), 8.92 (d, $J$ = 7.1 Hz, 1H), 8.62 (d, $J$ = 8.2 Hz, 1H), 8.55 (d, $J$ = 1.8 Hz, 1H), 8.27 (s, 1H), 7.94 (d, $J$ = 1.8 Hz, 1H), 7.77 (dd, $J$ = 8.6, 1.9 Hz, 1H), 7.69 (d, $J$ = 8.7 Hz, 1H), 7.57 - 7.49 (m, 3H), 7.23 (t, $J$ = 8.8 Hz, 2H), 5.53 - 5.43 (m, 1H), 4.76 (d, $J$ = 8.0 Hz, 1H), 4.67 - 4.61 (m, 1H), 3.93 (s, 3H), 2.72 - 2.55 (m, 1H), 2.35 (s, 1H).

**Example 103: Preparation of Compound 103**

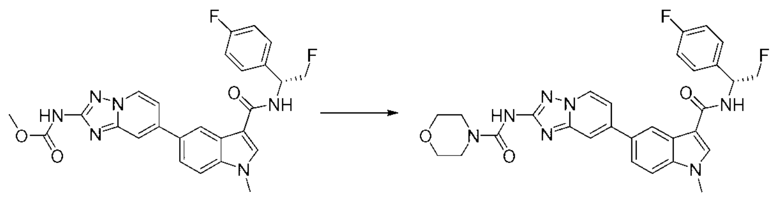

Compound 95-1

Compound 103

Preparation of Compound 103:

Compound 95-1 (100 mg, 0.2 mmol) was dissolved in acetonitrile (1 mL); morpholine (32 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 103 with a yield of 61%. MS (ESI, positive ion) m/z:560.2 $[M + H]^+$.

$^1$H NMR (400 MHz, DMSO-$d_6$) δ 9.63 (s, 1H), 8.84 (d, $J$ = 7.1 Hz, 1H), 8.69 (d, $J$ = 8.3 Hz, 1H), 8.54 (d, $J$ = 1.3 Hz, 1H), 8.26 (s, 1H), 7.86 (dd, $J$ = 1.9, 0.9 Hz, 1H), 7.75 (dd, $J$ = 8.7, 1.9 Hz, 1H), 7.72 (d, $J$ = 8.6 Hz, 1H), 7.59 - 7.53 (m, 2H), 7.43 (dd, $J$ = 7.1, 2.0 Hz, 1H), 7.21 (t, $J$ = 8.7 Hz, 2H), 5.49 (dq, $J$ = 14.9, 7.2 Hz, 1H), 4.76 (dd, $J$ = 6.6, 3.3 Hz, 1H), 4.68 - 4.60 (m, 1H), 3.93 (s, 3H), 3.55 (dd, $J$ = 7.6, 3.8 Hz, 4H), 3.31 (dd, $J$ = 7.4, 3.7Hz, 4H).

**Example 104: Preparation of Compound 104**

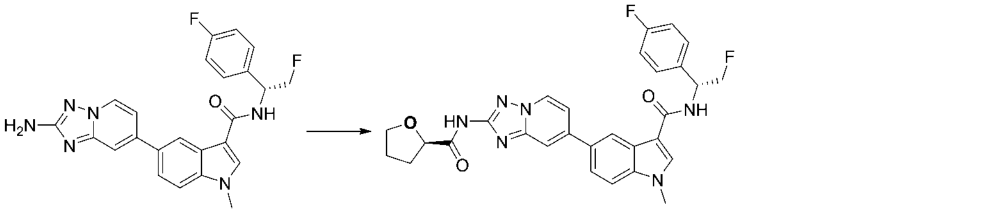

INT-15

化合物104
Compound 104

Preparation of Compound 104:

**INT-15** (89 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 104 with a yield of 71%. MS (ESI, positive ion) m/z: 545.2 $[M + H]^+$.

$^1$H NMR (400 MHz, DMSO-$d_6$) δ 9.63 (s, 1H), 8.84 (d, *J* = 7.1 Hz, 1H), 8.69 (d, *J* = 8.3 Hz, 1H), 8.54 (d, *J* = 1.3 Hz, 1H), 8.26 (s, 1H), 7.86 (dd, *J* = 1.9, 0.9 Hz, 1H), 7.75 (dd, *J* = 8.7, 1.9 Hz, 1H), 7.72 (d, *J* = 8.6 Hz, 1H), 7.59 - 7.53 (m, 2H), 7.43 (dd, *J* = 7.1, 2.0 Hz, 1H), 7.21 (t, *J* = 8.7 Hz, 2H), 5.49 (dq, *J* = 14.9, 7.2 Hz, 1H), 4.76 (dd, *J* = 6.6, 3.3 Hz, 1H), 4.68 - 4.60 (m, 1H), 3.93 (s, 3H), 3.67 (m, 2H), 3.53 (m, 1H), 1.88 (m, 4H).

**Example 105: Preparation of Compound 105**

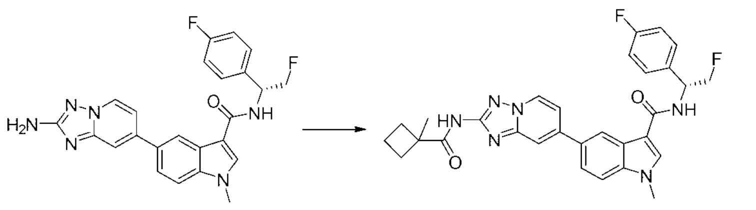

INT-15

Compound 105

Preparation of Compound 105:

**INT-15** (89 mg, 0.2 mmol) was dissolved in pyridine (1 mL), 1-methylcyclobutanecarboxylic acid (34 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 105 with a yield of 71%. MS (ESI, positive ion) m/z: 543.2 $[M + H]^+$.

$^1$H NMR (400 MHz, DMSO-$d_6$) δ 9.62 (s, 1H), 8.83 (d, *J* = 7.1 Hz, 1H), 8.62 (d, *J* = 8.3 Hz, 1H), 8.54 (d, *J* = 1.8 Hz, 1H), 8.27 (s, 1H), 7.85 (dd, *J* = 1.9, 0.9 Hz, 1H), 7.75 (dd, *J* = 8.7, 1.9 Hz, 1H), 7.69 (d, *J* = 8.6 Hz, 1H), 7.59 - 7.53 (m, 2H), 7.43 (dd, *J* = 7.1, 2.0 Hz, 1H), 7.23 (t, *J* = 8.9 Hz, 2H), 5.48 (dq, *J* = 14.9, 7.2 Hz, 1H), 4.75 (dd, *J* = 6.6, 3.3 Hz, 1H), 4.68 - 4.60 (m, 1H), 3.93 (s, 3H), 2.78 (m, 4H), 1.23(s, 3H).

**Example 106: Preparation of Compound 106**

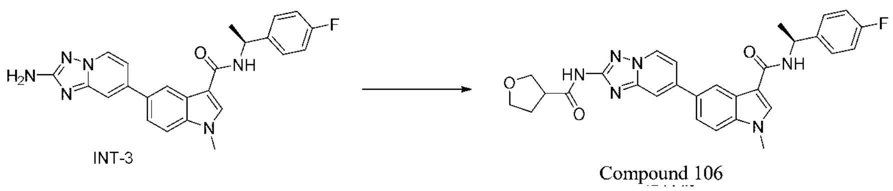

INT-3

Compound 106

Preparation of Compound 106:

Intermediate **INT-3** (85 mg, 0.2 mmol) was dissolved in pyridine (1 mL); 3-tetrahydrofuroic acid (30 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 106 with a yield of 55%. MS (ESI, positive ion) m/z: 527.2 $[M + H]^+$.

$^1$H NMR (400 MHz, DMSO-$d_6$) δ 10.44 (s, 1H), 8.79 (d, *J* = 7.4 Hz, 1H), 8.56 (d, *J* = 1.4 Hz, 1H), 8.34 (d, *J* = 8.1 Hz, 1H), 8.22 (s, 1H), 7.92 (d, *J* = 1.9 Hz, 1H), 7.72 (dd, *J* = 8.6, 1.8 Hz, 1H), 7.61 (d, *J* = 8.7 Hz, 1H), 7.43 (ddt, *J* = 5.8, 4.1, 1.7 Hz, 3H), 7.24 - 7.12 (m, 2H), 5.22 (p, *J* = 7.2 Hz, 1H), 3.91 (s, 3H), 3.27 (s, 2H), 2.56 (dd, *J* = 5.4, 3.6 Hz, 4H),2.1 (m, 2H), 1.87 (m, 1H), 1.50 (d, *J* = 7.0 Hz, 3H).

**Example 107: Preparation of Compound 107**

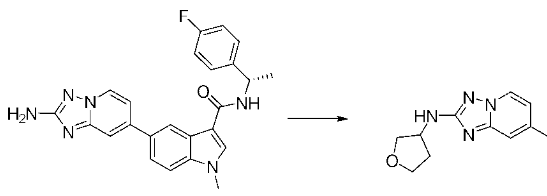

INT-15 Compound 107

Preparation of Compound 107:

**INT-15** (89 mg, 0.2 mmol) was dissolved in THF (1 mL), dihydro-3(2H)-furanone (0.2 mL) and tetraisopropyl titanate (0.1 mL) were added, and the mixture was heated to 80°C and stirred for 2 h. After cooling to room temperature, sodium borohydride (23 mg, 0.6 mmol) was added. After 1 h, the reaction mixture was concentrated under reduced pressure and purified by C18 reverse phase silica gel column (acetonitrile/0.1% aqueous ammonium bicarbonate solution = 60%) to obtain Compound 107 with a yield of 18%. MS (ESI, positive ion) m/z: 499.2 $[M + H]^+$.

$^1$H NMR (400 MHz, DMSO-$d_6$) δ 10.56 (s, 1H), 8.87 (d, *J* = 7.1 Hz, 1H), 8.53 (d, *J* = 1.9 Hz, 1H), 8.38 (d, *J* = 8.1 Hz, 1H), 8.22 (s, 1H), 7.90 (d, *J* = 1.9 Hz, 1H), 7.74 (dd, *J* = 8.6, 1.8 Hz, 1H), 7.67 (d, *J* = 8.7 Hz, 1H), 7.47 (ddt, *J* = 5.8, 4.1, 1.7 Hz, 3H), 7.23 - 7.11 (m, 2H), 5.24 (p, *J* = 7.2 Hz, 1H), 3.92 (s, 3H), 3.76 (m,1H), 2.56 (dd, *J* = 5.4, 3.4 Hz, 4H), 2.15 (m,2H), 1.50 (d, *J* = 7.0 Hz, 3H).

**Example 108: Preparation of Compound 108**

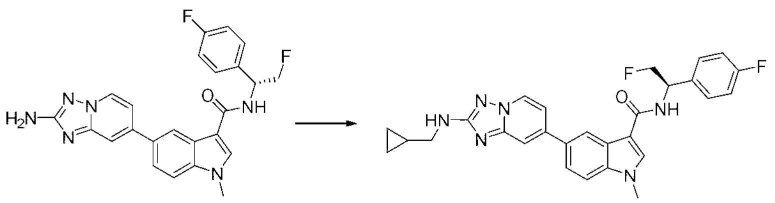

INT-15 Compound 108

Preparation of Compound 108:

**INT-15** (89 mg, 0.2 mmol) was dissolved in THF (1 mL), cyclopropanecarbaldehyde (0.2 mL) and tetraisopropyl titanate (0.1 mL) were added, and the mixture was heated to 80°C and stirred for 2 h. After cooling to room temperature, sodium borohydride (23 mg, 0.6 mmol) was added. After 1 h, the reaction mixture was concentrated under reduced pressure and purified by C18 reverse phase silica gel column (acetonitrile/0.1% aqueous ammonium bicarbonate solution = 60%) to obtain Compound 108 with a yield of 23%. MS (ESI, positive ion) m/z: 501.2 $[M + H]^+$.

$^1$H NMR (400 MHz, DMSO-$d_6$) δ 9.64 (s, 1H), 8.84 (d, *J* = 7.1 Hz, 1H), 8.65 (d, *J* = 8.2 Hz, 1H), 8.55 (d, *J* = 1.9 Hz, 1H), 8.28 (s, 1H), 7.83 (dd, *J* = 1.9, 0.9 Hz, 1H), 7.75 (dd, *J* = 8.6, 1.9 Hz, 1H), 7.71 (d, *J* = 8.6 Hz, 1H), 7.62 - 7.53 (m, 2H),

7.46 (dd, $J$ = 7.1, 2.0 Hz, 1H), 7.23 (t, $J$ = 8.8 Hz, 2H), 5.48 (dq, $J$ = 14.9, 7.2 Hz, 1H), 4.75 (dd, $J$ = 6.6, 3.3 Hz, 1H), 4.68 - 4.60 (m, 1H), 3.93 (s, 3H), 3.23 (m, 2H), 1.10 (m, 5H)

**Example 109: Preparation of Compound 109**

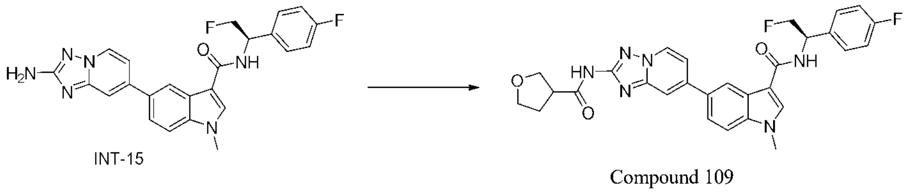

INT-15

Compound 109

Preparation of Compound 109:

Intermediate **INT-15** (88 mg, 0.2 mmol) was dissolved in pyridine (1 mL); 3-tetrahydrofuroic acid (30 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 109 with a yield of 65%. MS (ESI, positive ion) m/z: 545.2 $[M + H]^+$.

$^1H$ NMR (400 MHz, DMSO-$d_6$) δ 9.63 (s, 1H), 8.84 (d, $J$ = 7.1 Hz, 1H), 8.65 (d, $J$ = 8.2 Hz, 1H), 8.57 (d, $J$ = 1.9 Hz, 1H), 8.29 (s, 1H), 7.84 (dd, $J$ = 1.9, 0.9 Hz, 1H), 7.76 (dd, $J$ = 8.6, 1.9 Hz, 1H), 7.73 (d, $J$ = 8.6 Hz, 1H), 7.63 - 7.53 (m, 2H), 7.44 (dd, $J$ = 7.1, 2.0 Hz, 1H), 7.23 (t, $J$ = 8.8 Hz, 2H), 5.48 (dq, $J$ = 14.9, 7.2 Hz, 1H), 4.75 (dd, $J$ = 6.6, 3.3 Hz, 1H), 4.68 - 4.60 (m, 1H), 3.93 (s, 3H), 2.92 (m, 4H), 1.67 (m, 2H), 1.32 (m, 1H).

**Example 110: Preparation of Compound 110**

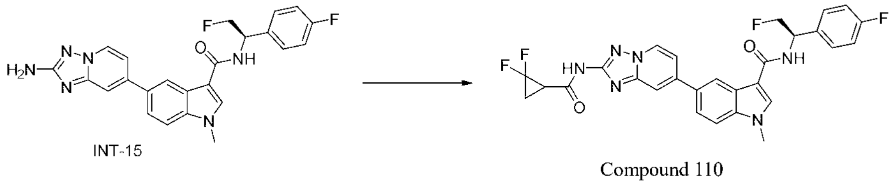

INT-15

Compound 110

Preparation of Compound 110:

Intermediate **INT-15** (88 mg, 0.2 mmol) was dissolved in pyridine (1 mL); 2,2-difluorocyclopropanecarboxylic acid (36 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 110 with a yield of 65%. MS (ESI, positive ion) m/z: 551.2 $[M + H]^+$.

$^1H$ NMR (400 MHz, DMSO-$d_6$) δ 9.64 (s, 1H), 8.83 (d, $J$ = 7.2 Hz, 1H), 8.64 (d, $J$ = 8.3 Hz, 1H), 8.55 (d, $J$ = 1.9 Hz, 1H), 8.30 (s, 1H), 7.83 (dd, $J$ = 2.2, 0.9 Hz, 1H), 7.73 (dd, $J$ = 8.6, 1.9 Hz, 1H), 7.71 (d, $J$ = 8.4 Hz, 1H), 7.63 - 7.53 (m, 2H), 7.44 (dd, $J$ = 7.1, 2.0 Hz, 1H), 7.23 (t, $J$ = 8.8 Hz, 2H), 5.48 (dq, $J$ = 14.9, 7.2 Hz, 1H), 4.75 (dd, $J$ = 6.6, 3.3 Hz, 1H), 4.68 - 4.60 (m, 1H), 3.93 (s, 3H), 2.02 (m, 2H), 1.86 (m, 1H).

**Example 111: Preparation of Compound 111**

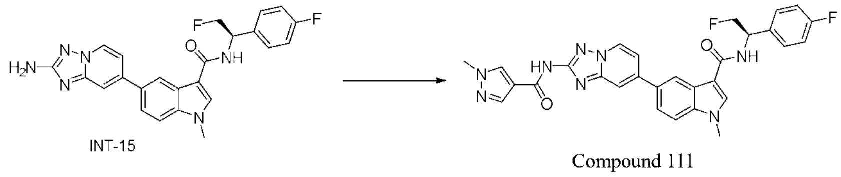

INT-15

Compound 111

Preparation of Compound 111:

Intermediate **INT-15** (88 mg, 0.2 mmol) was dissolved in pyridine (1 mL); 1-methylpyrazole-4-carboxylic acid (38 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column chromatography (acetonitrile/0.1% aqueous ammonium bicarbonate solution = 60%) to obtain Compound 111 with a yield of 45%. MS (ESI, positive ion) m/z: 555.2 $[M + H]^+$.

$^1H$ NMR (400 MHz, DMSO-$d_6$) δ 9.65 (s, 1H), 8.81 (d, *J* = 7.2 Hz, 1H), 8.64 (d, *J* = 8.3 Hz, 1H), 8.61 (d, *J* = 1.9 Hz, 1H), 8.32 (s, 1H), 8.16 (s, 1H), 7.95 (s, 1H), 7.83 (dd, *J* = 2.2, 0.9 Hz, 1H), 7.73 (dd, *J* = 8.6, 1.9 Hz, 1H), 7.71 (d, *J* = 8.4 Hz, 1H), 7.63 - 7.53 (m, 2H), 7.44 (dd, *J* = 7.1, 2.0 Hz, 1H), 7.23 (t, *J* = 8.8 Hz, 2H), 5.48 (dq, *J* = 14.9, 7.2 Hz, 1H), 4.75 (dd, *J* = 6.6, 3.3 Hz, 1H), 4.68 - 4.60 (m, 1H), 3.95 (s, 6H).

**Example 112: Preparation of Compound 112**

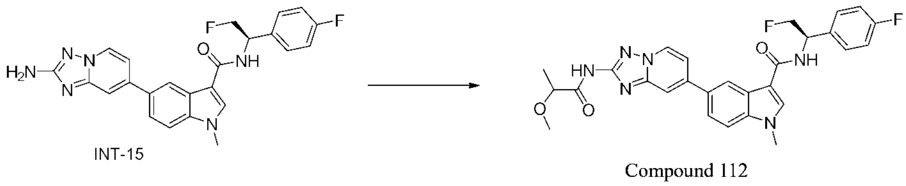

INT-15

Compound 112

Preparation of Compound 112:

Intermediate **INT-15** (88 mg, 0.2 mmol) was dissolved in pyridine (1 mL); 2-methoxypropionic acid (31 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column chromatography (acetonitrile/0.1% aqueous ammonium bicarbonate solution = 60%) to obtain Compound 112 with a yield of 45%. MS (ESI, positive ion) m/z: 533.2 $[M + H]^+$.

$^1H$ NMR (400 MHz, DMSO-$d_6$) δ 9.65 (s, 1H), 8.82 (d, *J* = 7.3 Hz, 1H), 8.63 (d, *J* = 8.5 Hz, 1H), 8.52 (d, *J* = 1.9 Hz, 1H), 8.30 (s, 1H), 7.83 (dd, *J* = 2.2, 0.9 Hz, 1H), 7.73 (dd, *J* = 8.5, 1.9 Hz, 1H), 7.72 (d, *J* = 8.2 Hz, 1H), 7.63 - 7.53 (m, 2H), 7.44 (dd, *J* = 7.2, 2.0 Hz, 1H), 7.22 (t, *J* = 8.8 Hz, 2H), 5.48 (dq, *J* = 14.9, 7.2 Hz, 1H), 4.75 (dd, *J* = 6.6, 3.3 Hz, 1H), 4.68 - 4.60 (m, 1H), 3.93 (s, 3H), 2.64 (s, 3H), 2.58 (m, 1H), 1.52 (d, *J* = 2.8 Hz, 3H).

**Example 113: Preparation of Compound 113**

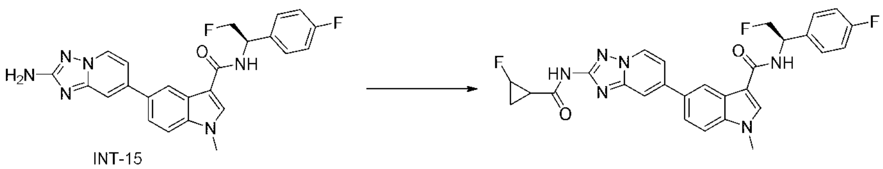

INT-15

Compound 113

Preparation of Compound 113:

Intermediate **INT-15** (88 mg, 0.2 mmol) was dissolved in pyridine (1 mL); 2-fluorocyclopropanecarboxylic acid (31 mg, 0.3 mmol) and 1-propylphosphonic anhydride (111 mg, 0.351 mmol) were added; the mixture was heated to 80°C

and stirred for 2 h. The reaction mixture was concentrated under reduced pressure and purified by C-18 reverse phase silica gel column (acetonitrile/0.1% aqueous ammonium bicarbonate solution = 60%) to obtain Compound 113 with a yield of 55%. MS (ESI, positive ion) m/z: 533.2 [M + H]$^+$.

$^1$H NMR (400 MHz, DMSO-$d_6$) δ 10.56 (s, 1H), 8.91 (d, *J* = 7.2 Hz, 1H), 8.57 (d, *J* = 1.6 Hz, 1H), 8.39 (d, *J* = 8.2 Hz, 1H), 8.22 (s, 1H), 7.92 (d, *J* = 1.9 Hz, 1H), 7.75 (dd, *J* = 8.4, 1.6 Hz, 1H), 7.65 (d, *J* = 8.7 Hz, 1H), 7.44 (ddt, *J* = 5.8, 4.1, 1.7 Hz, 3H), 7.21 - 7.12 (m, 2H), 5.23 (p, *J* = 7.2 Hz, 1H), 3.91 (s, 3H), 2.67 (m, 4H), 1.50 (d, *J* = 7.0 Hz, 3H), 1.20 (s, 3H).

**Example 114: Preparation of Compound 114**

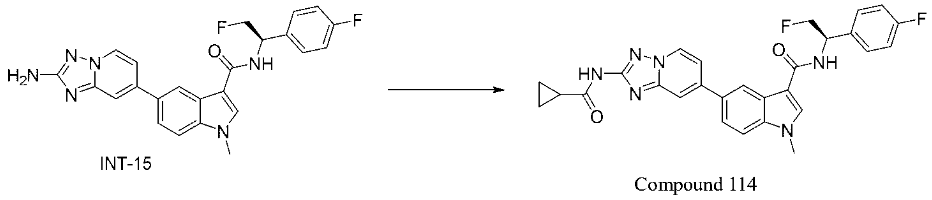

INT-15

Compound 114

Preparation of Compound 114:

Intermediate **INT-15** (88 mg, 0.2 mmol) was dissolved in pyridine (1 mL); cyclopropanecarboxylic acid (28 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 114 with a yield of 55%. MS (ESI, positive ion) m/z: 515.2 [M + H]$^+$.

$^1$H NMR (400 MHz, DMSO-$d_6$) δ 11.11 (s, 1H), 8.86 (d, *J* = 7.1 Hz, 1H), 8.67 (d, *J* = 8.3 Hz, 1H), 8.55 (d, *J* = 1.8 Hz, 1H), 8.30 (s, 1H), 7.90 (d, *J* = 2.0 Hz, 1H), 7.76 (dd, *J* = 8.6, 1.9 Hz, 1H), 7.69 (d, *J* = 8.7 Hz, 1H), 7.45 (ddd, *J* = 14.3, 6.8, 1.8 Hz, 2H), 7.40 - 7.33 (m, 2H), 7.20 - 7.11 (m, 1H), 5.51 (dq, *J* = 14.5, 6.6 Hz, 1H), 4.78 (d, *J* = 6.5 Hz, 1H), 4.66 (d, *J* = 6.5 Hz, 1H), 3.94 (s, 3H), 2.15 - 1.97 (m, 1H), 1.25 (d, *J* = 2.9 Hz, 4H).

**Example 115: Preparation of Compound 115**

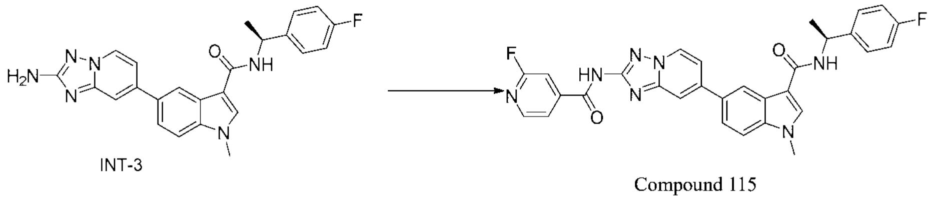

INT-3

Compound 115

Preparation of Compound 115:

Intermediate **INT-3** (83 mg, 0.2 mmol) was dissolved in pyridine (1 mL); cyclopropanecarboxylic acid (39 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 115 with a yield of 55%. MS (ESI, positive ion) m/z: 552.2 [M + H]$^+$.

$^1$H NMR (400 MHz, DMSO-$d_6$) δ 11.68 (s, 1H), 8.96 (dd, *J* = 7.1, 0.8 Hz, 1H), 8.58 (d, *J* = 1.8 Hz, 1H), 8.47 (d, *J* = 5.2 Hz, 1H), 8.38 (d, *J* = 8.0 Hz, 1H), 8.24 (s, 1H), 7.97 (dd, *J* = 2.0, 0.9 Hz, 1H), 7.90 (dt, *J* = 5.2, 1.7 Hz, 1H), 7.80 - 7.71 (m, 2H), 7.68 (d, *J* = 8.6 Hz, 1H), 7.54 (dd, *J* = 7.2, 2.0 Hz, 1H), 7.50 - 7.42 (m, 2H), 7.22 - 7.12 (m, 2H), 5.23 (p, *J* = 7.1 Hz, 1H), 3.92 (s, 3H), 1.51 (d, *J* = 7.1 Hz, 3H).

**Example 116: Preparation of Compound 116**

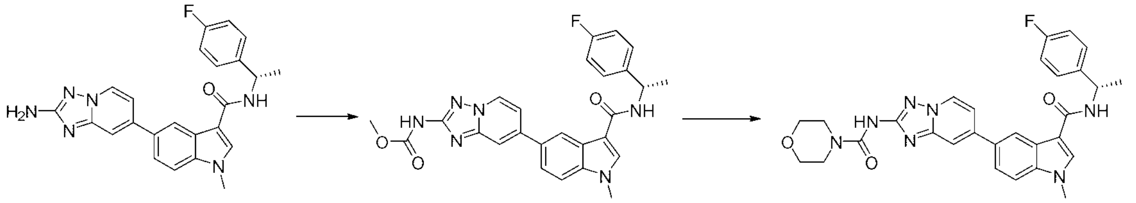

INT-3

Compound 116-1

Compound 116

Preparation of Compound 116-1:

Intermediate **INT-3** (42.8 g, 100 mmol) was dissolved in dichloromethane (1 L), methyl chloroformate (11.2 g, 120 mmol) and N,N-diisopropylethylamine (25.8 g, 200 mmol) were added, and the mixture was stirred at room temperature for 2 h. Petroleum ether (3 L) was added to the reaction mixture for crystallization; the solid was filtered with suction, washed with water, and dried to obtain Compound 116-1 with a yield of 65%. MS (ESI, positive ion) m/z: 487.2 $[M + H]^+$.

Preparation of Compound 116:

Compound 116-1 (97 mg, 0.2 mmol) was dissolved in acetonitrile (1 mL); morpholine (36 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 116 with a yield of 58%. MS (ESI, positive ion) m/z:542.2 $[M + H]^+$.

$^1$H NMR (400 MHz, DMSO-$d_6$) δ 9.60 (s, 1H), 8.85 - 8.79 (m, 1H), 8.60 - 8.48 (m, 1H), 8.37 (d, *J* = 7.9 Hz, 1H), 8.23 (s, 1H), 7.84 (dd, *J* = 2.1, 0.8 Hz, 1H), 7.74 (dd, *J* = 8.6, 1.9 Hz, 1H), 7.67 (d, *J* = 8.6 Hz, 1H), 7.49 - 7.45 (m, 2H), 7.43 (dd, *J* = 7.1, 2.0 Hz, 1H), 7.17 (t, *J* = 8.9 Hz, 2H), 5.21 (q, *J* = 7.2 Hz, 1H), 3.91 (s, 3H), 3.66 - 3.58 (m, 4H), 3.48 (t, *J* = 4.8 Hz, 4H), 1.50 (d, *J* = 7.1 Hz, 3H).

**Example 117: Preparation of Compound 117**

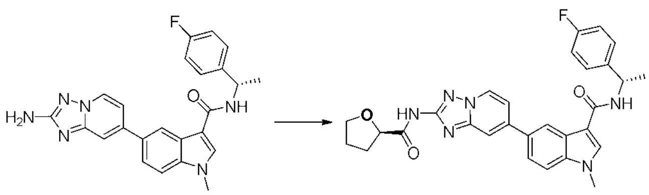

INT-3

Compound 117

Preparation of Compound 117:

**INT-3** (83 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 117 with a yield of 69%. MS (ESI, positive ion) m/z: 527.2 $[M + H]^+$.

$^1$H NMR (400 MHz, DMSO-$d_6$) δ 10.47 (s, 1H), 8.93 - 8.86 (m, 1H), 8.56 (d, *J* = 1.9 Hz, 1H), 8.37 (d, *J* = 8.0 Hz, 1H), 8.23 (s, 1H), 7.94 - 7.89 (m, 1H), 7.75 (dd, *J* = 8.7, 1.9 Hz, 1H), 7.68 (d, *J* = 8.6 Hz, 1H), 7.57 - 7.43 (m, 3H), 7.22 - 7.12 (m, 2H), 5.23 (p, *J* = 7.1 Hz, 1H), 4.54 (s, 1H), 4.02 (q, *J* = 6.9 Hz, 1H), 3.92 (s, 3H), 3.85 (ddd, *J* = 7.9, 7.0, 5.9 Hz, 1H), 2.31 - 2.18 (m, 1H), 2.06 - 1.95 (m, 1H), 1.95 - 1.81 (m, 2H), 1.51 (d, *J* = 7.1 Hz, 3H).

**Example 118: Preparation of Compound 118**

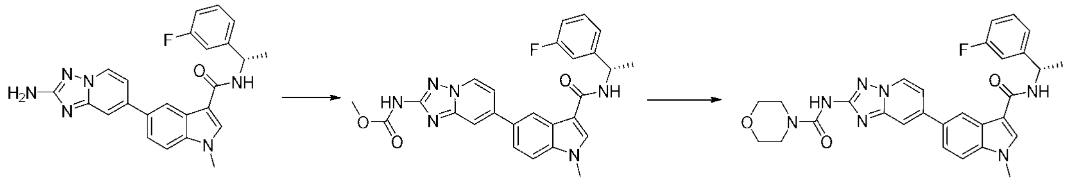

INT-4 Compound 118-1 Compound 118

Preparation of Compound 118-1:

Intermediate **INT-4** (42.8 g, 100 mmol) was dissolved in dichloromethane (1 L), methyl chloroformate (11.2 g, 120 mmol) and N,N-diisopropylethylamine (25.8 g, 200 mmol) were added, and the mixture was stirred at room temperature for 2 h. Petroleum ether (3 L) was added to the reaction mixture for crystallization; the solid was filtered with suction, washed with water, and dried to obtain Compound 118-1 with a yield of 65%. MS (ESI, positive ion) m/z: 487.2 $[M + H]^+$.

Preparation of Compound 118:

Compound 118-1 (97 mg, 0.2 mmol) was dissolved in acetonitrile (1 mL); morpholine (36 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 118 with a yield of 58%. MS (ESI, positive ion) m/z:542.2 $[M + H]^+$.

$^1H$ NMR (400 MHz, DMSO-$d_6$) δ 10.45 (s, 1H), 8.85 (d, *J* = 7.2 Hz, 1H), 8.53 (s, 1H), 8.42 (d, *J* = 8.0 Hz, 1H), 8.24 (s, 1H), 7.93 (s, 1H), 7.76 (d, *J* = 8.6 Hz, 1H), 7.69 (d, *J* = 8.7 Hz, 1H), 7.48 (d, *J* = 7.2 Hz, 1H), 7.42 (q, *J* = 7.5 Hz, 1H), 7.27 (t, *J* = 9.0 Hz, 2H), 7.11 - 7.02 (m, 1H), 5.25 (p, *J* = 7.2 Hz, 1H), 3.92 (s, 3H),3.50 (m, 4H),2.67 (m, 4H), 1.52 (d, *J* = 7.1 Hz, 3H).

**Example 119: Preparation of Compound 119**

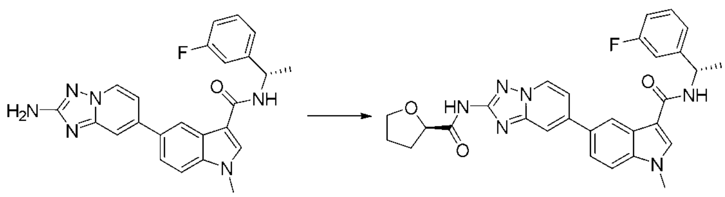

INT-4 Compound 119

Preparation of Compound 119:

**INT-4** (83 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 119 with a yield of 69%. MS (ESI, positive ion) m/z: 527.2 $[M + H]^+$.

$^1H$ NMR (400 MHz, DMSO-$d_6$) δ 10.47 (s, 1H), 8.89 (d, *J* = 7.2 Hz, 1H), 8.56 (s, 1H), 8.41 (d, *J* = 8.0 Hz, 1H), 8.25 (s, 1H), 7.91 (s, 1H), 7.75 (d, *J* = 8.6 Hz, 1H), 7.68 (d, *J* = 8.7 Hz, 1H), 7.49 (d, *J* = 7.2 Hz, 1H), 7.40 (q, *J* = 7.5 Hz, 1H), 7.27 (t, *J* = 9.0 Hz, 2H), 7.11 - 7.02 (m, 1H), 5.25 (p, *J* = 7.2 Hz, 1H), 4.01 (t, *J* = 7.1 Hz, 1H), 3.92 (s, 3H), 3.82 (dt, *J* = 14.0, 7.1 Hz, 2H), 2.22 (ddq, *J* = 26.5, 12.3, 7.3, 6.4 Hz, 2H), 1.93 - 1.84 (m, 3H), 1.52 (d, *J* = 7.1 Hz, 3H).

**Example 120: Preparation of Compound 120**

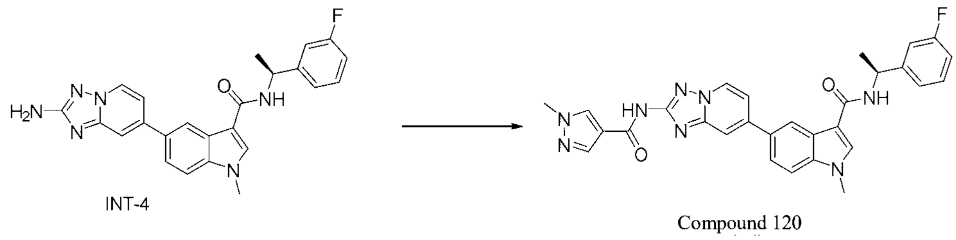

INT-4

Compound 120

Preparation of Compound 120:

Intermediate **INT-4** (83 mg, 0.2 mmol) was dissolved in pyridine (1 mL); 1-methylpyrazole-4-carboxylic acid (38 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 120 with a yield of 45%. MS (ESI, positive ion) m/z: 537.2 $[M + H]^+$.

$^1$H NMR (400 MHz, DMSO-$d_6$) δ 10.47 (s, 1H), 8.91 (dd, *J* = 7.2, 0.8 Hz, 1H), 8.65 (d, *J* = 1.9 Hz, 1H), 8.38 (d, *J* = 8.2 Hz, 1H), 8.18 (s, 1H), 7.95 (dd, *J* = 1.9, 0.9 Hz, 1H), 7.76 (dd, *J* = 8.7, 1.9 Hz, 1H), 7.69 (d, *J* = 8.6 Hz, 1H), 7.53 (dd, *J* = 7.2, 2.0 Hz, 1H), 7.32 (dd, *J* = 8.1, 5.4 Hz, 1H), 7.06 (td, *J* = 8.6, 7.6, 1.7 Hz, 2H), 5.60 (q, *J* = 8.1 Hz, 1H), 4.54 (s, 1H), 4.07 - 3.94 (m, 1H), 3.90 (s, 3H), 3.88 - 3.82 (m, 1H), 3.05 - 2.94 (m, 1H), 2.85 (dt, *J* = 16.1, 8.5 Hz, 1H), 2.56 (dd, *J* = 7.9, 3.1 Hz, 1H), 2.32 - 2.16 (m, 1H), 2.09 - 1.88 (m, 4H).

**Example 121: Preparation of Compound 121**

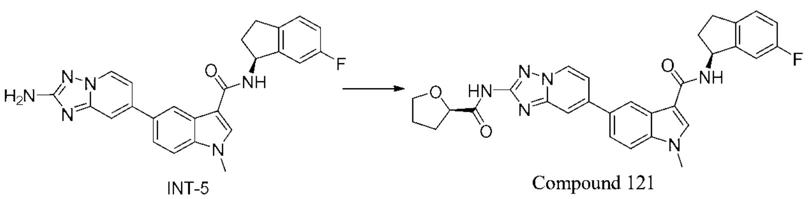

INT-5

Compound 121

Preparation of Compound 121:

**INT-5** (88 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 121 with a yield of 71%. MS (ESI, positive ion) m/z: 539.2 $[M + H]^+$.

$^1$H NMR (400 MHz, DMSO-$d_6$) δ 10.47 (s, 1H), 8.91 (dd, *J* = 7.2, 0.8 Hz, 1H), 8.65 (d, *J* = 1.9 Hz, 1H), 8.38 (d, *J* = 8.2 Hz, 1H), 8.18 (s, 1H), 7.95 (dd, *J* = 1.9, 0.9 Hz, 1H), 7.76 (dd, *J* = 8.7, 1.9 Hz, 1H), 7.69 (d, *J* = 8.6 Hz, 1H), 7.53 (dd, *J* = 7.2, 2.0 Hz, 1H), 7.32 (dd, *J* = 8.1, 5.4 Hz, 1H), 7.06 (td, *J* = 8.6, 7.6, 1.7 Hz, 2H), 5.60 (q, *J* = 8.1 Hz, 1H), 4.54 (s, 1H), 4.07 - 3.94 (m, 1H), 3.90 (s, 3H), 3.88 - 3.82 (m, 1H), 3.05 - 2.94 (m, 1H), 2.85 (dt, *J* = 16.1, 8.5 Hz, 1H), 2.56 (dd, *J* = 7.9, 3.1 Hz, 1H), 2.32 - 2.16 (m, 1H), 2.09 - 1.88 (m, 4H).

**Example 122: Preparation of Compound 122**

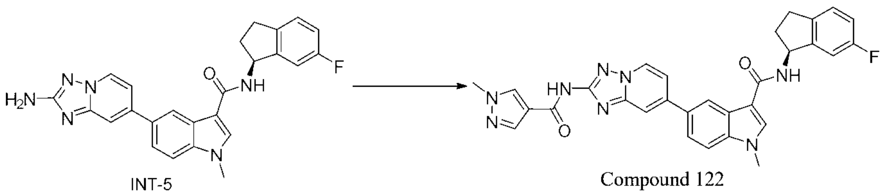

INT-5

Compound 122

Preparation of Compound 122:

Intermediate **INT-5** (88 mg, 0.2 mmol) was dissolved in pyridine (1 mL); 1-methylpyrazole-4-carboxylic acid (38 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 122 with a yield of 44%. MS (ESI, positive ion) m/z: 549.2 $[M + H]^+$.

$^1H$ NMR (400 MHz, DMSO-$d_6$) δ 10.92 (s, 1H), 8.93 (d, *J* = 7.1 Hz, 1H), 8.66 (d, *J* = 1.8 Hz, 1H), 8.44 (s, 1H), 8.39 (d, *J* = 8.3 Hz, 1H), 8.18 (s, 1H), 8.14 (s, 1H), 7.95 (d, *J* = 1.9 Hz, 1H), 7.77 (dd, *J* = 8.6, 1.9 Hz, 1H), 7.69 (d, *J* = 8.6 Hz, 1H), 7.53 (dd, *J* = 7.2, 2.0 Hz, 1H), 7.31 (dd, *J* = 8.8, 5.2 Hz, 1H), 7.06 (t, *J* = 7.8 Hz, 2H), 5.62 (t, *J* = 8.1 Hz, 1H), 3.91 (d, *J* = 7.9 Hz, 6H), 3.05 - 2.94 (m, 1H), 2.85 (dt, *J* = 16.4, 8.7 Hz, 1H), 2.57 (d, *J* = 8.8 Hz, 1H), 2.05 (dq, *J* = 12.3, 8.8 Hz, 1H).

**Example 123: Preparation of Compound 123**

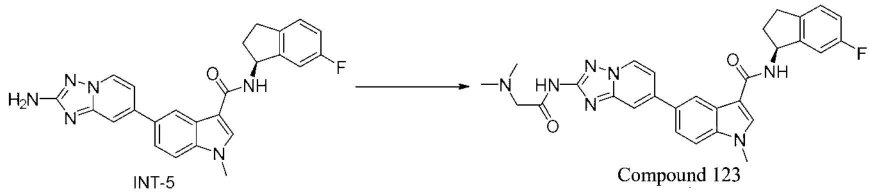

INT-5

Compound 123

Preparation of Compound 123:

Intermediate **INT-5** (88 mg, 0.2 mmol) was dissolved in pyridine (1 mL); 2-dimethylaminoacetic acid (31 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 123 with a yield of 54%. MS (ESI, positive ion) m/z: 526.2 $[M + H]^+$.

$^1H$ NMR (400 MHz, DMSO-$d_6$) δ 10.45 (s, 1H), 8.91 (d, *J* = 7.1 Hz, 1H), 8.65 (d, *J* = 1.9 Hz, 1H), 8.38 (d, *J* = 8.2 Hz, 1H), 8.18 (s, 1H), 7.97 - 7.92 (m, 1H), 7.77 (dd, *J* = 8.6, 1.9 Hz, 1H), 7.69 (d, *J* = 8.7 Hz, 1H), 7.52 (dd, *J* = 7.1, 2.0 Hz, 1H), 7.36 - 7.28 (m, 1H), 7.06 (d, *J* = 9.1 Hz, 2H), 5.61 (q, *J* = 8.1 Hz, 1H), 3.90 (s, 3H), 3.22 (s, 2H), 3.05 - 2.95 (m, 1H), 2.86 (dt, *J* = 16.0, 8.5 Hz, 1H), 2.56 (s, 1H), 2.33 (s, 6H), 2.05 (dq, *J* = 12.5, 8.8 Hz, 1H).

**Example 124: Preparation of Compound 124**

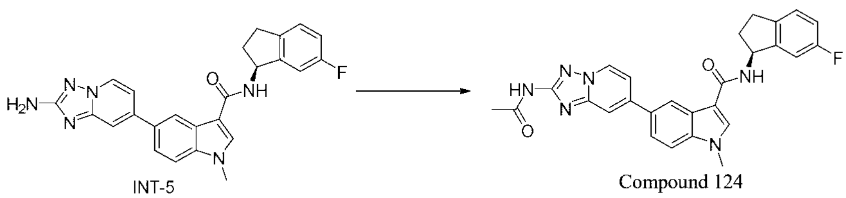

INT-5

Compound 124

Preparation of Compound 124:

Intermediate **INT-5** (88 mg, 0.2 mmol) was dissolved in pyridine (1 mL); acetic acid (18 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 124 with a yield of 55%. MS (ESI, positive ion) m/z: 483.2 $[M + H]^+$.

$^1H$ NMR (400 MHz, DMSO-$d_6$) δ 10.79 (s, 1H), 8.89 (dd, *J* = 7.1, 0.9 Hz, 1H), 8.64 (d, *J* = 1.9 Hz, 1H), 8.37 (d, *J* = 8.2 Hz, 1H), 8.17 (s, 1H), 7.93 (dd, *J* = 2.1, 0.9 Hz, 1H), 7.76 (dd, *J* = 8.6, 1.9 Hz, 1H), 7.69 (d, *J* = 8.7 Hz, 1H), 7.50 (dd, *J* = 7.1, 2.0 Hz, 1H), 7.35 - 7.29 (m, 1H), 7.06 (t, *J* = 8.4 Hz, 2H), 5.60 (q, *J* = 8.1 Hz, 1H), 3.90 (s, 3H), 3.00 (dd, *J* = 13.8, 8.6 Hz, 1H), 2.86 (dt, *J* = 16.0, 8.3 Hz, 1H), 2.55 (d, *J* = 5.6 Hz, 1H), 2.18 (s, 3H), 2.05 (dq, *J* = 12.4, 8.8 Hz, 1H).

**Example 125: Preparation of Compound 125**

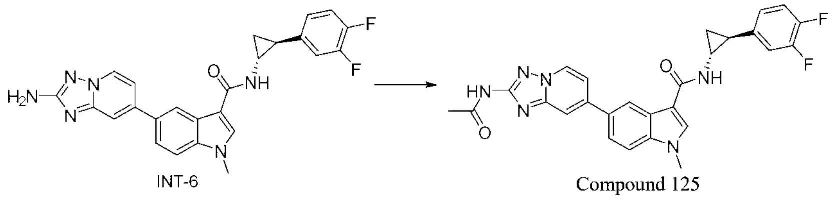

INT-6

Compound 125

Preparation of Compound 125:

Intermediate **INT-6** (91 mg, 0.2 mmol) was dissolved in pyridine (1 mL); acetic acid (18 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 125 with a yield of 55%. MS (ESI, positive ion) m/z: 501.2 $[M + H]^+$.

$^1H$ NMR (400 MHz, DMSO-$d_6$) δ 10.79 (s, 1H), 8.88 (dd, *J* = 7.1, 0.8 Hz, 1H), 8.57 (d, *J* = 1.8 Hz, 1H), 8.30 (d, *J* = 3.8 Hz, 1H), 8.07 (s, 1H), 7.91 (dd, *J* = 2.0, 0.9 Hz, 1H), 7.75 (dd, *J* = 8.6, 1.9 Hz, 1H), 7.67 (d, *J* = 8.7 Hz, 1H), 7.48 (dd, *J* = 7.2, 2.0 Hz, 1H), 7.36 (dt, *J* = 10.9, 8.6 Hz, 1H), 7.28 (ddd, *J* = 12.2, 7.8, 2.2 Hz, 1H), 7.13 - 7.06 (m, 1H), 3.90 (s, 3H), 3.06 - 3.00 (m, 1H), 2.21 (d, *J* = 21.2 Hz, 3H), 2.13 - 2.05 (m, 1H), 1.41 - 1.35 (m, 1H), 1.33 - 1.23 (m, 1H).

**Example 126: Preparation of Compound 126**

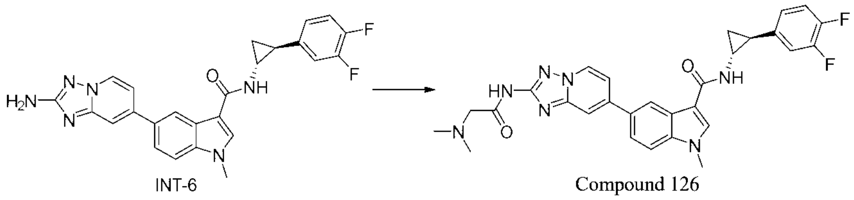

INT-6

Compound 126

Preparation of Compound 126:

Intermediate **INT-6** (91 mg, 0.2 mmol) was dissolved in pyridine (1 mL); 2-dimethylaminoacetic acid (31 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 126 with a yield of 54%. MS (ESI, positive ion) m/z: 544.2 $[M + H]^+$.

$^1H$ NMR (400 MHz, DMSO-$d_6$) δ 10.45 (s, 1H), 8.93 - 8.86 (m, 1H), 8.57 (d, *J* = 1.9 Hz, 1H), 8.30 (d, *J* = 3.9 Hz, 1H), 8.07 (s, 1H), 7.92 (dd, *J* = 2.0, 0.8 Hz, 1H), 7.75 (dd, *J* = 8.7, 1.9 Hz, 1H), 7.68 (d, *J* = 8.6 Hz, 1H), 7.50 (dd, *J* = 7.2, 2.0 Hz, 1H), 7.42 - 7.23 (m, 2H), 7.10 (s, 1H), 3.91 (s, 3H), 3.21 (s, 2H), 3.02 (dd, *J* = 8.0, 4.2 Hz, 1H), 2.33 (s, 6H), 2.13 - 2.04 (m, 1H), 1.38 (dt, *J* = 9.8, 5.3 Hz, 1H), 1.33 - 1.24 (m, 1H).

**Example 127: Preparation of Compound 127**

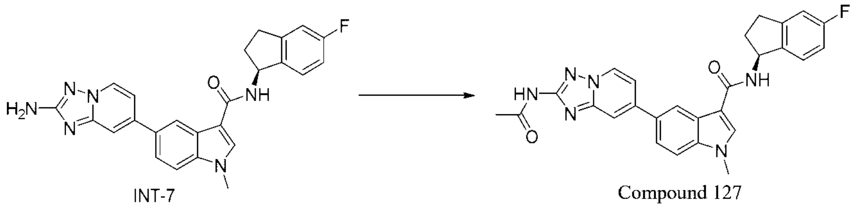

INT-7

Compound 127

Preparation of Compound 127:

Intermediate **INT-7** (88 mg, 0.2 mmol) was dissolved in pyridine (1 mL); acetic acid (18 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 127 with a yield of 53%. MS (ESI, positive ion) m/z: 483.2 $[M + H]^+$.

$^1$H NMR (400 MHz, DMSO-$d_6$) δ 10.80 (s, 1H), 8.90 (d, *J* = 7.0 Hz, 1H), 8.64 (d, *J* = 1.9 Hz, 1H), 8.33 (d, *J* = 8.3 Hz, 1H), 8.16 (s, 1H), 7.95 - 7.90 (m, 1H), 7.76 (dd, *J* = 8.6, 1.9 Hz, 1H), 7.68 (d, *J* = 8.7 Hz, 1H), 7.50 (dd, *J* = 7.1, 2.0 Hz, 1H), 7.31 (dd, *J* = 8.4, 5.4 Hz, 1H), 7.13 (d, *J* = 9.5 Hz, 1H), 7.03 (t, *J* = 8.8 Hz, 1H), 5.58 (q, *J* = 8.0 Hz, 1H), 3.89 (s, 3H), 3.03 (dd, *J* = 13.2, 10.0 Hz, 1H), 2.89 (dt, *J* = 16.3, 8.5 Hz, 1H), 2.60 - 2.54 (m, 1H), 2.18 (s, 3H), 2.05 (dq, *J* = 12.6, 8.7 Hz, 1H).

**Example 128: Preparation of Compound 128**

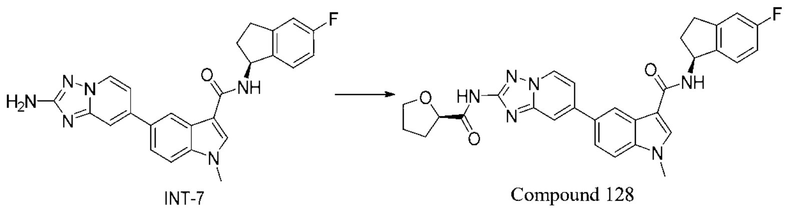

INT-7

Compound 128

Preparation of Compound 128:

**INT-7** (88 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 128 with a yield of 71%. MS (ESI, positive ion) m/z: 539.2 $[M + H]^+$.

$^1$H NMR (400 MHz, DMSO-$d_6$) δ 10.47 (s, 1H), 8.92 (d, *J* = 7.1 Hz, 1H), 8.64 (d, *J* = 1.8 Hz, 1H), 8.33 (d, *J* = 8.3 Hz, 1H), 8.16 (s, 1H), 7.97 - 7.92 (m, 1H), 7.76 (dd, *J* = 8.6, 1.9 Hz, 1H), 7.68 (d, *J* = 8.7 Hz, 1H), 7.53 (dd, *J* = 7.2, 2.0 Hz, 1H), 7.31 (dd, *J* = 8.3, 5.4 Hz, 1H), 7.13 (d, *J* = 9.4 Hz, 1H), 7.03 (t, *J* = 8.9 Hz, 1H), 5.58 (q, *J* = 7.9 Hz, 1H), 4.54 (s, 1H), 4.02 (q, *J* = 7.0 Hz, 1H), 3.89 (s, 3H), 3.85 (q, *J* = 7.2 Hz, 1H), 3.09 - 2.99 (m, 1H), 2.89 (dt, *J* = 16.3, 8.5 Hz, 1H), 2.59 - 2.54 (m, 1H), 2.31 - 2.16 (m, 1H), 2.11 - 1.83 (m, 4H).

**Example 129: Preparation of Compound 129**

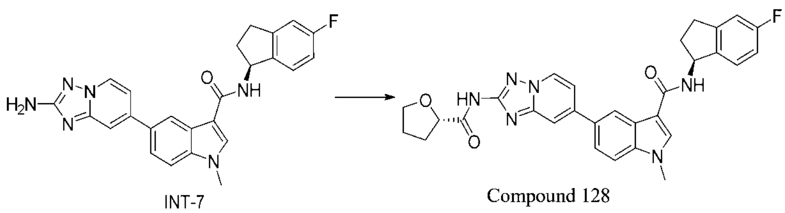

INT-7

Compound 128

Preparation of Compound 129:

**INT-7** (88 mg, 0.2 mmol) was dissolved in pyridine (1 mL), (S)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 129 with a yield of 61%. MS (ESI, positive ion) m/z: 539.2 $[M + H]^+$.

$^1$H NMR (400 MHz, DMSO-$d_6$) δ 10.48 (s, 1H), 8.95 - 8.89 (m, 1H), 8.65 (d, *J* = 1.9 Hz, 1H), 8.33 (d, *J* = 8.3 Hz, 1H), 8.16 (s, 1H), 7.95 (dd, *J* = 2.0, 0.9 Hz, 1H), 7.77 (dd, *J* = 8.6, 1.9 Hz, 1H), 7.69 (d, *J* = 8.6 Hz, 1H), 7.53 (dd, *J* = 7.2, 2.0 Hz, 1H), 7.31 (dd, *J* = 8.3, 5.4 Hz, 1H), 7.13 (d, *J* = 9.0 Hz, 1H), 7.03 (t, *J* = 8.9 Hz, 1H), 5.58 (q, *J* = 8.0 Hz, 1H), 4.54 (s, 1H), 4.02 (q, *J* = 7.0 Hz, 1H), 3.89 (s, 3H), 3.85 (q, *J* = 7.0 Hz, 1H), 3.09 - 2.99 (m, 1H), 2.89 (dt, *J* = 16.2, 8.4 Hz, 1H), 2.58 - 2.54 (m, 1H), 2.25 (dq, *J* = 11.3, 7.8 Hz, 1H), 2.09 - 1.87 (m, 4H).

**Example 130: Preparation of Compound 130**

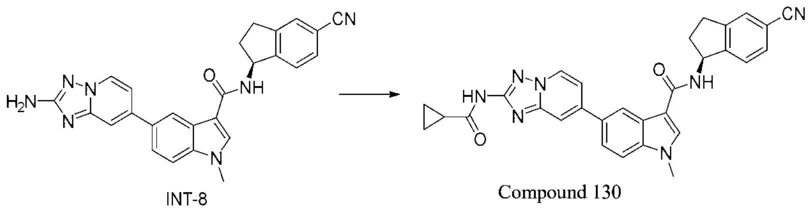

INT-8 Compound 130

Preparation of Compound 130:

**INT-8** (89 mg, 0.2 mmol) was dissolved in pyridine (1 mL), cyclopropanecarboxylic acid (26 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 130 with a yield of 61%. MS (ESI, positive ion) m/z: 516.2 $[M + H]^+$.

$^1$H NMR (400 MHz, DMSO-$d_6$) δ 11.08 (s, 1H), 8.88 (d, *J* = 7.0 Hz, 1H), 8.63 (d, *J* = 1.9 Hz, 1H), 8.43 (d, *J* = 8.2 Hz, 1H), 8.15 (s, 1H), 7.92 (d, *J* = 1.9 Hz, 1H), 7.80 - 7.73 (m, 2H), 7.69 (dd, *J* = 8.5, 3.1 Hz, 2H), 7.53 - 7.44 (m, 2H), 5.66 (q, *J* = 8.5 Hz, 1H), 3.90 (s, 3H), 3.13 - 3.03 (m, 1H), 2.95 (dt, *J* = 16.4, 8.7 Hz, 1H), 2.14 - 2.02 (m, 2H), 1.30 (ddd, *J* = 4.0, 3.0, 2.0 Hz, 1H), 0.86 (d, *J* = 6.1 Hz, 4H).

**Example 131: Preparation of Compound 131**

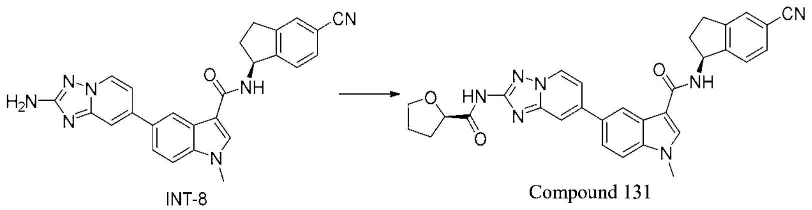

INT-8 Compound 131

Preparation of Compound 131:

**INT-8** (89 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 131 with a yield of 71%. MS (ESI, positive ion) m/z: 546.2 $[M + H]^+$.

$^1$H NMR (400 MHz, DMSO-$d_6$) δ 10.47 (s, 1H), 8.92 (d, *J* = 7.3 Hz, 1H), 8.64 (d, *J* = 1.9 Hz, 1H), 8.43 (d, *J* = 8.3 Hz, 1H), 8.15 (s, 1H), 7.95 (dd, *J* = 2.0, 0.8 Hz, 1H), 7.81 - 7.74 (m, 2H), 7.73 - 7.65 (m, 2H), 7.53 (dd, *J* = 7.2, 2.0 Hz, 1H), 7.48 (d,

*J* = 7.9 Hz, 1H), 5.66 (q, *J* = 8.3 Hz, 1H), 4.54 (s, 1H), 4.02 (q, *J* = 7.0 Hz, 1H), 3.90 (s, 3H), 3.85 (q, *J* = 7.0 Hz, 1H), 3.14 - 3.03 (m, 1H), 2.95 (dt, *J* = 16.5, 8.7 Hz, 1H), 2.59 - 2.54 (m, 1H), 2.32 - 2.18 (m, 1H), 2.14 - 1.83 (m, 4H).

**Example 132: Preparation of Compound 132**

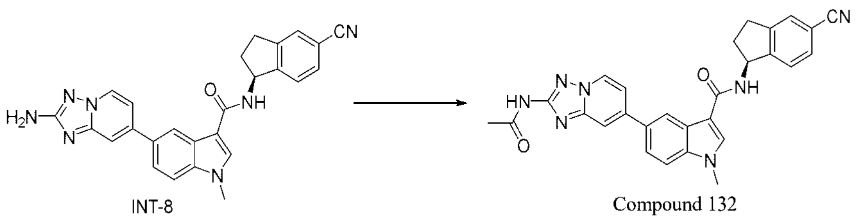

INT-8

Compound 132

Preparation of Compound 132:

Intermediate **INT-8** (89 mg, 0.2 mmol) was dissolved in pyridine (1 mL); acetic acid (18 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 132 with a yield of 53%. MS (ESI, positive ion) m/z: 490.2 $[M + H]^+$.

$^1H$ NMR (400 MHz, DMSO-$d_6$) δ 10.80 (s, 1H), 8.90 (dd, *J* = 7.1, 0.8 Hz, 1H), 8.63 (d, *J* = 2.1 Hz, 1H), 8.43 (d, *J* = 8.3 Hz, 1H), 8.15 (s, 1H), 7.93 (dd, *J* = 1.9, 0.9 Hz, 1H), 7.80 - 7.73 (m, 2H), 7.69 (dd, *J* = 8.9, 2.8 Hz, 2H), 7.53 - 7.44 (m, 2H), 5.66 (q, *J* = 8.3 Hz, 1H), 3.90 (s, 3H), 3.13 - 3.03 (m, 1H), 2.95 (dt, *J* = 16.2, 8.5 Hz, 1H), 2.57 (dd, *J* = 8.1, 2.7 Hz, 1H), 2.18 (s, 3H), 2.07 (dq, *J* = 12.4, 8.9 Hz, 1H).

**Example 133: Preparation of Compound 133**

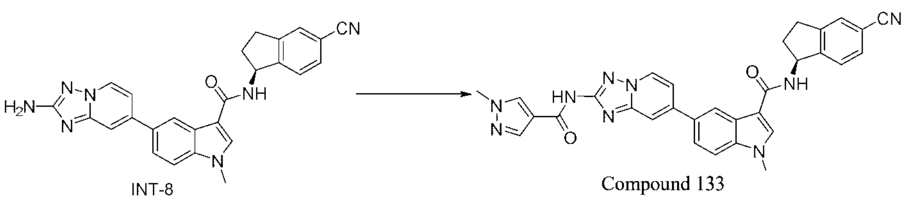

INT-8

Compound 133

Preparation of Compound 133:

Intermediate **INT-8** (89 mg, 0.2 mmol) was dissolved in pyridine (1 mL); 1-methylpyrazole-4-carboxylic acid (38 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 133 with a yield of 44%. MS (ESI, positive ion) m/z: 556.2 $[M + H]^+$.

$^1H$ NMR (400 MHz, DMSO-$d_6$) δ 10.92 (s, 1H), 8.94 (dd, *J* = 7.1, 0.8 Hz, 1H), 8.65 (d, *J* = 2.0 Hz, 1H), 8.44 (d, *J* = 7.3 Hz, 2H), 8.18 - 8.07 (m, 2H), 7.96 (dd, *J* = 2.0, 0.9 Hz, 1H), 7.82 - 7.75 (m, 2H), 7.73 - 7.65 (m, 2H), 7.53 (dd, *J* = 7.2, 2.0 Hz, 1H), 7.48 (d, *J* = 7.8 Hz, 1H), 5.66 (q, *J* = 8.4 Hz, 1H), 3.92 (s, 3H), 3.91 (s, 3H), 3.08 (dd, *J* = 15.5, 9.1 Hz, 1H), 2.95 (dt, *J* = 16.5, 8.7 Hz, 1H), 2.60 - 2.54 (m, 1H), 2.14 - 2.00 (m, 1H).

**Example 134: Preparation of Compound 134**

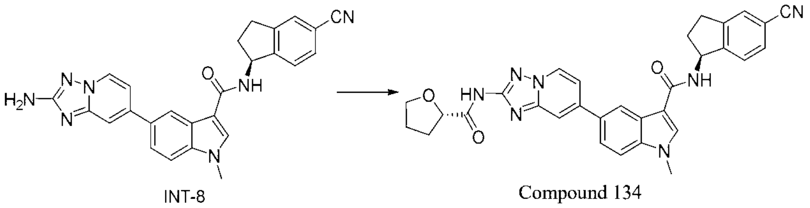

INT-8 Compound 134

Preparation of Compound 134:

**INT-8** (89 mg, 0.2 mmol) was dissolved in pyridine (1 mL), (S)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 134 with a yield of 61%. MS (ESI, positive ion) m/z: 546.2 $[M + H]^+$.

$^1H$ NMR (400 MHz, DMSO-$d_6$) δ 10.49 (s, 1H), 8.93 (d, *J* = 7.1 Hz, 1H), 8.64 (d, *J* = 1.8 Hz, 1H), 8.45 (d, *J* = 8.2 Hz, 1H), 8.16 (s, 1H), 7.98 - 7.93 (m, 1H), 7.81 - 7.74 (m, 2H), 7.73 - 7.66 (m, 2H), 7.53 (dd, *J* = 7.1, 2.0 Hz, 1H), 7.48 (d, *J* = 7.8 Hz, 1H), 5.67 (q, *J* = 8.3 Hz, 1H), 4.54 (s, 1H), 4.02 (q, *J* = 6.9 Hz, 1H), 3.91 (s, 3H), 3.85 (q, *J* = 7.1 Hz, 1H), 3.14 - 3.03 (m, 1H), 2.95 (dt, *J* = 16.4, 8.6 Hz, 1H), 2.60 - 2.55 (m, 1H), 2.32 - 2.19 (m, 1H), 2.14 - 1.84 (m, 4H).

**Example 135: Preparation of Compound 135**

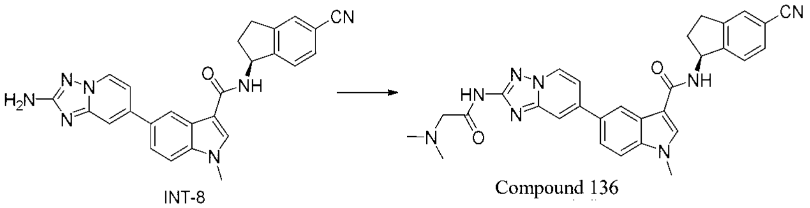

INT-8 Compound 136

Preparation of Compound 135:

Intermediate **INT-8** (89 mg, 0.2 mmol) was dissolved in pyridine (1 mL); 2-dimethylaminoacetic acid (31 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 135 with a yield of 54%. MS (ESI, positive ion) m/z: 533.2 $[M + H]^+$.

$^1H$ NMR (400 MHz, DMSO-$d_6$) δ 8.93 (d, *J* = 7.2 Hz, 1H), 8.64 (d, *J* = 1.8 Hz, 1H), 8.45 (d, *J* = 8.3 Hz, 1H), 8.16 (s, 1H), 7.99 - 7.94 (m, 1H), 7.81 - 7.74 (m, 2H), 7.73 - 7.66 (m, 2H), 7.55 (dd, *J* = 7.2, 2.0 Hz, 1H), 7.47 (d, *J* = 8.0 Hz, 1H), 5.66 (q, *J* = 8.4 Hz, 1H), 3.90 (s, 3H), 3.72 (s, 2H), 3.08 (dd, *J* = 15.1, 7.9 Hz, 1H), 2.95 (dt, *J* = 16.5, 8.6 Hz, 1H), 2.60 (d, *J* = 17.7 Hz, 6H), 2.58 - 2.55 (m, 1H), 2.14 - 2.00 (m, 1H).

**Example 136: Preparation of Compound 136**

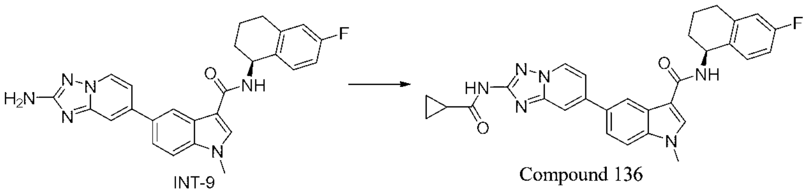

INT-9 Compound 136

Preparation of Compound 136:

**INT-9** (90 mg, 0.2 mmol) was dissolved in pyridine (1 mL), cyclopropanecarboxylic acid (26 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 136 with a yield of 61%. MS (ESI, positive ion) m/z: 523.2 $[M + H]^+$.

$^1H$ NMR (400 MHz, DMSO-$d_6$) δ 11.16 (s, 1H), 8.89 (d, *J*= 7.1 Hz, 1H), 8.67 (d, *J*= 1.8 Hz, 1H), 8.33 (d, *J* = 8.6 Hz, 1H), 8.19 (s, 1H), 7.94 (d, *J* = 2.4 Hz, 1H), 7.75 (dd, *J* = 8.7, 1.9 Hz, 1H), 7.67 (d, *J= 8.6* Hz, 1H), 7.50 (dd, *J*= 7.2, 2.0 Hz, 1H), 7.31 (dd, J= 8.2, 6.1 Hz, 1H), 6.99 (t, *J* = 8.8 Hz, 2H), 5.27 (q, *J* = 8.4, 7.5 Hz, 1H), 3.88 (s, 3H), 2.91 - 2.73 (m, 2H), 2.00 (p, *J* = 7.4, 6.5 Hz, 2H), 1.92 - 1.73 (m, 2H), 0.93 - 0.80 (m, 5H).

Example 137: Preparation of Compound 137

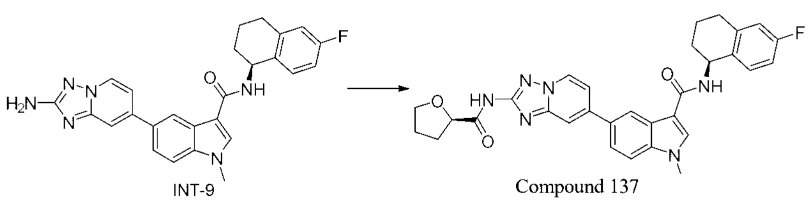

INT-9

Compound 137

Preparation of Compound 137:

INT-9 (90 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 137 with a yield of 71%. MS (ESI, positive ion) m/z: 553.2 $[M + H]^+$.

$^1H$ NMR (400 MHz, DMSO-$d_6$) δ 10.48 (s, 1H), 8.92 (dd, *J* = 7.2, 0.8 Hz, 1H), 8.66 (d, *J* = 1.8 Hz, 1H), 8.32 (d, *J* = 8.6 Hz, 1H), 8.18 (s, 1H), 7.94 (dd, *J* = 2.0, 0.9 Hz, 1H), 7.76 (dd, *J* = 8.6, 1.9 Hz, 1H), 7.68 (d, *J* = 8.6 Hz, 1H), 7.52 (dd, *J* = 7.1, 2.0 Hz, 1H), 7.36 - 7.27 (m, 1H), 6.99 (d, *J* = 9.2 Hz, 2H), 5.27 (t, *J* = 6.5 Hz, 1H), 4.02 (q, *J* = 7.0 Hz, 1H), 3.89 (s, 3H), 3.88 - 3.80 (m, 2H), 2.83 (dt, *J* = 7.3, 4.2 Hz, 2H), 1.99 (dt, *J* = 15.2, 8.1 Hz, 4H), 1.91 (q, *J* = 7.0 Hz, 2H), 1.84 (dd, *J* = 18.7, 10.8 Hz, 2H).

**Example 138: Preparation of Compound 138**

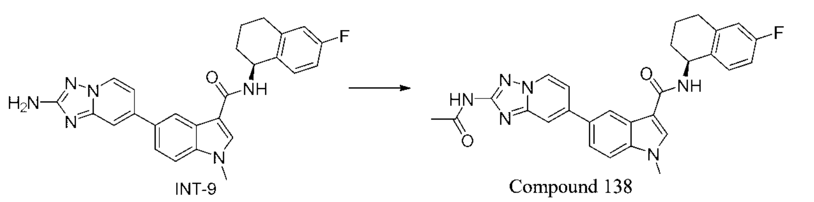

INT-9

Compound 138

Preparation of Compound 138:

**INT-9** (90 mg, 0.2 mmol) was dissolved in pyridine (1 mL), acetic acid (18 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 138 with a yield of 61%. MS (ESI, positive ion) m/z: 497.2 $[M + H]^+$.

$^1H$ NMR (400 MHz, DMSO-$d_6$) δ 10.80 (s, 1H), 8.90 (d, *J* = 7.0 Hz, 1H), 8.65 (d, *J* = 1.9 Hz, 1H), 8.32 (d, *J* = 8.6 Hz, 1H), 8.17 (s, 1H), 7.95 - 7.90 (m, 1H), 7.76 (dd, *J* = 8.7, 1.8 Hz, 1H), 7.68 (d, *J* = 8.6 Hz, 1H), 7.50 (dd, *J* = 7.1, 2.0 Hz, 1H), 7.36 - 7.28 (m, 1H), 7.00 (t, *J* = 8.5 Hz, 2H), 5.27 (s, 1H), 3.89 (s, 3H), 2.82 (d, *J* = 6.9 Hz, 2H), 2.18 (s, 3H), 2.00 (d, *J* = 4.9 Hz,

2H), 1.91 - 1.75 (m, 2H).

**Example 139: Preparation of Compound 139**

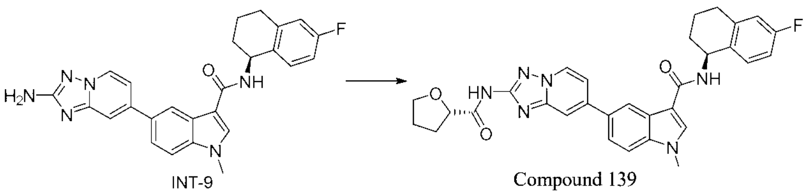
INT-9
Compound 139

Preparation of Compound 139:

**INT-9** (90 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 139 with a yield of 68%. MS (ESI, positive ion) m/z: 553.2 $[M + H]^+$.

$^1$H NMR (400 MHz, DMSO-$d_6$) δ 10.48 (s, 1H), 8.92 (dd, *J* = 7.2, 0.8 Hz, 1H), 8.66 (d, *J* = 1.8 Hz, 1H), 8.32 (d, *J* = 8.6 Hz, 1H), 8.18 (s, 1H), 7.94 (dd, *J* = 2.0, 0.9 Hz, 1H), 7.76 (dd, *J* = 8.6, 1.9 Hz, 1H), 7.68 (d, *J* = 8.6 Hz, 1H), 7.52 (dd, *J* = 7.1, 2.0 Hz, 1H), 7.36 - 7.27 (m, 1H), 6.99 (d, *J* = 9.2 Hz, 2H), 5.27 (t, *J* = 6.5 Hz, 1H), 4.02 (q, *J* = 7.0 Hz, 1H), 3.89 (s, 3H), 3.88 - 3.80 (m, 2H), 2.83 (dt, *J* = 7.3, 4.2 Hz, 2H), 1.99 (dt, *J* = 15.2, 8.1 Hz, 4H), 1.91 (q, *J* = 7.0 Hz, 2H), 1.84 (dd, *J* = 18.7, 10.8 Hz, 2H).

**Example 140: Preparation of Compound 140**

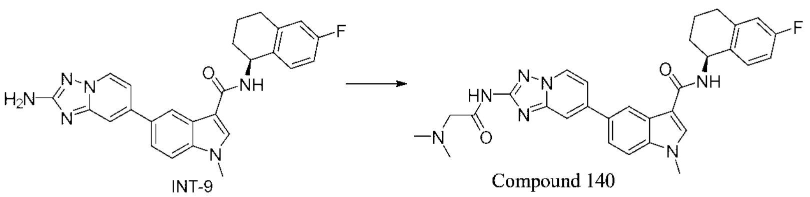
INT-9
Compound 140

Preparation of Compound 140:

**INT-9** (90 mg, 0.2 mmol) was dissolved in pyridine (1 mL), 2-(dimethylamino)acetic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 140 with a yield of 56%. MS (ESI, positive ion) m/z: 540.2 $[M + H]^+$.

$^1$H NMR (400 MHz, DMSO-$d_6$) δ 10.45 (s, 1H), 8.94 - 8.88 (m, 1H), 8.65 (d, *J* = 1.8 Hz, 1H), 8.31 (d, *J* = 8.6 Hz, 1H), 8.17 (s, 1H), 7.93 (dd, *J* = 2.0, 0.9 Hz, 1H), 7.76 (dd, *J* = 8.7, 1.9 Hz, 1H), 7.68 (d, *J* = 8.7 Hz, 1H), 7.51 (dd, *J* = 7.2, 2.0 Hz, 1H), 7.36 - 7.27 (m, 1H), 7.00 (t, *J* = 8.5 Hz, 2H), 5.27 (s, 1H), 3.89 (s, 3H), 3.21 (s, 2H), 2.82 (d, *J* = 6.4 Hz, 2H), 2.33 (s, 6H), 2.06 - 1.94 (m, 2H), 1.84 (td, *J* = 16.8, 14.5, 9.2 Hz, 2H).

**Example 141: Preparation of Compound 141**

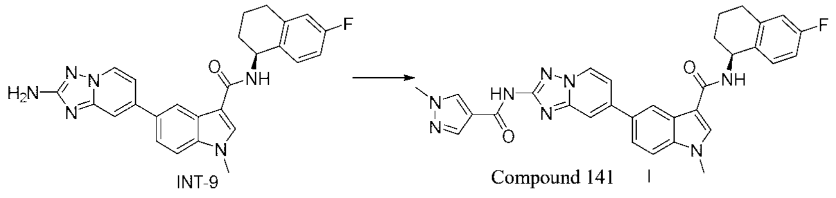
INT-9
Compound 141

Preparation of Compound 141:

**INT-9** (90 mg, 0.2 mmol) was dissolved in pyridine (1 mL); 1-methylpyrazole-4-carboxylic acid (38 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 141 with a yield of 62%. MS (ESI, positive ion) m/z: 563.2 $[M + H]^+$.

$^1$H NMR (400 MHz, DMSO-$d_6$) δ 10.93 (s, 1H), 8.94 (d, *J* = 7.1 Hz, 1H), 8.67 (s, 1H), 8.44 (s, 1H), 8.33 (d, *J* = 8.7 Hz, 1H), 8.18 (s, 1H), 8.13 (s, 1H), 7.98 - 7.93 (m, 1H), 7.81 - 7.74 (m, 1H), 7.69 (d, *J* = 8.7 Hz, 1H), 7.52 (dd, *J* = 7.3, 2.0 Hz, 1H), 7.36 - 7.28 (m, 1H), 6.99 (d, *J* = 9.2 Hz, 2H), 5.28 (s, 1H), 3.92 (s, 3H), 3.89 (s, 3H), 2.82 (s, 2H), 2.66 - 2.56 (m, 1H), 2.00 (d, *J* = 5.3 Hz, 2H), 1.83 (s, 1H).

**Example 142: Preparation of Compound 142**

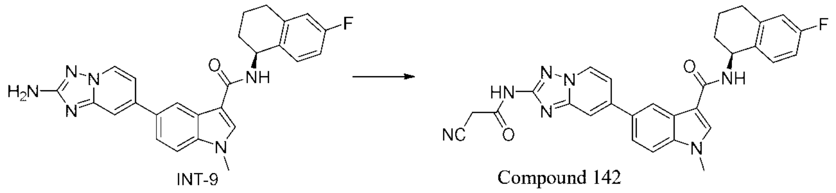
INT-9
Compound 142

Preparation of Compound 142:

**INT-9** (90 mg, 0.2 mmol) was dissolved in pyridine (1 mL), cyanoacetic acid (25 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 142 with a yield of 71%. MS (ESI, positive ion) m/z: 522.2 $[M + H]^+$.

$^1$H NMR (400 MHz, DMSO-$d_6$) δ 11.27 (s, -1H), 8.94 (d, *J* = 7.1 Hz, 1H), 8.66 (d, *J* = 1.9 Hz, 1H), 8.32 (d, *J* = 8.6 Hz, 1H), 8.17 (s, 1H), 7.97 (dd, *J* = 2.1, 0.9 Hz, 1H), 7.77 (dd, *J* = 8.7, 1.9 Hz, 1H), 7.68 (d, *J* = 8.7 Hz, 1H), 7.54 (dd, *J* = 7.1, 2.0 Hz, 1H), 7.36 - 7.28 (m, 1H), 7.00 (t, *J* = 8.4 Hz, 2H), 5.27 (s, 1H), 3.90 - 3.88 (m, 3H), 2.82 (d, *J* = 6.4 Hz, 2H), 2.80 - 2.57 (m, 1H), 2.50 - 2.26 (m, 1H), 2.04 - 1.97 (m, 2H), 1.84 (dq, *J* = 15.1, 8.2, 5.6 Hz, 2H).

**Example 143: Preparation of Compound 143**

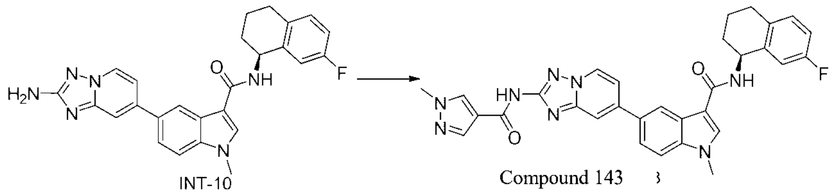
INT-10
Compound 143

Preparation of Compound 143:

**INT-10** (90 mg, 0.2 mmol) was dissolved in pyridine (1 mL); 1-methylpyrazole-4-carboxylic acid (38 mg, 0.3

mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 143 with a yield of 72%. MS (ESI, positive ion) m/z: 563.2 $[M + H]^+$.

$^1$H NMR (400 MHz, DMSO-$d_6$) δ 10.92 (s, 1H), 8.94 (dd, *J* = 7.1, 0.8 Hz, 1H), 8.67 (d, *J* = 1.9 Hz, 1H), 8.44 (s, 1H), 8.32 (d, *J* = 8.7 Hz, 1H), 8.18 (s, 1H), 8.13 (d, *J* = 0.8 Hz, 1H), 7.95 (dd, *J* = 2.0, 0.9 Hz, 1H), 7.77 (dd, *J* = 8.7, 1.9 Hz, 1H), 7.69 (d, *J* = 8.7 Hz, 1H), 7.52 (dd, *J* = 7.2, 2.0 Hz, 1H), 7.32 (dd, *J* = 8.2, 6.1 Hz, 1H), 7.00 (t, *J* = 8.7 Hz, 2H), 5.31 - 5.23 (m, 1H), 3.92 (s, 3H), 3.89 (s, 3H), 2.82 (d, *J* = 6.7 Hz, 2H), 2.01 (dd, *J* = 9.0, 4.7 Hz, 2H), 1.83 (ddd, *J* = 23.5, 11.1, 7.9 Hz, 2H).

**Example 144: Preparation of Compound 144**

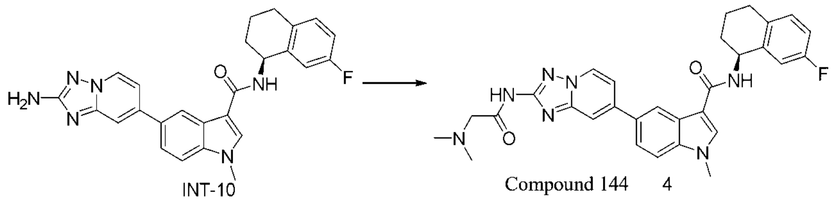
INT-10
Compound 144 4

Preparation of Compound 144:

**INT-10** (90 mg, 0.2 mmol) was dissolved in pyridine (1 mL), 2-(dimethylamino)acetic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 144 with a yield of 58%. MS (ESI, positive ion) m/z: 540.2 $[M + H]^+$.

$^1$H NMR (400 MHz, DMSO-$d_6$) δ 10.45 (s, 1H), 8.91 (d, *J* = 7.0 Hz, 1H), 8.65 (d, *J* = 1.8 Hz, 1H), 8.32 (d, *J* = 8.6 Hz, 1H), 8.17 (s, 1H), 7.93 (dd, *J* = 2.1, 0.9 Hz, 1H), 7.76 (dd, *J* = 8.7, 1.9 Hz, 1H), 7.68 (d, *J* = 8.6 Hz, 1H), 7.51 (dd, *J* = 7.2, 2.0 Hz, 1H), 7.36 - 7.28 (m, 1H), 7.00 (t, *J* = 8.4 Hz, 2H), 5.27 (s, 1H), 3.89 (s, 3H), 3.21 (s, 2H), 2.82 (d, *J* = 6.4 Hz, 2H), 2.33 (s, 6H), 2.04 - 1.97 (m, 2H), 1.86 (dd, *J* = 22.3, 13.8 Hz, 2H).

**Example 145: Preparation of Compound 145**

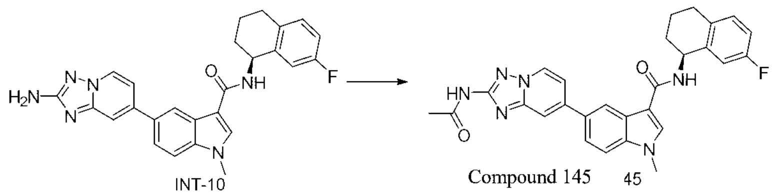
INT-10
Compound 145 45

Preparation of Compound 145:

**INT-10** (90 mg, 0.2 mmol) was dissolved in pyridine (1 mL); acetic acid (18 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 145 with a yield of 61%. MS (ESI, positive ion) m/z: 497.2 $[M + H]^+$.

$^1$H NMR (400 MHz, DMSO-$d_6$) δ 10.82 (s, 1H), 8.90 (dd, *J* = 7.1, 0.8 Hz, 1H), 8.66 (d, *J* = 1.9 Hz, 1H), 8.33 (d, *J* = 8.6 Hz, 1H), 8.18 (s, 1H), 7.93 (dd, *J* = 1.9, 0.9 Hz, 1H), 7.76 (dd, *J* = 8.6, 1.9 Hz, 1H), 7.68 (d, *J* = 8.6 Hz, 1H), 7.50 (dd, *J* = 7.1, 2.0 Hz, 1H), 7.36 - 7.27 (m, 1H), 7.00 (t, *J* = 8.5 Hz, 2H), 5.27 (s, 1H), 3.89 (s, 3H), 2.82 (d, *J* = 6.8 Hz, 2H), 2.18 (s, 3H), 2.06 - 1.95 (m, 2H), 1.85 (dt, *J* = 17.8, 10.0 Hz, 2H).

**Example 146: Preparation of Compound 146**

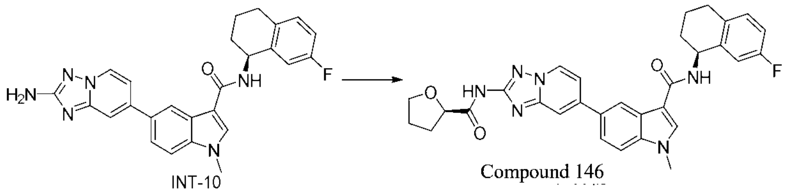

INT-10 Compound 146

Preparation of Compound 146:

**INT-10** (90 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 146 with a yield of 68%. MS (ESI, positive ion) m/z: 553.2 [M + H]$^+$.

$^1$H NMR (400 MHz, DMSO-$d_6$) δ 10.48 (s, 1H), 8.92 (d, *J* = 7.1 Hz, 1H), 8.66 (d, *J* = 1.8 Hz, 1H), 8.32 (d, *J* = 8.6 Hz, 1H), 8.18 (s, 1H), 7.95 (d, *J* = 2.0 Hz, 1H), 7.76 (dd, *J* = 8.7, 1.9 Hz, 1H), 7.69 (d, *J* = 8.6 Hz, 1H), 7.52 (dd, *J* = 7.2, 2.0 Hz, 1H), 7.36 - 7.29 (m, 1H), 7.00 (t, *J* = 8.4 Hz, 2H), 5.28 (s, 1H), 4.54 (s, 1H), 4.02 (q, *J* = 7.0 Hz, 1H), 3.89 (s, 3H), 3.88 - 3.82 (m, 1H), 2.82 (d, *J* = 6.6 Hz, 2H), 2.30 - 2.21 (m, 1H), 2.00 (q, *J* = 6.5, 6.1 Hz, 3H), 1.88 (ddd, *J* = 32.1, 17.2, 9.5 Hz, 4H).

**Example 147: Preparation of Compound 147**

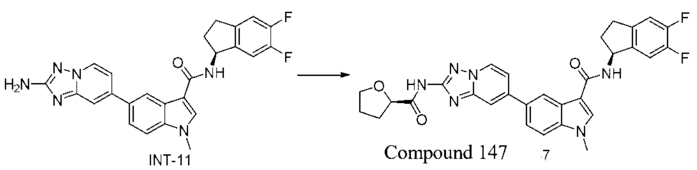

INT-11 Compound 147

Preparation of Compound 147:

**INT-11** (90 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 147 with a yield of 68%. MS (ESI, positive ion) m/z: 553.2 [M + H]$^+$.

$^1$H NMR (400 MHz, DMSO-$d_6$) δ 11.27 (s, -1H), 8.94 (d, *J* = 7.1 Hz, 1H), 8.66 (d, *J* = 1.9 Hz, 1H), 8.32 (d, *J* = 8.6 Hz, 1H), 8.17 (s, 1H), 7.97 (dd, *J* = 2.1, 0.9 Hz, 1H), 7.77 (dd, *J* = 8.7, 1.9 Hz, 1H), 7.68 (d, *J* = 8.7 Hz, 1H), 7.54 (dd, *J* = 7.1, 2.0 Hz, 1H), 7.36 - 7.28 (m, 1H), 7.00 (t, *J* = 8.4 Hz, 2H), 5.27 (s, 1H), 3.90 - 3.88 (m, 3H), 2.82 (d, *J* = 6.4 Hz, 2H), 2.80 - 2.57 (m, 1H), 2.50 - 2.26 (m, 1H), 2.04 - 1.97 (m, 2H), 1.84 (dq, *J* = 15.1, 8.2, 5.6 Hz, 2H).

**Example 148: Preparation of Compound 148**

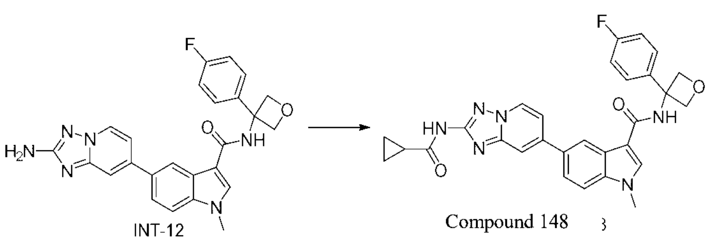

INT-12 Compound 148

Preparation of Compound 148:

INT-12 (91 mg, 0.2 mmol) was dissolved in pyridine (1 mL), cyclopropanecarboxylic acid (26 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 148 with a yield of 61%. MS (ESI, positive ion) m/z: 525.2 $[M + H]^+$.

$^1H$ NMR (400 MHz, DMSO-$d_6$) δ 11.10 (s, 1H), 9.22 (s, 1H), 8.93 (s, 1H), 8.63 (d, *J* = 1.8 Hz, 1H), 8.05 (s, 1H), 7.95 (d, *J* = 1.9 Hz, 1H), 7.81 (dd, *J* = 8.7, 1.9 Hz, 1H), 7.73 (d, *J* = 8.6 Hz, 1H), 7.61 - 7.57 (m, 2H), 7.51 (dd, *J* = 7.1, 2.0 Hz, 1H), 7.21 (d, *J* = 8.9 Hz, 2H), 5.13 (t, *J* = 5.9 Hz, 1H), 4.77 (d, *J* = 8.1 Hz, 1H), 4.29 (d, *J* = 8.1 Hz, 1H), 3.93 (s, 3H), 3.66 (d, *J* = 6.5 Hz, 2H), 0.86 (d, *J* = 6.2 Hz, 5H).

**Example 149: Preparation of Compound 149**

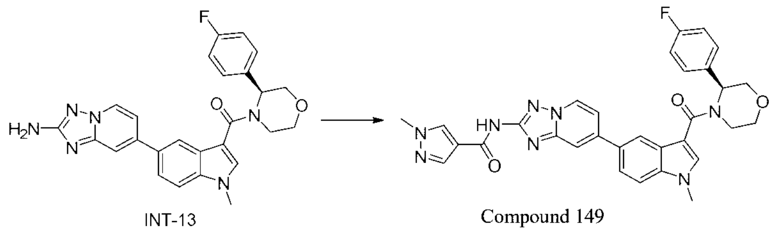

INT-13 Compound 149

Preparation of Compound 149:

**INT-13** (94 mg, 0.2 mmol) was dissolved in pyridine (1 mL); 1-methylpyrazole-4-carboxylic acid (38 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 149 with a yield of 62%. MS (ESI, positive ion) m/z: 579.2 $[M + H]^+$.

$^1H$ NMR (400 MHz, DMSO-$d_6$) δ 10.92 (s, 1H), 8.95 - 8.85 (m, 1H), 8.44 (s, 1H), 8.19 (dd, *J* = 9.3, 1.8 Hz, 1H), 8.13 (s, 1H), 7.97 - 7.92 (m, 1H), 7.88 (d, *J* = 9.6 Hz, 1H), 7.78 (td, *J* = 7.7, 6.8, 1.7 Hz, 1H), 7.73 - 7.64 (m, 1H), 7.61 - 7.38 (m, 4H), 7.24 (t, *J* = 8.9 Hz, 2H), 5.63 (s, 1H), 4.50 (d, *J* = 12.2 Hz, 1H), 4.10 (d, *J* = 13.4 Hz, 1H), 3.98 - 3.93 (m, 1H), 3.92 (s, 3H), 3.90 (s, 3H), 3.87 (d, *J* = 11.4 Hz, 2H), 3.68 - 3.58 (m, 1H).

**Example 150: Preparation of Compound 150**

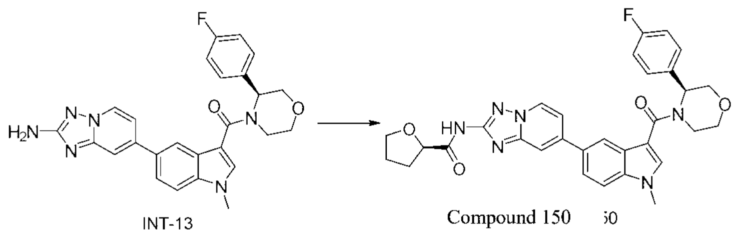

INT-13 Compound 150

Preparation of Compound 150:

**INT-13** (94 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 150 with a yield of 66%. MS (ESI, positive ion) m/z: 579.2 $[M + H]^+$.

$^1H$ NMR (400 MHz, DMSO-$d_6$) δ 10.47 (s, 1H), 8.90 (d, *J* = 7.2 Hz, 1H), 8.21 - 8.10 (m, 1H), 7.96 - 7.87 (m, 2H),

7.77 (dd, *J* = 8.7, 1.8 Hz, 1H), 7.69 (d, *J* = 8.6 Hz, 1H), 7.53 (dt, *J* = 9.5, 4.7 Hz, 2H), 7.48 (dd, *J* = 7.2, 2.0 Hz, 1H), 7.24 (t, *J* = 8.9 Hz, 2H), 5.63 (s, 1H), 4.49 (d, *J* = 12.3 Hz, 1H), 4.09 (d, *J* = 13.5 Hz, 1H), 4.02 (q, *J* = 7.0 Hz, 1H), 3.94 (dd, *J* = 12.2, 3.5 Hz, 2H), 3.89 (s, 3H), 3.85 (q, *J* = 7.1 Hz, 2H), 3.63 (td, *J* = 11.7, 2.9 Hz, 1H), 3.29 (d, *J* = 12.6 Hz, 1H), 2.32 - 1.95 (m, 2H), 1.94 - 1.80 (m, 2H).

**Example 151: Preparation of Compound 151**

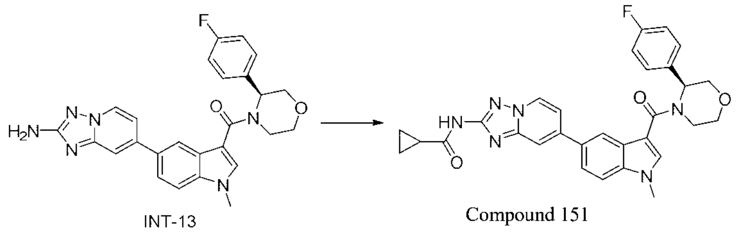

INT-13

Compound 151

Preparation of Compound 151:

**INT-13** (94 mg, 0.2 mmol) was dissolved in pyridine (1 mL), cyclopropanecarboxylic acid (26 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 151 with a yield of 56%. MS (ESI, positive ion) m/z: 539.2 $[M + H]^+$.

$^1H$ NMR (400 MHz, DMSO-$d_6$) δ 10.46 (s, 1H), 8.92 (d, *J* = 7.3 Hz, 1H), 8.22 - 8.11 (m, 1H), 7.97 - 7.88 (m, 2H), 7.76 (dd, *J* = 8.7, 1.8 Hz, 1H), 7.67 (d, *J* = 8.6 Hz, 1H), 7.53 (dt, *J* = 9.5, 4.7 Hz, 2H), 7.48 (dd, *J* = 7.2, 2.0 Hz, 1H), 7.24 (t, *J* = 8.9 Hz, 2H), 5.63 (s, 1H), 4.49 (d, *J* = 12.3 Hz, 1H), 3.94 (dd, *J* = 12.2, 3.5 Hz, 2H), 3.89 (s, 3H),, 3.29 (d, *J* = 12.6 Hz, 1H), 1.94 - 1.80 (m, 2H).1.35 (m, 5H).

**Example 152: Preparation of Compound 152**

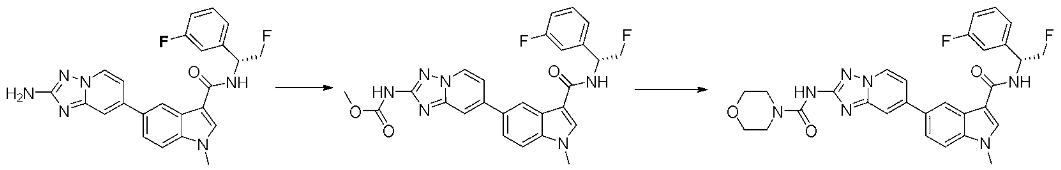

INT-14

Compound 152-1

Compound 152 2

Preparation of Compound 152-1:

Intermediate **INT-14** (44.6 g, 100 mmol) was dissolved in dichloromethane (1 L), methyl chloroformate (11.2 g, 120 mmol) and N,N-diisopropylethylamine (25.8 g, 200 mmol) were added, and the mixture was stirred at room temperature for 2 h. Petroleum ether (3 L) was added to the reaction mixture for crystallization; the solid was filtered with suction, washed with water, and dried to obtain Compound 152-1 with a yield of 71%. MS (ESI, positive ion) m/z: 505.2 $[M + H]^+$.

Preparation of Compound 152:

Compound 152-1 (100 mg, 0.2 mmol) was dissolved in acetonitrile (1 mL); morpholine (36 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 152 with a yield of 64%. MS (ESI, positive ion) m/z:560.2 $[M + H]^+$.

$^1H$ NMR (400 MHz, DMSO-$d_6$) δ 9.66 (s, 1H), 9.21 (d, *J* = 8.6 Hz, 1H), 8.86 (d, *J* = 7.1 Hz, 1H), 8.52 (s, 1H), 8.03 - 7.97 (m, 1H), 7.98 - 7.90 (m, 2H), 7.63 - 7.54 (m, 2H), 7.49 - 7.42 (m, 1H), 7.22 (t, *J* = 8.9 Hz, 2H), 5.50 (dq, *J* = 13.5, 8.4, 6.8

Hz, 1H), 5.01 - 4.73 (m, 1H), 4.69 (ddd, $J$ = 46.3, 9.5, 5.6 Hz, 1H), 4.24 (s, 3H), 3.66 - 3.59 (m, 4H), 3.47 (t, $J$ = 4.7 Hz, 4H).

**Example 153: Preparation of Compound 153**

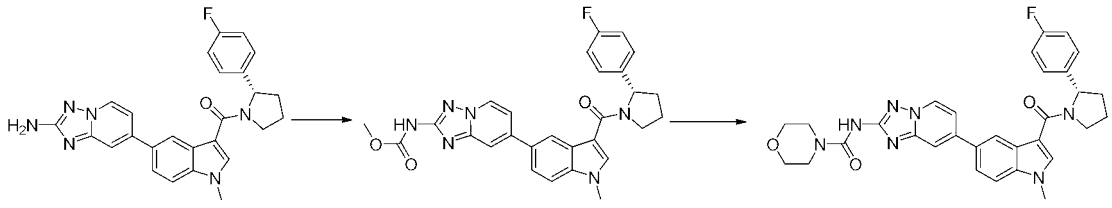

INT-16 Compound 153-1 Compound 153 3

Preparation of Compound 153-1:

Intermediate **INT-16** (4.5 g, 10 mmol) was dissolved in dichloromethane (0.3 L), methyl chloroformate (1.12 g, 12 mmol) and N,N-diisopropylethylamine (2.58 g, 20 mmol) were added, and the mixture was stirred at room temperature for 2 h. Petroleum ether (3 L) was added to the reaction mixture for crystallization; the solid was filtered with suction, washed with water, and dried to obtain Compound 153-1 with a yield of 71%. MS (ESI, positive ion) m/z: 513.2 [M + H]$^+$.

Preparation of Compound 153:

Compound 152-1 (100 mg, 0.2 mmol) was dissolved in acetonitrile (1 mL); morpholine (36 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 153 with a yield of 64%. MS (ESI, positive ion) m/z:568.2 [M + H]$^+$.

1H NMR (400 MHz, DMSO-d6) δ 11.07 (s, 1H), 8.82 (m, 1H), 8.48 (d, J = 1.8 Hz, 1H), 8.35 (s, 1H),7.85 (m, 2H), 7.73 (d, J = 8.6 Hz, 1H),7.59 - 7.49 (m, 1H), 7.42 (dd, J = 7.1, 2.0 Hz, 1H), 7.32 (m, 1H), 7.13 (t, J = 8.7 Hz, 2H),, 4.71-4.52 (m, 2H), 3.74 (s, 3H), 3.55-3.31 (m, 10H), 2.10-1.74 (m, 4H).

**Example 154: Preparation of Compound 154**

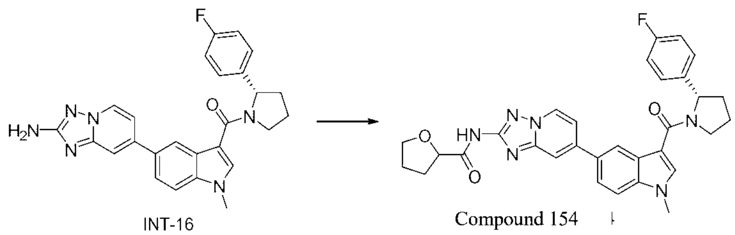

INT-16 Compound 154

Preparation of Compound 154:

**INT-16** (90 mg, 0.2 mmol) was dissolved in pyridine (1 mL); tetrahydrofuran-2-carboxylic acid (35 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 154 with a yield of 78%. MS (ESI, positive ion) m/z: 553.2 [M + H]$^+$.

1H NMR (400 MHz, DMSO-d6) δ 11.07 (s, 1H), 8.82 (m, 1H), 8.48 (d, J = 1.8 Hz, 1H), 8.35 (s, 1H),7.85 (m, 2H), 7.73 (d, J = 8.6 Hz, 1H),7.59 - 7.49 (m, 1H), 7.42 (dd, J = 7.1, 2.0 Hz, 1H), 7.32 (m, 1H), 7.13 (t, J = 8.7 Hz, 2H),, 4.71-4.52 (m, 2H), 3.86-3.70 (m, 5H), 3.51-3.40 (m, 2H), 2.10-1.74 (m, 6H).

**Example 155: Preparation of Compound 155**

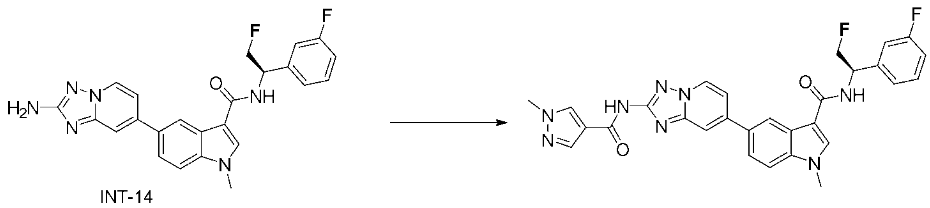

INT-14

Compound 155

Preparation of Compound 155:

Intermediate **INT-14** (86 mg, 0.2 mmol) was dissolved in pyridine (1 mL); 1-methylpyrazole-4-carboxylic acid (38 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 155 with a yield of 45%. MS (ESI, positive ion) m/z: 555.2 [M + H]$^+$.

$^1$H NMR (400 MHz, DMSO-$d_6$) δ 10.88 (s, 1H), 8.85 (d, *J* = 7.2 Hz, 1H), 8.61 (d, *J* = 8.3 Hz, 1H), 8.50 (d, *J* = 1.9 Hz, 1H), 8.36 (s, 1H), 8.25 (s, 1H), 8.06 (s, 1H), 7.86 (s, 1H), 7.70 (d, *J* = 1.8 Hz, 1H), 7.64 (d, *J* = 8.7 Hz, 1H), 7.45 - 7.35 (m, 2H), 7.33 - 7.26 (m, 2H), 7.09 (t, *J* = 8.6 Hz, 1H), 5.44 (dq, *J* = 15.0, 7.0 Hz, 1H), 4.71 (d, *J* = 6.5 Hz, 1H), 4.60 (d, *J* = 6.4 Hz, 1H), 3.87 (s, 3H), 3.85 (s, 3H).

**Example 156: Preparation of Compound 156**

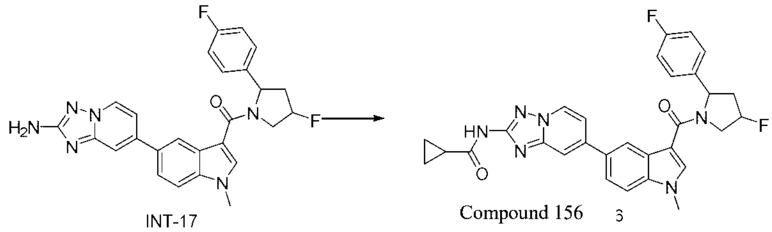

INT-17

Compound 156

Preparation of Compound 156:

**INT-17** (94 mg, 0.2 mmol) was dissolved in pyridine (1 mL), cyclopropanecarboxylic acid (26 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 156 with a yield of 66%. MS (ESI, positive ion) m/z: 541.2 [M + H]$^+$.

**Example 157: Preparation of Compound 157**

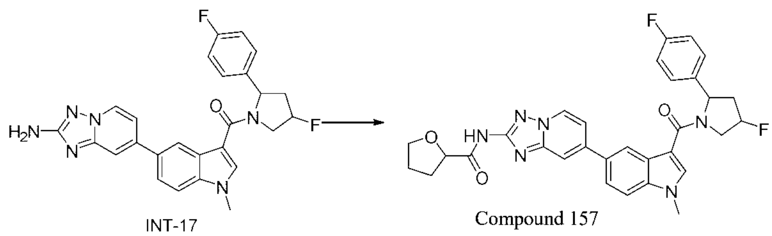

INT-17

Compound 157

Preparation of Compound 157:

**INT-17** (90 mg, 0.2 mmol) was dissolved in pyridine (1 mL); tetrahydrofuran-2-carboxylic acid (35 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 157 with a yield of 78%. MS (ESI, positive ion) m/z: 570.2 $[M + H]^+$.

$^1H$ NMR (400 MHz, DMSO-d6) δ 11.07 (s, 1H), 8.82 (m, 1H), 8.35 (s, 1H),7.85 (m, 2H), 7.73 (d, J = 8.6 Hz, 1H),7.59 - 7.49 (m, 2H), 7.42 (dd, J = 7.1, 2.0 Hz, 1H), 7.32 (dd, J = 8.5, 5.5 Hz, 2H), 7.13 (t, J = 8.7 Hz, 2H), 4.81-4.75 (m, 1H), 3.8-3.75 (m, 7H), 2.51-1.83 (m, 4H).

**Example 158: Preparation of Compound 158**

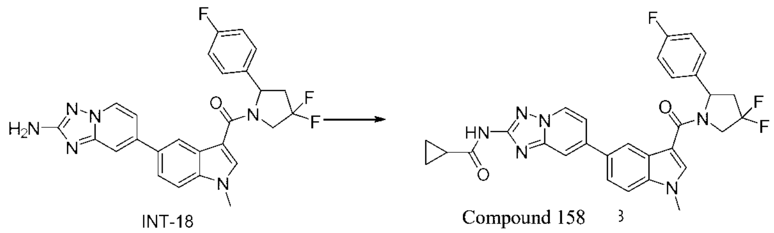

INT-18 Compound 158

Preparation of Compound 158:

**INT-18** (98 mg, 0.2 mmol) was dissolved in pyridine (1 mL), cyclopropanecarboxylic acid (26 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 158 with a yield of 56%. MS (ESI, positive ion) m/z: 559.2 $[M + H]^+$.

$^1H$ NMR (400 MHz, DMSO-d6) δ 11.07 (s, 1H), 8.82 (m, 1H), 8.35 (s, 1H),7.85 (m, 2H), 7.73 (d, J = 8.6 Hz, 1H),7.59 - 7.49 (m, 2H), 7.42 (dd, J = 7.1, 2.0 Hz, 1H), 7.32 (dd, J = 8.5, 5.5 Hz, 2H), 7.13 (t, J = 8.7 Hz, 2H), 4.81-4.75 (m, 1H), 3.8-3.75 (s, 5H), 2.51-2.42 (m, 2H), 1.51-1.43 (m, 1H),1.12-1.06 (m, 4H).

**Example 159: Preparation of Compound 159**

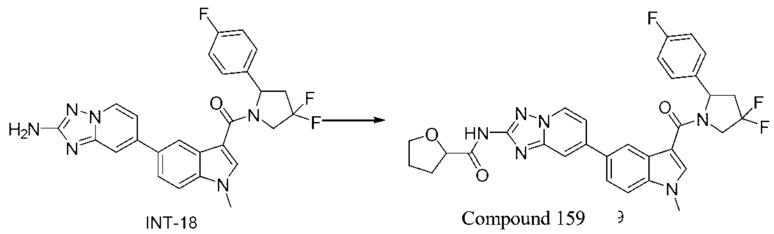

INT-18 Compound 159

Preparation of Compound 159:

**INT-18** (90 mg, 0.2 mmol) was dissolved in pyridine (1 mL); tetrahydrofuran-2-carboxylic acid (35 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 159 with a yield of 72%. MS (ESI, positive ion) m/z: 589.2 $[M + H]^+$.

$^1H$ NMR (400 MHz, DMSO-d6) δ 11.07 (s, 1H), 8.82 (m, 1H), 8.35 (s, 1H),7.85 (m, 2H), 7.73 (d, J = 8.6 Hz, 1H),7.59 - 7.49 (m, 2H), 7.42 (dd, J = 7.1, 2.0 Hz, 1H), 7.32 (dd, J = 8.5, 5.5 Hz, 2H), 7.13 (t, J = 8.7 Hz, 2H), 4.81-4.75 (m, 1H), 3.8-3.75 (m, 7H), 2.51-1.83 (m, 4H).

**Example 160: Preparation of Compound 160**

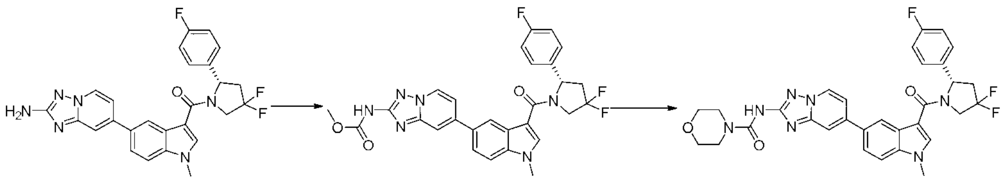

INT-18

Compound 160-1

Compound 160

Preparation of Compound 160-1:

Intermediate **INT-18** (490 mg, 1 mmol) was dissolved in dichloromethane (0.3 L); methyl chloroformate (112 mg, 1.2 mmol) and N,N-diisopropylethylamine (258 mg, 2.0 mmol) were added, and the mixture was stirred at room temperature for 2 h. Petroleum ether (0.3 L) was added to the reaction mixture for crystallization; the solid was filtered with suction, washed with water, and dried to obtain Compound 160-1 with a yield of 61%. MS (ESI, positive ion) m/z: 549.2 $[M + H]^+$.

Preparation of Compound 160:

Compound 160-1 (110 mg, 0.2 mmol) was dissolved in acetonitrile (1 mL); morpholine (36 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 160 with a yield of 68%. MS (ESI, positive ion) m/z:604.2 $[M + H]^+$.

$^1$H NMR (400 MHz, DMSO-d6) δ 11.07 (s, 1H), 8.82 (m, 1H), 8.35 (s, 1H),7.85 (m, 2H), 7.73 (d, J = 8.6 Hz, 1H),7.59 - 7.49 (m, 2H), 7.42 (dd, J = 7.1, 2.0 Hz, 1H), 7.32 (dd, J = 8.5, 5.5 Hz, 2H), 7.13 (t, J = 8.7 Hz, 2H), 4.82 - 4.70 (m, 1H), 3.89-3.71 (m, 3H), 3.64 (m, 4H), 3.31 (m, 4H), 2.52 - 2.28 (m, 2H).

**Example 161: Preparation of Compound 161**

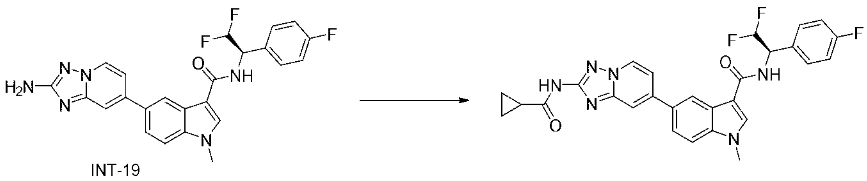

INT-19

Compound 161

Preparation of Compound 161:

Intermediate **INT-19** (93 mg, 0.2 mmol) was dissolved in pyridine (1 mL); cyclopropanecarboxylic acid (28 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 161 with a yield of 65%. MS (ESI, positive ion) m/z: 533.2 $[M + H]^+$.

$^1$H NMR (400 MHz, DMSO-$d_6$) δ 11.28 (s, 1H), 8.92 (d, *J* = 7.2 Hz, 1H), 8.78 (d, *J* = 9.1 Hz, 1H), 8.55 (d, *J* = 1.9 Hz, 1H), 8.36 (s, 1H), 7.94 (d, *J* = 1.9 Hz, 1H), 7.77 (dd, *J* = 8.7, 1.9 Hz, 1H), 7.70 (d, *J* = 8.7 Hz, 1H), 7.66 - 7.62 (m, 2H), 7.50 (dd, *J* = 7.1, 2.0 Hz, 1H), 7.27 (t, *J* = 8.9 Hz, 2H), 6.37-6.12 (m, 2H), 4.02 (d, *J* = 8.5 Hz, 5H), 3.85 (q, *J* = 7.0 Hz, 1H), 2.32 - 2.19 (m, 1H), 2.07 - 1.84 (m, 3H), 1.50 (d, *J* = 7.0 Hz, 3H).

**Example 162: Preparation of Compound 162**

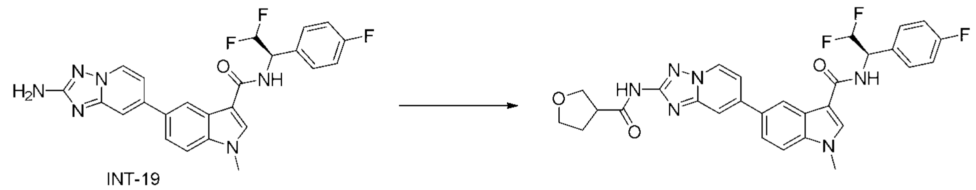

INT-19

Compound 162

Preparation of Compound 162:

Intermediate **INT-19** (93 mg, 0.2 mmol) was dissolved in pyridine (1 mL); 3-tetrahydrofuroic acid (30 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 162 with a yield of 55%. MS (ESI, positive ion) m/z: 564.2 $[M + H]^+$.

$^1H$ NMR (400 MHz, DMSO-$d_6$) δ 11.28 (s, 1H), 8.92 (d, *J* = 7.2 Hz, 1H), 8.78 (d, *J* = 9.1 Hz, 1H), 8.55 (d, *J* = 1.9 Hz, 1H), 8.36 (s, 1H), 7.94 (d, *J* = 1.9 Hz, 1H), 7.77 (dd, *J* = 8.7, 1.9 Hz, 1H), 7.70 (d, *J* = 8.7 Hz, 1H), 7.66 - 7.62 (m, 2H), 7.50 (dd, *J* = 7.1, 2.0 Hz, 1H), 7.27 (t, *J* = 8.9 Hz, 2H), 6.37-6.12 (m, 2H), 4.12-3.75 (m, 7H), 2.72 - 2.63 (m, 1H), 2.17 - 1.94 (m, 2H).

**Example 163: Preparation of Compound 163**

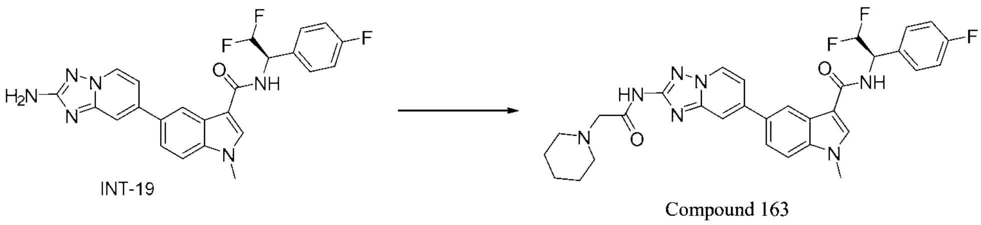

INT-19

Compound 163

Preparation of Compound 163:

Intermediate **INT-19** (93 mg, 0.2 mmol) was dissolved in pyridine (1 mL); 1-piperidineacetic acid (42 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 163 with a yield of 55%. MS (ESI, positive ion) m/z: 590.2 $[M + H]^+$.

$^1H$ NMR (400 MHz, DMSO-$d_6$) δ 11.28 (s, 1H), 8.92 (d, *J* = 7.2 Hz, 1H), 8.78 (d, *J* = 9.1 Hz, 1H), 8.55 (d, *J* = 1.9 Hz, 1H), 8.36 (s, 1H), 7.94 (d, *J* = 1.9 Hz, 1H), 7.77 (dd, *J* = 8.7, 1.9 Hz, 1H), 7.70 (d, *J* = 8.7 Hz, 1H), 7.66 - 7.62 (m, 2H), 7.50 (dd, *J* = 7.1, 2.0 Hz, 1H), 7.27 (t, *J* = 8.9 Hz, 2H), 6.37-6.12 (m, 2H), 3.81 (s, 3H), 3.35 (s, 2H), 2.51-3.36 (m, 4H), 1.62-1.42 (m, 6H).

**Example 164: Preparation of Compound 164**

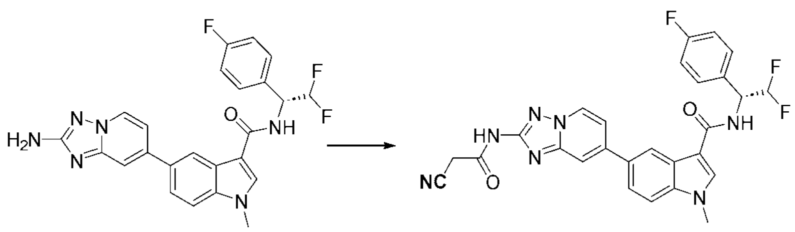

INT-19

Compound 164

Preparation of Compound 164:

**INT-19** (93 mg, 0.2 mmol) was dissolved in pyridine (1 mL), cyanoacetic acid (29 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 164 with a yield of 58%. MS (ESI, positive ion) m/z: 532.2 $[M + H]^+$.

$^1H$ NMR (400 MHz, DMSO-$d_6$) δ 11.28 (s, 1H), 8.92 (d, *J* = 7.2 Hz, 1H), 8.78 (d, *J* = 9.1 Hz, 1H), 8.55 (d, *J* = 1.9 Hz, 1H), 8.36 (s, 1H), 7.94 (d, *J* = 1.9 Hz, 1H), 7.77 (dd, *J* = 8.7, 1.9 Hz, 1H), 7.70 (d, *J* = 8.7 Hz, 1H), 7.66 - 7.62 (m, 2H), 7.50 (dd, *J* = 7.1, 2.0 Hz, 1H), 7.27 (t, *J* = 8.9 Hz, 2H), 6.37 (td, *J* = 55.5, 4.2 Hz, 1H), 5.61 (dt, *J* = 14.6, 9.8 Hz, 1H), 4.06 (s, 2H), 3.94 (s, 3H).

**Example 165: Preparation of Compound 165**

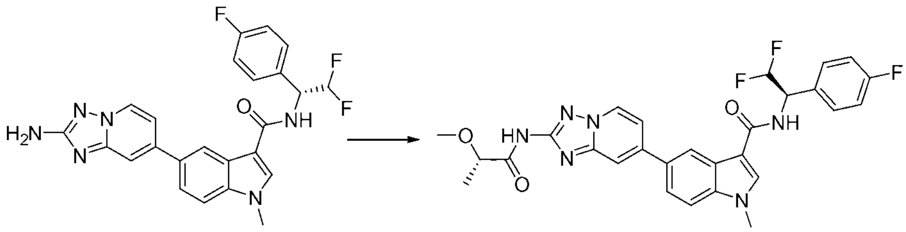

INT-19 Compound 165

Preparation of Compound 165:

**INT-19** (93 mg, 0.2 mmol) was dissolved in pyridine (1 mL), (S)-2-methoxypropanoic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 165 with a yield of 62%. MS (ESI, positive ion) m/z: 551.2 $[M + H]^+$.

$^1H$ NMR (400 MHz, DMSO-$d_6$) δ 10.72 (s, 1H), 8.91 (d, *J* = 7.1 Hz, 1H), 8.78 (d, *J* = 9.0 Hz, 1H), 8.55 (d, *J* = 1.8 Hz, 1H), 8.36 (s, 1H), 7.92 (d, *J* = 2.0 Hz, 1H), 7.77 (dd, *J* = 8.7, 1.9 Hz, 1H), 7.74 - 7.60 (m, 3H), 7.49 (dd, *J* = 7.2, 2.0 Hz, 1H), 7.27 (t, *J* = 8.9 Hz, 2H), 6.37 (td, *J* = 55.6, 4.1 Hz, 1H), 5.66 - 5.58 (m, 1H), 4.05 (s, 1H), 3.94 (s, 3H), 3.32 (s, 3H), 1.34 (d, *J* = 6.7 Hz, 3H).

**Example 166: Preparation of Compound 166**

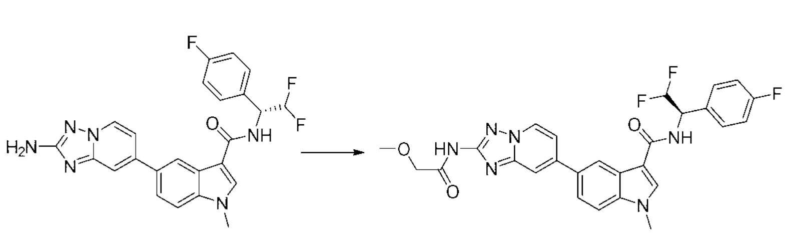

INT-19 Compound 166

Preparation of Compound 166:

**INT-19** (93 mg, 0.2 mmol) was dissolved in pyridine (1 mL), 2-methoxyacetic acid (26 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 166 with a yield of 69%. MS (ESI, positive ion) m/z: 537.2 $[M + H]^+$.

$^1H$ NMR (400 MHz, DMSO-$d_6$) δ 10.68 (s, 1H), 8.90 (d, *J* = 7.1 Hz, 1H), 8.77 (d, *J* = 9.1 Hz, 1H), 8.55 (d, *J* = 1.8 Hz, 1H), 8.36 (s, 1H), 7.92 (d, *J* = 1.9 Hz, 1H), 7.77 (dd, *J* = 8.5, 1.9 Hz, 1H), 7.70 (d, *J* = 8.6 Hz, 1H), 7.64 (dd, *J* = 8.5, 5.5 Hz, 2H), 7.49 (dd, *J* = 7.2, 1.9 Hz, 1H), 7.27 (t, *J* = 8.8 Hz, 2H), 6.37 (td, *J* = 55.5, 4.1 Hz, 1H), 5.66 - 5.58 (m, 1H), 4.18 (s, 2H), 3.94 (s, 3H), 3.39 (s, 3H).

**Example 167: Preparation of Compound 167**

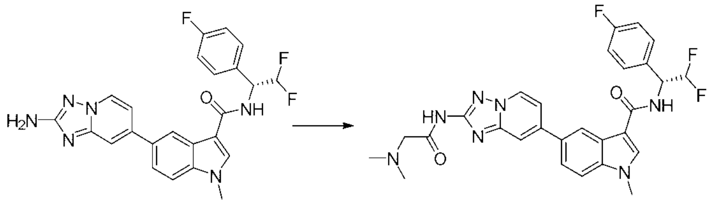

INT-19

Compound 167

Preparation of Compound 167:

**INT-19** (93 mg, 0.2 mmol) was dissolved in pyridine (1 mL), 2-(dimethylamino)acetic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 167 with a yield of 73%. MS (ESI, positive ion) m/z: 550.2 [M + H]$^+$.

$^1H$ NMR (400 MHz, DMSO-$d_6$) δ 10.45 (s, 1H), 8.89 (d, *J* = 7.2 Hz, 1H), 8.76 (d, *J* = 9.0 Hz, 1H), 8.53 (s, 1H), 8.35 (s, 1H), 7.90 (s, 1H), 7.76 (d, *J* = 8.8 Hz, 1H), 7.72 - 7.59 (m, 3H), 7.47 (d, *J* = 7.1 Hz, 1H), 7.26 (t, *J* = 8.7 Hz, 2H), 6.53 - 6.19 (m, 1H), 5.61 (d, *J* = 12.9 Hz, 1H), 3.93 (s, 3H), 3.20 (s, 2H), 2.31 (s, 6H).

**Example 168: Preparation of Compound 168**

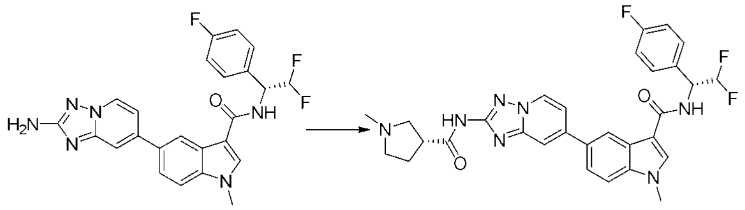

INT-19

Compound 168

Preparation of Compound 168:

**INT-19** (93 mg, 0.2 mmol) was dissolved in pyridine (1 mL), (R)-1-methylpyrrolidine-3-carboxylic acid (39 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 168 with a yield of 68%. MS (ESI, positive ion) m/z: 576.2 [M + H]$^+$.

$^1H$ NMR (400 MHz, DMSO-$d_6$) δ 10.91 (s, 1H), 8.89 (d, *J* = 7.1 Hz, 1H), 8.78 (d, *J* = 9.0 Hz, 1H), 8.55 (s, 1H), 8.36 (s, 1H), 7.90 (d, *J* = 1.9 Hz, 1H), 7.77 (dd, *J* = 8.7, 1.9 Hz, 1H), 7.70 (d, *J* = 8.6 Hz, 1H), 7.64 (dd, *J* = 8.4, 5.4 Hz, 2H), 7.48 (dd, *J* = 7.1, 2.0 Hz, 1H), 7.27 (t, *J* = 8.7 Hz, 2H), 6.55 - 6.19 (m, 1H), 5.65 - 5.58 (m, 1H), 3.94 (s, 3H), 2.82 (dt, *J* = 8.1, 5.0 Hz, 2H), 2.45 (s, 3H), 2.36 (s, 1H), 2.07 (ddd, *J* = 20.3, 16.3, 9.0 Hz, 2H), 1.37 (d, *J* = 6.1 Hz, 2H).

**Example 169: Preparation of Compound 169**

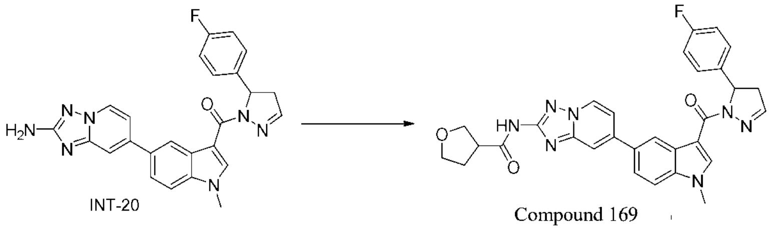

INT-20

Compound 169

Preparation of Compound 162:

Intermediate **INT-20** (90 mg, 0.2 mmol) was dissolved in pyridine (1 mL); 3-tetrahydrofuroic acid (30 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 169 with a yield of 55%. MS (ESI, positive ion) m/z: 552.2 $[M + H]^+$.

$^1H$ NMR (400 MHz, DMSO-d6) δ 11.07 (s, 1H), 8.82 (m, 2H), 8.35 (s, 1H),7.85 (m, 2H), 7.73 (d, J = 8.6 Hz, 1H),7.59 - 7.49 (m, 1H), 7.42 (dd, J = 7.1, 2.0 Hz, 1H), 7.32 (dd, J = 8.5, 5.5 Hz, 2H), 7.13 (t, J = 8.7 Hz, 2H), 4.02 (d, *J* = 8.5 Hz, 5H), 3.85 (q, *J* = 7.0 Hz, 1H), 2.32 - 2.19 (m, 1H), 2.07 - 1.84 (m, 3H), 1.50 (d, *J* = 7.0 Hz, 3H).

**Example 170: Preparation of Compound 170**

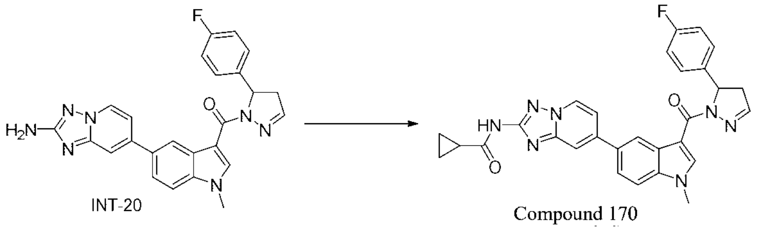

INT-20

Compound 170

Preparation of Compound 170:

Intermediate **INT-20** (93 mg, 0.2 mmol) was dissolved in pyridine (1 mL); cyclopropanecarboxylic acid (28 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 170 with a yield of 61%. MS (ESI, positive ion) m/z: 522.2 $[M + H]^+$.

$^1H$ NMR (400 MHz, DMSO-d6) δ 11.07 (s, 1H), 8.82 (m, 2H), 8.35 (s, 1H),7.85 (m, 2H), 7.73 (d, J = 8.6 Hz, 1H),7.59 - 7.49 (m, 1H), 7.42 (dd, J = 7.1, 2.0 Hz, 1H), 7.32 (dd, J = 8.5, 5.5 Hz, 2H), 7.13 (t, J = 8.7 Hz, 2H), 4.82 - 4.70 (m, 1H), 3.83 (s, 3H), 2.81 - 2.56 (m, 2H), 1.45 - 1.38 (m, 1H), 1.12-0.96 (m, 4H).

**Example 171: Preparation of Compound 171**

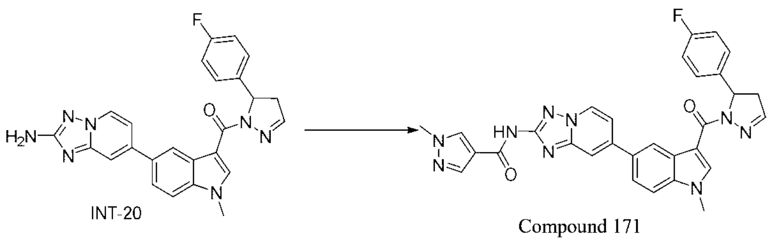

INT-20

Compound 171

Preparation of Compound 171:

**INT-20** (94 mg, 0.2 mmol) was dissolved in pyridine (1 mL); 1-methylpyrazole-4-carboxylic acid (38 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 171 with a yield of 62%. MS (ESI, positive ion) m/z: 562.2 $[M + H]^+$.

$^1H$ NMR (400 MHz, DMSO-d6) δ 11.07 (s, 1H), 8.82 (m, 2H), 8.35 (s, 1H),7.85 (m, 2H), 7.73 (d, J = 8.6 Hz, 1H),7.59 - 7.49 (m, 1H), 7.42 (dd, J = 7.1, 2.0 Hz, 1H), 7.32 (dd, J = 8.5, 5.5 Hz, 2H), 7.13 (t, J = 8.7 Hz, 2H), 4.82 - 4.70 (m, 1H), 3.95 (s, 3H), 3.73 (s, 3H), 2.81 - 2.56 (m, 2H).

**Example 172: Preparation of Compound 172**

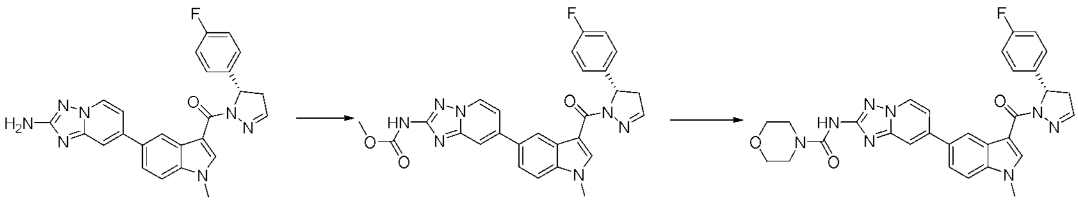

INT-20 Compound 172-1 Compound 172

Preparation of Compound 172-1:

Intermediate **INT-20** (490 mg, 1 mmol) was dissolved in dichloromethane (0.3 L); methyl chloroformate (112 mg, 1.2 mmol) and N,N-diisopropylethylamine (258 mg, 2.0 mmol) were added, and the mixture was stirred at room temperature for 2 h. Petroleum ether (0.3 L) was added to the reaction mixture for crystallization; the solid was filtered with suction, washed with water, and dried to obtain Compound 172-1 with a yield of 61%. MS (ESI, positive ion) m/z: 512.2 $[M + H]^+$.

Preparation of Compound 172:

Compound 172-1 (102 mg, 0.2 mmol) was dissolved in acetonitrile (1 mL); morpholine (36 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 172 with a yield of 68%. MS (ESI, positive ion) m/z: 567.2 $[M + H]^+$.

$^1H$ NMR (400 MHz, DMSO-d6) δ 11.07 (s, 1H), 8.82 (m, 2H), 8.35 (s, 1H),7.85 (m, 2H), 7.73 (d, J = 8.6 Hz, 1H),7.59 - 7.49 (m, 1H), 7.42 (dd, J = 7.1, 2.0 Hz, 1H), 7.32 (dd, J = 8.5, 5.5 Hz, 2H), 7.13 (t, J = 8.7 Hz, 2H), 4.82 - 4.70 (m, 1H), 3.83 (s, 3H), 3.64 (m, 4H), 3.31 (m, 4H), 2.81 - 2.56 (m, 2H).

**Example 173: Preparation of Compound 173**

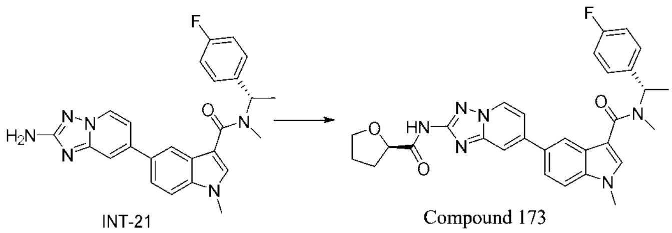

INT-21 Compound 173

Preparation of Compound 173:

**INT-21** (88 mg, 0.2 mmol) was dissolved in pyridine (1 mL), (R)-tetrahydrofuran-2-carboxylic acid (35 mg, 0.3

mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 173 with a yield of 71%. MS (ESI, positive ion) m/z: 543.2 $[M + H]^+$.

$^1H$ NMR (400 MHz, DMSO-$d_6$) δ 10.47 (s, 1H), 8.89 (d, *J* = 7.1 Hz, 1H), 8.29 (d, *J* = 1.8 Hz, 1H), 7.95 - 7.87 (m, 2H), 7.76 (dd, *J* = 8.6, 1.9 Hz, 1H), 7.67 (d, *J* = 8.6 Hz, 1H), 7.48 (dd, *J* = 7.2, 2.0 Hz, 1H), 7.43 (dd, *J* = 8.5, 5.5 Hz, 2H), 7.27 - 7.17 (m, 2H), 4.02 (q, *J* = 7.0 Hz, 1H), 3.90 (s, 3H), 3.86 (t, *J* = 7.0 Hz, 1H), 2.86 (s, 3H), 2.31 - 2.18 (m, 1H), 2.07 - 1.81 (m, 3H), 1.62 (d, *J* = 7.0 Hz, 3H), 1.29 (dd, *J* = 10.4, 6.7 Hz, 1H).

**Example 174: Preparation of Compound 174**

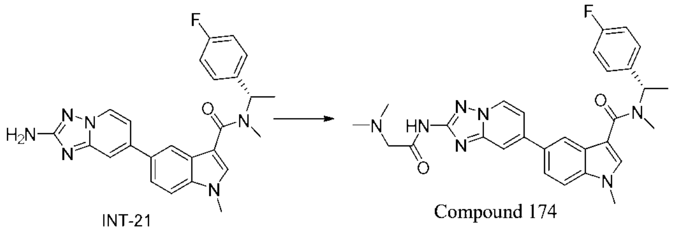

INT-21 Compound 174

Preparation of Compound 174:

**INT-21** (88 mg, 0.2 mmol) was dissolved in pyridine (1 mL), 2-(dimethylamino)acetic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 174 with a yield of 74%. MS (ESI, positive ion) m/z: 527.2 $[M + H]^+$.

$^1H$ NMR (400 MHz, DMSO-$d_6$) δ 10.44 (s, 1H), 8.88 (d, *J* = 7.2 Hz, 1H), 8.28 (s, 1H), 7.90 (d, *J* = 7.6 Hz, 2H), 7.79 - 7.72 (m, 1H), 7.67 (d, *J* = 8.6 Hz, 1H), 7.45 (ddd, *J* = 14.6, 7.6, 3.6 Hz, 3H), 7.22 (t, *J* = 8.7 Hz, 2H), 3.90 (s, 3H), 3.22 (s, 2H), 2.86 (s, 3H), 2.57 - 2.49 (m, 6H), 1.62 (d, *J* = 7.1 Hz, 3H).

**Example 175: Preparation of Compound 175**

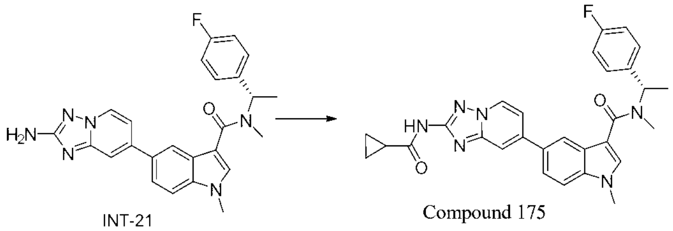

INT-21 Compound 175

Preparation of Compound 175:

Intermediate **INT-21** (93 mg, 0.2 mmol) was dissolved in pyridine (1 mL); cyclopropanecarboxylic acid (28 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 175 with a yield of 61%. MS (ESI, positive ion) m/z: 511.2 $[M + H]^+$.

$^1H$ NMR (400 MHz, DMSO-$d_6$) δ 11.07 (s, 1H), 8.88 - 8.84 (m, 1H), 8.28 (d, *J* = 1.8 Hz, 1H), 7.91 (dd, *J* = 3.5, 1.9 Hz, 2H), 7.77 (dd, *J* = 8.6, 1.9 Hz, 1H), 7.68 (d, *J* = 8.6 Hz, 1H), 7.48 - 7.41 (m, 3H), 7.23 (t, *J* = 8.9 Hz, 2H), 5.88 (s, 1H), 3.90 (s, 3H), 2.86 (s, 3H), 1.63 (d, *J*= 7.0 Hz, 3H), 0.86 (d, *J* = 5.6 Hz, 5H).

**Example 176: Preparation of Compound 176**

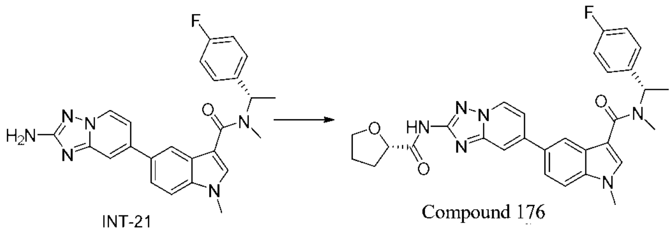

INT-21 Compound 176

Preparation of Compound 176:

**INT-21** (88 mg, 0.2 mmol) was dissolved in pyridine (1 mL), (S)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 176 with a yield of 71%. MS (ESI, positive ion) m/z: 543.2 $[M + H]^+$.

$^1$H NMR (400 MHz, DMSO-$d_6$) δ 10.46 (s, 1H), 8.89 (d, $J$ = 7.1 Hz, 1H), 8.28 (d, $J$ = 1.6 Hz, 1H), 7.95 - 7.87 (m, 2H), 7.77 (dd, $J$ = 8.7, 1.8 Hz, 1H), 7.68 (d, $J$ = 8.6 Hz, 1H), 7.48 (dd, $J$ = 7.2, 2.0 Hz, 1H), 7.43 (dd, $J$ = 8.5, 5.5 Hz, 2H), 7.22 (t, $J$ = 8.8 Hz, 2H), 5.88 (s, 1H), 4.53 (s, 1H), 4.01 (q, $J$ = 7.0 Hz, 1H), 3.90 (s, 3H), 3.89 - 3.80 (m, 1H), 2.86 (s, 3H), 2.31 - 1.93 (m, 2H), 1.90 (dt, $J$ = 13.5, 6.6 Hz, 2H), 1.62 (d, $J$ = 7.0 Hz, 3H).

**Example 177: Preparation of Compound 177**

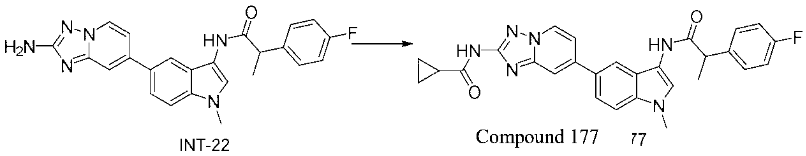

INT-22 Compound 177 77

Preparation of Compound 177:

Intermediate **INT-22** (84 mg, 0.2 mmol) was dissolved in pyridine (1 mL); cyclopropanecarboxylic acid (28 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 177 with a yield of 61%. MS (ESI, positive ion) m/z: 497.2 $[M + H]^+$.

$^1$H NMR (400 MHz, DMSO-$d_6$) δ 11.08 (s, 1H), 10.16 (s, 1H), 8.92 (d, $J$ = 7.1 Hz, 1H), 8.37 (d, $J$ = 1.8 Hz, 1H), 7.92 (d, $J$ = 1.9 Hz, 1H), 7.86 (s, 1H), 7.72 (dd, $J$ = 8.7, 1.8 Hz, 1H), 7.56 (d, $J$ = 8.7 Hz, 1H), 7.50 (td, $J$ = 5.6, 2.3 Hz, 3H), 7.25 - 7.15 (m, 2H), 4.04 (q, $J$ = 7.0 Hz, 1H), 3.80 (s, 3H), 2.10 (s, 1H), 1.49 (d, $J$ = 7.0 Hz, 3H), 0.87 (d, $J$ = 6.2 Hz, 4H).

**Example 178: Preparation of Compound 178**

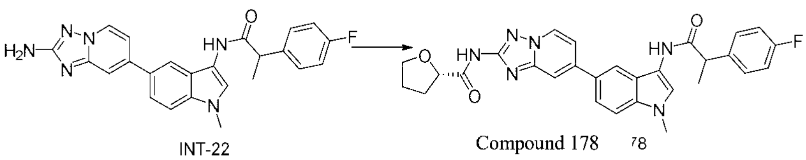

INT-22 Compound 178 78

Preparation of Compound 178:

**INT-22** (84 mg, 0.2 mmol) was dissolved in pyridine (1 mL), (S)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 178 with a yield of 71%. MS (ESI, positive ion) m/z: 527.2 $[M + H]^+$.

$^1$H NMR (400 MHz, DMSO-$d_6$) δ 10.45 (s, 1H), 10.17 (s, 1H), 8.94 (d, *J* = 7.1 Hz, 1H), 8.38 (d, *J* = 1.8 Hz, 1H), 7.94 (d, *J* = 2.0 Hz, 1H), 7.85 (s, 1H), 7.72 (dd, *J* = 8.7, 1.8 Hz, 1H), 7.58 - 7.48 (m, 4H), 7.21 - 7.16 (m, 2H), 4.55 (s, 1H), 4.10 - 3.97 (m, 2H), 3.88 - 3.83 (m, 1H), 3.80 (s, 3H), 2.33 - 2.19 (m, 1H), 2.02 (td, *J* = 7.1, 5.5 Hz, 1H), 1.98 - 1.93 (m, 1H), 1.49 (d, *J* = 7.0 Hz, 3H), 1.44 (s, 1H).

**Example 179: Preparation of Compound 179**

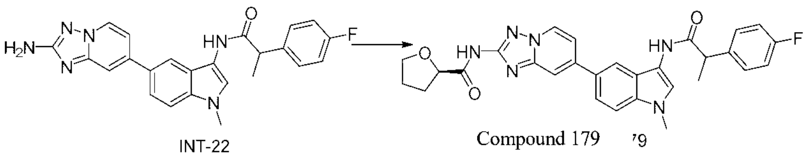

INT-22

Compound 179

Preparation of Compound 179:

**INT-22** (84 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 179 with a yield of 77%. MS (ESI, positive ion) m/z: 527.2 $[M + H]^+$.

$^1$H NMR (400 MHz, DMSO-$d_6$) δ 10.72 (s, 1H), 10.47 (s, 1H), 8.94 (dd, *J* = 7.1, 0.8 Hz, 1H), 8.22 (s, 1H), 7.96 - 7.89 (m, 2H), 7.73 (d, *J* = 8.9 Hz, 1H), 7.52 (dd, *J* = 8.4, 5.5 Hz, 2H), 7.43 (dd, *J* = 7.2, 2.0 Hz, 1H), 7.21 (t, *J* = 8.8 Hz, 2H), 4.54 (s, 1H), 4.02 (d, *J* = 8.5 Hz, 6H), 3.85 (q, *J* = 7.0 Hz, 1H), 2.32 - 2.19 (m, 1H), 2.07 - 1.84 (m, 3H), 1.50 (d, *J* = 7.0 Hz, 3H).

**Example 180: Preparation of Compound 180**

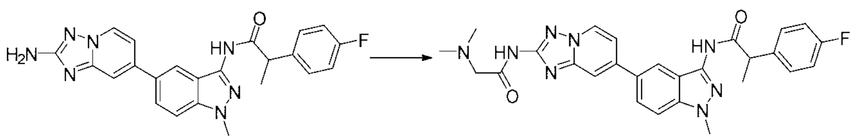

INT-23

Compound 180

Preparation of Compound 180:

**INT-23** (86 mg, 0.2 mmol) was dissolved in pyridine (1 mL), 2-(dimethylamino)acetic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 180 with a yield of 73%. MS (ESI, positive ion) m/z: 515.2 $[M + H]^+$.

$^1$H NMR (400 MHz, DMSO-$d_6$) δ 10.76 (s, 2H), 8.94 (d, *J* = 7.1 Hz, 1H), 8.23 (s, 1H), 7.91 (d, *J* = 6.5 Hz, 2H), 7.72 (d, *J* = 8.9 Hz, 1H), 7.52 (t, *J* = 6.9 Hz, 2H), 7.43 (d, *J* = 7.2 Hz, 1H), 7.21 (t, *J* = 8.7 Hz, 2H), 4.06 (s, 1H), 4.00 (s, 3H), 3.39 (s, 2H), 2.42 (s, 6H), 1.50 (d, *J* = 6.8 Hz, 3H).

**Example 181: Preparation of Compound 181**

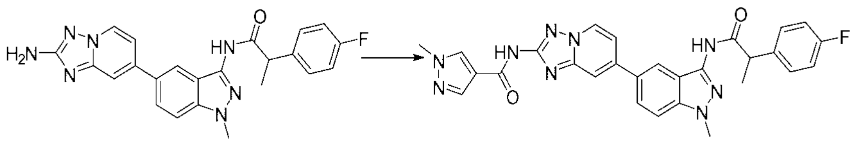

INT-23 Compound 181

Preparation of Compound 181:

**INT-23** (86 mg, 0.2 mmol) was dissolved in pyridine (1 mL); 1-methylpyrazole-4-carboxylic acid (38 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 181 with a yield of 62%. MS (ESI, positive ion) m/z: 538.2 $[M + H]^+$.

$^1$H NMR (400 MHz, DMSO-$d_6$) δ 10.92 (s, 1H), 10.74 (s, 1H), 8.96 (d, *J* = 7.1 Hz, 1H), 8.44 (s, 1H), 8.22 (s, 1H), 8.13 (s, 1H), 7.93 (td, *J* = 4.9, 4.4, 1.8 Hz, 2H), 7.73 (d, *J* = 8.9 Hz, 1H), 7.52 (dd, *J* = 8.5, 5.6 Hz, 2H), 7.42 (dd, *J* = 7.1, 2.0 Hz, 1H), 7.22 (t, *J* = 8.8 Hz, 2H), 4.04 (d, *J* = 7.2 Hz, 2H), 4.01 (s, 3H), 3.92 (s, 3H), 1.50 (d, *J* = 7.0 Hz, 3H).

**Example 182: Preparation of Compound 182**

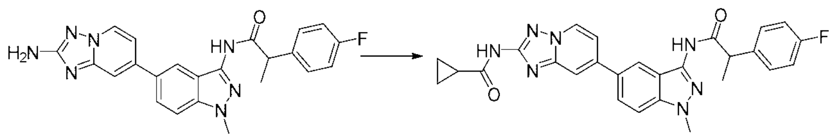

INT-23 Compound 182

Preparation of Compound 182:

Intermediate **INT-23** (86 mg, 0.2 mmol) was dissolved in pyridine (1 mL); cyclopropanecarboxylic acid (28 mg, 0.3 mmol) and 1-propylphosphonic anhydride (111 mg, 0.351 mmol) were added; the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 182 with a yield of 61%. MS (ESI, positive ion) m/z: 498.2 $[M + H]^+$.

$^1$H NMR (400 MHz, DMSO-$d_6$) δ 11.07 (s, 1H), 10.71 (s, 1H), 8.90 (d, *J* = 7.0 Hz, 1H), 8.21 (s, 1H), 7.90 (d, *J* = 8.9 Hz, 2H), 7.71 (d, *J* = 8.9 Hz, 1H), 7.52 (t, *J* = 6.9 Hz, 2H), 7.39 (d, *J* = 7.2 Hz, 1H), 7.21 (t, *J* = 8.6 Hz, 2H), 4.01 (d, *J* = 11.9 Hz, 4H), 2.05 (d, *J* = 41.0 Hz, 1H), 1.50 (d, *J* = 7.1 Hz, 3H), 0.86 (d, *J* = 6.3 Hz, 4H).

**Example 183: Preparation of Compound 183**

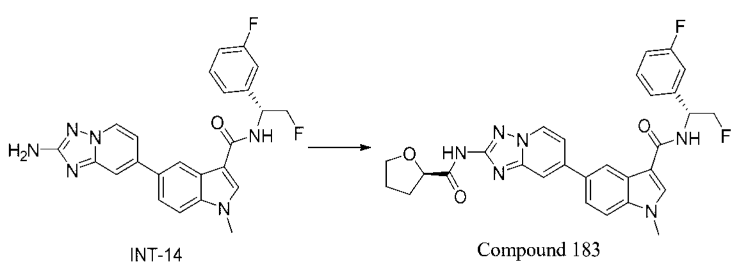

INT-14 Compound 183

Preparation of Compound 183:

**INT-14** (86 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel

column (acetonitrile/0.1% aqueous ammonium bicarbonate solution = 60%) to obtain Compound 183 with a yield of 67%. MS (ESI, positive ion) m/z: 545.2 [M + H]+.

$^1$H NMR (400 MHz, DMSO-$d_6$) δ 10.49 (s, 1H), 8.90 (d, *J* = 7.1 Hz, 1H), 8.65 (d, *J* = 8.3 Hz, 1H), 8.55 (d, *J* = 1.8 Hz, 1H), 8.30 (s, 1H), 7.92 (dd, *J* = 2.0, 0.9 Hz, 1H), 7.77 (dd, *J* = 8.7, 1.8 Hz, 1H), 7.70 (d, *J* = 8.6 Hz, 1H), 7.52 - 7.47 (m, 1H), 7.44 (dd, *J* = 7.9, 6.2 Hz, 1H), 7.36 (d, *J* = 8.4 Hz, 2H), 7.19 - 7.12 (m, 1H), 5.50 (dt, *J* = 14.8, 7.4 Hz, 1H), 4.78 (d, *J* = 6.4 Hz, 1H), 4.66 (d, *J* = 6.4 Hz, 1H), 4.53 (s, 1H), 4.01 (q, *J* = 7.1, 6.7 Hz, 1H), 3.94 (s, 3H), 3.84 (q, *J* = 7.2 Hz, 1H), 2.30 - 2.19 (m, 1H), 2.01 - 1.89 (m, 3H).

**Example 184: Preparation of Compound 184**

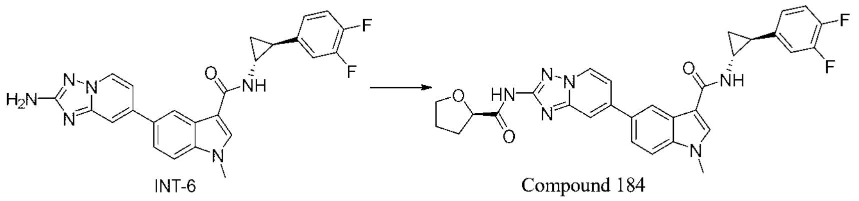

INT-6 Compound 184

Preparation of Compound 184:

**INT-6** (91 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 184 with a yield of 71%. MS (ESI, positive ion) m/z: 557.2 [M + H]+.

$^1$H NMR (400 MHz, DMSO-$d_6$) δ 10.47 (s, 1H), 8.90 (d, *J* = 7.2 Hz, 1H), 8.58 (d, *J* = 1.8 Hz, 1H), 8.31 (d, *J* = 3.8 Hz, 1H), 8.08 (s, 1H), 7.93 (d, *J* = 1.9 Hz, 1H), 7.75 (dd, *J* = 8.6, 1.9 Hz, 1H), 7.68 (d, *J* = 8.7 Hz, 1H), 7.51 (dd, *J* = 7.1, 2.0 Hz, 1H), 7.42 - 7.23 (m, 2H), 7.09 (dq, *J* = 6.7, 2.8, 2.2 Hz, 1H), 4.54 (s, 1H), 4.02 (dt, *J* = 8.1, 6.7 Hz, 1H), 3.91 (s, 3H), 3.89 - 3.80 (m, 1H), 3.02 (dq, *J* = 8.0, 3.9 Hz, 1H), 2.25 (dtd, *J* = 11.4, 7.9, 6.1 Hz, 1H), 2.09 (ddd, *J* = 9.5, 6.2, 3.3 Hz, 1H), 2.05 - 1.81 (m, 3H), 1.39 (dt, *J* = 9.7, 5.3 Hz, 1H), 1.29 (dt, *J* = 7.9, 5.8 Hz, 1H).

**Example 185: Preparation of Compound 185**

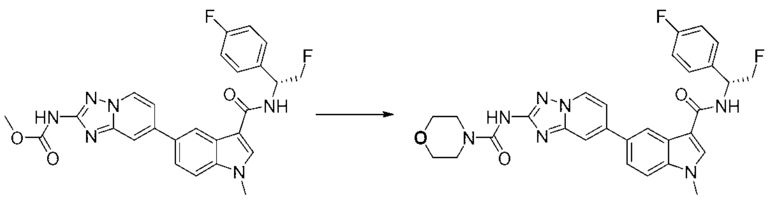

Compound 95-1 Compound 185 5

Preparation of Compound 185:

Compound 95-1 (100 mg, 0.2 mmol) was dissolved in acetonitrile (1 mL); morpholine (36 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 185 with a yield of 66%. MS (ESI, positive ion) m/z: 560.2 [M + H]+.

$^1$H NMR (400 MHz, DMSO-$d_6$) δ 10.53 (s, 1H), 8.90 (dd, *J* = 7.1, 0.8 Hz, 1H), 8.62 (d, *J* = 8.3 Hz, 1H), 8.55 (d, *J* = 1.8 Hz, 1H), 8.27 (s, 1H), 7.91 (dd, *J* = 1.9, 0.9 Hz, 1H), 7.76 (dd, *J* = 8.7, 1.8 Hz, 1H), 7.69 (d, *J* = 8.6 Hz, 1H), 7.60 - 7.53 (m, 2H), 7.48 (dd, *J* = 7.2, 2.0 Hz, 1H), 7.28 - 7.18 (m, 2H), 5.49 (dq, *J* = 15.3, 7.3 Hz, 1H), 4.82 - 4.70 (m, 1H), 4.70 - 4.60 (m, 1H), 3.93 (s, 3H), 3.64 (t, *J* = 4.6 Hz, 4H), 3.27 (s, 2H), 2.58 - 2.55 (m, 4H).

**Example 186: Preparation of Compound 186**

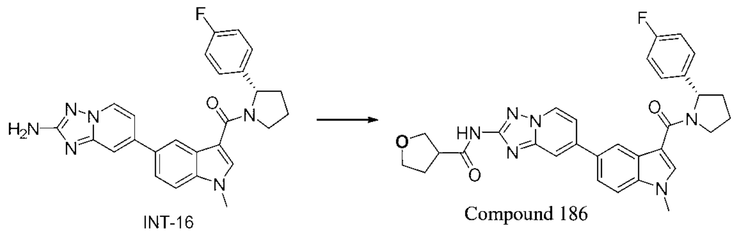

INT-16

Compound 186

Preparation of Compound 186:

INT-16 (90 mg, 0.2 mmol) was dissolved in pyridine (1 mL), tetrahydrofuran-3-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 186 with a yield of 62%. MS (ESI, positive ion) m/z: 553.2 $[M + H]^+$.

1H NMR (400 MHz, DMSO-d6) δ 11.07 (s, 1H), 8.82 (m, 1H), 8.48 (d, J = 1.8 Hz, 1H), 8.35 (s, 1H),7.85 (m, 2H), 7.73 (d, J = 8.6 Hz, 1H),7.59 - 7.49 (m, 1H), 7.42 (dd, J = 7.1, 2.0 Hz, 1H), 7.32 (m, 1H), 7.13 (t, J = 8.7 Hz, 2H), 4.71-4.52 (m, 2H), 4.23 (s, 3H), 4.16-3.70 (m, 4H), 2.81 (m, 1H), 2.23-2.04 (m, 2H).

**Example 187: Preparation of Compound 187**

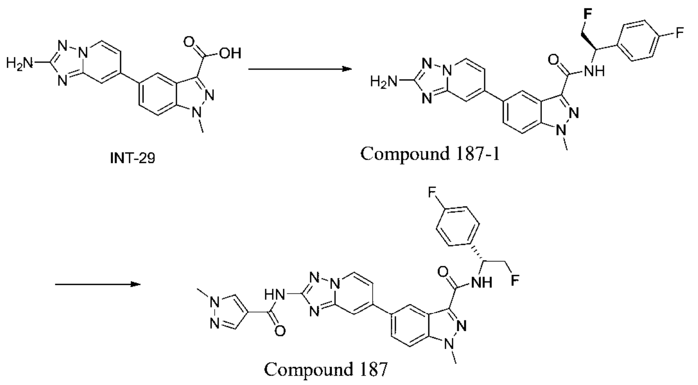

INT-29

Compound 187-1

Compound 187

Preparation of Compound 187-1:

Intermediate **INT-29** (321 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (R)-2-fluoro-1-(3-fluorophenyl)ethan-1-amine (188 mg, 1.2 mmol) was added, and the mixture was stirred for reaction at room temperature for 2 h. The reaction mixture was added dropwise to water and stirred for 0.5 h. The mixture was filtered and dried to obtain Compound 187-1 with a yield of 63%. MS (ESI, positive ion) m/z: 448.3 $[M + H]^+$.

Preparation of Compound 187:

Compound 187-1 (89 mg, 0.2 mmol) was dissolved in pyridine (1 mL), 1-methylpyrazole-4-carboxylic acid (38 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 187 with a yield of 52%. MS (ESI, positive ion) m/z: 556.2 $[M + H]^+$.

$^1$H NMR (400 MHz, DMSO-$d_6$) δ 10.94 (s, 1H), 8.94 (dd, *J* = 7.1, 0.8 Hz, 1H), 8.89 (d, *J* = 8.4 Hz, 1H), 8.78 (s, 1H), 8.55 (dd, *J* = 1.8, 0.8 Hz, 1H), 8.43 (s, 1H), 8.13 (d, *J* = 0.8 Hz, 1H), 8.05 - 7.98 (m, 2H), 7.93 (d, *J* = 9.1 Hz, 1H), 7.78 (s, 1H),

7.52 (ddd, *J* = 8.7, 3.6, 1.9 Hz, 3H), 7.17 (t, *J* = 8.9 Hz, 2H), 5.26 (m, 1H), 4.71-4.52 (m, 2H), 4.23 (s, 3H), 3.86 (s, 3H).

**Example 188: Preparation of Compound 188**

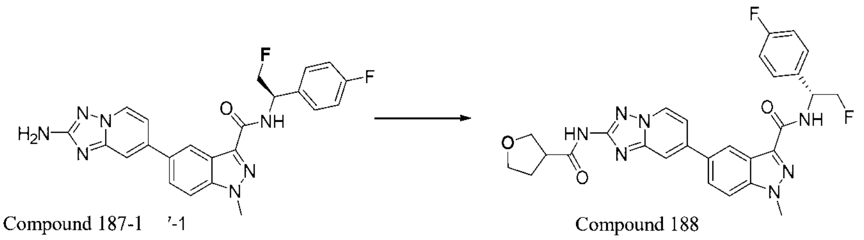

Compound 187-1 '-1 Compound 188

Preparation of Compound 188:

Compound 187-1 (89 mg, 0.2 mmol) was dissolved in pyridine (1 mL), tetrahydrofuran-3-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 188 with a yield of 52%. MS (ESI, positive ion) m/z: 546.2 $[M + H]^+$.

$^1$H NMR (400 MHz, DMSO-$d_6$) δ 10.94 (s, 1H), 8.94 (dd, *J* = 7.1, 0.8 Hz, 1H), 8.89 (d, *J* = 8.4 Hz, 1H), 8.55 (dd, *J* = 1.8, 0.8 Hz, 1H), 8.43 (s, 1H), 8.13 (d, *J* = 0.8 Hz, 1H), 8.05 - 7.98 (m, 2H), 7.93 (d, *J* = 9.1 Hz, 1H), 7.52 (ddd, *J* = 8.7, 3.6, 1.9 Hz, 3H), 7.17 (t, *J* = 8.9 Hz, 2H), 5.26 (m, 1H), 4.71-4.52 (m, 2H), 4.23 (s, 3H), 4.16-3.70 (m, 4H), 2.81 (m, 1H), 2.23-2.04 (m, 2H).

**Example 189: Preparation of Compound 189**

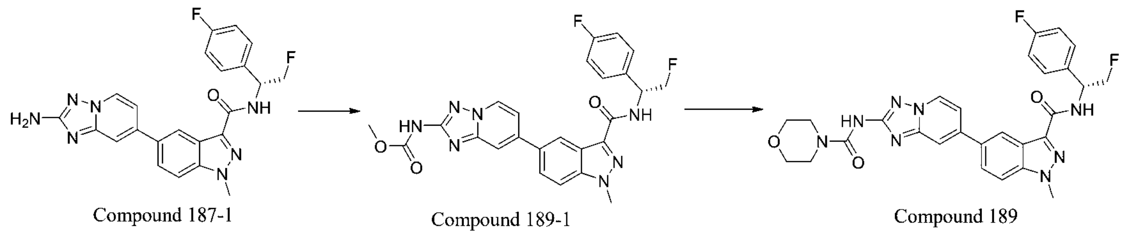

Compound 187-1 Compound 189-1 Compound 189

Preparation of Compound 189-1:

Intermediate 187-1 (447 mg, 1 mmol) was dissolved in dichloromethane (0.3 L); methyl chloroformate (112 mg, 1.2 mmol) and N,N-diisopropylethylamine (258 mg, 2.0 mmol) were added, and the mixture was stirred at room temperature for 2 h. Petroleum ether (0.3 L) was added to the reaction mixture for crystallization; the solid was filtered with suction, washed with water, and dried to obtain Compound 189-1 with a yield of 61%. MS (ESI, positive ion) m/z: 506.2 $[M + H]^+$.

Preparation of Compound 189:

Compound 189-1 (101 mg, 0.2 mmol) was dissolved in acetonitrile (1 mL); morpholine (36 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 189 with a yield of 58%. MS (ESI, positive ion) m/z: 561.2 $[M + H]^+$.

$^1$H NMR (400 MHz, DMSO-$d_6$) δ 9.65 (s, 1H), 9.20 (d, *J* = 8.6 Hz, 1H), 8.85 (d, *J* = 7.1 Hz, 1H), 8.52 (s, 1H), 8.03 - 7.97 (m, 1H), 7.97 - 7.90 (m, 2H), 7.62 - 7.54 (m, 2H), 7.49 - 7.43 (m, 1H), 7.22 (t, *J* = 8.9 Hz, 2H), 5.50 (dq, *J* = 13.5, 8.4, 6.8 Hz, 1H), 5.00 - 4.73 (m, 1H), 4.69 (ddd, *J* = 46.3, 9.5, 5.6 Hz, 1H), 4.24 (s, 3H), 3.66 - 3.59 (m, 4H), 3.47 (t, *J* = 4.7 Hz, 4H).

**Example 190: Preparation of Compound 190**

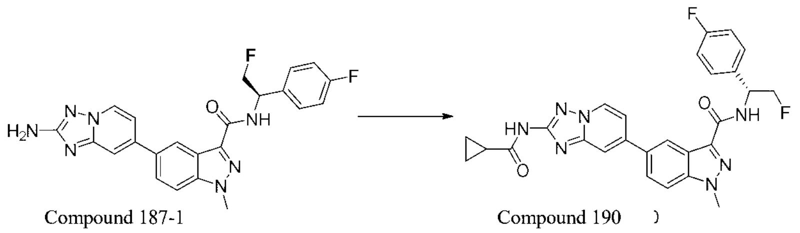

Compound 187-1 Compound 190

Preparation of Compound 190:

Compound 187-1 (89 mg, 0.2 mmol) was dissolved in pyridine (1 mL), cyclopropanecarboxylic acid (28 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 190 with a yield of 72%. MS (ESI, positive ion) m/z: 516.2 $[M + H]^+$.

$^1$H NMR (400 MHz, DMSO-$d_6$) δ 10.94 (s, 1H), 8.94 (dd, *J* = 7.1, 0.8 Hz, 1H), 8.89 (d, *J* = 8.4 Hz, 1H), 8.55 (dd, *J* = 1.8, 0.8 Hz, 1H), 8.43 (s, 1H), 8.13 (d, *J*= 0.8 Hz, 1H), 8.05 - 7.98 (m, 2H), 7.93 (d, *J* = 9.1 Hz, 1H), 7.52 (ddd, *J* = 8.7, 3.6, 1.9 Hz, 3H), 7.17 (t, *J* = 8.9 Hz, 2H), 5.26 (p, *J* = 7.4 Hz, 1H), 4.23 (s, 3H), 4.04-3.93 (m, 2H), 1.55 (d, *J* = 7.1 Hz, 3H).

**Example 191: Preparation of Compound 191**

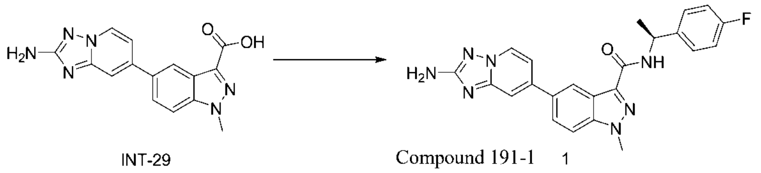

INT-29 Compound 191-1 1

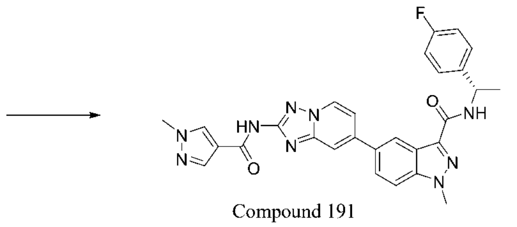

Compound 191

Preparation of Compound 191-1:

Intermediate **INT-29** (321 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-1-(3-fluorophenyl)ethan-1-amine (171 mg, 1.2 mmol) was added, and the mixture was stirred for reaction at room temperature for 2 h. The reaction mixture was added dropwise to water and stirred for 0.5 h. The mixture was filtered and dried to obtain Compound 191-1 with a yield of 61%. MS (ESI, positive ion) m/z: 430.3 $[M + H]^+$.

Preparation of Compound 191:

Compound 191-1 (86 mg, 0.2 mmol) was dissolved in pyridine (1 mL), 1-methylpyrazole-4-carboxylic acid (38 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 191 with a yield of 52%. MS (ESI, positive ion) m/z: 538.2 $[M + H]^+$.

$^1$H NMR (400 MHz, DMSO-$d_6$) δ 10.94 (s, 1H), 8.94 (dd, *J* = 7.1, 0.8 Hz, 1H), 8.89 (d, *J* = 8.4 Hz, 1H), 8.55 (dd, *J* =

1.8, 0.8 Hz, 1H), 8.43 (s, 1H), 8.13 (d, *J* = 0.8 Hz, 1H), 8.05 - 7.98 (m, 2H), 7.93 (d, *J* = 9.1 Hz, 1H), 7.52 (ddd, *J* = 8.7, 3.6, 1.9 Hz, 3H), 7.17 (t, *J* = 8.9 Hz, 2H), 5.26 (p, *J* = 7.4 Hz, 1H), 4.23 (s, 3H), 3.92 (s, 3H), 1.55 (d, *J* = 7.1 Hz, 3H).

**Example 192: Preparation of Compound 192**

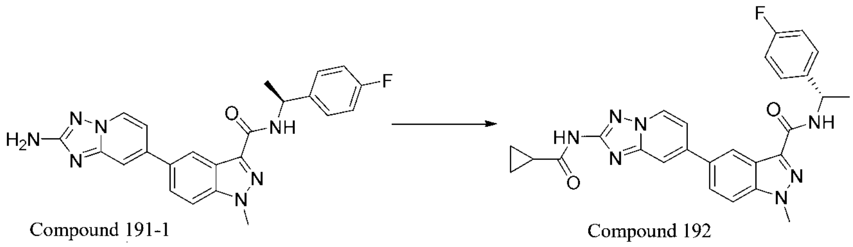

Compound 191-1

Compound 192

Preparation of Compound 192:

Compound 191-1 (86 mg, 0.2 mmol) was dissolved in pyridine (1 mL), cyclopropanecarboxylic acid (28 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 192 with a yield of 62%. MS (ESI, positive ion) m/z: 498.2 $[M + H]^+$.

$^1H$ NMR (400 MHz, DMSO-$d_6$) δ 11.11 (s, 1H), 8.92 - 8.86 (m, 2H), 8.56 - 8.51 (m, 1H), 8.04 - 7.95 (m, 2H), 7.92 (d, *J* = 8.9 Hz, 1H), 7.56 - 7.46 (m, 3H), 7.22 - 7.13 (m, 2H), 5.26 (p, *J* = 7.3 Hz, 1H), 4.22 (s, 3H), 2.45 - 1.65 (m, -1H), 1.55 (d, *J* = 7.0 Hz, 3H), 0.86 (d, *J* = 6.2 Hz, 4H).

**Example 193: Preparation of Compound 193**

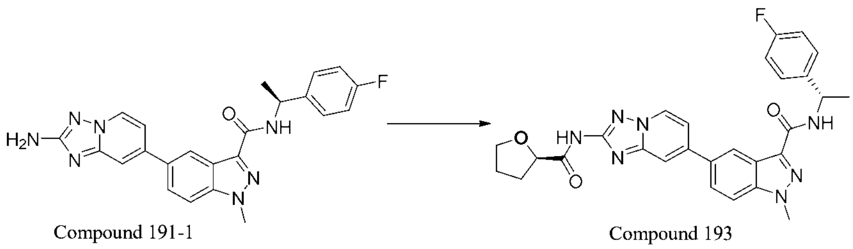

Compound 191-1

Compound 193

Preparation of Compound 193:

Compound 191-1 (86 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 193 with a yield of 72%. MS (ESI, positive ion) m/z: 528.2 $[M + H]^+$.

$^1H$ NMR (400 MHz, DMSO-$d_6$) δ 10.49 (s, 1H), 8.92 (dd, *J* = 7.1, 0.8 Hz, 1H), 8.87 (d, *J* = 8.4 Hz, 1H), 8.56 - 8.51 (m, 1H), 8.00 (ddd, *J* = 5.5, 3.9, 1.8 Hz, 2H), 7.92 (d, *J* = 8.9 Hz, 1H), 7.54 - 7.50 (m, 3H), 7.22 - 7.12 (m, 2H), 5.26 (p, *J* = 7.1 Hz, 1H), 4.54 (s, 1H), 4.22 (s, 3H), 4.02 (dt, *J* = 8.0, 6.7 Hz, 1H), 3.90 - 3.80 (m, 1H), 2.25 (dtd, *J* = 11.4, 8.0, 6.1 Hz, 1H), 2.03 - 1.88 (m, 3H), 1.56 (d, *J* = 7.1 Hz, 3H).

**Example 194: Preparation of Compound 194**

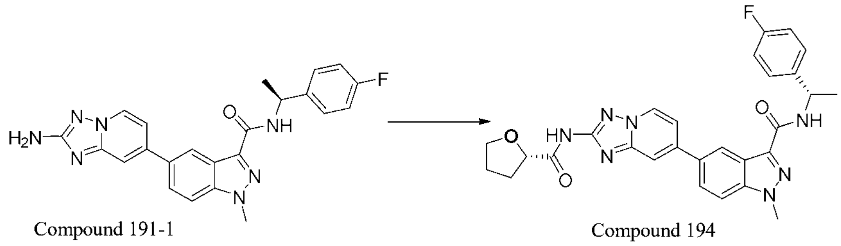

Compound 191-1 Compound 194

Preparation of Compound 194:

Compound 191-1 (86 mg, 0.2 mmol) was dissolved in pyridine (1 mL), (S)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 194 with a yield of 66%. MS (ESI, positive ion) m/z: 528.2 $[M + H]^+$.

$^1$H NMR (400 MHz, DMSO-$d_6$) δ 10.50 (s, 1H), 8.95 - 8.85 (m, 2H), 8.56 - 8.50 (m, 1H), 8.03 - 7.96 (m, 2H), 7.92 (d, *J* = 9.0 Hz, 1H), 7.52 (td, *J* = 5.6, 2.6 Hz, 3H), 7.22 - 7.12 (m, 2H), 5.26 (p, *J* = 7.2 Hz, 1H), 4.53 (s, 1H), 4.22 (s, 3H), 4.01 (q, *J* = 6.9 Hz, 1H), 3.84 (td, *J* = 7.6, 6.5 Hz, 1H), 2.30 - 1.92 (m, 2H), 1.97 - 1.82 (m, 2H), 1.55 (d, *J* = 7.1 Hz, 3H).

**Example 195: Preparation of Compound 195**

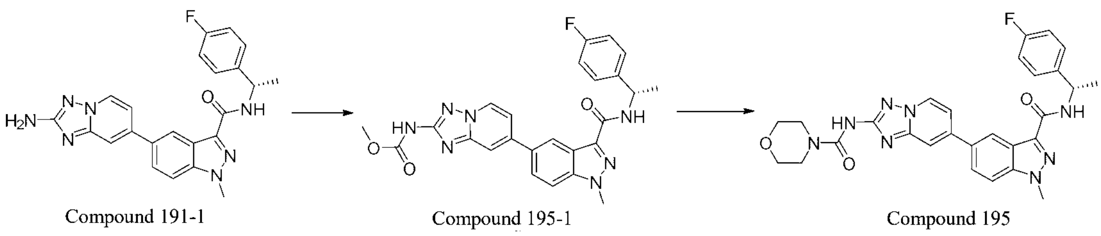

Compound 191-1 Compound 195-1 Compound 195

Preparation of Compound 195-1:

Intermediate 191-1 (429 mg, 1 mmol) was dissolved in dichloromethane (0.3 L); methyl chloroformate (112 mg, 1.2 mmol) and N,N-diisopropylethylamine (258 mg, 2.0 mmol) were added, and the mixture was stirred at room temperature for 2 h. Petroleum ether (0.3 L) was added to the reaction mixture for crystallization; the solid was filtered with suction, washed with water, and dried to obtain Compound 195-1 with a yield of 61%. MS (ESI, positive ion) m/z: 488.2 $[M + H]^+$.

Preparation of Compound 195:

Compound 195-1 (97 mg, 0.2 mmol) was dissolved in acetonitrile (1 mL); morpholine (36 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 195 with a yield of 58%. MS (ESI, positive ion) m/z: 543.2 $[M + H]^+$.

$^1$H NMR (400 MHz, DMSO-$d_6$) δ 9.63 (s, 1H), 8.86 (t, *J* = 7.3 Hz, 2H), 8.52 (d, *J* = 1.8 Hz, 1H), 7.99 (dd, *J* = 8.9, 1.8 Hz, 1H), 7.95 - 7.85 (m, 2H), 7.57 - 7.49 (m, 2H), 7.46 (dd, *J* = 7.2, 2.0 Hz, 1H), 7.21 - 7.12 (m, 2H), 5.26 (p, *J* = 7.3 Hz, 1H), 4.22 (s, 3H), 3.63 (t, *J* = 4.7 Hz, 4H), 3.48 (t, *J* = 4.8 Hz, 4H), 1.55 (d, *J* = 7.0 Hz, 3H).

**Example 196: Preparation of Compound 196**

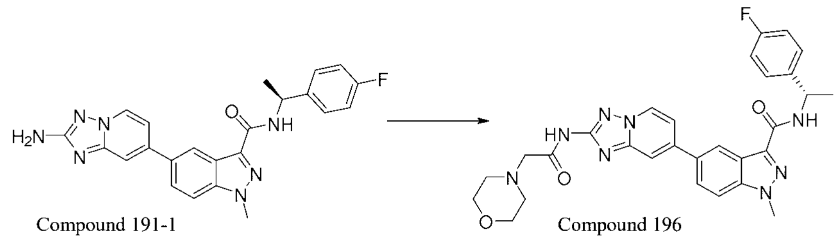

Compound 191-1

Compound 196

Preparation of Compound 196:

Compound 191-1 (86 mg, 0.2 mmol) was dissolved in pyridine (1 mL), morpholineacetic acid (43 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 196 with a yield of 71%. MS (ESI, positive ion) m/z: 557.2 $[M + H]^+$.

$^1$H NMR (400 MHz, DMSO-$d_6$) δ 10.53 (s, 1H), 8.85 (dd, *J* = 17.4, 7.7 Hz, 2H), 8.49 (d, *J* = 1.7 Hz, 1H), 7.93 (dd, *J* = 6.5, 1.9 Hz, 2H), 7.86 (d, *J* = 8.9 Hz, 1H), 7.53 - 7.45 (m, 3H), 7.14 (t, *J* = 8.9 Hz, 2H), 5.24 (t, *J* = 7.5 Hz, 1H), 4.18 (s, 3H), 3.62 (t, *J* = 4.5 Hz, 4H), 3.26 (s, 2H), 2.54 (t, *J* = 4.6 Hz, 4H), 1.53 (d, *J* = 7.0 Hz, 3H).

**Example 197: Preparation of Compound 197**

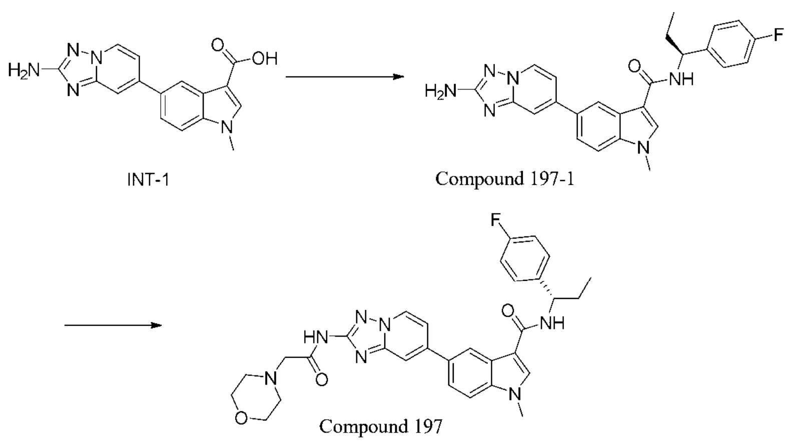

INT-1

Compound 197-1

Compound 197

Preparation of Compound 197-1:

Intermediate **INT-1** (306 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-1-(3-fluorophenyl)propan-1-amine (188 mg, 1.2 mmol) was added, and the mixture was stirred for reaction at room temperature for 2 h. The reaction mixture was added dropwise to water and stirred for 0.5 h. The mixture was filtered and dried to obtain Compound 197-1 with a yield of 64%. MS (ESI, positive ion) m/z: 443.3 $[M + H]^+$.

Preparation of Compound 197:

Compound 197-1 (88 mg, 0.2 mmol) was dissolved in pyridine (1 mL), morpholineacetic acid (43 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 197 with a yield of 61%. MS (ESI, positive ion) m/z: 570.2 $[M + H]^+$.

$^1$H NMR (400 MHz, DMSO-$d_6$) δ 10.54 (s, 1H), 8.89 (d, *J* = 7.1 Hz, 1H), 8.55 (d, *J* = 1.9 Hz, 1H), 8.38 (d, *J* = 8.1 Hz, 1H), 8.23 (s, 1H), 7.90 (d, *J* = 1.9 Hz, 1H), 7.74 (dd, *J* = 8.6, 1.8 Hz, 1H), 7.67 (d, *J* = 8.7 Hz, 1H), 7.47 (ddt, *J* = 5.8, 4.1, 1.7 Hz, 3H), 7.22 - 7.12 (m, 2H), 5.22 (p, *J* = 7.2 Hz, 1H), 3.91 (s, 3H), 3.64 (t, *J* = 4.6 Hz, 4H), 3.27 (s, 2H), 2.56 (dd, *J* = 5.4, 3.4 Hz, 4H), 1.50 (d, *J* = 7.0 Hz, 3H).

**Example 198: Preparation of Compound 198**

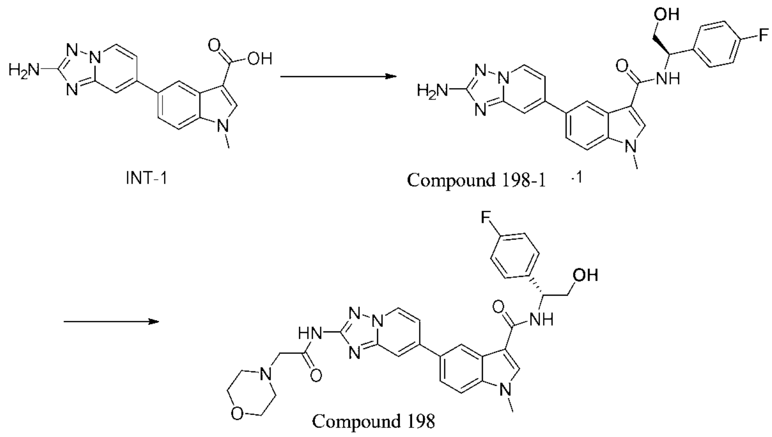

INT-1

Compound 198-1

Compound 198

Preparation of Compound 198-1:

Intermediate **INT-1** (306 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-1-(3-fluorophenyl)ethanol-1-amine (190 mg, 1.2 mmol) was added, and the mixture was stirred for reaction at room temperature for 2 h. The reaction mixture was added dropwise to water and stirred for 0.5 h. The mixture was filtered and dried to obtain Compound 198-1 with a yield of 54%. MS (ESI, positive ion) m/z: 443.3 $[M + H]^+$.

Preparation of Compound 198:

Compound 198-1 (88 mg, 0.2 mmol) was dissolved in pyridine (1 mL), morpholineacetic acid (43 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 198 with a yield of 61%. MS (ESI, positive ion) m/z: 571.2 $[M + H]^+$.

$^1$H NMR (400 MHz, DMSO-$d_6$) δ 10.54 (s, 1H), 8.89 (d, *J* = 7.1 Hz, 1H), 8.55 (d, *J* = 1.9 Hz, 1H), 8.38 (d, *J* = 8.1 Hz, 1H), 8.23 (s, 1H), 7.90 (d, *J* = 1.9 Hz, 1H), 7.74 (dd, *J* = 8.6, 1.8 Hz, 1H), 7.67 (d, *J* = 8.7 Hz, 1H), 7.47 (ddt, *J* = 5.8, 4.1, 1.7 Hz, 3H), 7.22 - 7.12 (m, 2H), 5.22 (p, *J* = 7.2 Hz, 1H), 3.95 (s, 1H), 3.81-3.72(m, 2H), 3.64 (t, *J* = 4.6 Hz, 4H), 3.27 (s, 2H), 2.56 (dd, *J* = 5.4, 3.4 Hz, 4H), 1.50 (d, *J* = 7.0 Hz, 3H).

**Example 199: Preparation of Compound 199**

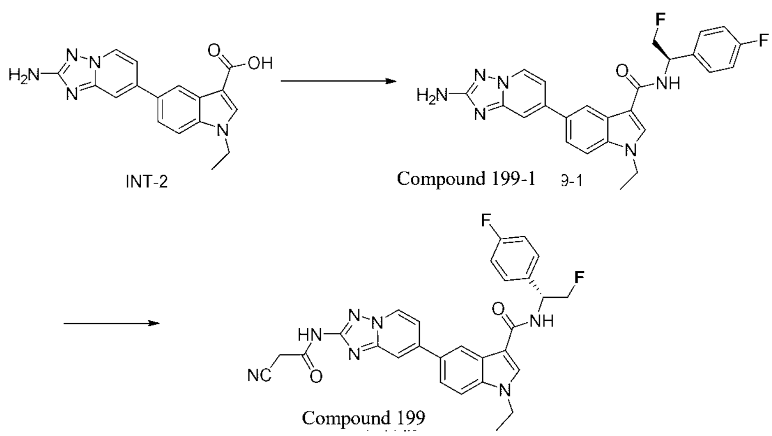

INT-2

Compound 199-1

Compound 199

Preparation of Compound 199-1:

Intermediate **INT-2** (320 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (R)-2-fluoro-1-(3-fluorophenyl)ethan-1-amine (188 mg, 1.2 mmol) was added, and the mixture was stirred for reaction at room temperature for 2 h. The reaction mixture was added dropwise to water and stirred for 0.5 h. The mixture was filtered and dried to obtain Compound 199-1 with a yield of 67%. MS (ESI, positive ion) m/z: 461.3 $[M + H]^+$.

Preparation of Compound 199:

Compound 199-1 (92 mg, 0.2 mmol) was dissolved in pyridine (1 mL), cyanoacetic acid (25 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 199 with a yield of 57%. MS (ESI, positive ion) m/z: 527.2 $[M + H]^+$.

$^1$H NMR (400 MHz, DMSO-$d_6$) δ 11.29 (s, 1H), 8.92 (d, *J* = 7.1 Hz, 1H), 8.62 (d, *J* = 8.2 Hz, 1H), 8.55 (d, *J* = 1.8 Hz, 1H), 8.27 (s, 1H), 7.94 (d, *J* = 1.8 Hz, 1H), 7.77 (dd, *J* = 8.6, 1.9 Hz, 1H), 7.69 (d, *J* = 8.7 Hz, 1H), 7.57 - 7.49 (m, 3H), 7.23 (t, *J* = 8.8 Hz, 2H), 5.53 - 5.43 (m, 1H), 4.76 (d, *J* = 8.0 Hz, 1H), 4.67 - 4.61 (m, 1H), 4.04-3.93 (m, 2H), 2.72 - 2.55 (m, 1H), 2.35 (s, 1H), 1.50 (d, *J* = 7.2 Hz, 3H).

**Example 200: Preparation of Compound 200**

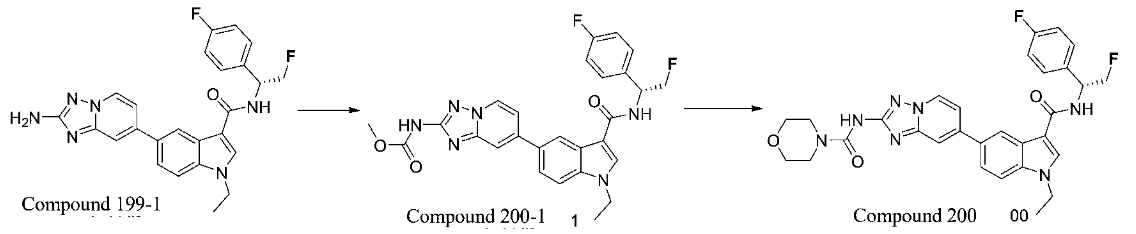

Compound 199-1

Compound 200-1

Compound 200

Preparation of Compound 200-1:

Intermediate 199-1 (460 mg, 1 mmol) was dissolved in dichloromethane (0.3 L); methyl chloroformate (112 mg, 1.2 mmol) and N,N-diisopropylethylamine (258 mg, 2.0 mmol) were added, and the mixture was stirred at room temperature for 2 h. Petroleum ether (0.3 L) was added to the reaction mixture for crystallization; the solid was filtered with suction, washed with water, and dried to obtain Compound 200-1 with a yield of 55%. MS (ESI, positive ion) m/z: 519.2 $[M + H]^+$.

Preparation of Compound 200:

Compound 200-1 (104 mg, 0.2 mmol) was dissolved in acetonitrile (1 mL); morpholine (36 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 200 with a yield of 58%. MS (ESI, positive ion) m/z: 574.2 $[M + H]^+$.

$^1$H NMR (400 MHz, DMSO-$d_6$) δ 11.29 (s, 1H), 8.92 (d, *J* = 7.1 Hz, 1H), 8.62 (d, *J* = 8.2 Hz, 1H), 8.55 (d, *J* = 1.8 Hz, 1H), 8.27 (s, 1H), 7.94 (d, *J* = 1.8 Hz, 1H), 7.77 (dd, *J* = 8.6, 1.9 Hz, 1H), 7.69 (d, *J* = 8.7 Hz, 1H), 7.57 - 7.49 (m, 3H), 7.23 (t, *J* = 8.8 Hz, 2H), 5.53 - 5.43 (m, 1H), 4.76 (d, *J* = 8.0 Hz, 1H), 4.67 - 4.61 (m, 1H), 3.66 - 3.58 (m, 4H), 3.48 (t, *J* = 4.8 Hz, 4H), 2.72 - 2.55 (m, 1H), 2.35 (s, 1H), 1.50 (d, *J* = 7.2 Hz, 3H).

**Example 201: Preparation of Compound 201**

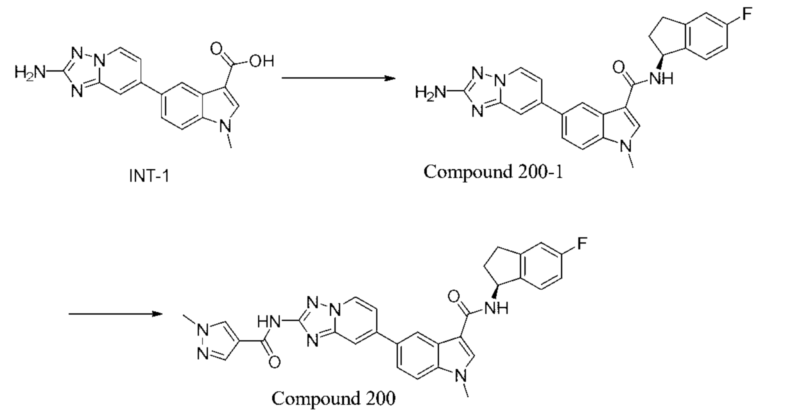

INT-1 Compound 200-1

Compound 200

Preparation of Compound 201-1:

Intermediate **INT-1** (306 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-5-fluoro-2,3-dihydro-1H-inden-1-amine (181 mg, 1.2 mmol) was added, and the mixture was stirred for reaction at room temperature for 2 h; the reaction mixture was added dropwise into water and stirred for 0.5 h; the mixture was filtered and dried to obtain Compound 201-1 with a yield of 58%. MS (ESI, positive ion) m/z: 441.3 $[M + H]^+$.

Preparation of Compound 201:

Compound 201-1 (88 mg, 0.2 mmol) was dissolved in pyridine (1 mL), 1-methylpyrazole-4-carboxylic acid (38 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 201 with a yield of 53%. MS (ESI, positive ion) m/z: 549.2 $[M + H]^+$.

$^1$H NMR (400 MHz, DMSO-$d_6$) δ 10.92 (s, 1H), 8.94 (dd, *J* = 7.1, 0.8 Hz, 1H), 8.66 (d, *J* = 1.7 Hz, 1H), 8.43 (s, 1H), 8.34 (d, *J* = 8.4 Hz, 1H), 8.18 - 8.11 (m, 2H), 7.95 (dd, *J* = 2.0, 0.9 Hz, 1H), 7.78 (dd, *J* = 8.7, 1.9 Hz, 1H), 7.69 (d, *J* = 8.6 Hz, 1H), 7.53 (dd, *J* = 7.2, 2.0 Hz, 1H), 7.31 (dd, *J* = 8.3, 5.4 Hz, 1H), 7.16 - 7.10 (m, 1H), 7.07 - 6.99 (m, 1H), 5.58 (q, *J* = 8.2 Hz, 1H), 3.92 (s, 3H), 3.90 (s, 3H), 3.04 (t, *J* = 11.8 Hz, 1H), 2.89 (dt, *J* = 16.5, 8.4 Hz, 1H), 2.58 - 2.54 (m, 1H), 2.12 - 1.98 (m, 1H).

**Example 202: Preparation of Compound 202**

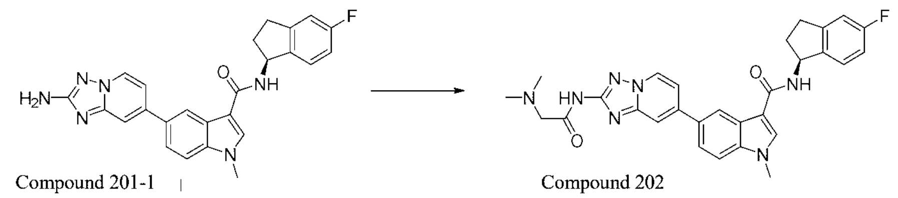

Compound 201-1 Compound 202

Preparation of Compound 202:

Compound 201-1 (88 mg, 0.2 mmol) was dissolved in pyridine (1 mL), 2-(dimethylamino)acetic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 202 with a yield of 58%. MS (ESI, positive ion) m/z: 526.2 $[M + H]^+$.

$^1$H NMR (400 MHz, DMSO-$d_6$) δ 10.45 (s, 1H), 8.91 (d, *J* = 7.3 Hz, 1H), 8.64 (d, *J* = 1.9 Hz, 1H), 8.33 (d, *J* = 8.4 Hz, 1H), 8.16 (s, 1H), 7.93 (d, *J* = 2.0 Hz, 1H), 7.76 (dd, *J* = 8.6, 1.9 Hz, 1H), 7.68 (d, *J* = 8.6 Hz, 1H), 7.51 (dd, *J* = 7.1, 2.0 Hz, 1H), 7.31 (dd, *J* = 8.4, 5.4 Hz, 1H), 7.13 (d, *J* = 9.9 Hz, 1H), 7.03 (t, *J* = 8.9 Hz, 1H), 5.58 (q, *J* = 7.9 Hz, 1H), 3.89 (s, 4H), 3.21 (s, 2H), 3.09 - 2.99 (m, 1H), 2.89 (dt, *J* = 16.4, 8.4 Hz, 1H), 2.33 (s, 5H), 2.04 (dq, *J* = 12.5, 8.7 Hz, 1H).

**Example 203: Preparation of Compound 203**

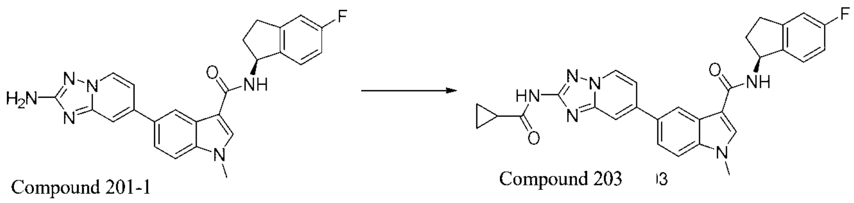

Compound 201-1

Compound 203

Preparation of Compound 203:

Compound 201-1 (88 mg, 0.2 mmol) was dissolved in pyridine (1 mL), cyclopropanecarboxylic acid (28 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 203 with a yield of 68%. MS (ESI, positive ion) m/z: 509.2 $[M + H]^+$.

$^1$H NMR (400 MHz, DMSO-$d_6$) δ 11.09 (s, 1H), 8.89 (d, *J* = 7.0 Hz, 1H), 8.65 (d, *J* = 1.8 Hz, 1H), 8.34 (d, *J* = 8.3 Hz, 1H), 8.16 (s, 1H), 7.93 (d, *J* = 2.0 Hz, 1H), 7.77 (dd, *J* = 8.7, 2.0 Hz, 1H), 7.69 (d, *J* = 8.7 Hz, 1H), 7.50 (dd, *J* = 7.1, 2.0 Hz, 1H), 7.32 (dd, *J* = 8.4, 5.3 Hz, 1H), 7.14 (d, *J* = 10.0 Hz, 1H), 7.08 - 6.99 (m, 1H), 5.58 (q, *J* = 8.0 Hz, 1H), 3.90 (s, 3H), 3.05 (dd, *J* = 17.5, 8.1 Hz, 1H), 2.90 (dt, *J* = 16.5, 8.5 Hz, 1H), 2.60 - 2.55 (m, 1H), 2.23 - 1.93 (m, 2H), 0.87 (d, *J* = 6.1 Hz, 4H).

**Example 204: Preparation of Compound 204**

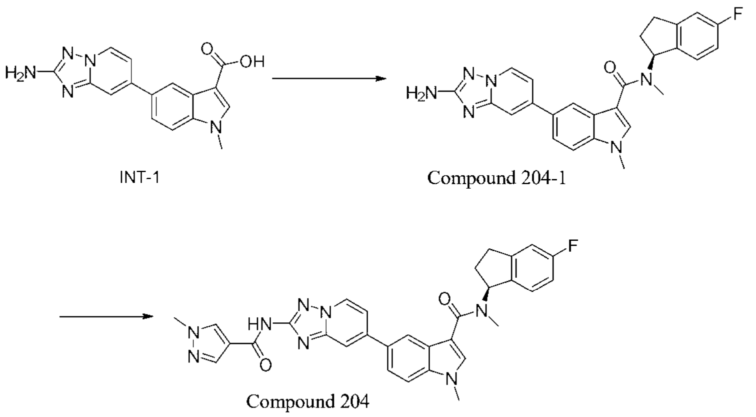

INT-1

Compound 204-1

Compound 204

Preparation of Compound 204-1:

Intermediate **INT-1** (306 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-5-fluoro-2,3-dihydro-1H-inden-1-amine-1-methyl (197 mg, 1.2 mmol) was added, and the mixture was stirred for reaction at room temperature for 2 h; the reaction mixture was added dropwise into water and stirred for 0.5 h; the mixture was filtered and dried to obtain Compound 204-1 with a yield of 49%. MS (ESI, positive ion) m/z: 454.3 $[M + H]^+$.

Preparation of Compound 204:

Compound 204-1 (91 mg, 0.2 mmol) was dissolved in pyridine (1 mL), 1-methylpyrazole-4-carboxylic acid (38 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 204 with a yield of 51%. MS (ESI, positive ion) m/z: 563.2 $[M + H]^+$.

$^1$H NMR (400 MHz, DMSO-$d_6$) δ 10.92 (s, 1H), 8.93 (d, *J* = 7.1 Hz, 1H), 8.66 (d, *J* = 1.8 Hz, 1H), 8.44 (s, 1H), 8.39

(d, $J$ = 8.3 Hz, 1H), 8.18 (s, 1H), 8.14 (s, 1H), 7.95 (d, $J$ = 1.9 Hz, 1H), 7.77 (dd, $J$ = 8.6, 1.9 Hz, 1H), 7.69 (d, $J$ = 8.6 Hz, 1H), 7.53 (dd, $J$ = 7.2, 2.0 Hz, 1H), 7.31 (dd, $J$ = 8.8, 5.2 Hz, 1H), 7.06 (t, $J$ = 7.8 Hz, 2H), 5.62 (t, $J$ = 8.1 Hz, 1H), 3.91 (d, $J$ = 7.9 Hz, 6H), 3.05 - 2.94 (m, 1H), 3.26 (s, 3H), 2.85 (dt, $J$ = 16.4, 8.7 Hz, 1H), 2.57 (d, $J$ = 8.8 Hz, 1H), 2.05 (dq, $J$ = 12.3, 8.8 Hz, 1H).

**Example 205: Preparation of Compound 205**

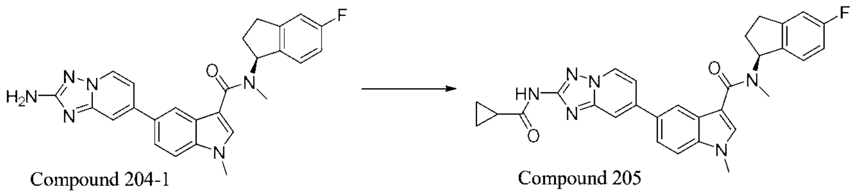

Compound 204-1

Compound 205

Preparation of Compound 205:

Compound 204-1 (91 mg, 0.2 mmol) was dissolved in pyridine (1 mL), cyclopropanecarboxylic acid (28 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 205 with a yield of 67%. MS (ESI, positive ion) m/z: 523.2 $[M + H]^+$.

$^1$H NMR (400 MHz, DMSO-$d_6$) δ 11.09 (s, 1H), 8.89 (d, $J$ = 7.0 Hz, 1H), 8.65 (d, $J$ = 1.8 Hz, 1H), 8.34 (d, $J$ = 8.3 Hz, 1H), 8.16 (s, 1H), 7.93 (d, $J$ = 2.0 Hz, 1H), 7.77 (dd, $J$ = 8.7, 2.0 Hz, 1H), 7.50 (dd, $J$ = 7.1, 2.0 Hz, 1H), 7.32 (dd, $J$ = 8.4, 5.3 Hz, 1H), 7.14 (d, $J$ = 10.0 Hz, 1H), 7.08 - 6.99 (m, 1H), 5.58 (q, $J$ = 8.0 Hz, 1H), 3.90 (s, 3H), 3.26 (s, 3H), 3.05 (dd, $J$ = 17.5, 8.1 Hz, 1H), 2.90 (dt, $J$ = 16.5, 8.5 Hz, 1H), 2.60 - 2.55 (m, 1H), 2.23 - 1.93 (m, 2H), 0.87 (d, $J$ = 6.1 Hz, 4H).

**Example 206: Preparation of Compound 206**

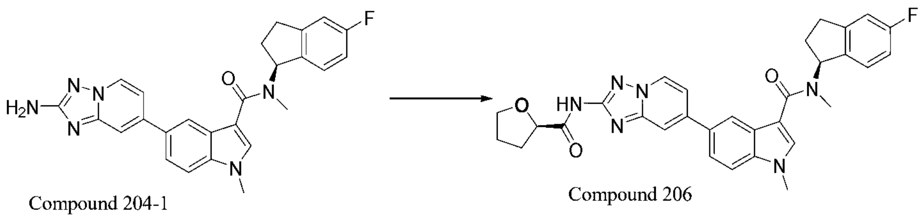

Compound 204-1

Compound 206

Preparation of Compound 206:

Compound 204-1 (91 mg, 0.2 mmol) was dissolved in pyridine (1 mL), (R)-tetrahydrofuran-2-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 206 with a yield of 67%. MS (ESI, positive ion) m/z: 553.2 $[M + H]^+$.

$^1$H NMR (400 MHz, DMSO-$d_6$) δ 11.09 (s, 1H), 8.89 (d, $J$ = 7.0 Hz, 1H), 8.65 (d, $J$ = 1.8 Hz, 1H), 8.34 (d, $J$ = 8.3 Hz, 1H), 8.16 (s, 1H), 7.93 (d, $J$ = 2.0 Hz, 1H), 7.77 (dd, $J$ = 8.7, 2.0 Hz, 1H), 7.50 (dd, $J$ = 7.1, 2.0 Hz, 1H), 7.32 (dd, $J$ = 8.4, 5.3 Hz, 1H), 7.14 (d, $J$ = 10.0 Hz, 1H), 7.08 - 6.99 (m, 1H), 5.58 (q, $J$ = 7.9 Hz, 1H), 4.54 (s, 1H), 4.02 (q, $J$ = 7.0 Hz, 1H), 3.89 (s, 3H), 3.31 (s, 3H), 3.85 (q, $J$ = 7.2 Hz, 1H), 3.09 - 2.99 (m, 1H), 2.89 (dt, $J$ = 16.3, 8.5 Hz, 1H), 2.59 - 2.54 (m, 1H), 2.31 - 2.16 (m, 1H), 2.11 - 1.83 (m, 4H).

**Example 207: Preparation of Compound 207**

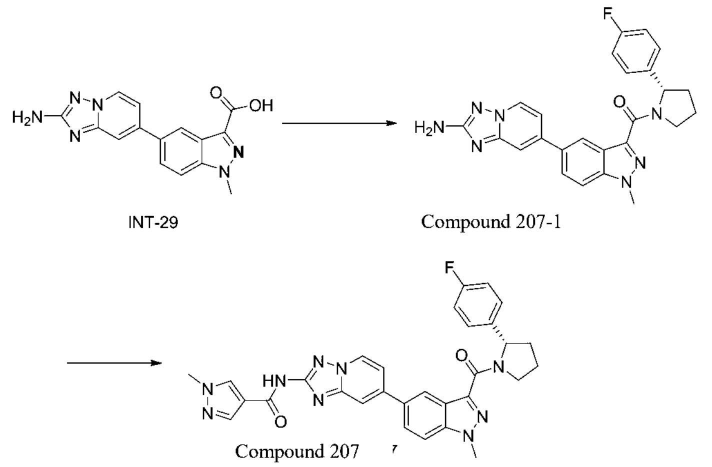

INT-29

Compound 207-1

Compound 207

Preparation of Compound 207-1:

Intermediate **INT-29** (307 mg, 1.0 mmol) was dissolved in dimethyl sulfoxide (10 mL); N,N-diisopropylethylamine (258 mg, 2 mmol) and HATU (570 mg, 1.5 mmol) were added, and the mixture was stirred at room temperature for 1 h. (S)-2-(4-fluorophenyl)pyrrolidine (198 mg, 1.2 mmol) was added, and the mixture was stirred for reaction at room temperature for 2 h; the reaction mixture was added dropwise into water and stirred for 0.5 h; the mixture was filtered and dried to obtain Compound 207-1 with a yield of 49%. MS (ESI, positive ion) m/z: 456.3 $[M + H]^+$.

Preparation of Compound 207:

Compound 207-1 (91 mg, 0.2 mmol) was dissolved in pyridine (1 mL), 1-methylpyrazole-4-carboxylic acid (38 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 207 with a yield of 51%. MS (ESI, positive ion) m/z: 564.2 $[M + H]^+$.

1H NMR (400 MHz, DMSO-d6) δ 11.07 (s, 1H), 8.82 (m, 2H), 8.48 (d, J = 1.8 Hz, 1H), 7.85 (m, 2H), 7.73 (d, J = 8.6 Hz, 1H),7.59 - 7.49 (m, 1H), 7.42 (dd, J = 7.1, 2.0 Hz, 1H), 7.32 (dd, J = 8.5, 5.5 Hz, 2H), 7.13 (t, J = 8.7 Hz, 2H), 5.05 (m, 1H), 3.95 (s, 3H), 3.71 (s, 3H), 1.89 (d, *J* = 6.8 Hz, 3H).

**Example 208: Preparation of Compound 208**

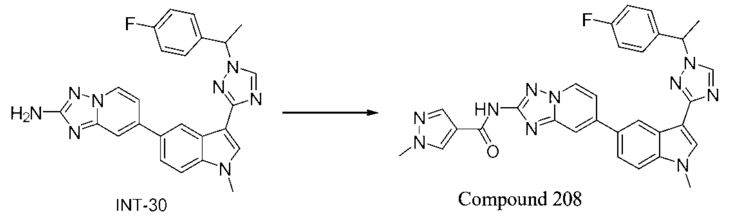

INT-30

Compound 208

Preparation of Compound 208:

Compound **INT-30** (90 mg, 0.2 mmol) was dissolved in pyridine (1 mL), 1-methylpyrazole-4-carboxylic acid (38 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 208 with a yield of 53%. MS (ESI, positive ion) m/z: 561.2 $[M + H]^+$.

$^1H$ NMR (400 MHz, DMSO-$d_6$) δ 11.08 (s, 1H), 8.86 (d, *J* = 7.0 Hz, 1H), 8.50 (m, 2H), 8.19 (s, 1H), 7.95 (s, 1H), 7.89 (d, *J* = 1.9 Hz, 1H), 7.82 (m, 2H), 7.73 (d, *J* = 8.6 Hz, 1H), 7.42 (m, 2H), 7.36 (dd, *J* = 8.7, 5.5 Hz, 2H), 7.18 (t, *J* = 8.9 Hz, 2H), 5.05 (m, 1H), 3.95 (s, 3H), 3.71 (s, 3H), 1.89 (d, *J* = 6.8 Hz, 3H).

**Example 209: Preparation of Compound 209**

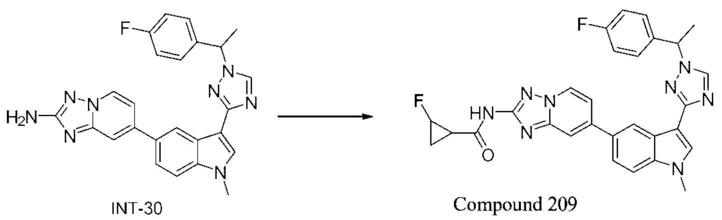


Preparation of Compound 209:

Compound **INT-30** (90 mg, 0.2 mmol) was dissolved in pyridine (1 mL), 2-fluorocyclopropanecarboxylic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 209 with a yield of 56%. MS (ESI, positive ion) m/z: 539.2 $[M + H]^+$.

$^1$H NMR (400 MHz, DMSO-$d_6$) δ 11.08 (s, 1H), 8.86 (d, *J* = 7.0 Hz, 1H), 8.50 (d, *J* = 1.8 Hz, 1H), 8.19 (s, 1H), 7.95 (s, 1H), 7.89 (d, *J* = 1.9 Hz, 1H), 7.82 (m, 2H), 7.73 (d, *J* = 8.6 Hz, 1H), 7.42 (dd, *J* = 7.2, 2.0 Hz, 1H), 7.36 (dd, *J* = 8.7, 5.5 Hz, 2H), 7.18 (t, *J* = 8.9 Hz, 2H), 6.10 (d, *J* = 6.8 Hz, 1H), 4.71 - 4.59 (m, 1H), 3.95 (s, 3H), 1.89 (d, *J* = 6.8 Hz, 3H), 0.92-0.78(m, 3H).

**Example 210: Preparation of Compound 210**

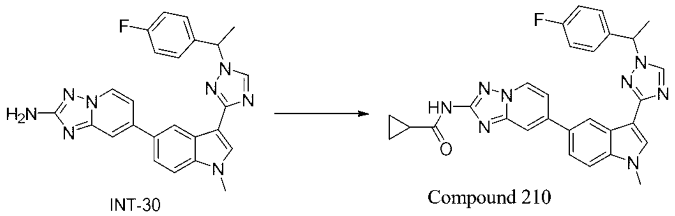


Preparation of Compound 210:

Compound **INT-30** (90 mg, 0.2 mmol) was dissolved in pyridine (1 mL), cyclopropanecarboxylic acid (28 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 210 with a yield of 69%. MS (ESI, positive ion) m/z: 521.2 $[M + H]^+$.

$^1$H NMR (400 MHz, DMSO-$d_6$) δ 11.08 (s, 1H), 8.86 (d, *J* = 7.0 Hz, 1H), 8.50 (d, *J* = 1.8 Hz, 1H), 8.19 (s, 1H), 7.95 (s, 1H), 7.89 (d, *J* = 1.9 Hz, 1H), 7.82 (m, 2H), 7.73 (d, *J* = 8.6 Hz, 1H), 7.42 (dd, *J* = 7.2, 2.0 Hz, 1H), 7.36 (dd, *J* = 8.7, 5.5 Hz, 2H), 7.18 (t, *J* = 8.9 Hz, 2H), 6.10 (d, *J* = 6.8 Hz, 1H), 3.95 (s, 3H), 1.89 (d, *J* = 6.8 Hz, 3H), 0.92-0.78(m, 5H).

**Example 211: Preparation of Compound 211**

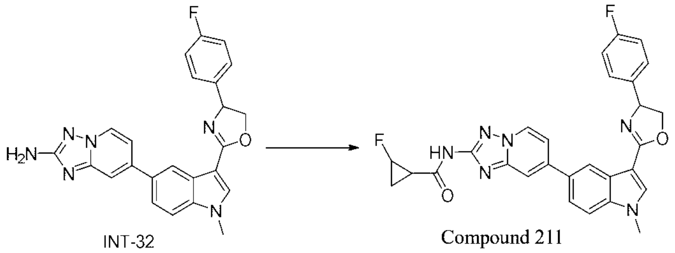

INT-32 Compound 211

Preparation of Compound 211:

Compound **INT-32** (85 mg, 0.2 mmol) was dissolved in pyridine (1 mL), 2-fluorocyclopropanecarboxylic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 211 with a yield of 64%. MS (ESI, positive ion) m/z: 513.2 $[M + H]^+$.

$^1H$ NMR (400 MHz, DMSO-$d_6$) δ 10.53 (s, 1H), 8.76 (m, 1H), 8.55 (d, *J* = 1.8 Hz, 1H), 8.27 (s, 1H), 7.91 (dd, *J* = 1.9, 0.9 Hz, 1H), 7.76 (s, 1H), 7.69 (d, *J* = 8.6 Hz, 1H), 7.60 - 7.53 (m, 1H), 7.48 (m, 2H), 7.28 - 7.18 (m, 2H), 5.54 - 5.49 (m, 1H), 4.71 - 4.59 (m, 3H), 3.72 (s, 3H), 2.60 - 2.55 (m, 1H), 1.42 (m, 1H), 1.21-1.06 (m, 2H).

**Example 212: Preparation of Compound 212**

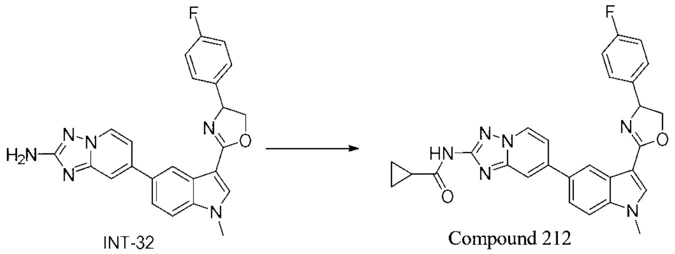

INT-32 Compound 212

Preparation of Compound 212:

Compound **INT-32** (85 mg, 0.2 mmol) was dissolved in pyridine (1 mL), cyclopropanecarboxylic acid (28 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 212 with a yield of 68%. MS (ESI, positive ion) m/z: 495.2 $[M + H]^+$.

$^1H$ NMR (400 MHz, DMSO-$d_6$) δ 10.53 (s, 1H), 8.76 (m, 1H), 8.55 (d, *J* = 1.8 Hz, 1H), 8.27 (s, 1H), 7.91 (dd, *J* = 1.9, 0.9 Hz, 1H), 7.76 (s, 1H), 7.69 (d, *J* = 8.6 Hz, 1H), 7.60 - 7.53 (m, 1H), 7.48 (m, 2H), 7.28 - 7.18 (m, 2H), 5.54 - 5.49 (m, 1H), 4.71 - 4.59 (m, 2H), 3.72 (s, 3H), 2.60 - 2.55 (m, 1H), 1.42 (m, 1H), 0.72-0.56 (m, 4H).

**Example 213: Preparation of Compound 213**

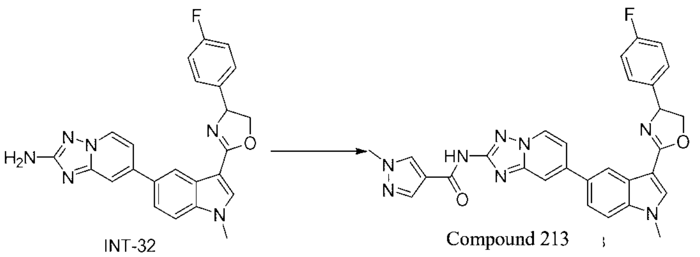

INT-32 Compound 213

Preparation of Compound 213:

Compound **INT-32** (85 mg, 0.2 mmol) was dissolved in pyridine (1 mL), 1-methylpyrazole-4-carboxylic acid (38 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 213 with a yield of 58%. MS (ESI, positive ion) m/z: 535.2 $[M + H]^+$.

$^1H$ NMR (400 MHz, DMSO-$d_6$) δ 10.53 (s, 1H), 8.76 (m, 2H), 8.55 (d, *J* = 1.8 Hz, 1H), 8.27 (s, 1H), 7.91 (dd, *J* = 1.9, 0.9 Hz, 1H), 7.76 (s, 1H), 7.69 (d, *J* = 8.6 Hz, 1H), 7.60 - 7.53 (m, 2H), 7.48 (m, 2H), 7.28 - 7.18 (m, 2H), 5.54 - 5.49 (m, 1H), 4.71 - 4.59 (m, 2H), 3.92 (s, 3H), 3.71 (s, 3H).

**Example 214: Preparation of Compound 214**

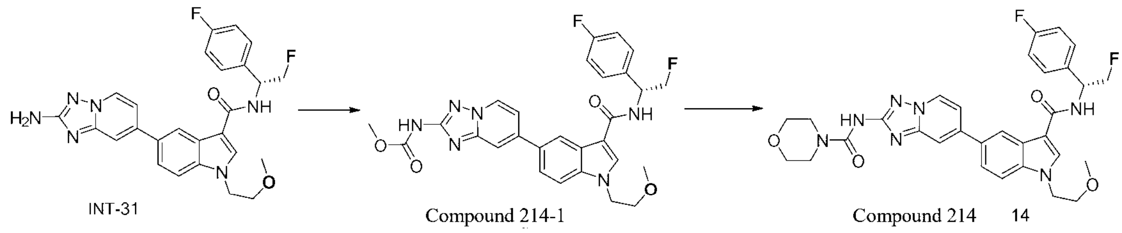

INT-31 Compound 214-1 Compound 214 14

Preparation of Compound 214-1:

Intermediate **INT-31** (490 mg, 1 mmol) was dissolved in dichloromethane (0.3 L); methyl chloroformate (112 mg, 1.2 mmol) and N,N-diisopropylethylamine (258 mg, 2.0 mmol) were added, and the mixture was stirred at room temperature for 2 h. Petroleum ether (0.3 L) was added to the reaction mixture for crystallization; the solid was filtered with suction, washed with water, and dried to obtain Compound 214-1 with a yield of 65%. MS (ESI, positive ion) m/z: 549.2 $[M + H]^+$.

Preparation of Compound 214:

Compound 214-1 (110 mg, 0.2 mmol) was dissolved in acetonitrile (1 mL); morpholine (36 mg, 0.4 mmol) was added, and the mixture was heated to 80°C and stirred for 6 h. The reaction mixture was concentrated under reduced pressure and purified by C-18 reverse phase silica gel column (acetonitrile/0.1% aqueous ammonium bicarbonate solution = 60%) to obtain Compound 214 with a yield of 58%. MS (ESI, positive ion) m/z: 604.2 $[M + H]^+$.

$^1H$ NMR (400 MHz, DMSO-$d_6$) δ 10.53 (s, 1H), 8.90 (dd, *J* = 7.1, 0.8 Hz, 1H), 8.62 (d, *J* = 8.3 Hz, 1H), 8.55 (d, *J* = 1.8 Hz, 1H), 8.27 (s, 1H), 7.91 (dd, *J* = 1.9, 0.9 Hz, 1H), 7.76 (dd, *J* = 8.7, 1.8 Hz, 1H), 7.69 (d, *J* = 8.6 Hz, 1H), 7.60 - 7.53 (m, 2H), 7.48 (dd, *J* = 7.2, 2.0 Hz, 1H), 7.28 - 7.18 (m, 2H), 5.49 (dq, *J* = 15.3, 7.3 Hz, 1H), 4.70 - 4.60 (m, 3H), 3.64 (m, 6H), 3.43 (s, 3H), 3.27 (s, 2H), 2.58 - 2.55 (m, 4H).

**Example 215: Preparation of Compound 215**

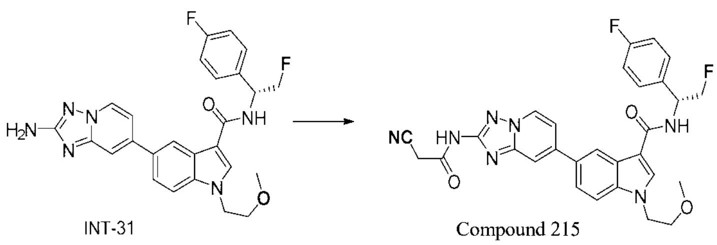

INT-31 Compound 215

Preparation of Compound 215:

Compound **INT-31** (85 mg, 0.2 mmol) was dissolved in pyridine (1 mL), cyanoacetic acid (25 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 215 with a yield of 58%. MS (ESI, positive ion) m/z: 558.2 $[M + H]^+$.

$^1H$ NMR (400 MHz, DMSO-$d_6$) δ 10.53 (s, 1H), 8.90 (dd, *J* = 7.1, 0.8 Hz, 1H), 8.62 (d, *J* = 8.3 Hz, 1H), 8.55 (d, *J* = 1.8 Hz, 1H), 8.27 (s, 1H), 7.91 (dd, *J* = 1.9, 0.9 Hz, 1H), 7.76 (dd, *J* = 8.7, 1.8 Hz, 1H), 7.69 (d, *J* = 8.6 Hz, 1H), 7.60 - 7.53 (m, 2H), 7.48 (dd, *J* = 7.2, 2.0 Hz, 1H), 7.28 - 7.18 (m, 2H), 5.49 (dq, *J* = 15.3, 7.3 Hz, 1H), 4.70 - 4.60 (m, 3H), 3.71 - 3.60 (m, 2H),3.34 (s, 2H), 3.23 (s, 3H), 3.27 (s, 2H).

**Example 216: Preparation of Compound 216**

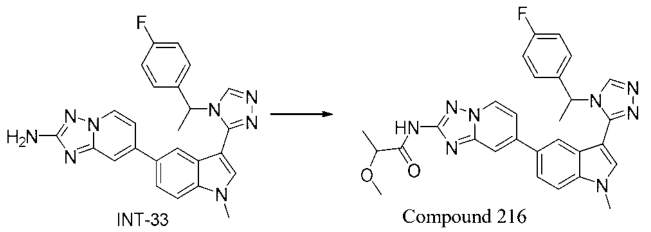

INT-33 Compound 216

Preparation of Compound 216:

**INT-33** (90 mg, 0.2 mmol) was dissolved in pyridine (1 mL), 2-methoxypropanoic acid (31 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 216 with a yield of 66%. MS (ESI, positive ion) m/z: 539.2 $[M + H]^+$.

$^1H$ NMR (400 MHz, DMSO-$d_6$) δ 10.50 (s, 1H), 8.95 - 8.85 (m, 2H), 8.66 (s, 1H), 8.56 - 8.50 (m, 1H), 8.03 - 7.96 (m, 2H), 7.92 (d, *J* = 9.0 Hz, 1H), 7.52 (m, 3H), 7.22 - 7.12 (m, 2H), 5.26 (p, *J* = 7.2 Hz, 1H), 4.18 (m, 1H), 3.39 (s, 3H), 1.55 (d, *J* = 7.1 Hz, 3H), 1.35 (d, *J* = 6.6 Hz, 3H).

**Example 217: Preparation of Compound 217**

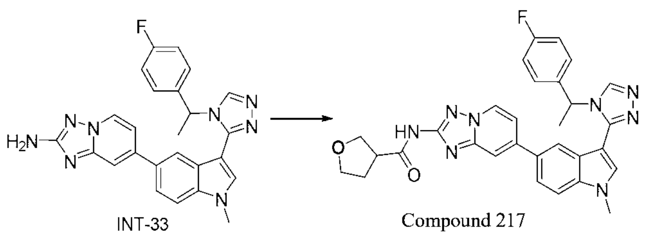

INT-33 Compound 217

Preparation of Compound 217:

**INT-33** (90 mg, 0.2 mmol) was dissolved in pyridine (1 mL), tetrahydrofuran-3-carboxylic acid (35 mg, 0.3 mmol) and 1-propylphosphonic anhydride (111 mg, 0.351 mmol) were added, and the mixture was heated to 80°C and stirred for 2 h. The reaction mixture was concentrated under reduced pressure and purified by C-18 reverse phase silica gel column (acetonitrile/0.1% aqueous ammonium bicarbonate solution = 60%) to obtain Compound 217 with a yield of 76%. MS (ESI, positive ion) m/z: 551.2 $[M + H]^+$.

$^1H$ NMR (400 MHz, DMSO-$d_6$) δ 10.50 (s, 1H), 8.95 - 8.85 (m, 2H), 8.66 (s, 1H), 8.56 - 8.50 (m, 1H), 8.03 - 7.96 (m, 2H), 7.92 (d, *J* = 9.0 Hz, 1H), 7.52 (m, 3H), 7.22 - 7.12 (m, 2H), 5.26 (p, *J* = 7.2 Hz, 1H), 4.53 (s, 1H), 4.22 (s, 3H), 4.01 (q, *J* =

6.9 Hz, 1H), 3.84 (td, *J* = 7.6, 6.5 Hz, 1H), 2.30 - 1.92 (m, 2H), 1.97 - 1.82 (m, 2H), 1.55 (d, *J* = 7.1 Hz, 3H).

**Example 218: Preparation of Compound 218**

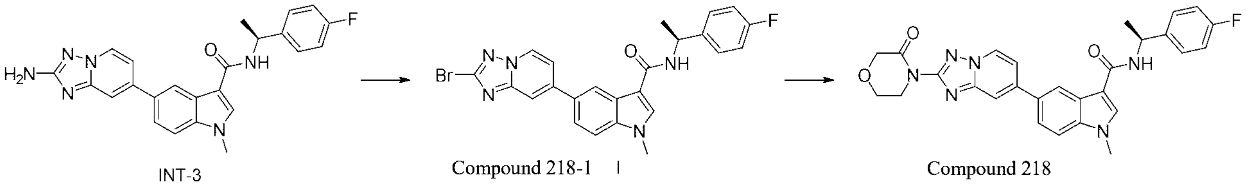

INT-3 Compound 218-1 Compound 218

Preparation of Compound 218-1:

Under an argon atmosphere, Intermediate **INT-3** (2 g, 4.7 mmol) and copper bromide (3 g, 14.1 mmol) were dissolved in acetonitrile (40 mL), and the mixture was heated to reflux. Tert-butyl nitrite (0.63 g, 6.1 mmol) was added dropwise. After the addition was completed, the reaction mixture was stirred for reaction for 1 h. The reaction mixture was concentrated under reduced pressure and purified by silica gel column (dichloromethane/methanol = 40/1) to obtain Compound 218-1 with a yield of 35%. MS (ESI, positive ion) m/z: 492.2 $[M + H]^+$.

Preparation of Compound 218:

Under an argon atmosphere, 3-morpholinone (155 mg, 1.5 mmol) was dissolved in ethylene glycol dimethyl ether (10 mL), sodium hydride (60 mg, 1.5 mmol, 60%) was added at 0°C-5°C, and the mixture was stirred at this temperature for 0.5 h. Intermediate **INT-2** (246 mg, 0.5 mmol) and $PdCl_2(dppf)_2$ (25 mg) were added, and the mixture was heated to reflux and stirred for reaction for 2 h. The reaction mixture was concentrated under reduced pressure and purified by silica gel column (dichloromethane/methanol = 20/1) to obtain Compound 218 with a yield of 21%. MS (ESI, positive ion) m/z: 513.2 $[M + H]^+$.

$^1H$ NMR (400 MHz, DMSO-$d_6$) δ 8.96 (d, *J* = 7.1 Hz, 1H), 8.56 (d, *J* = 1.8 Hz, 1H), 8.37 (d, *J* = 8.0 Hz, 1H), 8.23 (s, 1H), 8.01 (d, *J* = 1.9 Hz, 1H), 7.75 (dd, *J* = 8.7, 1.8 Hz, 1H), 7.68 (d, *J* = 8.6 Hz, 1H), 7.55 (dd, *J* = 7.2, 1.9 Hz, 1H), 7.52 - 7.43 (m, 2H), 7.22 - 7.12 (m, 2H), 5.23 (p, *J* = 7.2 Hz, 1H), 4.32 (s, 2H), 4.09 - 3.95 (m, 4H), 3.92 (s, 3H), 1.51 (d, *J* = 7.1 Hz, 3H).

**Example 219: Preparation of Compound 219**

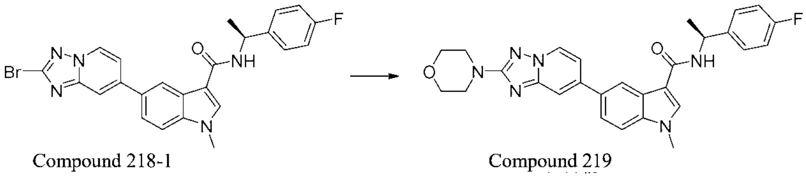

Compound 218-1 Compound 219

Preparation of Compound 219:

Under an argon atmosphere, morpholine (87 mg, 1 mmol), Compound 218-1 (246 mg, 0.5 mmol), $Pd_2(dba)_3$ (20 mg), and 4,5-bis(diphenylphosphino)-9,9-dimethylxanthene (20 mg) were dissolved in 1,4-dioxane (3 mL), cesium carbonate (325 mg, 1 mmol, 60%) was added, and the mixture was heated to reflux and stirred for reaction overnight. The reaction mixture was concentrated under reduced pressure and purified by silica gel column (dichloromethane/-methanol = 30/1) to obtain Compound 219 with a yield of 24%. MS (ESI, positive ion) m/z: 499.2 $[M + H]^+$.

$^1H$ NMR (400 MHz, DMSO-$d_6$) δ 8.69 (d, *J* = 7.0 Hz, 1H), 8.52 - 8.48 (m, 1H), 8.34 (d, *J* = 7.9 Hz, 1H), 8.21 (d, *J* = 6.7 Hz, 1H), 7.73 - 7.62 (m, 3H), 7.49 - 7.45 (m, 2H), 7.29 (dd, *J* = 7.1, 2.0 Hz, 1H), 7.20 - 7.14 (m, 2H), 5.22 (t, *J* = 7.4 Hz, 1H), 3.91 (d, *J* = 4.6 Hz, 3H), 3.74 (t, *J* = 4.8 Hz, 4H), 3.50 (t, *J* = 4.8 Hz, 4H), 1.50 (d, *J* = 7.1 Hz, 3H).

**Test Example 1: *In Vitro* Kinase Assay and Assay for Protection Against Necroptosis for Compound in the Present Invention**

*In Vitro* RIPK1 Kinase Assay:

This assay was completed using the Kinase Profiler service provided by Eurofins. The experimental method was as follows:
The small molecule compound to be tested (0.001-10 μM) and the protein kinase to be tested were incubated with a buffer containing a substrate, 10 mM magnesium acetate, and [$\gamma$-$^{33}$P-ATP]. The reaction was initiated by adding a Mg\ATPmix. After incubation at room temperature for a period of time, a 3% phosphate solution was added to the buffer to terminate the reaction. Subsequently, 10 μL of the reaction mixture was quantitatively pipetted and dropped onto P30 filter paper. The paper was washed three times with 75 mM phosphate solution and once with methanol. After drying, scintillation fluid was added for scinticounting. The inhibitory activity of the compound was expressed as the half-maximal inhibitory concentration ($IC_{50}$). The $IC_{50}$ values were derived by fitting the inhibition rates corresponding to the respective concentration gradients.
The in vitro MLK2/3 kinase assay utilized the ADP-Glo method provided by ICE Bioscience INC. A drug screening system based on MLK2/3 kinase was employed to evaluate the inhibiting ability of small molecule compounds against kinase activity. The experimental method is as follows:
ADP-Glo Method

1. 2 × ATP/substrate solution and 2 × kinase solution were prepared using kinase reaction buffer.
2. 40 nL of the compound diluent was transferred into a 384-well plate using an Echo 655. After centrifugation, 2 μL of 2 × kinase solution was added to the 384-well plate, centrifuged at 1000 rpm for 1 min, and incubated at 25°C for 10 min;
3.2 μL of 2 × substrate and ATP solution was added into the 384-well plate, centrifuged at 1000 rpm for 1 min, and incubated at 25°C for 60 min.
4. 4 μL of ADP-Glo was transferred to a 384-well plate, centrifuged at 1000 rpm for 1 min, and incubated at 25°C for 40 min.
5. 8 μL of test solution was transferred to a 384-well plate, centrifuged at 1000 rpm for 1 min, and incubated at 25°C for 40 min.
6. The luminescence signal was read using a multifunctional microplate reader.
7. Data Analysis

The negative control reading was set at 0% inhibition rate and the positive control reading was set at 100% inhibition rate to calculate the inhibition rate of each test solution.

$$\%\text{Compound Inhibition Rate}=\left[1-\frac{\text{Data}_{\text{Compound}}-\overline{\text{Data}}_{\text{Positive Control}}}{\overline{\text{Data}}_{\text{Negative Control}}-\overline{\text{Data}}_{\text{Positive Control}}}\right]*100\%$$

$\text{Data}_{\text{Positive Control}}$: Mean value of positive control wells
$\text{Data}_{\text{Negative Control}}$: Mean value of negative control wells

The $IC_{50}$ (half inhibitory concentration) of a compound was obtained using the following non-linear fitting formula:

$$Y=\text{Bottom} + (\text{Top-Bottom})/(1+10^{\wedge}((\text{LogIC}_{50}\text{-X})*\text{HillSlope}))$$

X: Compound concentration log value
Y: Compound inhibition rate (% inh)

Equation for Z' factor calculation:

$$Z'=1-3(\text{SDmin}+\text{SDmax})/(\text{AVEmax}-\text{AVEmin})$$

wherein:

Min is the positive control Data, and Max is the negative control DMSO Data.
SD is the standard deviation, and AVE is the average.

*In Vitro* Assay for Protection of HT-29 Cells from Necroptosis:

1) Experimental Materials

DMEM medium was purchased from Gibco; penicillin-streptomycin was purchased from Hyclone; TNFα was purchased from Peprotech; Smac mimetic and Z-VAD-FMK were purchased from Selleck; and CCK-8 was purchased from Medchem Express.

2) Experimental Methods

HT-29 cells (colon cancer cells) were cultured in DMEM + 10% FBS + penicillin/streptomycin. For the experiment, HT-29 cells in the logarithmic phase were collected and seeded into 96-well plates at a specific density (typically $8 \times 10^3$ cells/well for adherent cells). The cells were cultured overnight in an incubator at 37°C with 5% $CO_2$. On the following day, the test compound was diluted with culture medium to the corresponding concentrations and added into the 96-well plate, with 3 replicate wells for each sample. A solvent control group and a blank control group containing only culture medium were set simultaneously. The spiked cells were induced with a combination of TNFα/Smac mimetic/Z-VAD-FMK for 24 h. Subsequently, 10 μL of CCK8 solution was added to each well, and the cells were incubated in an incubator for 1-3 h. Absorbance was measured at 495 nm using a microplate reader, and the cell necrosis protection rate of the drug was calculated using the following formula:

$$\text{Cell necrosis protection rate} = [(X - C_0)/(C - C_0)] \times 100\%$$

where C, $C_0$, and X represent the average absorbance values of the solvent control group, the blank control group, and the drug treatment group, respectively. Finally, a cell viability curve was fitted using Graphpad Prism 5.0 software, and the $EC_{50}$ value of the test compound for inhibiting necroptosis was calculated.

The results are shown in Table 1, wherein "+++++" represents $IC_{50}$ or $EC_{50} < 0.001$ μM; "++++" represents 0.01 μM > $IC_{50}$ or $EC_{50} \geq 0.001$ μM; "+++" represents 0.1 μM > $IC_{50}$ or $EC_{50} \geq 0.01$ μM; "++" represents 1 μM > $IC_{50}$ or $EC_{50} \geq$ 0.1 μM; and "+" represents 10 μM > $IC_{50}$ or $EC_{50} \geq 1$ μM.

Table 1 Inhibitory Activity of Compound in the Present Invention Against RIPK1 Kinase

| **Compound No.** | **RIPK1($IC_{50}$)** | **Compound No.** | **RIPK1($IC_{50}$)** |
|---|---|---|---|
| 3 | +++ | 71 | +++ |
| 6 | +++ | 73 | +++ |
| 8 | +++ | 74 | +++ |
| 9 | +++ | 75 | ++++ |
| 14 | +++ | 106 | +++ |
| 18 | +++ | 121 | +++ |
| 27 | +++ | 122 | ++ |
| 29 | +++ | 124 | +++ |
| 32 | +++ | 127 | ++ |
| 38 | +++ | 130 | +++ |
| 39 | +++ | 138 | +++ |
| 40 | +++ | 156 | +++ |
| 42 | +++ | 161 | ++++ |
| 46 | +++ | 175 | +++ |
| 51 | +++ | 182 | ++ |
| 59 | +++ | 196 | +++ |
| 60 | +++ | 210 | + |
| 68 | +++ | | |

Table 2 Inhibitory Activity of Compound in the Present Invention Against MLK2/3 Kinase

| Compound No. | MLK2/3 ($IC_{50}$) |
|---|---|
| 73 | +++ |
| 74 | +++ |
| 75 | +++ |

Table 3 Protective Activity of Compound in the Present Invention Against Necroptosis

| Compound No. | HT-29 ($EC_{50}$) | Compound No. | HT-29 ($EC_{50}$) |
|---|---|---|---|
| 1 | +++ | 111 | ++++ |
| 2 | - | 112 | +++++ |
| 3 | +++ | 113 | ++++ |
| 4 | +++ | 114 | +++++ |
| 5 | +++++ | 115 | +++++ |
| 6 | +++++ | 116 | +++++ |
| 7 | +++++ | 117 | +++++ |
| 8 | +++++ | 118 | +++++ |
| 9 | +++++ | 119 | +++++ |
| 10 | ++++ | 120 | +++++ |
| 11 | ++++ | 121 | +++++ |
| 12 | +++++ | 122 | ++ |
| 13 | +++++ | 123 | ++++ |
| 14 | +++++ | 124 | ++++ |
| 15 | +++++ | 125 | + |
| 16 | +++ | 126 | - |
| 17 | +++++ | 127 | + |
| 18 | +++++ | 128 | - |
| 19 | +++++ | 129 | - |
| 20 | +++++ | 130 | ++++ |
| 21 | +++++ | 131 | - |
| 22 | ++++ | 132 | ++ |
| 23 | +++++ | 133 | - |
| 24 | +++++ | 134 | - |
| 25 | +++++ | 135 | - |
| 26 | +++++ | 136 | +++ |
| 27 | +++++ | 137 | +++ |
| 28 | ++++ | 138 | - |
| 29 | +++++ | 139 | ++++ |
| 30 | +++++ | 140 | - |
| 31 | +++ | 141 | ++++ |
| 32 | +++++ | 142 | +++ |
| 33 | + | 143 | ++++ |

(continued)

| Compound No. | HT-29 ($EC_{50}$) | Compound No. | HT-29 ($EC_{50}$) |
|---|---|---|---|
| 34 | +++++ | 144 | - |
| 35 | +++++ | 145 | ++++ |
| 36 | +++++ | 146 | +++ |
| 37 | ++++ | 147 | ++++ |
| 38 | +++++ | 148 | +++ |
| 39 | +++++ | 149 | +++++ |
| 40 | +++++ | 150 | +++++ |
| 41 | +++++ | 151 | +++++ |
| 42 | +++++ | 152 | - |
| 43 | +++++ | 153 | +++++ |
| 44 | ++++ | 154 | +++++ |
| 45 | +++++ | 155 | +++++ |
| 46 | +++++ | 156 | +++++ |
| 47 | +++++ | 157 | +++++ |
| 48 | +++ | 158 | + |
| 49 | ++++ | 159 | +++++ |
| 50 | +++++ | 160 | +++++ |
| 51 | +++++ | 161 | +++++ |
| 52 | +++++ | 162 | +++++ |
| 53 | +++++ | 163 | +++++ |
| 54 | - | 164 | +++++ |
| 55 | - | 165 | +++++ |
| 56 | - | 166 | +++++ |
| 57 | +++ | 167 | +++++ |
| 58 | +++++ | 168 | +++++ |
| 59 | +++++ | 169 | +++++ |
| 60 | +++++ | 170 | +++++ |
| 61 | ++++ | 171 | ++++ |
| 62 | +++++ | 172 | ++++ |
| 63 | + | 173 | +++++ |
| 64 | +++++ | 174 | +++++ |
| 65 | +++++ | 175 | +++++ |
| 66 | +++++ | 176 | +++++ |
| 67 | +++++ | 177 | +++ |
| 68 | +++++ | 178 | ++ |
| 69 | +++++ | 179 | ++ |
| 70 | +++++ | 180 | + |
| 71 | +++++ | 181 | + |
| 72 | +++++ | 182 | +++ |

(continued)

| Compound No. | HT-29 ($EC_{50}$) | Compound No. | HT-29 ($EC_{50}$) |
|---|---|---|---|
| 73 | +++++ | 183 | +++++ |
| 74 | +++++ | 184 | ++ |
| 75 | +++++ | 185 | +++++ |
| 76 | +++++ | 186 | +++++ |
| 77 | +++++ | 187 | +++++ |
| 78 | +++++ | 188 | +++++ |
| 79 | +++++ | 189 | +++++ |
| 80 | +++++ | 190 | +++++ |
| 81 | +++++ | 191 | +++++ |
| 82 | +++++ | 192 | +++++ |
| 83 | +++++ | 193 | +++++ |
| 84 | +++++ | 194 | - |
| 85 | +++++ | 195 | +++++ |
| 86 | +++++ | 196 | +++++ |
| 87 | +++++ | 197 | +++++ |
| 88 | ++++ | 198 | ++ |
| 89 | - | 199 | +++++ |
| 90 | + | 200 | +++++ |
| 91 | + | 201 | - |
| 92 | ++ | 202 | - |
| 93 | ++ | 203 | - |
| 94 | +++++ | 204 | +++ |
| 95 | +++++ | 205 | ++++ |
| 96 | +++++ | 206 | + |
| 97 | +++++ | 207 | +++++ |
| 98 | +++++ | 208 | ++ |
| 99 | +++++ | 209 | ++ |
| 100 | +++++ | 210 | ++ |
| 101 | +++++ | 211 | +++ |
| 102 | +++++ | 212 | +++ |
| 103 | +++++ | 213 | +++ |
| 104 | + | 214 | +++ |
| 105 | +++ | 215 | +++ |
| 106 | +++++ | 216 | +++ |
| 107 | +++++ | 217 | +++ |
| 108 | +++++ | 218 | +++ |
| 109 | ++++ | 219 | ++++ |
| 110 | +++++ | | |
| Note: "-" indicates not tested. | | | |

The experimental results in Tables 1 and 2 showed that the exemplary compound in the present invention has significant inhibitory activities against both RIPK1 kinase and MLK2/3 kinase, with $IC_{50}$ value for RIPK1 kinase activity inhibition being less than 1 μM, less than 0.1 μM, or even less than 0.01 μM. The $IC_{50}$ value for MLK2/3 kinase activity inhibition was less than 1 μM. Table 3 further showed that the compound in the present invention, having inhibitory activity against RIPK1 and/or MLK2/3 kinases, also exhibits significant protection of HT-29 cells from necroptosis.

The aforementioned description of specific exemplary embodiments in the present invention is presented for purposes of explanation and illustration. These descriptions are not intended to limit the present invention to the precise forms disclosed, and obviously, many modifications and variations can be made in light of the above teachings. The exemplary embodiments are chosen and described in order to explain the specific principles of the present invention and its practical application, thereby enabling those skilled persons in the art to implement and utilize various exemplary embodiments in the present invention with various alterations and modifications. The scope of the present invention is intended to be defined by the claims and their equivalents.

## Claims

**1.** Compound of formula (I), pharmaceutically acceptable salt, solvate or stereoisomer thereof:

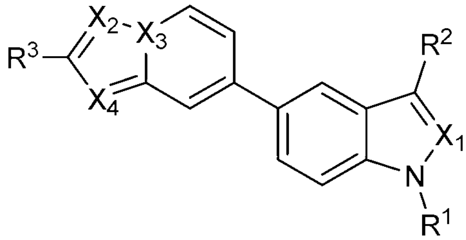

(I)

wherein,

X1 is selected from N or CH;
X2. X3 and X4 are each independently selected from N or CH, provided that at least one of X1, X2 and X3 is N;
$R^1$ is selected from $C_{1-6}$ alkyl, $C_{1-6}$ alkoxy, -$C_{1-6}$ alkyl-$C_{1-6}$ alkoxy or $C_{3-6}$ cycloalkyl, wherein the said $C_{1-6}$ alkyl is optionally substituted with halogen;
$R^2$ is selected from

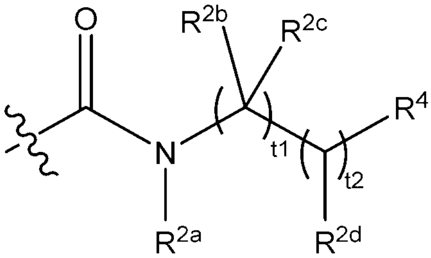

or

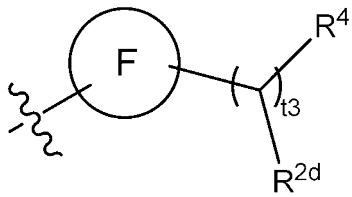

;

wherein, $R^4$ is selected from $C_{1-6}$ alkyl, $C_{5-8}$ aryl, 8- to 12-membered partially unsaturated bicyclic group and 5- or 6-membered monocyclic heteroaryl, wherein the said $C_{5-8}$ aryl, 8- to 12-membered partially unsaturated bicyclic group and 5- or 6-membered monocyclic heteroaryl are optionally substituted with one or more substituents selected from $R^{41}$;
$R^{41}$ is selected from halogen, cyano, $-NR^{4a}R^{4b}$, $C_{1-6}$ alkyl and $C_{3-6}$ cycloalkyl;
Ring F is selected from 5- or 6-membered monocyclic heteroaryl and 4- to 8-membered nitrogen-containing partially unsaturated heterocyclyl;
$R^{2a}$. $R^{2b}$, $R^{2c}$ and $R^{2d}$ are each independently selected from hydrogen, deuterium, halogen, $C_{1-6}$ alkyl and $C_{3-6}$ cycloalkyl, wherein the said $C_{1-6}$ alkyl and $C_{3-6}$ cycloalkyl are optionally substituted with one or more halogen or hydroxyl;
Alternatively, $R^{2b}$ and $R^{2c}$, together with the carbon atom to which they are attached, form $C_{3-6}$ cycloalkyl or 4- to 12-membered heterocycloalkyl;
Alternatively, $R^{2a}$ and $R^{2b}$, together with the carbon atom and nitrogen atom to which they are attached, form 4- to

12-membered nitrogen-containing heterocycloalkyl or 4- to 8-membered nitrogen-containing partially unsaturated heterocyclyl;
Alternatively, $R^{2b}$ and $R^{2d}$, together with the carbon atom to which they are attached, form $C_{3-6}$ cycloalkyl or 4- to 12-membered heterocycloalkyl;
The said $C_{3-6}$ cycloalkyl, 4- to 12-membered heterocycloalkyl, 4- to 12-membered nitrogen-containing heterocycloalkyl or 4- to 8-membered nitrogen-containing partially unsaturated heterocyclyl is optionally substituted with one or more substituents selected from halogen;
t1. t2 and t3 are each independently selected from integers of 0 to 6;
$R^3$ is selected from 4- to 12-membered heterocycloalkyl, -NH-$C_{1-6}$ alkyl, -NH-4- to 12-membered heterocycloalkyl or -NH-C(O)$R^5$, wherein the said $C_{1-6}$ alkyl or 4- to 12-membered heterocycloalkyl is optionally substituted with one or more $C_{3-6}$ cycloalkyl;
$R^5$ is selected from hydrogen, deuterium, $C_{1-6}$ alkyl, $C_{1-6}$ alkoxy, $C_{3-6}$ cycloalkyl, 4- to 12-membered heterocycloalkyl, 5- or 6-membered heteroaryl, -$NR^{5a}R^{5b}$, -C(O)O-$C_{1-6}$ alkyl, -C(O)$NR^{5a}R^{5b}$,-$NR^{5a}R^{5b}$-C(O)O-$C_{1-6}$ alkyl, -C(O)-4- to 12-membered heterocycloalkyl, -$C_{1-6}$ alkyl-$(OCH_2CH_2)_mOCH_3$, -$C_{1-6}$ alkyl-$NR^{5a}R^{5b}$, -$C_{1-6}$ alkyl-NH-$C_{1-6}$ alkyl-O-$C_{1-6}$ alkyl, -$C_{1-6}$ alkyl-4- to 12-membered heterocycloalkyl, -$C_{1-6}$ alkyl-NH-4- to 12-membered heterocycloalkyl, -$C_{1-6}$ alkyl-NH-C1-6 alkyl-4- to 12-membered heterocycloalkyl, or -C1-6 alkyl-5- or 6-membered heteroaryl, wherein the said $C_{1-6}$ alkyl, $C_{1-6}$ alkoxy, $C_{3-6}$ cycloalkyl, 4- to 12-membered heterocycloalkyl, and 5-or 6-membered heteroaryl are optionally substituted with one or more substituents selected from deuterium, halogen, cyano, oxo, hydroxyl, carboxyl, $C_{1-6}$ alkyl, -$C_{1-6}$ alkyl-OH, $C_{1-6}$ alkoxy, $C_{3-6}$ cycloalkyl, -C(O)-$C_{1-6}$ alkyl, -C(O)O-$C_{1-6}$ alkyl, -C(O)-$C_{3-6}$ cycloalkyl, or 4- to 12-membered heterocycloalkyl, wherein two hydrogen atoms on an optional carbon atom of the said $C_{1-6}$ alkyl are substituted to form a $C_{3-6}$ cycloalkyl;
m is an integer selected from 1 to 4;
$R^{4a}$. $R^{4b}$, $R^{5a}$ and $R^{5b}$ are each independently selected from hydrogen, halogen, $C_{1-6}$ alkyl or $C_{1-6}$ alkoxy, wherein the said $C_{1-6}$ alkyl is optionally substituted with one or more substituents selected from halogen, hydroxyl or C3-6 cycloalkyl.

2. The compound according to claim 1, wherein $R^1$ is selected from $C_{1-6}$ alkyl, $C_{1-6}$ alkoxy, -$C_{1-6}$ alkyl-$C_{1-6}$ alkoxy, or $C_{3-6}$ cycloalkyl;

preferably, $R^1$ is selected from $C_{1-6}$ alkyl or -$C_{1-3}$ alkyl-$C_{1-3}$ alkoxy;
preferably, $R^1$ is selected from methyl, ethyl or -$CH_2CH_2OCH_3$.

3. The compound according to claim 1 or 2, wherein $R^2$ is selected from

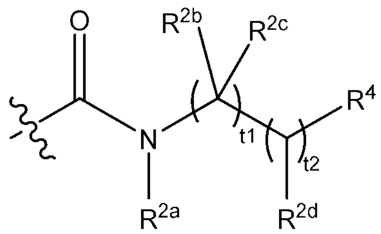

or

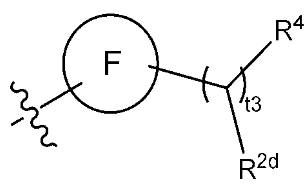

;

$R^4$ is selected from $C_{1-6}$ alkyl,

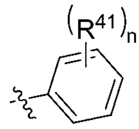, 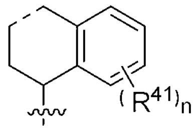 or 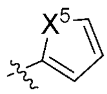,

wherein the said $R^{41}$ is as defined for the compound of formula (I); $X^5$ is selected from N, O or S;
preferably, $R^{41}$ is selected from halogen, cyano or amino;
preferably, $R^{41}$ is selected from fluoro, chloro or cyano;
preferably, ring F is selected from a 5- or 6-membered monocyclic heteroaryl or a 4- to 8-membered nitrogen-containing partially unsaturated heterocyclyl, wherein the said 5- or 6-membered monocyclic heteroaryl or 4- to 8-membered nitrogen-containing partially unsaturated heterocyclyl optionally contains 1, 2 or 3 heteroatoms selected from N and O as ring atoms;
preferably, ring F is selected from triazolyl or dihydrooxazolyl;
preferably, $R^{2a}$, $R^{2b}$, $R^{2c}$ and $R^{2d}$ are each independently selected from hydrogen, deuterium, halogen, $C_{1-6}$ alkyl, halo-substituted $C_{1-6}$ alkyl or hydroxy-substituted $C_{1-6}$ alkyl; more preferably selected from hydrogen, deuterium, halogen, methyl, ethyl, fluoroethyl, difluoroethyl or hydroxymethyl;
preferably, $R^{2a}$ is selected from hydrogen, deuterium or methyl;
preferably, $R^{2b}$, $R^{2c}$ and $R^{2d}$ are each independently selected from methyl, ethyl, fluoroethyl, difluoroethyl or hydroxymethyl;
preferably, $R^{2b}$ and $R^{2c}$, together with the carbon atom to which they are attached, form $C_{3-6}$ cycloalkyl or 4- to 12-membered heterocycloalkyl, wherein the said 4- to 12-membered heterocycloalkyl is preferably 4- to 6-membered oxygen-containing heterocycloalkyl, more preferably oxetanyl;
Alternatively, $R^{2a}$ and $R^{2b}$, together with the carbon atom and nitrogen atom to which they are attached, form a 4- to 12-membered nitrogen-containing heterocycloalkyl or a 4- to 8-membered nitrogen-containing partially unsaturated heterocyclyl, wherein the said 4- to 12-membered nitrogen-containing heterocycloalkyl is preferably azetidinyl or morpholinyl; the said 4- to 8-membered nitrogen-containing partially unsaturated heterocyclyl is preferably dihydropyrazolyl, and the said azetidinyl is optionally substituted with one or more halogens;
Alternatively, $R^{2b}$, $R^{2d}$ and the carbon atom to which they are attached together form $C_{3-6}$ cycloalkyl; preferably, R2 is selected from

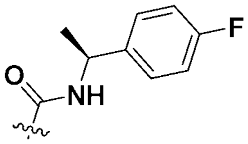, 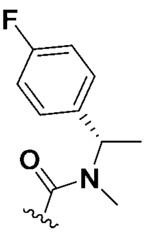, 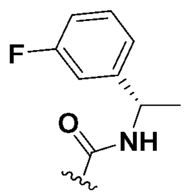,

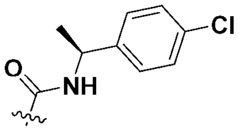, 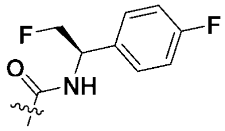, 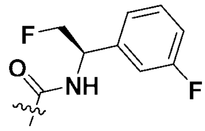, 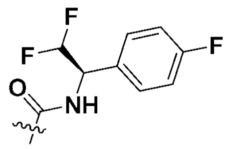, 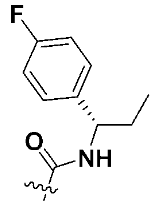,

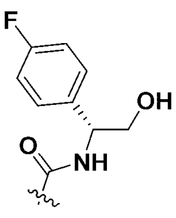, 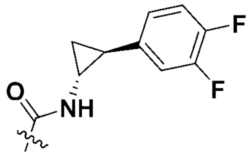, 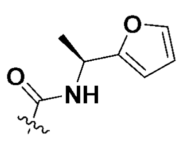, 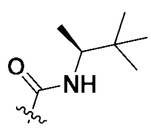, 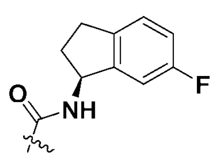,

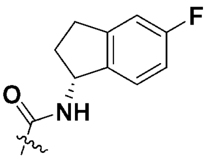, 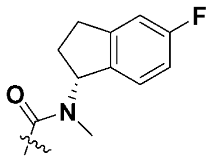, 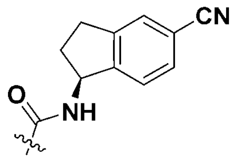, 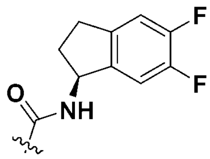,

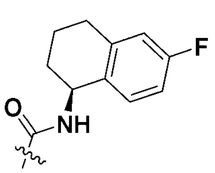, 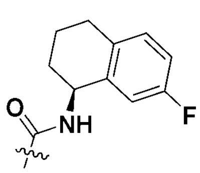, 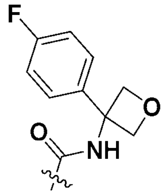, 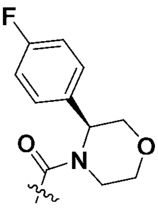, 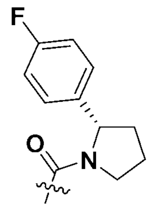,

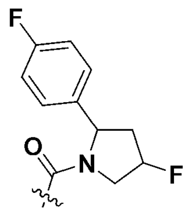, 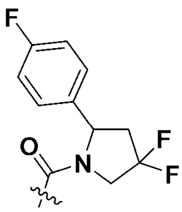, 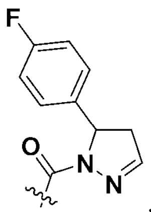, 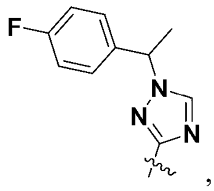, 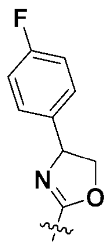 or 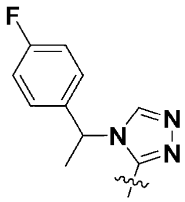.

**4.** The compound according to any one of claims 1 to 3, wherein $R^3$ is selected from 4- to 6-membered heterocycloalkyl, -NH-$C_{1-6}$ alkyl, -NH-4- to 12-membered heterocycloalkyl or -NH-C(O)$R^5$, wherein the said $C_{1-6}$ alkyl is optionally substituted with one or more $C_{3-6}$ cycloalkyl, and the said 4- to 6-membered heterocycloalkyl is optionally substituted with one or more oxo;

preferably, $R^3$ is selected from morpholinyl or oxomorpholinyl;
preferably, $R^3$ is selected from -NH-isopropyl, -NH-$CH_2$-cyclopropyl or -NH-tetrahydrofuran-3-yl;
preferably, $R^5$ is selected from methyl, ethoxy, -$CH_2CH_2COOH$, -$CH_2Cl$, -$CH_2CN$, -$CH_2OCH_3$,-$CH(CH_3)OCH_3$,

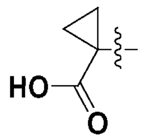,

-$C(O)OCH_2CH_3$, -$C(O)NH_2$, -$CH_2(OCH_2CH_2)_2OCH_3$,-$CH_2(OCH_2CH_2)_3OCH_3$,

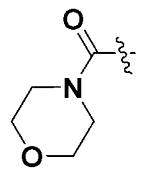, 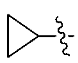, 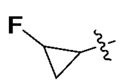, 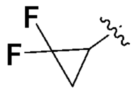, 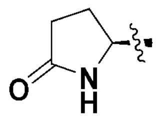, 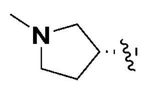,

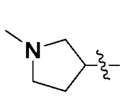, 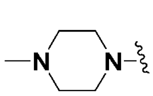, 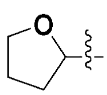, 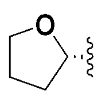, 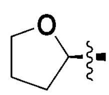, 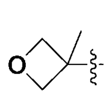, 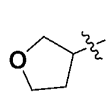, 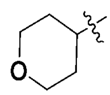,

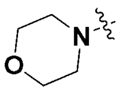, 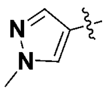, 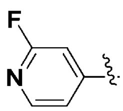, 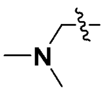, 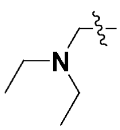, 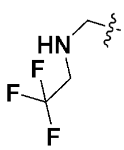, 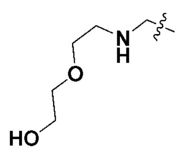, 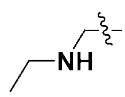,

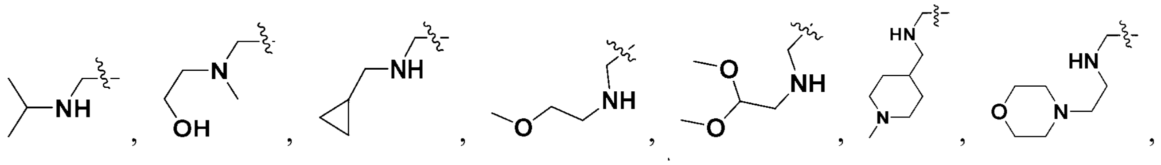
, , , , , , ,

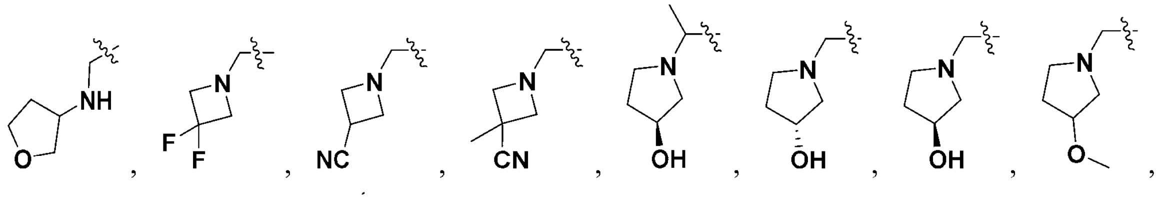
, , , , , , , ,

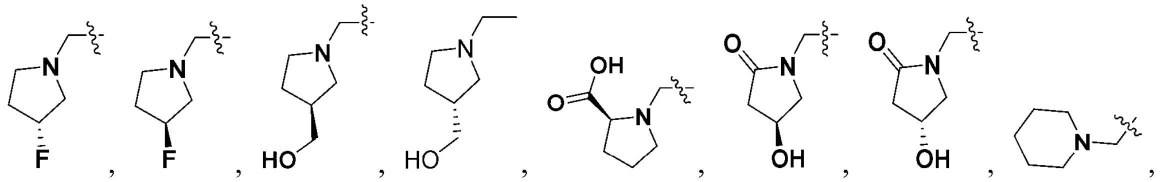
, , , , , , , ,

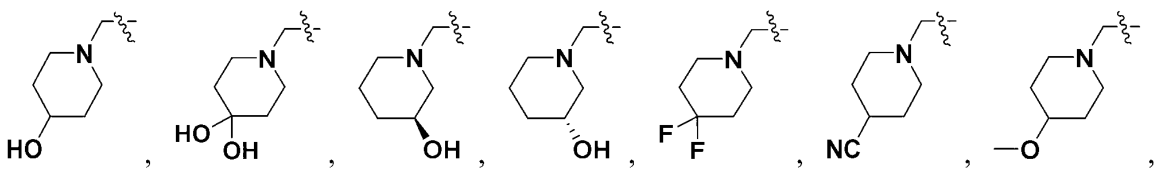
, , , , , , ,

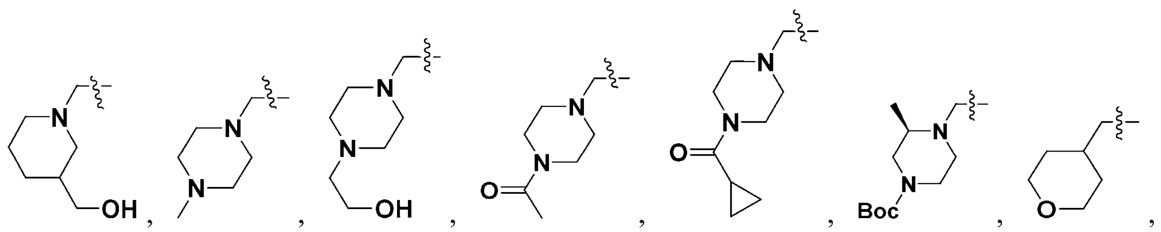
, , , , , , ,

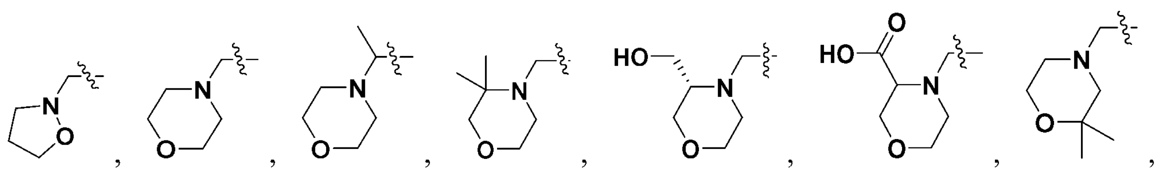
, , , , , , ,

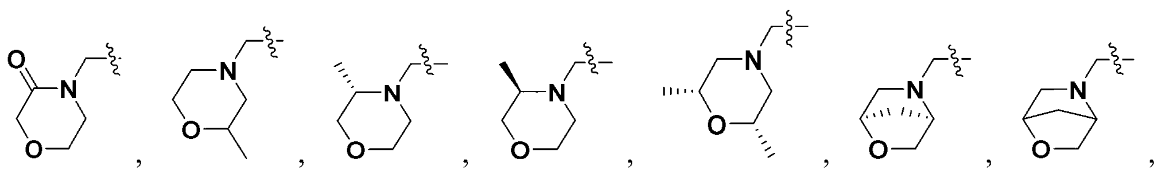
, , , , , , ,

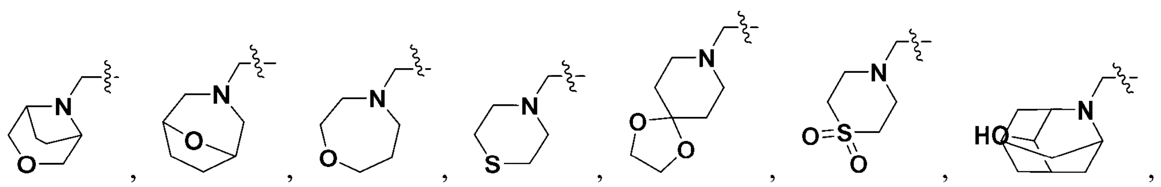
, , , , , , ,

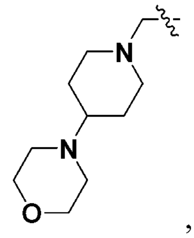, 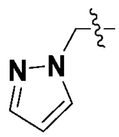, 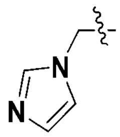, 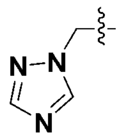 or 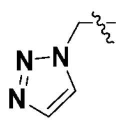;

preferably, $R^3$ is selected from:

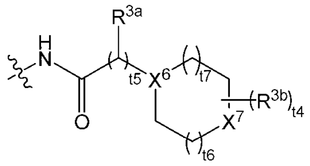, 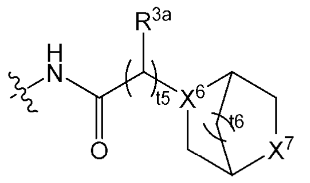, 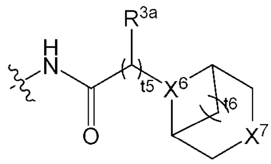,

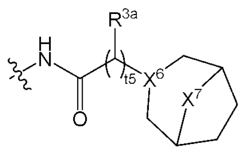, 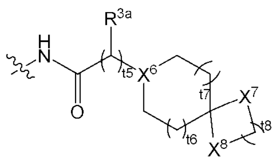, 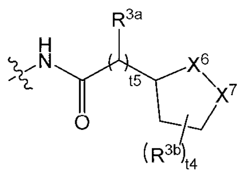, 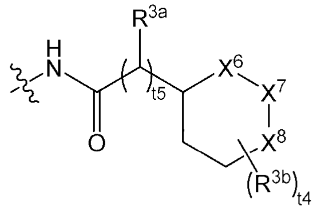,

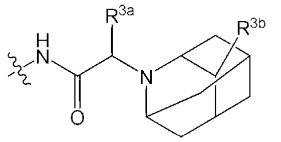, 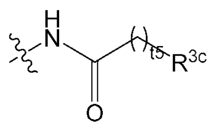, 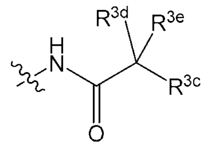, 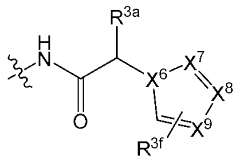,

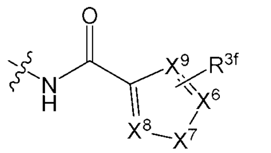, 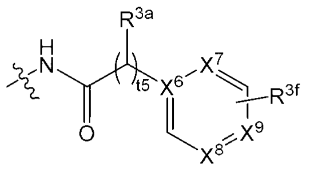, 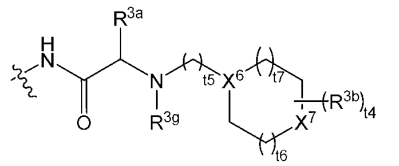,

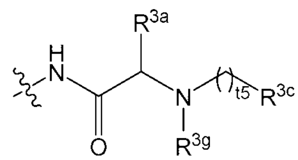;

preferably, $R^3$ is selected from:

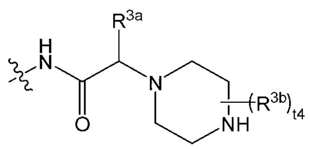, 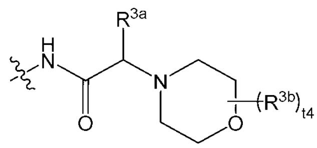, 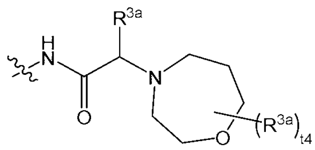,

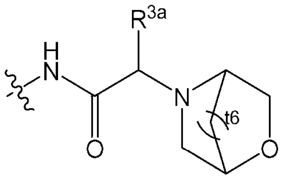, 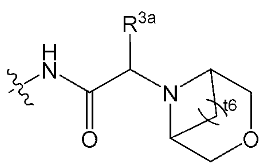, 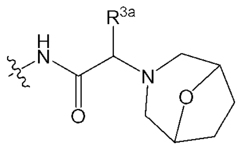, 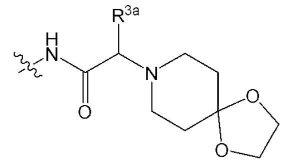,

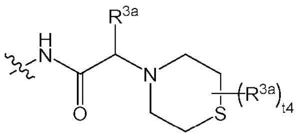, 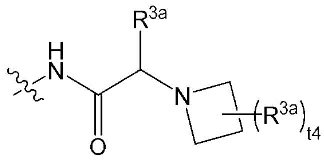, 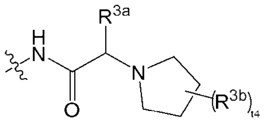,

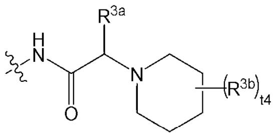, 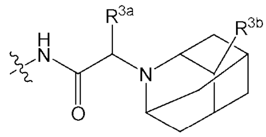, 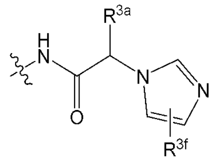, 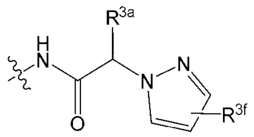,

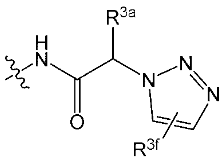, 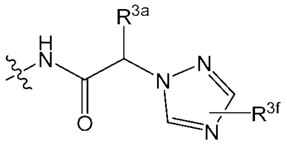, 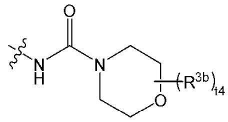, 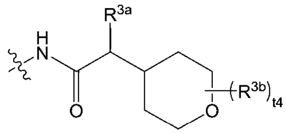,

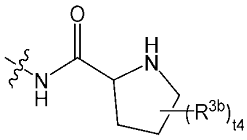, 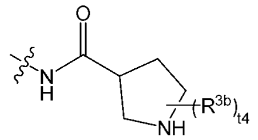, 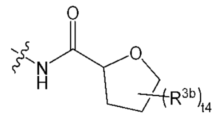, 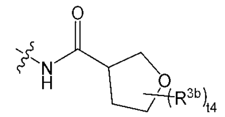,

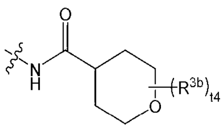, 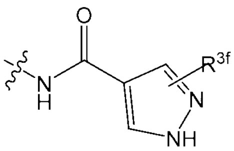, 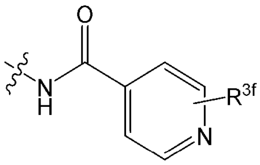, 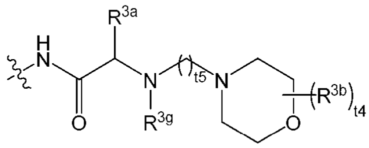,

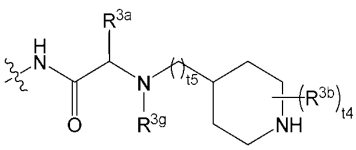, 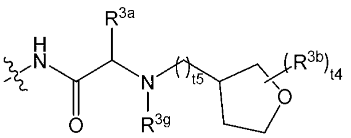, 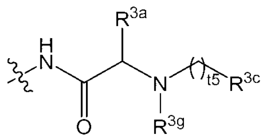,

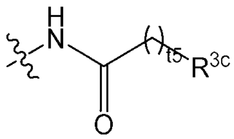, 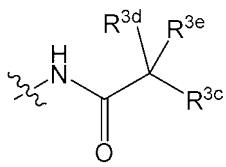;

wherein, $X^8$, $X^7$, $X^8$, and $X^9$ are each independently selected from N, NH, O, S, CH or $CH_2$;
t4 and t5 are each independently selected from integers of 0 to 6;
t6 and t7 are each independently selected from 0, 1 or 2;
$R^{3a}$. $R^{3b}$, $R^{3c}$, $R^{3d}$, $R^{3e}$, $R^{3f}$, and $R^{3g}$ are each independently selected from hydrogen, deuterium, halogen, cyano, oxo, hydroxyl, carboxyl, $C_{1-6}$ alkyl, $C_{1-6}$ alkoxy, $C_{3-6}$ cycloalkyl, 4- to 12-membered heterocycloalkyl,

$-(OCH_2CH_2)_mOCH_3$, $-NR^{X3a}R^{X3b}$, $-C(O)R^{X3c}$, wherein the said $C_{1-6}$ alkyl, $C_{1-6}$ alkoxy, $C_{3-6}$ cycloalkyl are optionally substituted with one or more substituents selected from halogen, hydroxyl, $C_{1-6}$ alkoxy;

or. $R^{3d}$, $R^{3e}$ and the carbon atom to which they are attached together form $C_{3-6}$ cycloalkyl;

$R^{X3a}$, $R^{X3b}$, and $R^{X3c}$ are each independently selected from hydrogen, $C_{1-6}$ alkyl, $C_{1-6}$ alkoxy or $C_{3-6}$ cycloalkyl;

m is an integer selected from 1 to 4; preferably 2 or 3;

preferably, $R^{3a}$ is selected from hydrogen, deuterium, oxo, or $C_{1-6}$ alkyl; preferably from hydrogen, oxo, or methyl;

preferably, $R^{3b}$ is selected from hydrogen, deuterium, halogen, cyano, oxo, hydroxyl, carboxyl, $C_{1-6}$ alkyl, $C_{1-6}$ alkoxy, $-C_{1-6}$ alkyl-OH, $-C(O)C_{1-6}$ alkyl, $-C(O)C_{3-6}$ cycloalkyl, $C_{3-6}$ cycloalkyl, or 4- to 12-membered heterocycloalkyl; wherein the said 4- to 12-membered heterocycloalkyl optionally contains 1, 2, or 3 heteroatoms selected from N and O as ring atoms;

preferably, $R^{3b}$ is selected from hydrogen, deuterium, halogen, cyano, oxo, hydroxyl, carboxyl, methyl, methoxyl, $-CH_2OH$, $-C(O)CH_3$, -C(O)cyclopropyl, cyclopropyl, or morpholinyl;

preferably, $R^{3c}$ is selected from hydrogen, deuterium, halogen, cyano, oxo, hydroxyl, carboxyl, amino, $-(OCH_2CH_2)_mOCH_3$, $-NR^{X3a}R^{X3b}$, $C_{1-6}$ alkyl, $C_{1-6}$ alkoxy, halogen-substituted $C_{1-6}$ alkyl, $C_{3-6}$ cycloalkyl and halogen-substituted $C_{3-6}$ cycloalkyl, wherein the said $C_{1-6}$ alkyl or $C_{1-6}$ alkoxy is optionally substituted with one or more substituents selected from hydroxyl and $C_{1-6}$ alkoxy;

preferably, $R^{3c}$ is selected from hydrogen, deuterium, halogen, cyano, oxo, hydroxyl, carboxyl, amino, dimethylamino, diethylamino, trifluoromethyl, methoxy, ethoxy, isopropyl, cyclopropyl, halogen-substituted cyclopropyl, $-CH(OCH_3)_2$, $-OCH_2CH_2OH$, $-(OCH_2CH_2)_2OCH_3$ or $-(OCH_2CH_2)_3OCH_3$;

Alternatively, $R^{3d}$ and $R^{3e}$ together with the carbon atom to which they are attached form $C_{3-6}$ cycloalkyl; preferably, $R^{3d}$ and $R^{3e}$ together with the carbon atom to which they are attached form cyclopropyl;

preferably, $R^{3f}$ is hydrogen.

**5.** The compound according to claim 1, wherein the compound of formula (I) is a compound represented by formula (II) or formula (III):

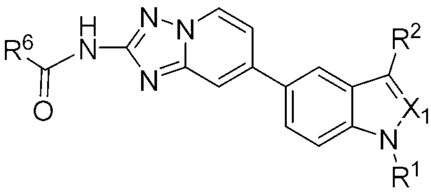

(II)

wherein $X^1$, $R^1$ and $R^2$ are each as defined for the compound of formula (I);

$R^6$ is selected from hydrogen, deuterium, $C_{1-6}$ alkyl, $C_{1-6}$ alkoxy, $C_{3-6}$ cycloalkyl, 4- to 12-membered heterocycloalkyl, 5- or 6-membered heteroaryl, $-C(O)NR^{5a}R^{5b}$, $-C(O)O-C_{1-6}$ alkyl or -C(O)-(4- to 12-membered heterocycloalkyl), wherein the said $C_{1-6}$ alkyl, $C_{3-6}$ cycloalkyl, 4- to 12-membered heterocycloalkyl and 5- or 6-membered heteroaryl are optionally substituted with one or more substituents selected from halogen, oxo, hydroxyl, carboxyl, cyano, $C_{1-6}$ alkyl, $-C_{1-6}$ alkyl-OH, $C_{1-6}$ alkoxy, $C_{3-6}$ cycloalkyl, $-C(O)-C_{1-6}$ alkyl, $-C(O)O-C_{1-6}$ alkyl, $-C(O)-C_{3-6}$ cycloalkyl or 4- to 12-membered heterocycloalkyl, wherein two hydrogen atoms on any one carbon atom of the said $C_{1-6}$ alkyl are substituted to form $C_{3-6}$ cycloalkyl;

$R^{5a}$ and $R^{5b}$ are each independently selected from hydrogen, $C_{1-6}$ alkyl or $C_{1-6}$ alkoxy, wherein the said $C_{1-6}$ alkyl is optionally substituted with one or more halogens or $C_{3-6}$ cycloalkyl;

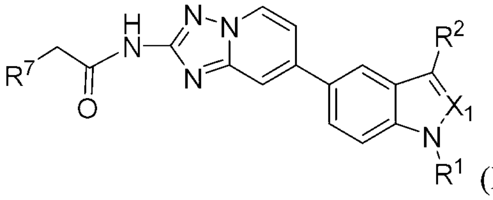

(III)

wherein $X^1$, $R^1$ and $R^2$ are each as defined for the compound of formula (I);

$R^7$ is selected from $-NR^{5a}R^{5b}$, $-(OCH_2CH_2)_mOCH_3$, $-NH-C_{1-6}$ alkyl-O-$C_{1-6}$ alkyl, -4- to 12-membered heterocycloalkyl, -NH-4- to 12-membered heterocycloalkyl, $-NH-C_{1-6}$ alkyl-4- to 12-membered heterocycloalkyl or 5- or 6-membered heteroaryl, wherein the said $C_{1-6}$ alkyl, $C_{3-6}$ cycloalkyl, 4- to 12-membered heterocycloalkyl and 5- or 6-membered heteroaryl are optionally substituted with one or more substituents selected from halogen, oxo, hydroxyl, carboxyl, cyano, $C_{1-6}$ alkyl, $-C_{1-6}$ alkyl-OH, $C_{1-6}$ alkoxy, $C_{3-6}$ cycloalkyl, $-C(O)-C_{1-6}$ alkyl, $-C(O)O-C_{1-6}$

alkyl, -C(O)-$C_{3-6}$ cycloalkyl or 4- to 12-membered heterocycloalkyl;
$R^{5a}$ and $R^{5b}$ are each independently selected from hydrogen, $C_{1-6}$ alkyl or $C_{1-6}$ alkoxy, wherein the said $C_{1-6}$ alkyl is optionally substituted with one or more halogens or $C_{3-6}$ cycloalkyl;
preferably, the compound of formula (III) is a compound represented by formula (IIIa) or formula (IIIb):

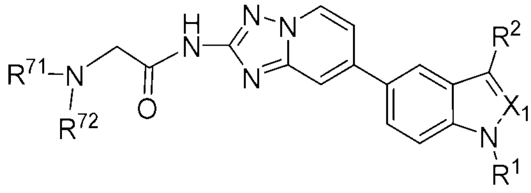
(IIIa)

wherein $X^1$, $R^1$ and $R^2$ are each as defined for the compound of formula (I);
$R^{71}$ and $R^{72}$ are each independently selected from hydrogen, $C_{1-6}$ alkyl, $C_{1-6}$ alkoxy, 4- to 12-membered heterocycloalkyl and 5- or 6-membered heteroaryl, wherein the said $C_{1-6}$ alkyl is optionally substituted with one or more substituents selected from halogen, $C_{1-6}$ alkoxy, $C_{3-6}$ cycloalkyl, 4- to 12-membered heterocycloalkyl or $-CH(OCH_3)_2$;

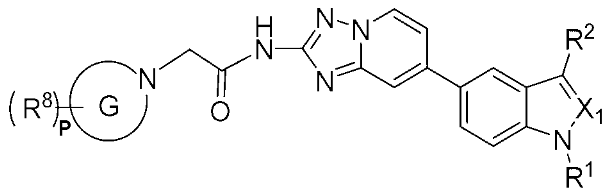
(IIIb)

wherein $X^1$, $R^1$ and $R^2$ are each as defined for the compound of formula (I);
Ring G is selected from 5- or 6-membered nitrogen-containing heteroaryl or 5- to 12-membered nitrogen-containing heterocycloalkyl;
$R^8$ is selected from hydrogen, halogen, cyano, hydroxyl, carboxyl, oxo, $C_{1-6}$ alkyl, $C_{1-6}$ alkoxy, -$C_{1-6}$ alkyl-OH, -C(O)-$C_{1-6}$ alkyl, -C(O)O-$C_{1-6}$ alkyl or -C(O)-$C_{3-6}$ cycloalkyl;
p is selected from 0, 1, 2 or 3;
preferably, $R^8$ is selected from hydrogen, fluoro, cyano, hydroxyl, carboxyl, oxo, methyl, methoxy, hydroxymethyl, $-C(O)CH_3$, -C(O)-cyclopropyl or $-C(O)OCH(CH_3)_3$;
preferably, ring G is selected from

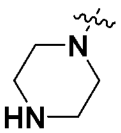 , 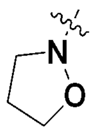 , 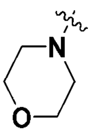 , 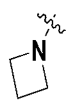 , 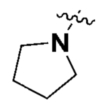 , 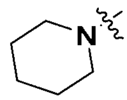 ,

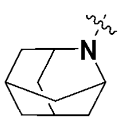 , 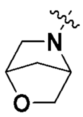 , 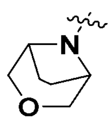 , 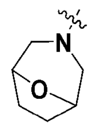 , 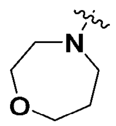 , 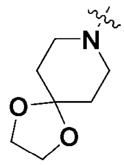 , 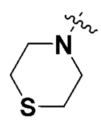 , 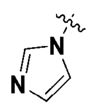 , 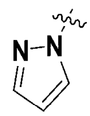 ,

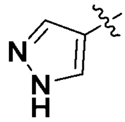 , 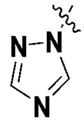 , 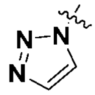 or 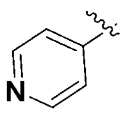 .

**6.** The compound according to any one of claims 1-5, wherein the said 4- to 12-membered heterocycloalkyl is selected from 4- to 7-membered monocyclic heterocycloalkyl, 6- to 8-membered bicyclic heterocycloalkyl, 6- to 10-membered bridged heterocycloalkyl, or 6- to 10-membered spiroheterocycloalkyl; the said 4- to 7-membered monocyclic heterocycloalkyl, 6- to 8-membered bicyclic heterocycloalkyl, 6- to 10-membered bridged heterocycloalkyl, or 6-

to 10-membered spiroheterocycloalkyl preferably contains 1, 2, or 3 heteroatoms selected from N, S, or O as ring atoms;

preferably, the said 4- to 12-membered heterocycloalkyl is selected from pyrrolidinyl, piperidinyl, piperazinyl, oxetanyl, tetrahydrofuranyl, oxetanyl, isoxazolidinyl, morpholinyl, oxazepanyl, azetidinyl, pyrrolidinyl, piperidinyl, azaadamantyl, oxa-azabicycloheptyl, oxa-azabicyclooctyl, oxa-azaspirodecyl or thiomorpholinyl;
preferably, the said 4- to 12-membered heterocycloalkyl is selected from

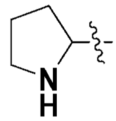 , 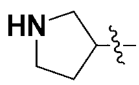 ,

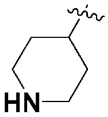 , 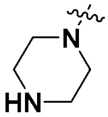 , 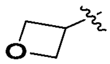 , 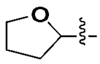 , 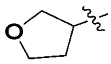 , 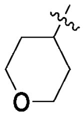 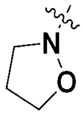 , 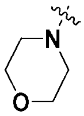 , 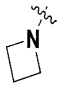 , 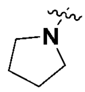 ,

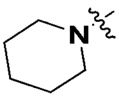 , 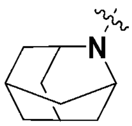 , 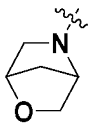 , 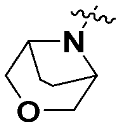 , 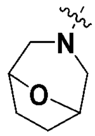 , 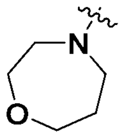 , 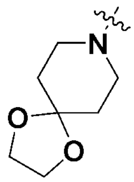 or 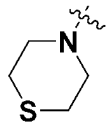 ;

Alternatively, the said 5- or 6-membered heteroaryl is selected from 5- or 6-membered nitrogen-containing heteroaryl, wherein the said 5- or 6-membered nitrogen-containing heteroaryl optionally contains 1, 2 or 3 nitrogen atoms as ring atoms;
preferably, the said 5- or 6-membered heteroaryl is selected from imidazolyl, pyrazolyl, triazolyl or pyridyl;
preferably, the said 5- or 6-membered heteroaryl is selected from

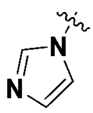 , 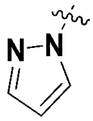 , 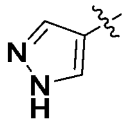 ,

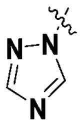 , 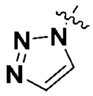 or 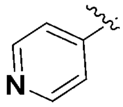 .

**7.** The following compounds, or pharmaceutically acceptable salts, solvates or stereoisomers thereof:

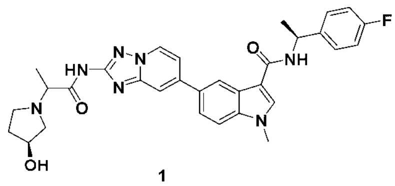
1 ,

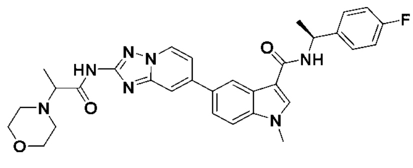
2 ,

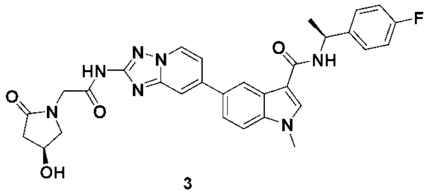
3
,

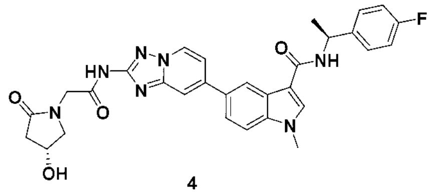
4
,

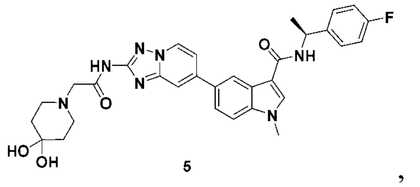
5
,

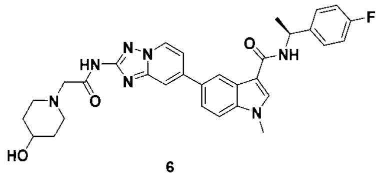
6
,

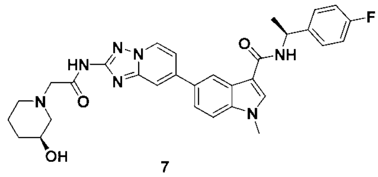
7
,

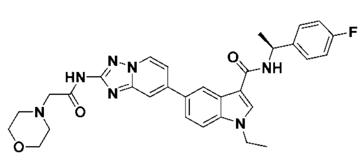
8
,

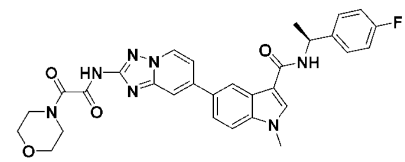
9
,

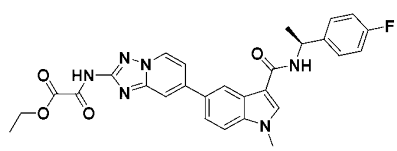
10
,

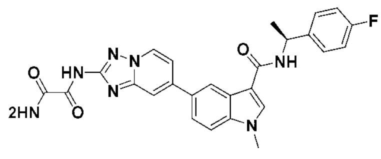
11

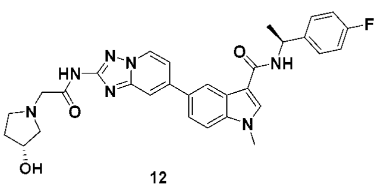
12
,

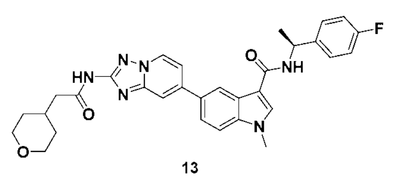
13
,

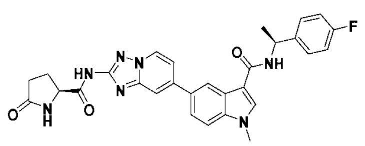
14
,

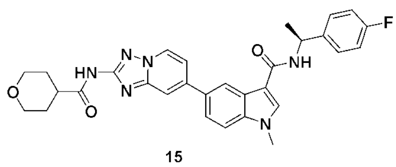
15
,

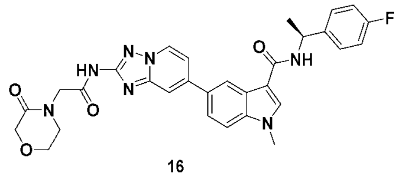
16
,

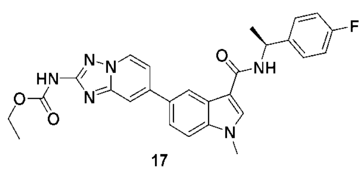
17
,

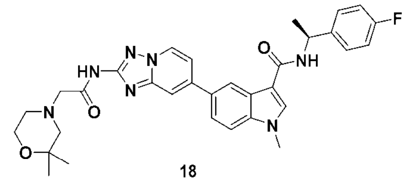
18
,

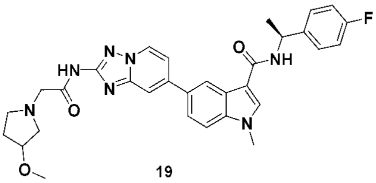
19
,

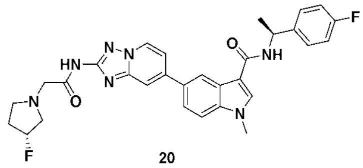
20
,

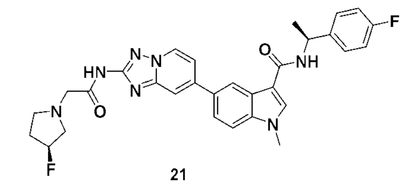
21
,

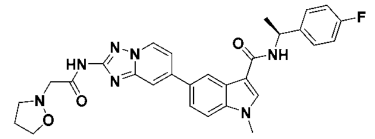
22
,

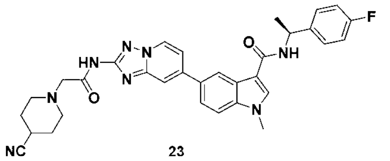

23,

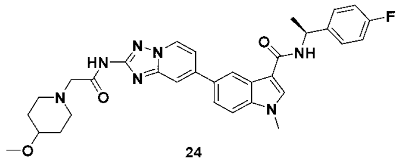

24,

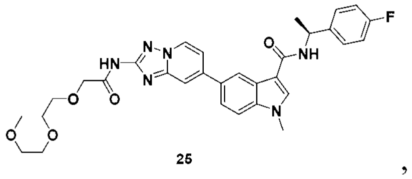

25,

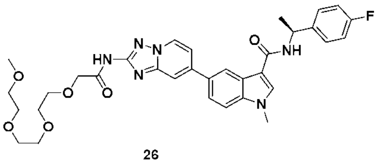

26,

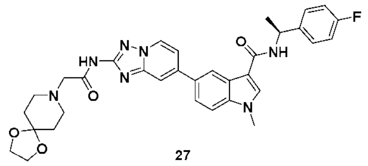

27,

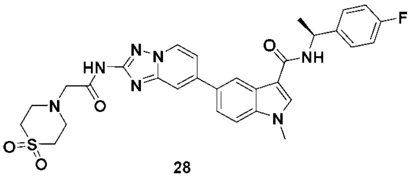

28,

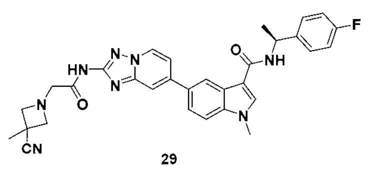

29,

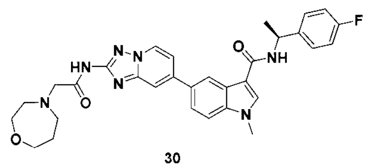

30,

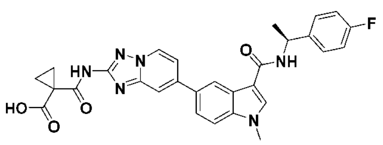

31,

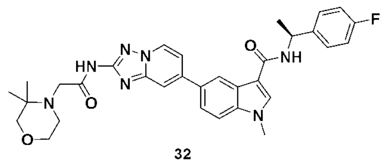

32

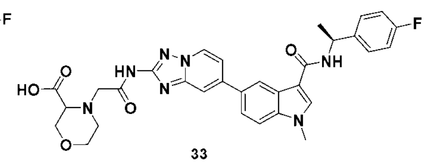

33,

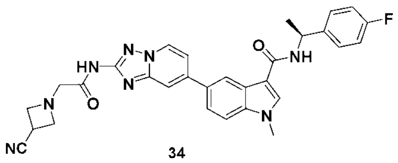

34,

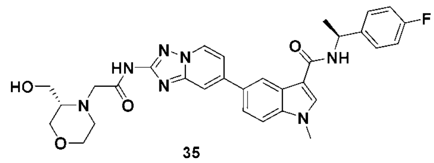

35,

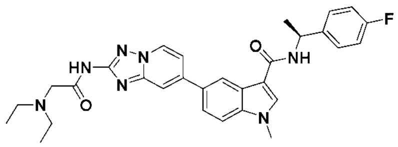

36,

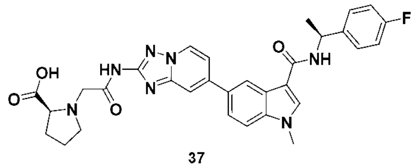

37,

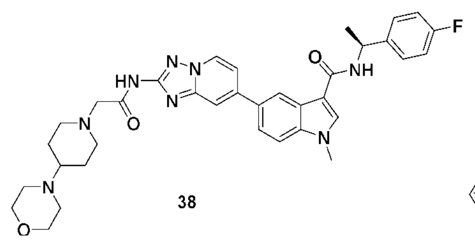

38

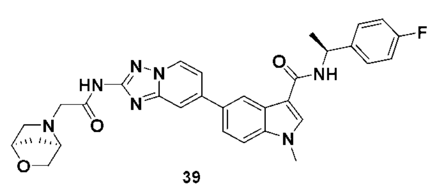

39

,

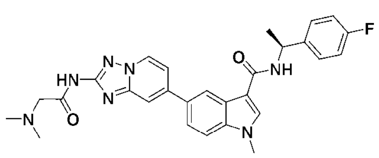

40,

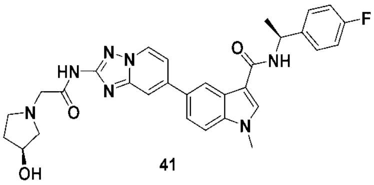

41,

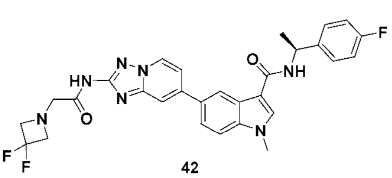

42,

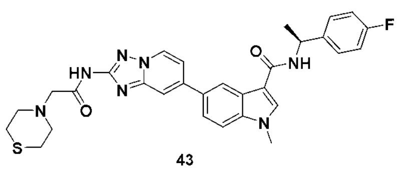

43

,

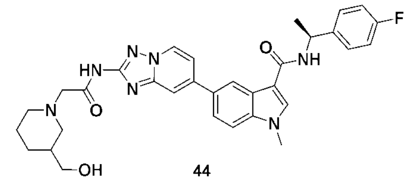

44

,

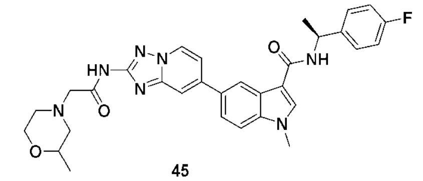

45

,

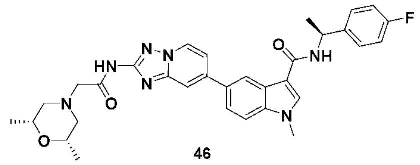

46

,

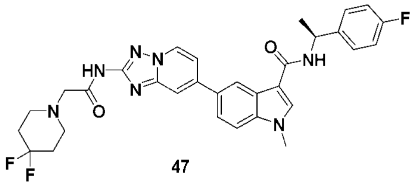

47

,

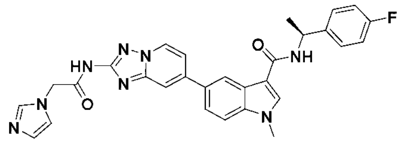

48

,

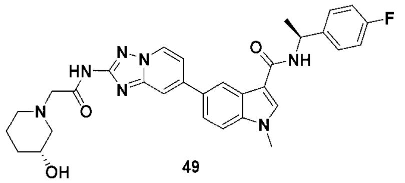

49

,

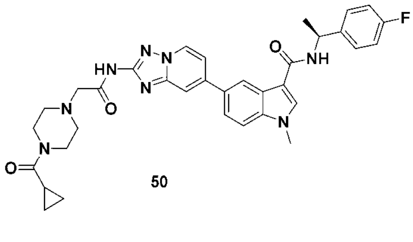

50

,

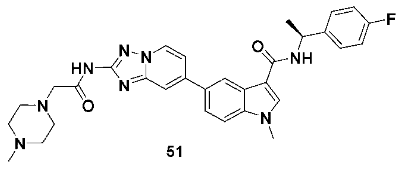

51

,

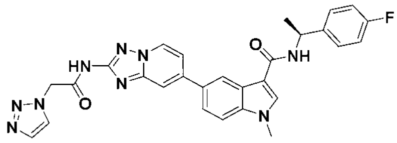

52

,

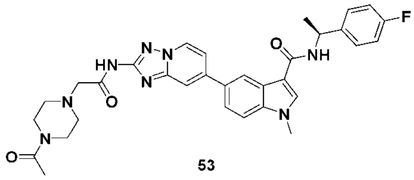

53

,

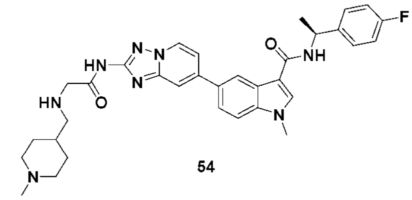

54

,

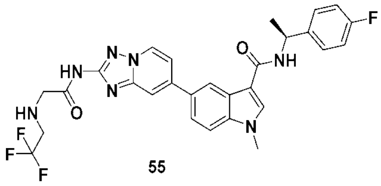

55

,

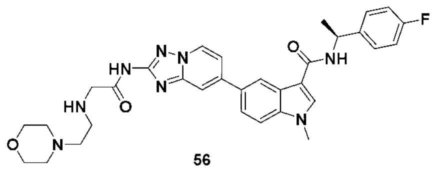

56

,

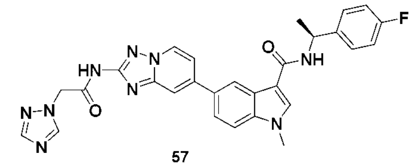
57
,

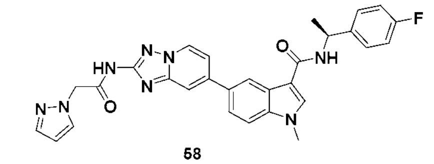
58
,

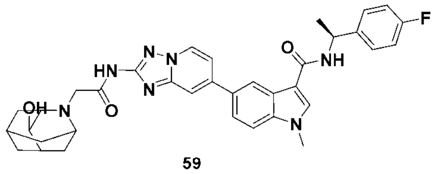
59
,

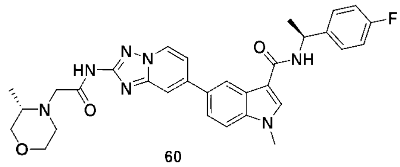
60
,

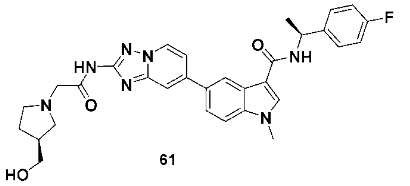
61
,

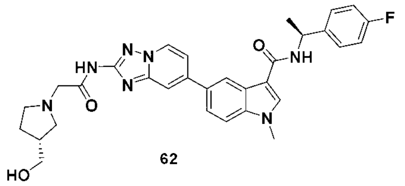
62
,

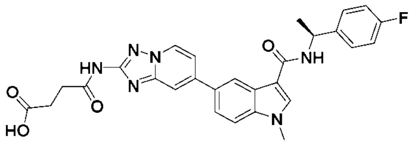
63
,

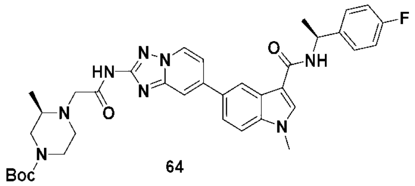
64
,

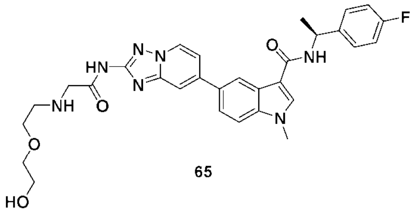
65

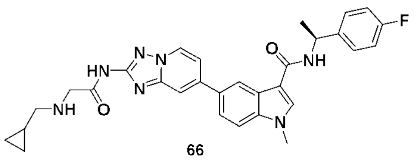
66
,

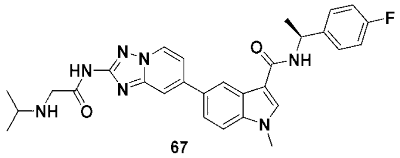
67
,

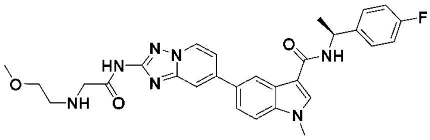
68

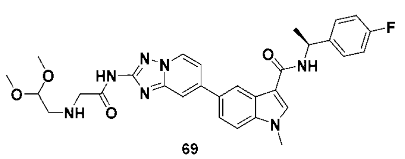
69
,

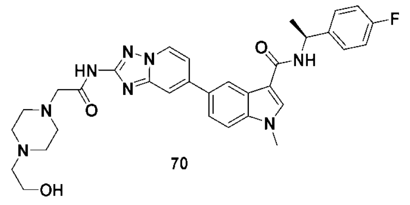
70
,

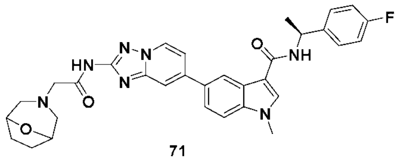
71

,

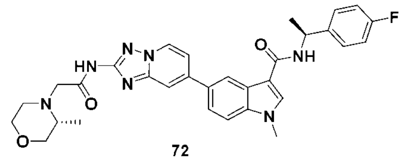
72

,

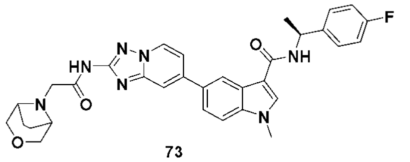
73

,

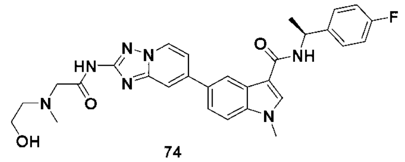
74

,

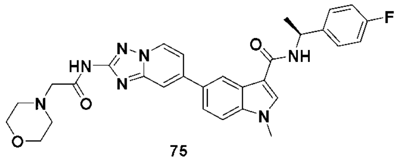
75

,

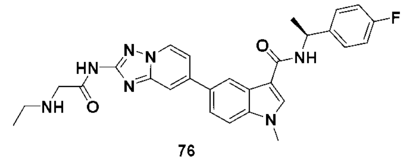
76

,

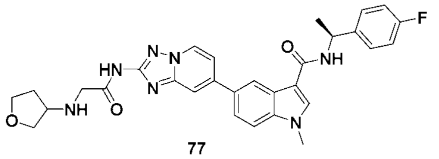
77

,

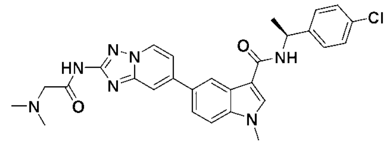
78

,

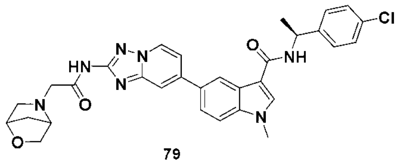
79

,

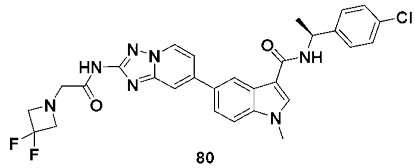
80

,

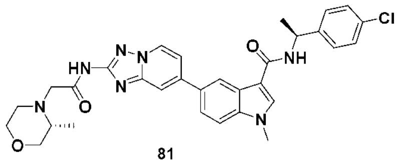
81

,

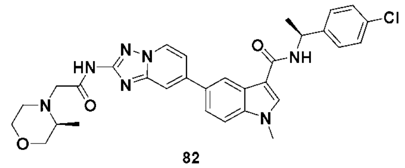
82

,

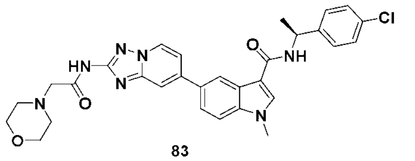
83

,

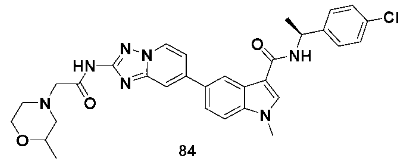
84

,

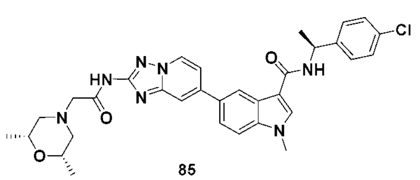
85

,

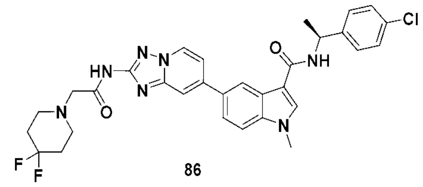
86

,

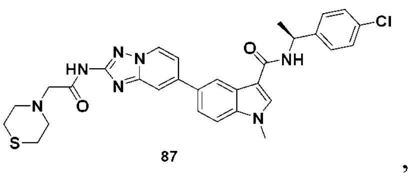
87
,
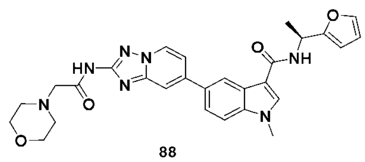
88
,
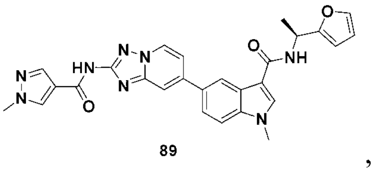
89
,

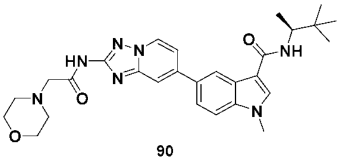
90
,
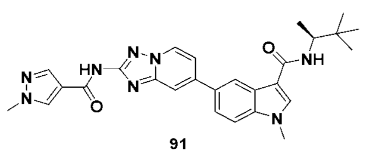
91
,
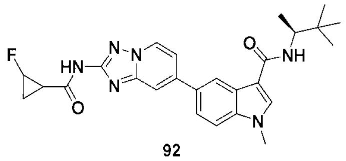
92
,

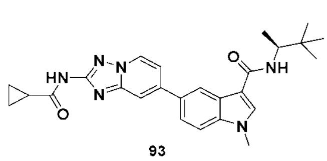
93
,
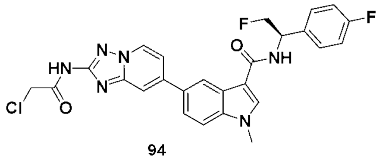
94
,
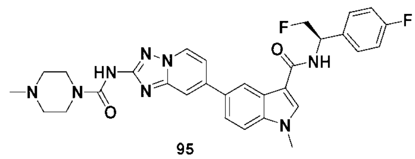
95
,

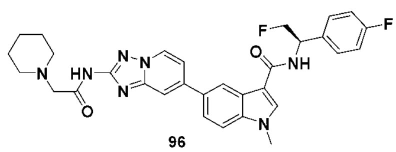
96
,
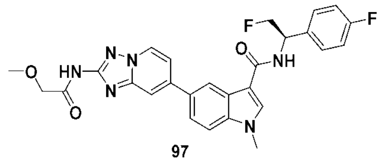
97
,
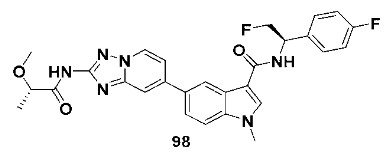
98
,

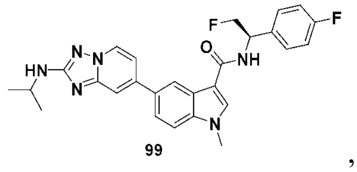
99
,
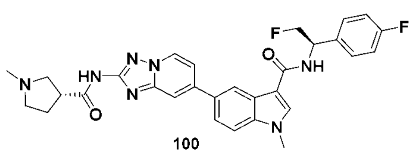
100
,
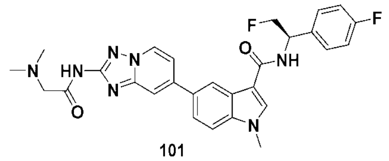
101
,

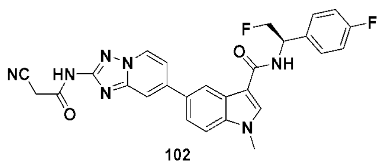
102
,
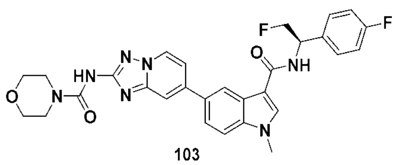
103
,
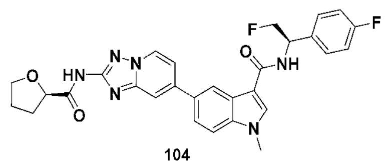
104
,

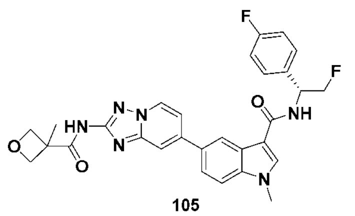
105
,
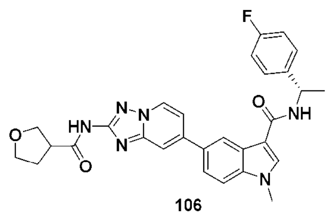
106
,
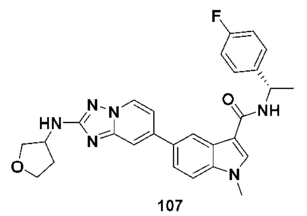
107
,

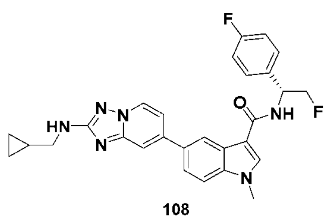
108
,
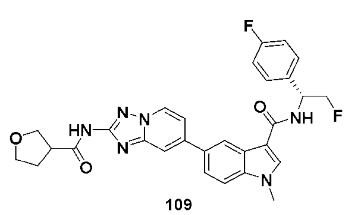
109
,
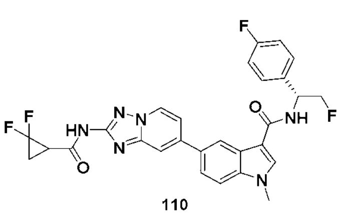
110
,

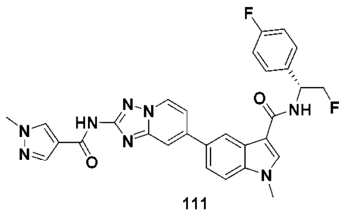
111
,
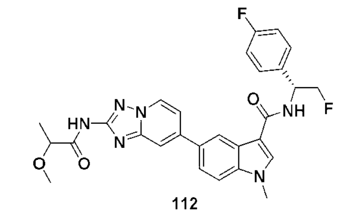
112
,
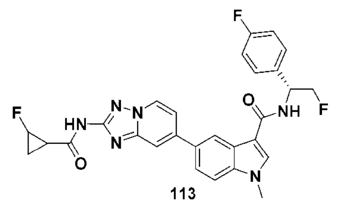
113
,
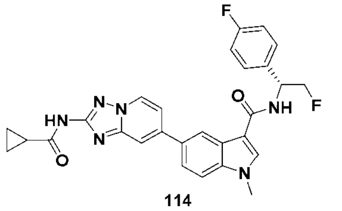
114
,
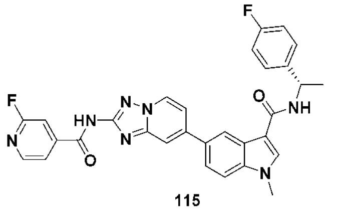
115
,
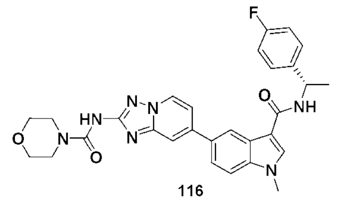
116
,
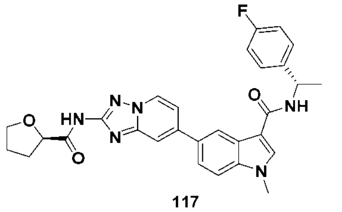
117
,
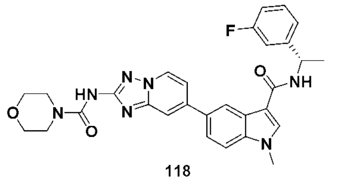
118
,
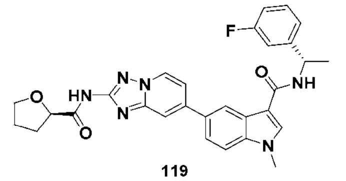
119
,
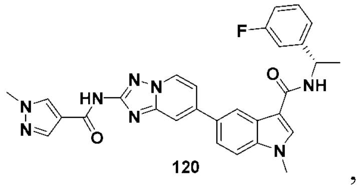
120
,
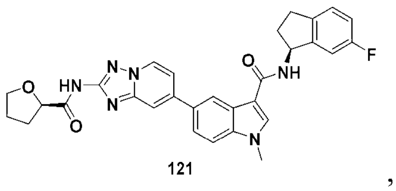
121
,
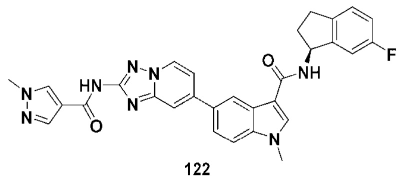
122
,
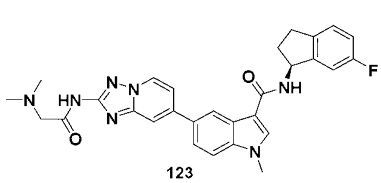
123
,
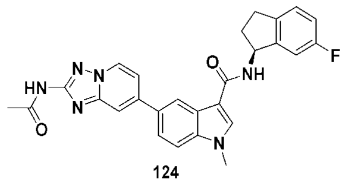
124
,
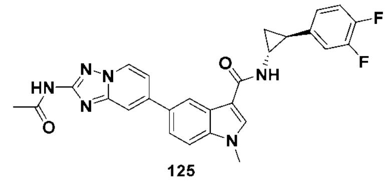
125
,
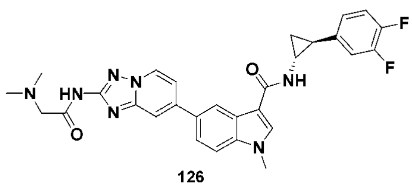
126
,
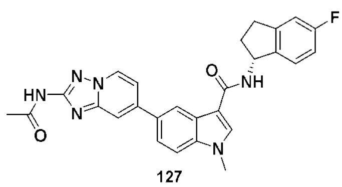
127
,
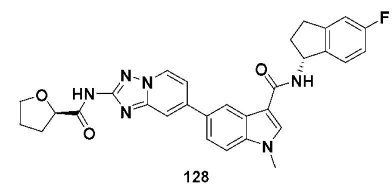
128
,
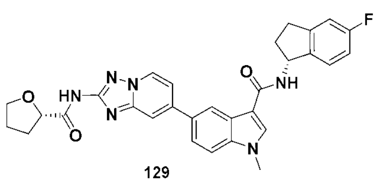
129
,
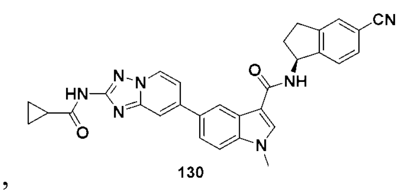
130
,
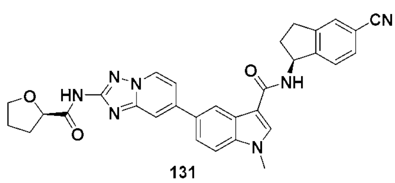
131
,

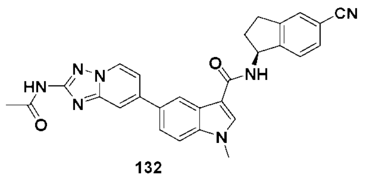
132 ,

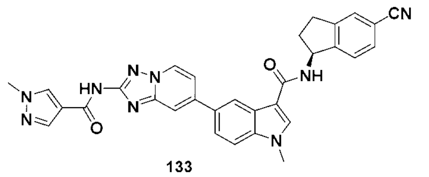
133 ,

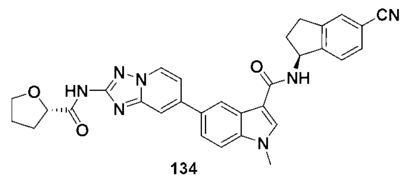
134 ,

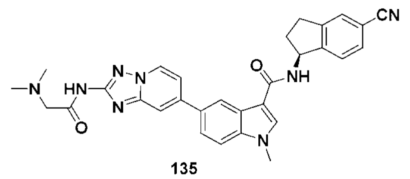
135 ,

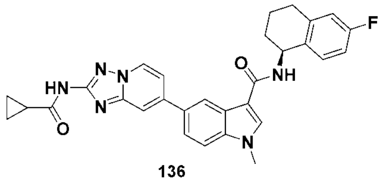
136 ,

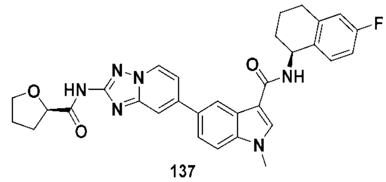
137 ,

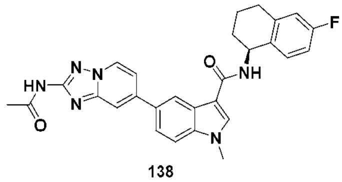
138 ,

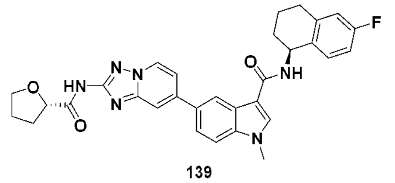
139 ,

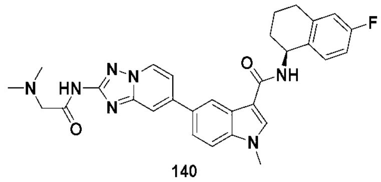
140 ,

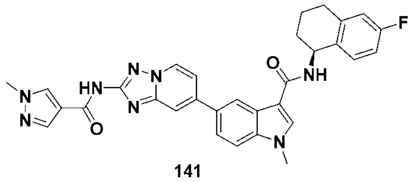
141 ,

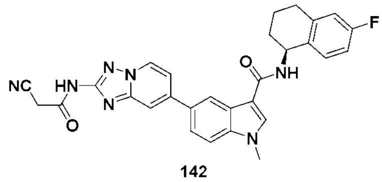
142 ,

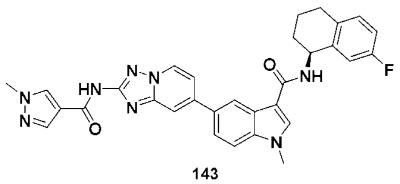
143 ,

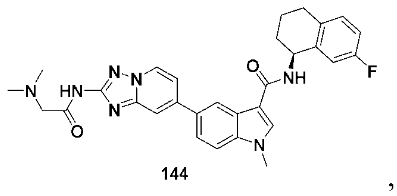
144 ,

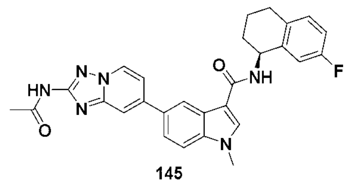
145 ,

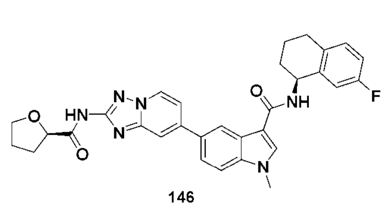
146 ,

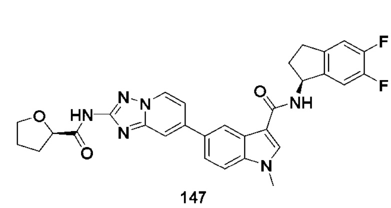
147 ,

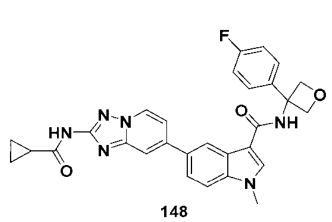
148 ,

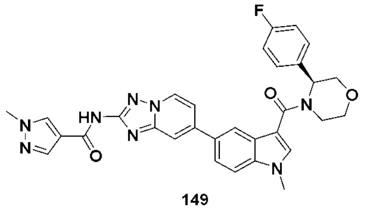
149,
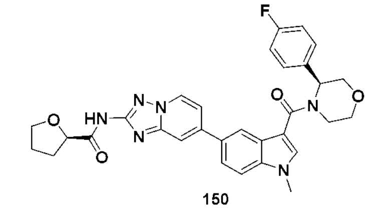
150,
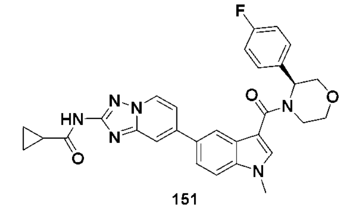
151,

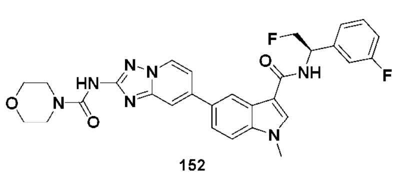
152,
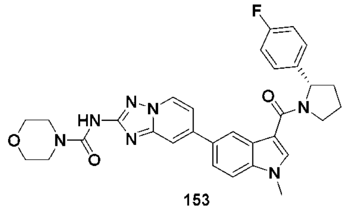
153,
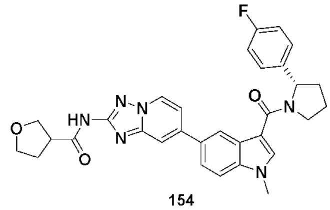
154,

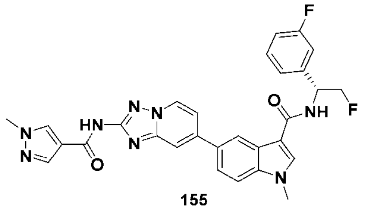
155,
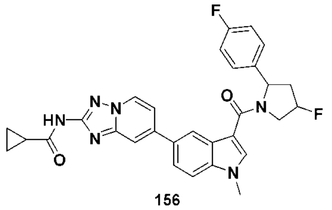
156,
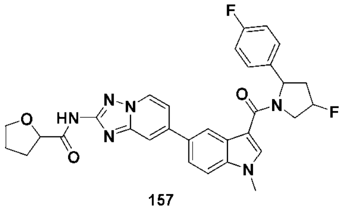
157,

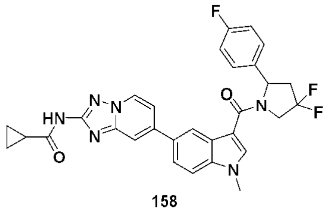
158,
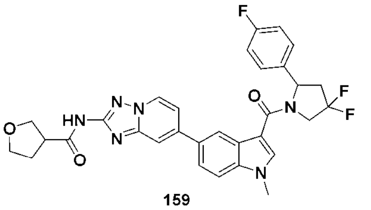
159,
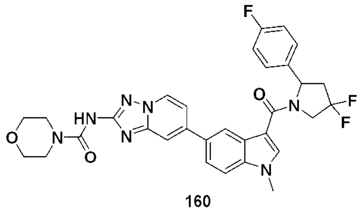
160,

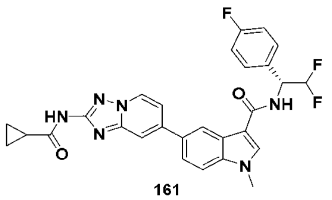
161,
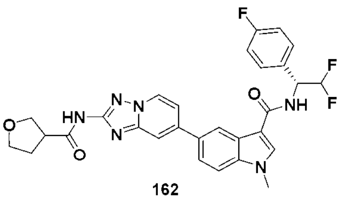
162,
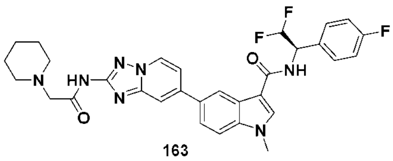
163,

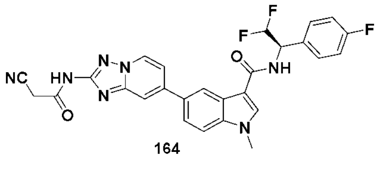
164,
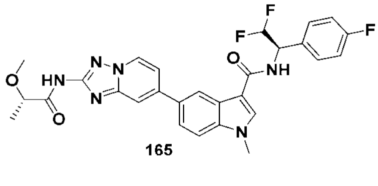
165,
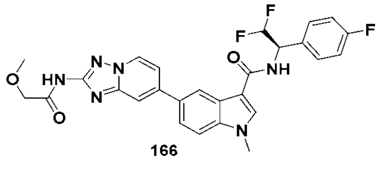
166,

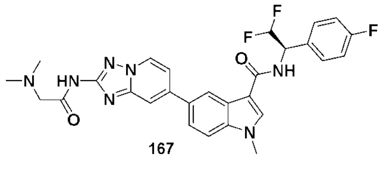
167,
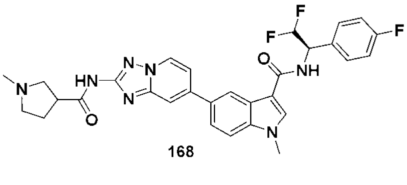
168,
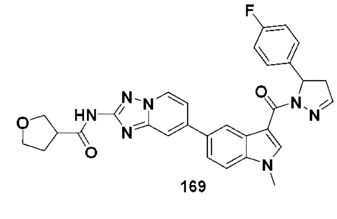
169,

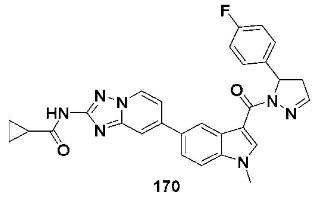
170 ,

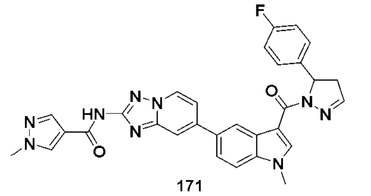
171 ,

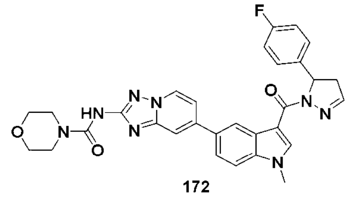
172 ,

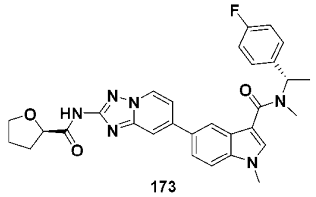
173 ,

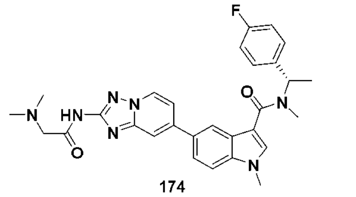
174 ,

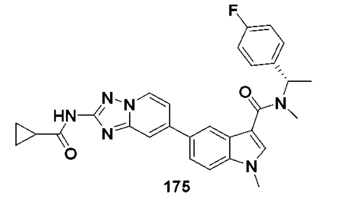
175 ,

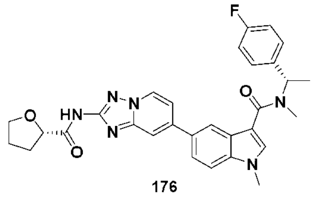
176 ,

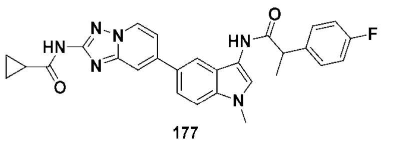
177 ,

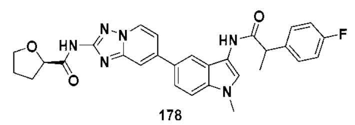
178 ,

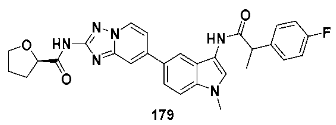
179 ,

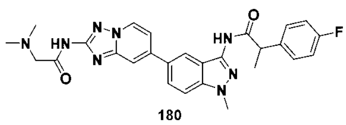
180 ,

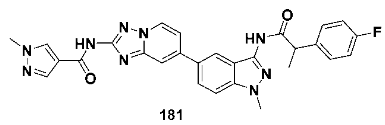
181 ,

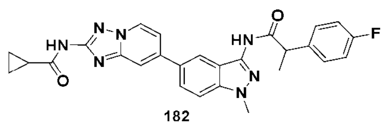
182 ,

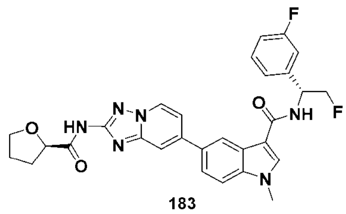
183 ,

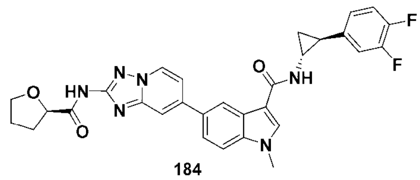
184 ,

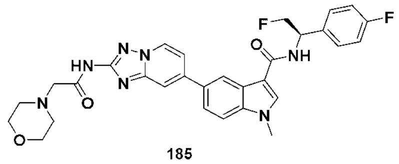
185 ,

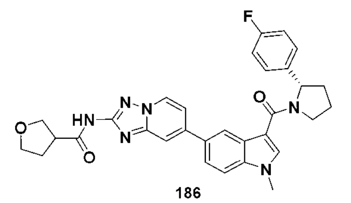
186 ,

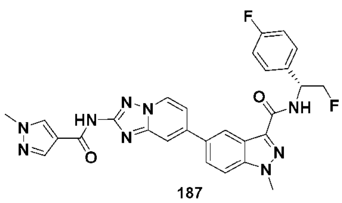
187 ,

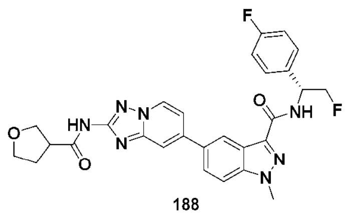
188 ,

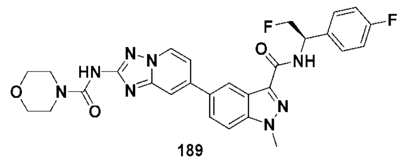
189 ,

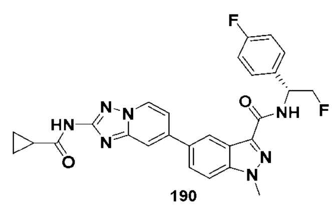
190 ,

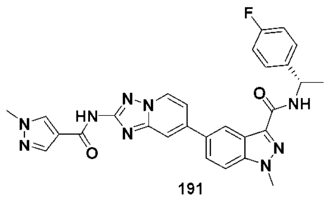
191 ,

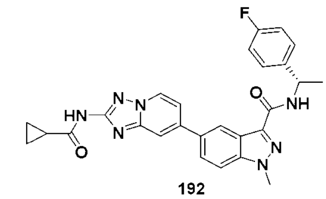
192 ,

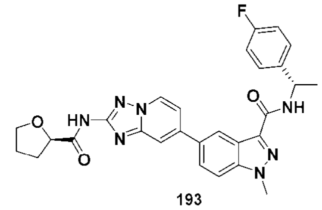
193 ,

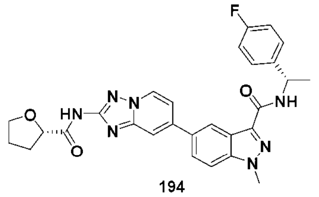
194 ,

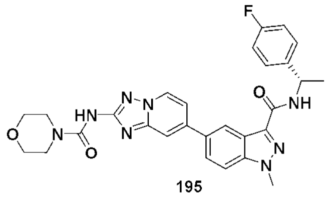
195 ,

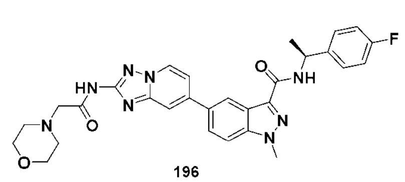
196 ,

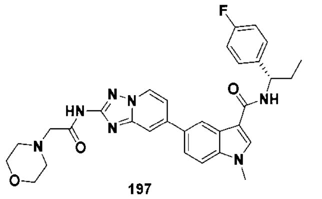
197 ,

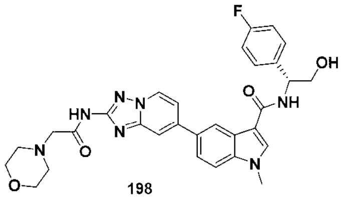
198 ,

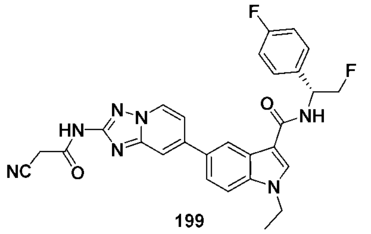
199 ,

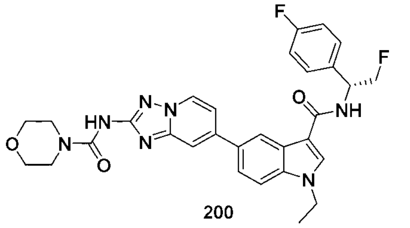
200 ,

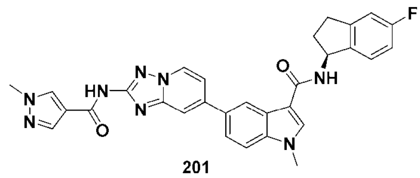
201

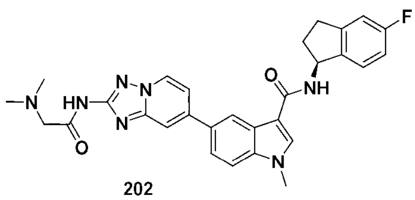
202 ,

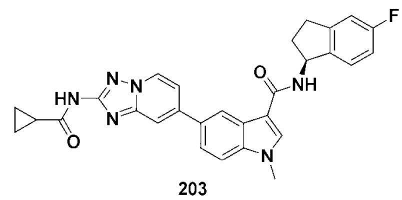
203 ,

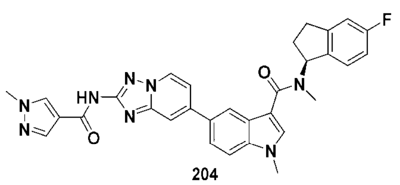
204 ,

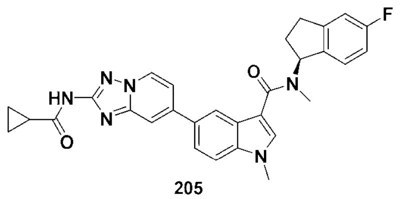
205 ,

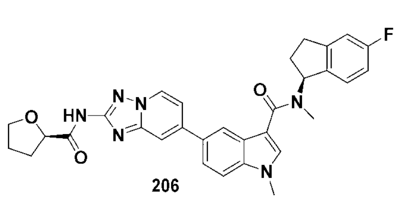
206 ,

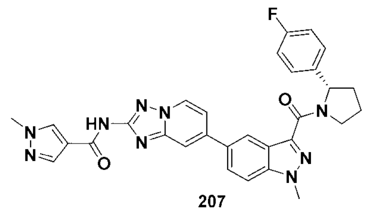
207 ,

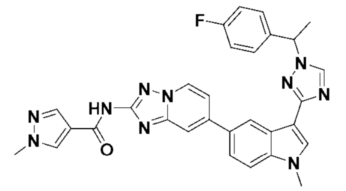
208 ,

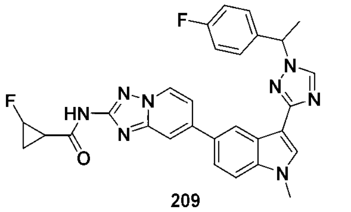
209 ,

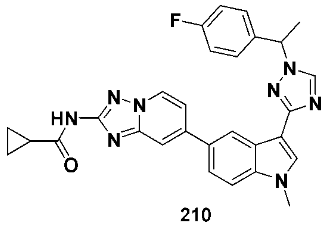
210 ,

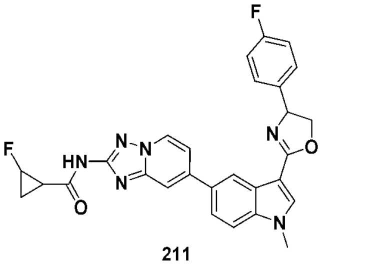
211 ,

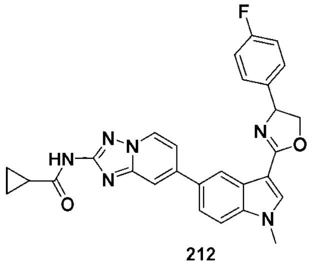
212 ,

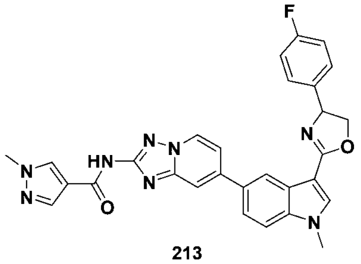
213 ,

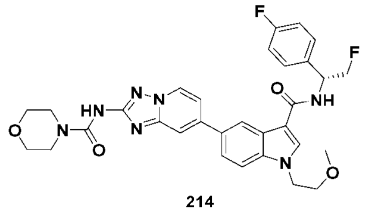
214 ,

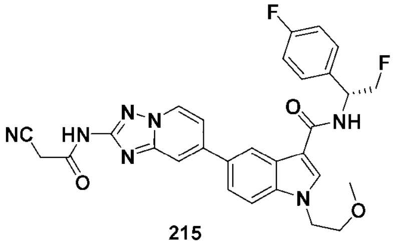
215 ,

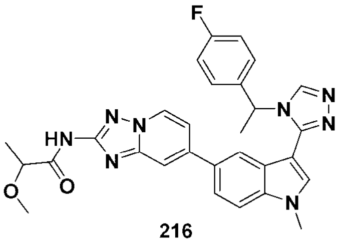
216 ,

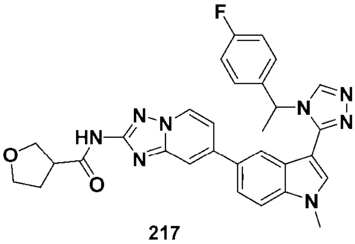
217 ,

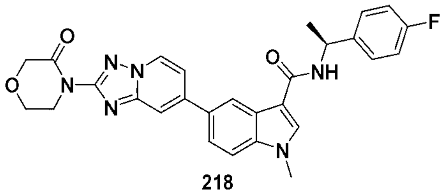
218 ,

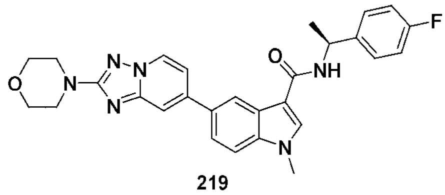
219 .

8. A pharmaceutical composition, comprising the compound according to any one of claims 1-7, or a pharmaceutically acceptable salt, solvate or stereoisomer thereof.

9. The aforementioned compounds, or pharmaceutically acceptable salts, solvates or stereoisomers thereof according to any one of claims 1-7 or pharmaceutical composition according to claim 8, in the manufacture of a drug for preventing and/or treating inflammation, autoimmune diseases, neurodegenerative diseases and tumor-related diseases.

10. Use of the compound according to any one of claims 1-7, or a pharmaceutically acceptable salt, solvate or stereoisomer thereof, or the pharmaceutical composition according to claim 8 in the preparation of RIPK1 and/or MLK inhibitors;
Preferably, the said MLK inhibitor is an MLK2/3 inhibitor.

## INTERNATIONAL SEARCH REPORT

International application No.
**PCT/CN2024/117673**

**A. CLASSIFICATION OF SUBJECT MATTER**

C07D471/04(2006.01)i; C07D519/00(2006.01)i; A61K31/437(2006.01)i; A61K31/5377(2006.01)i; A61K31/4545(2006.01)i; A61K31/438(2006.01)i; A61P35/00(2006.01)i; A61P37/06(2006.01)i; A61P25/28(2006.01)i; A61P29/00(2006.01)i

According to International Patent Classification (IPC) or to both national classification and IPC

**B. FIELDS SEARCHED**

Minimum documentation searched (classification system followed by classification symbols)

IPC:C07D A61K A61P

Documentation searched other than minimum documentation to the extent that such documents are included in the fields searched

Electronic data base consulted during the international search (name of data base and, where practicable, search terms used)

CNTXT; CNABS; WPABS; ENTXT; ENTXTC; DWPI; VEN; VCN; STN; CJFD; CNKI; ISI-Web of Science; 超星读秀, DUXIU; 万方, WANFANG: 吡啶并三氮唑, 吡啶, 三氮唑, 抑制剂, pyridin+, triazole, inhibit+, RIPK1, MLK, 式(I)化合物

**C. DOCUMENTS CONSIDERED TO BE RELEVANT**

| Category* | Citation of document, with indication, where appropriate, of the relevant passages | Relevant to claim No. |
|---|---|---|
| X | CN 114230565 A (ORIGIANT PHARMACEUTICAL CO., LTD.) 25 March 2022 (2022-03-25)<br>description, table 1, and paragraphs [0006]-[0091] and [0328]-[0336] | 1-10 |
| X | LI, Yueshan et al. "Generative Deep Learning Enables the Discovery of a Potent and Selective RIPK1 Inhibitor"<br>*Nature Communications*, No. 13, 12 November 2022 (2022-11-12), 6891<br>page 6, compound RI-962 | 1-10 |
| A | CN 111434661 A (ACCRO BIOSCIENCE INC.) 21 July 2020 (2020-07-21)<br>entire document | 1-10 |
| A | WO 2011050245 A1 (YIN YAN et al.) 28 April 2011 (2011-04-28)<br>entire document | 1-10 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 December 2024** | **18 December 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/117673**

| Patent document cited in search report | Publication date (day/month/year) | Patent family member(s) | Publication date (day/month/year) |
|---|---|---|---|
| CN 114230565 A | 25 March 2022 | US 2023365546 A1 | 16 November 2023 |
| | | CA 3192271 A1 | 17 March 2022 |
| | | JP 2023541263 A | 29 September 2023 |
| | | EP 4212525 A1 | 19 July 2023 |
| | | AU 2021341865 A1 | 04 May 2023 |
| | | AU 2021341865 B2 | 06 June 2024 |
| | | WO 2022052861 A1 | 17 March 2022 |
| | | CN 114230565 B | 27 October 2023 |
| CN 111434661 A | 21 July 2020 | EP 3908277 A1 | 17 November 2021 |
| | | EP 3908277 A4 | 28 December 2022 |
| | | WO 2020146858 A1 | 16 July 2020 |
| | | US 2022112188 A1 | 14 April 2022 |
| | | JP 2022517977 A | 11 March 2022 |
| | | JP 7410954 B2 | 10 January 2024 |
| | | CN 111434661 B | 12 September 2023 |
| WO 2011050245 A1 | 28 April 2011 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023112185953
- CN 2024109393705


### Non-patent literature cited in the description


- *Journal of Neuroscience the Official Journal of the Society for Neuroscience*, 2013, vol. 33.24, 9998-10010
- *Journal of Neuroscience*, 2009, vol. 29 (28), 9078-9089
- *Oncogene*, 2010, vol. 29.31, 4399-4411